# EUROPEAN PATENT SPECIFICATION

(11) **EP 3 456 190 B1**
(45) Date of publication and mention of the grant of the patent: **24.11.2021**
(21) Application number: 18183980.4
(22) Date of filing: 29.06.2009
(51) Int. Cl.: A01K 67/027, C12N 15/13, C07K 16/00, C12N 15/85, C12N 5/10

(54) **ANTIBODY PRODUCING TRANSGENIC MURINE ANIMAL**
ANTIKÖRPERPRODUZIERENDE TRANSGENE MURINE TIERE
ANIMAL MURIN TRANSGÉNIQUES PRODUISANT UN ANTICORPS

(30) Priority: 27.06.2008 WO PCT/NL2008/050430
(43) Date of publication of application: 20.03.2019
(62) Divisional of application: 12175544.1
(73) Proprietor: Merus N.V., 3584 CM Utrecht (NL)
(72) Inventor: Houtzager, Erwin, 3702 SC Zeist (NL); Pinto, Rui Daniel, 3581 JS Utrecht (NL); Logtenberg, Ton, 3584 CM Utrecht (NL); Throsby, Mark, 3584 CM Utrecht (NL)
(74) Representative: Lahrtz, Fritz

(56) References cited:
- EP-A- 1 439 234
- EP-A2- 0 814 159
- WO-A1-02/066630
- WO-A1-2004/106375
- WO-A2-2005/068622
- WO-A2-2006/117699
- SIRAC C ET AL: "Role of the monoclonal k chain V domain and reversibility of renal damage in a transgenic model of acquired Fanconi syndrome", BLOOD, AMERICAN SOCIETY OF HEMATOLOGY, US, vol. 108, no. 2, 15 July 2006 (2006-07-15) , pages 536-543, XP003030514, ISSN: 0006-4971, DOI: 10.1182/BLOOD-2005-11-4419
- PELANDA R ET AL: "A prematurely expressed Ig(kappa) transgene, but not V(kappa)J(kappa) gene segment targeted into the Ig(kappa) locus, can rescue B cell development in lambda5-deficient mice.", IMMUNITY SEP 1996, vol. 5, no. 3, September 1996 (1996-09), pages 229-239, XP002556633, ISSN: 1074-7613
- LONBERG NILS: "Human antibodies from transgenic animals", NATURE BIOTECHNOLOGY, NATURE PUBLISHING GROUP, NEW YORK, NY, US, vol. 23, no. 9, 1 September 2005 (2005-09-01), pages 1117-1125, XP002442149, ISSN: 1087-0156
- PRESTA ET AL: "Engineering of therapeutic antibodies to minimize immunogenicity and optimize function", ADVANCED DRUG DELIVERY REVIEWS, ELSEVIER BV, AMSTERDAM, NL, vol. 58, no. 5-6, 7 August 2006 (2006-08-07), pages 640-656, XP024892146, ISSN: 0169-409X [retrieved on 2006-08-07]
- WEINER LOUIS M: "Fully human therapeutic monoclonal antibodies", JOURNAL OF IMMUNOTHERAPY, LIPPINCOTT WILLIAMS & WILKINS, HAGERSTOWN, MD, US, vol. 29, no. 1, 1 January 2006 (2006-01-01), pages 1-9, XP009118693, ISSN: 1524-9557
- JAKOBOVITS AYA ET AL: "From XenoMouse technology to panitumumab, the first fully human antibody product from transgenic mice.", NATURE BIOTECHNOLOGY OCT 2007, vol. 25, no. 10, October 2007 (2007-10), pages 1134-1143, XP002556637, ISSN: 1087-0156
- DE KRUIF JOHN ET AL: "Human immunoglobulin repertoires against tetanus toxoid contain a large and diverse fraction of high-affinity promiscuous V(H) genes.", JOURNAL OF MOLECULAR BIOLOGY 3 APR 2009, vol. 387, no. 3, 3 April 2009 (2009-04-03) , pages 548-558, XP002556330, ISSN: 1089-8638
- L M Carlson ET AL: "Templated insertions in the rearranged chicken IgL V gene segment arise by intrachromosomal gene conversion.", GENES AND DEVELOPMENT., vol. 4, no. 4, 1 April 1990 (1990-04-01), pages 536-547, XP055654273, US ISSN: 0890-9369, DOI: 10.1101/gad.4.4.536
- Ivan J. Fuss ET AL: "Isolation of Whole Mononuclear Cells from Peripheral Blood and Cord Blood" In: "Current Protocols in Immunology", 1 May 2001 (2001-05-01), John Wiley & Sons, Inc., Hoboken, NJ, USA, XP055072167, ISBN: 978-0-47-114273-7 DOI: 10.1002/0471142735.im0701s85,
- Horwitz David A ET AL: "Distinctive Functional Properties of Human Blood L Lymphocytes: A Comparison with T Lymphocytes, B Lymphocytes, and Monocytes", The Journal of immunology (1950), 1 May 1977 (1977-05-01), pages 1712-1721, XP055816820, United States Retrieved from the Internet: URL:https://www.jimmunol.org/content/jimmu nol/118/5/1712.full.pdf [retrieved on 2021-06-22]
- FALKOFF R M ET AL: "T cell enrichment and depletion of human peripheral blood mononuclear cell preparations. Unexpected findings in the study of the functional activities of the separated populations", JOURNAL OF IMMUNOLOGICAL METHODS, ELSEVIER SCIENCE PUBLISHERS B.V.,AMSTERDAM, NL, vol. 50, no. 1, 16 April 1982 (1982-04-16) , pages 39-49, XP023676462, ISSN: 0022-1759, DOI: 10.1016/0022-1759(82)90302-7 [retrieved on 1982-04-16]
- KERSTIN B MEYER ET AL: "The importance of the 3'-enhancer region in immunoglobulin x gene expression", NUCLEIC ACIDS RESEARCH, OXFORD UNIVERSITY PRESS, GB, vol. 18, no. 19, 11 October 1990 (1990-10-11), pages 5609-5615, XP001525496, ISSN: 0305-1048, DOI: 10.1093/NAR/18.19.5609
- BETZ A G ET AL: "Elements regulating somatic hypermutation of an immunoglobulin @k gene: Critical role for the intron enhancer/matrix attachment region", CELL, ELSEVIER, AMSTERDAM NL, vol. 77, no. 2, 22 April 1994 (1994-04-22) , pages 239-248, XP024245265, ISSN: 0092-8674, DOI: 10.1016/0092-8674(94)90316-6 [retrieved on 1994-04-22]
- JUDDE J-G ET AL: "Characterization of the human immunoglobulin kappa gene 3' enhancer: functional importance of three motifs that demonstrate B-cell-specific in vivo footprints", MOLECULAR AND CELLULAR BIOLOGY, AMERICAN SOCIETY FOR PHARMACOLOGY AND EXPERIMENTAL THERAPEUTICS, US, vol. 12, no. 11, 1 November 1992 (1992-11-01), pages 5206-5216, XP001069601, ISSN: 0270-7306
- FAUSTINO N. A. ET AL: "Pre-mRNA splicing and human disease", GENES & DEVELOPMENT, vol. 17, no. 4, 15 February 2003 (2003-02-15), pages 419-437, XP055816814, DOI: 10.1101/gad.1048803 Retrieved from the Internet: URL:http://genesdev.cshlp.org/content/17/4 /419.full.pdf>
- CARTEGNI L ET AL: "Listening to silence and understanding nonsense: exonic mutations that affect splicing", NATURE REVIEWS GENETICS, MACMILLAN MAGAZINES, GB, vol. 3, no. 4, 1 April 2002 (2002-04-01), pages 285-298, XP002262849, DOI: 10.1038/NRG775
- WOODLEY L ET AL: "REGULATION OF ALTERNATIVE PRE-MRNA SPLICING", BRIEFINGS IN FUNCTIONAL GENOMICS AND PROTEOMICS, HENRY STEWART, LONDON, GB, vol. 1, no. 3, 1 October 2002 (2002-10-01) , pages 266-277, XP001168605, ISSN: 1473-9550
- FATHIMA SHAMILA ET AL: "Comparison of Manual Versus Automated Data Collection Method for Haematological Parameters", BIOMEDICAL JOURNAL OF SCIENTIFIC & TECHNICAL RESEARCH, vol. 15, no. 3, 1 March 2019 (2019-03-01), XP055816823, DOI: 10.26717/BJSTR.2019.15.002702 Retrieved from the Internet: URL:https://biomedres.us/pdfs/BJSTR.MS.ID. 002702.pdf>
- GOODNOW CHRISTOPHER C ET AL: "Altered immunoglobulin expression and functional silencing of self-reactive B lymphocytes in transgenic mice", NATURE, MACMILLAN JOURNALS LTD., ETC, LONDON, vol. 334, no. 6184, 1 August 1988 (1988-08-01), pages 676-682, XP037103628, ISSN: 0028-0836, DOI: 10.1038/334676A0 [retrieved on 1988-08-01]
- LARRICK J W ET AL: "Rapid cloning of rearranged immunoglobulin genes from human hybridoma cells using mixed primers and the polymerase chain reaction", BIOCHEMICAL AND BIOPHYSICAL RESEARCH COMMUNICATIONS, ELSEVIER, AMSTERDAM NL, vol. 160, no. 3, 15 May 1989 (1989-05-15), pages 1250-1256, XP000293053, ISSN: 0006-291X, DOI: 10.1016/S0006-291X(89)80138-X

## Description

### TECHNICAL FIELD

The invention relates to the production and use of transgenic murine hosts capable of producing antibodies or derivatives thereof, which are expressed from at least partially exogenous nucleic acids (transgenes). Transgenes to produce such transgenic murine hosts and methods to produce such heterologous antibodies; methods and vectors for producing such transgenic murine hosts are disclosed.

### BACKGROUND OF THE INVENTION

B cells mediate humoral immunity by producing specific antibodies. The basic structural subunit of an antibody (Ab) is an immunoglobulin (Ig) molecule. Ig molecules consist of a complex of two identical heavy (H) and two identical light (L) polypeptide chains. At the amino terminus of each H chain and L chain is a region that varies in amino acid sequence named the variable (V) region. The remaining portion of the H and L chains is relatively constant in amino acid sequence and is named the constant (C) region. In an Ig molecule, the H and L chain V regions (VH and VL) are juxtaposed to form the potential antigen-binding site. The genes that encode H and L chain V regions are assembled somatically from segments of germline DNA during precursor B (pre-B) cell differentiation: V, D and J gene segments for the H chain and V and J gene segments for the L chain. Within Ig V regions are three regions of greatest amino acid sequence variability that interact to form the antigen-recognition site and are thus referred to as complementarity determining regions (CDRs).

The V gene segment encodes the bulk of the V region domain, including CDR1 and CDR2. Diversity in CDR1 and CDR2 derives from sequence heterogeneity among multiple different germline-encoded V segments. CDR3 is encoded by sequences that are formed by the joining of H chain V, D, and J gene segments and L chain V and J segments and by mechanisms that create nucleotide sequence heterogeneity where these segments are combined. Additional diversity may be derived from pairing of different H and L chain V regions. Collectively these processes yield a primary repertoire of antibodies encoded by germline gene segments and expressed by newly-formed B cells.

An additional source of antibody diversity is imposed on top of the diversity generated by recombination of Ig gene segments. B cells are able to introduce mutations into the antibody V regions that they express, a process called somatic hypermutation. Thus, when an animal first encounters an antigen, the antigen binds to a specific B cell which happens to carry antibodies which have a V domain which binds the antigen. This primary response may activate this B cell to go on to secrete the cognate antibody. These activated B cells can also now target a somatic mutation process to their rearranged antibody gene segments and thus allow the production of daughter cells which make variants of the antibodies of the primary response. A selection process amplifies those variant B cell descendants which make an antibody of improved affinity of the antigen. In B cells, somatic hypermutations are targeted to a restricted genomic region including both the rearranged VH and VL genes. Thus somatic mutation allows affinity maturation - the production and selection of high affinity antibodies. Therefore, somatic mutation is important for the generation of high affinity antibodies.

The exquisite specificity and high affinity of antibodies and the discovery of hybridoma technology allowing the generation of monoclonal antibodies (mAbs) has generated great expectations for their utilization as targeted therapeutics for human diseases. MAbs are identical because they are produced by a single B cell and its progeny. MAbs are made by fusing the spleen cells from a mouse that has been immunized with the desired antigen with myeloma cells to generate immortalized hybridomas. One of the major impediments facing the development of in vivo applications for mAbs in humans is the intrinsic immunogenicity of non-human Igs. Patients respond to therapeutic doses of mouse mAbs by making antibodies against the mouse Ig sequences (Human Anti Mouse Antibodies; HAMA), causing acute toxicity, alter their biodistribution and accelerate clearance, thus reducing the efficacy of subsequent administrations (Mirick, et al., (2004) Q. Nucl. Med. Mol. Imaging 48, 251-257).

To circumvent the generation of HAMA, antibody humanization methods have been developed in an attempt to produce mAbs with decreased immunogenicity when applied to humans. These endeavors have yielded various recombinant DNA-based approaches aimed at increasing the content of human amino acid sequences in mAbs while retaining the specificity and affinity of the parental non-human antibody. Humanization began with the construction of mouse-human chimeric mAbs (Morrison, S. L., et al., (1984). Proc. Natl. Acad. Sci. USA., 81, 6851-5), in which the Ig C regions in murine mAbs were replaced by human C regions. Chimeric mAbs contain 60-70% of human amino acid sequences and are considerably less immunogenic than their murine counterparts when injected into humans, albeit that a human anti-chimeric antibody response was still observed (Hwang, W. Y., et al. (2005). Methods, 36, 3-10).

In attempts to further humanize murine mAbs, CDR grafting was developed. In CDR grafting, murine antibodies are humanized by grafting their CDRs onto the VL and VH frameworks of human Ig molecules, while retaining those murine framework residues deemed essential for specificity and affinity (Jones, P.T., et al., (1986). Nature, 321, 522). Overall, CDR-grafted antibodies consist of more than 80% human amino acid sequences (Queen, C. et al. (1989) Proc. Natl. Acad. Sci. U. S. A. 86, 10029; Carter, P. et al. (1992) Proc. Natl. Acad. Sci. U. S. A. 89, 4285). Despite these efforts, CDR-grafted, humanized antibodies were shown to still evoke an antibody response against the grafted V region (Hwang, W. Y., et al. (2005). Methods, 36, 3).

Subsequently to CDR grafting, humanization methods based on different paradigms such as resurfacing (Padlan, E. A., et al., (1991). Mol. Immunol., 28, 489), superhumanization (Tan, P., D. A., et al., (2002) J. Immunol., 169, 1119), human string content optimization (Lazar, G. A., et al., (2007). Mol. Immunol., 44, 1986) and humaneering have been developed in an attempt to further decrease the content of non-human sequences in therapeutic mAbs (Almagro, J. C., et al., (2008). Frontiers in Bioscience 13, 1619). As in CDR grafting approaches, these methods rely on analyses of the antibody structure and sequence comparison of the non-human and human mAbs in order to evaluate the impact of the humanization process into immunogenicity of the final product. When comparing the immunogenicity of chimeric and humanized antibodies, humanization of variable regions appears to decrease immunogenicity further (Hwang, W. Y., et al. (2005). Methods, 36, 3-10).

De-immunization is another approach developed to reduce the immunogenicity of chimeric or mouse antibodies. It involves the identification of linear T-cell epitopes in the antibody of interest, using bioinformatics, and their subsequent replacement by site-directed mutagenesis to human or non-immunogenic sequences (WO09852976A1). Although de-immunized antibodies exhibited reduced immunogenicity in primates, compared with their chimeric counterparts, some loss of binding affinity was observed (Jain, M., et al., (2007). Trends in Biotechnol. 25, 307).

The development of phage display technology complemented and extended humanization approaches in attempts to obtain less immunogenic mAbs for therapy in humans. In phage display, large collections ('libraries') of human antibody VH and VL regions are expressed on the surface of filamentous bacteriophage particles. From these libraries, rare phages are selected through binding interaction with antigen; soluble antibody fragments are expressed from infected bacteria and the affinity of binding of selected antibodies is improved by mutation (Winter, G., et al. (1994). Annu. Rev. Immunol. 12, 433). The process mimics immune selection, and antibodies with many different bindings specificities have been isolated using this approach (Hoogenboom, H. R., et al. (2005). Nat. Biotechnol., 23, 1105). Various sources of H and L chain V regions have been used to construct phage display libraries including those isolated from non-immune or immune donors. In addition, phage display libraries have been constructed of V regions that contain artificially randomized synthetic CDR regions in order to create additional diversity. Often, antibodies obtained from phage display libraries are subjected to in vitro affinity maturation to obtain high affinity antibodies (Hoogenboom, H. R., et al. (2005). Nat. Biotechnol., 23, 1105).

The creation of transgenic mouse strains producing human antibodies in the absence of mouse antibodies has provided another technology platform for the generation of specific and high affinity human mAbs for application in humans. In these transgenic animals, the endogenous mouse antibody machinery is inactivated and replaced by human Ig loci to substantially reproduce the human humoral immune system in mice (Jakobovits, A., et al. (2007). Nat. Biotechnol. 25, 1134. Lonberg, N. (2005). Nat. Biotechnol. 23, 1117). B cell development as well as Ig diversification by recombination of gene segments is faithfully reproduced in these mice, leading to a diverse repertoire of murine B cells expressing human Igs. By immunizing these mice with antigens, it was further demonstrated that these transgenic animals accumulated somatic mutations in the V regions of both heavy and light chains to produce a wide diversity of high-affinity human mAbs (Lonberg, N. (2005). Nat. Biotechnol. 23, 1117).

The question, whether "fully human" mAbs such as derived from phage display libraries or transgenic mice are less immunogenic than humanized mAbs cannot be answered yet, because full immunogenicity data are available for just two human mAbs. An anti-tumor necrosis factor mAb, developed from phage-displayed human libraries induced antibody responses in 12% of patients - at the higher end of the incidence of anti-antibody responses of the humanized antibodies (Hwang, W. Y., et al. (2005). Methods, 36, 3-10).

Evaluation of the immunogenicity of the first registered human mAb generated by the transgenic approach demonstrated that mAb treatment resulted in the generation of antibodies in approximately 5.5% of treated cancer patients (Jakobovits, A., et al. (2007). Nat. Biotechnol. 25, 1134., Lofgren, J. A., et al. (2007). J. Immunol. 178, 7467). EP0814159 discloses a theoretical experiment for the production of a transgenic mouse for the production of antibodies, said mouse comprising a rearranged human V domain linked to a human light chain constant domain. No transgenic mice have been produced.

A need therefore remains for a method and means for producing antibodies that are specific for their targets, but are less immunogenic. As described herein, the reduction of immunogenicity is at least partially achieved by providing a transgenic murine host comprising, at least in its B cell lineage, a nucleic acid encoding at least an immunoglobulin light chain, wherein the light chain encoding sequence is provided with a means that renders it resistant to DNA rearrangements and/or somatic hypermutations, preferably such a murine host is a mouse. The nucleic acid encodes a rearranged human VL region of a human immunoglobulin.

As claimed, the present invention relates to a transgenic murine host capable of producing immunoglobulins comprising a human VL region, said murine host comprising integrated in its germline a rearranged nucleic acid comprising one human germline V and one human germline J gene segment encoding a rearranged human VL region and a light chain constant region of any animal species, wherein said light chain variable region is capable of pairing with at least two different heavy chains encoded by the host, such that variety in specificity of antibodies comprising said human VL region is retained through rearrangements and hypermutations in the heavy chains.

As claimed, the present invention further relates to a method for producing a desired antibody comprising exposing a host according to the invention to an antigen such that an antibody response is induced and isolating the antibodies specific for the antigen.

As claimed, the present invention further relates to a method for producing a desired antibody comprising exposing a host according to the invention to an antigen such that an antibody response is induced and isolating cells producing such antibodies, culturing and harvesting said antibodies.

As claimed, the present invention further relates to a method for producing a desired antibody comprising exposing a host according to the invention to an antigen such that an antibody response is induced and isolating a nucleic acid encoding at least part of such an antibody, inserting said nucleic acid or a copy or a derivative thereof in an expression cassette and expressing said antibody in a host cell.

In the remainder of the specification mice are typically used as examples of the transgenic murine hosts. The transgenic murine hosts are capable of mounting an immune response to an antigen, where the response produces antibodies having human light chain variable regions. Mice have been used for the production of B-lymphocytes for immortalization for the production of antibodies. Since mice are easy to handle, can be bred in large numbers, and are known to have an extensive immune repertoire, mice will usually be the animal of choice. Therefore, in the following discussion, the discussion will refer to mice, but it is described that it should be understood that other animals, particularly non-primate mammals, may be readily substituted for the mice, following the same procedures.

The reason for preventing rearrangements and hypermutation is that in this manner a non-immunogenic polypeptide can be chosen beforehand knowing that this polypeptide chain will remain non-immunogenic. At least the light chain of the resulting immunoglobulin is thus less immunogenic. The resulting antibody needs to have (usually) both a light- and a heavy chain. The non-immunogenic chain must therefore be capable of pairing with the other chain. The other chain may be an endogenous chain, an exogenous chain or a hybrid of both. For human therapy, the non-immunogenic chain should be as close to human as possible.

A means for rendering a gene encoding an immunoglobulin chain (or chains) resistant to DNA rearrangement and/or mutation is of course removal of all genetic elements responsible for said rearrangement and/or mutation. The drawback thereof is that the variability of the two chains is eliminated, whereas the invention preferably retains the variability in the heavy chain and inhibits and/or prevents the rearrangement-mutation of the light chain.

The elements for rearrangement and/or hypermutation characterized so far are located within the loci for immunoglobulins. Therefore the means for rendering the immunoglobulin encoding sequence resistant to DNA rearrangement and/or mutation is inserting the gene in a locus outside the immunoglobulin loci.

Thus, according to the invention a transgenic murine host is provided wherein the light chain encoding sequence is integrated in the genome of the murine host in a locus outside the immunoglobulin loci. In one aspect of the invention, the insertion is in a locus that is resistant to gene silencing. According to one aspect of the invention, the integration is in the Rosa-locus.

It is preferred to provide an expression cassette which can be inserted in a Rosa locus or comparable locus with a means that allows expression of the immunoglobulin chain(s) essentially limited to cells of B cell lineage, preferably with a means that allows expression of the light chain encoding nucleic acid during a certain stage of the development of B cells. The term "essentially limited expression" indicates that expression is predominantly in cells of the B-cell lineage, but that lower levels of expression in other cells, as compared to the level of expression in B-cells, is possible. In a preferred embodiment, the term "essentially limited expression" indicates that the expression is exclusively present in cells of the B-cell lineage. Such means typically and preferably include B cell (developmental stage) specific promoters such as CD19, CD20, µHC (all V-genes), VpreB1, VpreB2, VpreB3, λ5, Igα, Igβ, κLC (all genes), λLC (all genes), BSAP (Pax5). Although it is very well possible to direct the expression of the DNA rearrangement and/or mutation resistant chain by such promoters, they are relatively weak. A strong promoter will typically be required to ensure adequate surface expression of the B cell receptor (made up of the membrane attached Ig H and L chain) and to compete with the expression and pairing of endogenous chains (if present) through allelic exclusion. Such a promoter, however is usually not tissue specific. To confer tissue specificity, an indirect system employing Cre/lox or the like is preferred. The desired chain is put under control of a strong promoter inhibited by an element that can be removed by the action of a Cre-protein, leading to activation of the desired immunoglobulin encoding gene. This system is described in detail in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universitat zu Köln; http://deposit.ddb.de/cgi-bin/dokserv?idn= 97557230x&dok_var=d1&dok_ext=pdf&filename=97557230x.pdf.

The immunoglobulin chain is a light chain capable of pairing with different heavy chains encoded by the murine host. The light chain will then be the same (and less immunogenic) in all antibodies, but variety in specificity is retained through rearrangements and hypermutations in the heavy chains. It may in that case be preferable to silence at least one of the endogenous loci encoding a light chain, although allelic exclusion may render this unnecessary.

According to this embodiment, preferably the endogenous kappa (κ) light chain locus is functionally silenced.

If the endogenous κ light chain locus is silenced, but also for other reasons, it is preferred that the resistant light chain is a κ light chain that has a germline sequence. According to the invention such a light chain would lead to an antibody with reduced immunogenicity. The preferred germline sequence is based on the human IGKV1-39 (012) as this light chain is very frequently observed in the human repertoire (de Wildt et al. 1999. J. Mol. Biol. 285(3):895) and has superior thermodynamic stability, yield and solubility (Ewert et al. 2003. J. Mol. Biol. 325(3):531).

The following gives more specific embodiments of the expression cassette with which the murine host can be provided according to the invention. This is typically advantageous for immunoglobulins.

Thus the invention provides in a specific embodiment a transgenic murine host wherein the light chain encoding nucleic acid comprises in 5'-3' direction: a B cell specific promoter, a leader, a rearranged human V gene, optionally a MoEκi enhancer, a constant region (κ) and optionally a (truncated) MoEκ3' enhancer. Neuberger identified and examined a novel B-cell specific enhancer located downstream of the kappa constant region (EP004690251). This enhancer has been shown to play a crucial role in the expression of kappa genes as removal of the 808bp enhancer strongly reduced expression. Deletion of the 3' kappa enhancer also strongly reduced the level of somatic hypermutations (SHM). In transgenic and cell expression studies it has been revealed that reduced, mutated or deleted 3' kappa enhancers not only lowered expression levels but also decreased the level of somatic hypermutations. Currently, it can not be determined whether the 3' kappa enhancer is involved in SHM processes, expression regulation or both (review Odegard, V. H., et al. (2006). Nat. Rev. Immunol. 6, 573; Inlay, M., et al. (2002). Nat. Immunol. 3, 463.).

Detailed expression studies using engineered variants of the 3' kappa enhancer indicated that a 50 nucleotide region is sufficient to drive expression. However for proper expression a reduced sequence of 145 nucleotides is preferred (EP04690251; Meyer, K. B., et al. (1990) Nucleic Acids Res. 18(19):5609-15)

Thus the invention in one aspect provides in the context of the claimed transgenic murine host and in the context of the claimed method, use of a nucleic acid for insertion into the genome of a murine host that is an expression cassette for the expression of a desired proteinaceous molecule in cells developing into mature B cells during a certain stage of development, said cassette comprising means for preventing silencing of expression of the desired proteinaceous molecule after introduction into a host cell, and means for timing expression of the desired proteinaceous molecule with the desired developmental stage of the host cell.

An expression cassette is defined as a nucleic acid that has been provided with means for introduction into the genome of a host cell, such as sequences which allow for homologous recombination with a certain site in the genome. Usually the nucleic acid will be DNA, typically double stranded. Typically the expression cassette will be provided to the cell in a vector from which it is transferred to the genome of the cell. The expression cassette further comprises all elements necessary for expression of the gene in a host cell, although in certain embodiments some of such elements may be present on a second nucleic acid to be introduced, whereby these elements act in trans. Elements necessary for expression in a host cell include promoters, enhancers and other regulatory elements. Only those elements are necessary that are not provided by the host cell.

According to one aspect of the invention it is important that the expression of the gene of interest is not silenced in the genome of the host cell, especially not in the development stage where expression is required. This can be done by various means, such as insertion into the endogenous locus or by providing the cassette with nucleic acid elements that prevent silencing (Kwaks et al. (2006) Trends Biotechnol. 24(3), p. 137-142). It is preferred that the expression cassette is inserted in a locus that is not silenced in the host cells (EP 01439234).

Said means for prevention of silencing comprise STabilizing Anti-Repression-sequences (STAR®-sequences) and Matrix Attachment Regions (MARs). A STAR sequence is a nucleic acid sequence that comprises a capacity to influence transcription of genes in cis. Typically, although not necessarily, a STAR sequence does not code by itself for a functional protein element. In one embodiment one STAR element is used. Preferably, however, more than one STAR element is used. In a particularly preferred embodiment an expression cassette for use in the invention is provided with two STAR sequences; one STAR sequence at the 5' side of the coding sequence of the immunoglobulin gene and one STAR sequence at the 3' side of the coding sequence of the immunoglobulin gene. MARs are DNA sequences that are involved in anchoring DNA/chromatin to the nuclear matrix and they have been described in both mammalian and plant species. MARs possess a number of features that facilitate the opening and maintenance of euchromatin. MARs can increase transgene expression and limit position-effects.

According to one aspect of the invention it is important that expression from the cassette only occurs during a certain period in the development of a cell, in particular a developing B cell, more in particular a B cell in a transgenic murine host, in particular a mouse. In this particular case the developmental period is chosen such that the expression of the light-chain does not significantly interfere with the normal differentiation and/or maturation of the cell and when applicable, allows for pairing of the polypeptide chain produced with its counterpart.

This may be achieved by providing a nucleic acid useful in the context of the invention, wherein said means for timing expression is a promoter of which the activity is essentially limited to the certain stage of development. In a developing B cell, which, e.g. after immunization is maturing and/or differentiating, the expression of the gene of interest, which is the rearranged human VL region of a human immunoglobulin, must not interfere (significantly) with said maturation and/or differentiation and it needs to be timed such that the resulting polypeptide can pair with its counterparts. Therefore also described is a nucleic acid useful in the invention wherein said certain stage starts at a stage immediately preceding or coinciding with the onset of the expression of light chain molecules by said cells at a certain stage of development into a mature B cell.

This may be achieved by selecting a promoter which is active only during said suitable period. Such a promoter may be a CD 19 promoter, the Ig-α promoter, the Ig-β promoter, the µhc (all genes) promoter, the Vk promoter or analogues or homologues thereof.

The promoter as disclosed above may not drive the expression of the gene of interest directly. Instead it drives the expression of a gene of which the product activates in trans the expression of the gene of interest. Such an activating gene may be a gene encoding a so-called Cre recombinase or Cre-like protein. The expression cassette for the gene of interest may e.g. be provided with a sequence that inhibits expression of the gene of interest. Said sequence can be removed by the action of the Cre recombinase, which is under control of the desired promoter (active during the proper stage of development). A set of expression cassettes may be required.

Therefore described is a set of nucleic acids that are expression cassettes, wherein one nucleic acid comprises an expression cassette encoding a Cre-like protein under control of a promoter active during the desired stage of development of the host cell and the second nucleic acid comprises a sequence encoding a desired proteinaceous molecule under control of a constitutive promoter which can be activated by the action of a Cre-like protein. Said activation may be achieved by removal of a stop sequence flanked by loxP sites. The Cre/lox system is described in detail in Rajewsky et al. (1996) J. Clin. Invest. 98, p.600-603. Such systems are reviewed in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universitat zu Köln; http://deposit.ddb.de/cgi-bin/dokserv?idn=97557230x&dok_var=d1&dok_ext=pdf&filename=9755723 0x.pd.

The invention provides a transgenic murine host as defined in the claims, which has been provided with an expression cassette, wherein the desired proteinaceous molecule is a rearranged human VL region of a human immunoglobulin, wherein the VL region is encoded by one human germline V and one human germline J gene segmentA most preferred polypeptide is 012, preferably the rearranged germline kappa light chain IGKV1-39*01/IGKJ1*01 (nomenclature according to the IMGT database, http://www.imgt.org).

It is further described that the polypeptide chain is rendered essentially incapable of rearrangement and/or of excluded of any sequence modification such as normally operating on Ig during the process of B cell affinity maturation. Therefore, the invention describes a transgenic murine host which has been provided with an expression cassette, wherein said re-arrrangement and/or sequence modifications are prevented by the absence of elements at least partially responsible for somatic hypermutation such as, for example, the MoEκi enhancer.

An expression cassette comprises means for prevention of silencing.Said means for prevention of silencing are means for insertion into a locus in the genome of the host cell that is resistant to silencing. Said means for insertion are preferably means for homologous recombination into said site resistant to silencing. A preferred locus when the murine host is a mouse is the rosa-locus.

A further preferred expression cassette comprises in 5'-3' direction: a Vκ promoter, a mouse leader, a human V gene, optionally a MoEκi enhancer, a rat constant region (Cκ) and optionally a (truncated) MoEκ3' enhancer.

Yet a further preferred expression cassette comprises in 5'-3' direction: a Vκ promoter, a human leader, a human V gene, optionally a MoEκi enhancer, a rat constant region (Cκ) and optionally a (truncated) MoEκ3' enhancer.

Of course the ultimate goal of the invention is to produce antibodies to be used in human therapeutics. Thus the invention provides a method for producing a desired antibody comprising exposing a murine host according to the invention to an antigen such that an antibody response is induced and isolating the antibodies specific for the antigen.

In an alternative embodiment, the invention provides a method for producing a desired antibody comprising exposing a murine host according to the invention to an antigen such that an antibody response is induced and isolating cells producing such antibodies, culturing and optionally immortalizing said cells and harvesting said antibodies.

In a further embodiment, the invention provides a method for producing a desired antibody comprising exposing a murine hostaccording to the invention to an antigen such that an antibody response is induced and isolating a nucleic acid encoding at least part of such an antibody, inserting said nucleic acid or a copy or a derivative thereof in an expression cassette and expressing said antibody in a host cell.

The methods for producing antibodies from transgenic mice are known to a person skilled in the art. Particularly preferred are methods for production of mixtures of antibodies from one cell, whereby the nucleic acids encoding these antibodies have been derived from mice according to the invention.

These so-called oligoclonics are disclosed in WO04106375 and WO05068622.

The present invention provides transgenic murine hosts, preferably mice, capable of generating specific and high affinity hybrid mouse-human antibodies with human immunoglobulin light chain variable (VL) regions in germline configuration and preferably murine immunoglobulin heavy chain variable (VH) regions that may have accumulated somatic mutations during the process of antigen-driven affinity maturation. It is envisaged that the murine VH regions of the hybrid antibodies may be subjected to humanization procedures to yield mAbs that have reduced immunogenicity when applied in humans based on germline or near-germline VL regions and murine VH regions that have been humanized.

In particular, it has been shown in the present invention that transgenic mice that harbor a DNA expression construct that encodes a rearranged human VL region under the control of cis-acting genetic elements that provide timely and regulated expression of the transgene on a significant proportion of B cells during B cell development, yet lack elements that direct the somatic hypermutation machinery to the transgene, are capable of generating specific and high affinity mouse-human hybrid antibodies with essentially unmutated L chains. It is shown that the rearranged human transgene is capable of pairing with a diversity of endogenous murine immunoglobulin H chains to form mouse-human hybrid immunoglobulins expressed on the surface of B cells and to sufficiently facilitate murine B cell development to obtain a sizeable and diverse peripheral B cell compartment.

In a preferred embodiment, the transgene expression construct harbors the coding sequences of a human rearranged L chain V region under the control of a human VL promoter to direct B-cell specific expression. In addition, the construct harbors the murine 3' Ck enhancer sequence for B cell specific and inducible and high level expression of the transgene. Furthermore, the construct is designed to lack regulatory elements that facilitate the recruitment of the somatic hypermutation machinery to the transgene, such as the intron enhancer and the 3' C-kappa enhancer.

In a related embodiment, the rearranged human VL gene is inserted in the murine Rosa26 locus by site-specific integration. The Rosa26 locus is useful in the context of the "targeted transgenesis" approach for efficient generation of transgenic organisms (such as mice) with a predictable transgene expression pattern.

In a preferred embodiment, the rearranged human VL region is selected for its capacity to pair with at least two different murine VH genes so as to ensure the generation of a population of B cells with a diverse VH gene repertoire. A method of obtaining such VL regions comprises amplifying a repertoire of rearranged VH genes from the B cells of mice and a repertoire of human rearranged germline VL regions from the B cells of humans and cloning them into phagemid display vectors to prepare diverse libraries of hybrid immunoglobulins in bacteria. By nucleotide sequence analysis of collections of unselected and antigen-selected VH/VL pairs, human germline VL genes that pair with many different murine VH genes are identified. A collection of human germline VL genes with this capacity is described.

It is shown that upon immunization with antigen, the B cells are capable of mounting an immune response, leading to the generation of B cells that secrete hybrid antibodies with high specificity and affinity. The V regions encoding these antibodies are characterized by the human transgenic light chain that harbors no or very few mutations and a murine heavy chain that harbors a variable number of mutations introduced by the somatic hypermutation machinery.

Strategies to obtain high affinity hybrid monoclonal antibodies from the transgenic mice by hybridoma and display technologies are contemplated as well as procedures to humanize the murine VH regions to obtain less immunogenic antibodies for application in humans.

The invention may involve use of an immunoglobulin L chain transgene construct comprising DNA sequences that encode a human immunoglobulin VL region in combination with a light chain constant region (CL) of an animal immunoglobulin protein, which sequences are operably linked to transcription regulatory sequences that, when integrated in a murine transgenic host, produce an Ig VL-CL polypeptide with a human VL region that is not or marginally subject to somatic hypermutation. The Ig VL is capable of pairing with rearranged VH-CH polypeptides that are generated during B cell development in the murine transgenic host, with said VH-CH polypeptides retaining the capacity to undergo somatic hypermutation upon stimulation. The CL region may be of any animal species and is generally capable of pairing with the CH regions of the murine transgenic host.

Also described is the use of a transgene construct as above in producing a transgenic murine host capable of the production of hybrid antibodies consisting of VL-CL polypeptides and VH-CH polypeptides in which the VL region is of human origin and the CL, VH and CH may be of any animal species, including human. Upon immunization, these transgenic animals are capable of generating high affinity antibodies encoded by somatically hypermutated VH genes and essentially non-mutated VL genes encoded by the transgene.

Another aspect describes a process for the production of a transgenic murine host capable of the production of hybrid antibodies in response to antigenic challenge, comprising functionally disrupting the endogenous immunoglobulin light chain locus and inserting into the transgenic murine host genome a transgene construct as described.

The invention describes the use of murine hosts obtainable by this process in the production of B cells that produce immunoglobulin having human VL light chain. There is described a process for the production of B cells that produce immunoglobulin having human VL and binding to a selected antigen, comprising challenging a murine host obtainable by a process as above with said antigen and screening for B cells from said animal that bind said antigen. Further described are B cells obtainable by this process and hybridomas obtainable by immortalizing such B cells, e.g. hybridomas obtained by fusing B cells as above with myeloma cells. The invention also describes a process for producing monoclonal antibody comprising cultivating such a hybridoma. Also described is the use of the above B cells in producing a hybridoma or corresponding monoclonal antibody.

The invention describes a process for the production of immunoglobulin having human VL chain and binding to a selected antigen, comprising challenging a murine host obtainable as above with said antigen and obtaining immunoglobulin there from.

In one strategy, as an individual step, a rearranged VL region encoded by human germline V and J gene segments and a light chain constant region of any animal species but preferably a murine constant region is introduced into the mouse germ line. The transgene DNA may be introduced into the pronuclei of fertilized oocytes or embryonic stem cells. The integration may be random or homologous depending on the particular strategy to be employed. For example, the VL transgene may be introduced by random insertion, resulting in mice that bear one or multiple copies of the transgene in the genome. Alternatively, the human VL transgene may be targeted to a specific genomic locus using site-specific recombination as described in the art.

In one preferred embodiment, the VL transgene is targeted to the murine ROSA26 locus which is a suitable integration site allowing strong and predictable expression of inserted transgenes (EP 1439234). The targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the murine host is predictable. Furthermore, random X inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes. Thus, it may be envisaged that 2 or more different rearranged germline human VL regions are inserted into the Rosa26 locus to further increase the diversity of the repertoire of hybrid or human antibodies.

In another embodiment, a rearranged human VL region may be targeted to the murine Ig kappa or lambda light chain locus so as to functionally inactivate the endogenous locus or mice containing the rearranged human VL region may be bred with mice that lack functional kappa or lambda Ig loci or both. Thus, by using transformation, using repetitive steps or in combination with breeding, transgenic murine hosts may be obtained which are able to produce antibodies harboring the human VL transgene in the substantial absence of endogenous host immunoglobulin light chains.

In one embodiment, a human VL transgene is selected for its capacity to pair with a substantial portion of murine VH regions to form a diverse repertoire of functional mouse-human hybrid antibodies expressed on the surface of B cells. By a substantial portion of murine VH regions is meant that the human VL pairs with at least with 0.1% of the murine VH regions generated during B cell development, more preferably with at least 1% and most preferably with at least 10%. Methods to identify human VL genes with this characteristic include randomly pairing a repertoire of human VL regions with a repertoire of murine VH regions, co-expression of VH and VL regions in appropriate eukaryotic or prokaryotic expression vectors and screening for human VL regions that pair with a substantial portion of murine VH regions. Phagemid vectors may be used to direct expression of mouse-human antibody fragments in bacterial cells or to the surface of filamentous phage and analysis of binding capacity of antibody fragments by methods known in the art.

In another embodiment, a human VL transgene is selected for its capacity to pair with a substantial portion of human VH regions to form a diverse repertoire of human antibodies expressed on the surface of B cells. By a substantial portion of human VH regions is meant that the human VL pairs with at least with 0.1% of the human VH regions generated during B cell development, more preferably with at least 1% and most preferably with at least 10%.

In the latter embodiment, the human VL transgenic mice are crossed with mice that harbor functional rearranged or non-rearranged human H chain immunoglobulin loci and functionally inactivated endogenous H chain Ig loci as described in the art. The functional inactivation of the two copies of each of the three host Ig loci (heavy chain, kappa and lambda light chain), where the host contains the human IgH and the rearranged human VL transgene would allow for the production of purely human antibody molecules without the production of host or host human chimeric antibodies. Such a host strain, by immunization with specific antigens, would respond by the production of mouse B-cells producing specific human antibodies, which B-cells are subsequently fused with mouse myeloma cells or are immortalized in any other manner for the continuous stable production of human monoclonal antibodies. Alternatively, said population of B cells is used as a source of VH regions that can be obtained by constructing cDNA libraries or by PCR amplification using primers for human VH regions as is known in the art.

A human rearranged VL gene is reconstructed in an appropriate eukaryotic or prokaryotic microorganism and the resulting DNA fragments can be introduced into pronuclei of fertilized mouse oocytes or embryonic stem cells. Various constructs that direct B cell specific expression of VL transgenes have been described in the art and have the following general format: a leader sequence and relevant upstream sequences to direct B cell specific expression of the transgene, a coding sequence of a human VL transgene, an enhancer sequence that directs B cell specific and high level expression of the transgene and a murine constant region gene. In a preferred format, the enhancer is the C-kappa 3' enhancer because it directs high level expression in B-lineage cells, but does not recruit somatic hypermutation when used in transgene constructs.

In one embodiment, murine hosts, preferably mice, comprising one or multiple copies of the transgene in the genome are isolated and analyzed for stable expression. Murine hosts are selected that show stable expression of the transgene over longer periods of time, preferably in B-cells. If required, different murine host lines comprising independent insertions of one or multiple copies of the transgene, preferably on different chromosomes, are crossed to obtain murine hosts with different insertions of one or multiple copies of the transgene to increase expression of the transgene in murine hosts, preferably in B-cells.

Further described is progeny of a transgenic murine host according to the invention, the progeny comprising, at least in its B-cell lineage, a light chain encoding sequence together with a means that renders the sequence resistant to DNA rearrangements and/or somatic hypermutations.

Further described is progeny of a transgenic murine host according to the invention, the progeny comprising an expression cassette for the expression of a desired proteinaceous molecule in cells during a certain stage of development in cells developing into mature B cells.

Additionally described is a cell that is isolated from a transgenic murine host according to the invention, the cell comprising a light chain encoding sequence together with a means that renders the sequence resistant to DNA rearrangements and/or somatic hypermutations.

Additionally described is a cell that is isolated from a transgenic murine host according to the invention, the cell comprising an expression cassette for the expression of a desired proteinaceous molecule in cells during a certain stage of development in cells developing into mature B cells. A cell, possibly an antibody-producing B-cell or a cell that is capable of differentiating or maturating into an antibody-producing B-cell, can be used for *in vitro* production of antibodies, as is known to the skilled person, for example from Gascan et al. 1991. J. Exp. Med. 173: 747-750. Methods for immortalization of a cell are known in the art and include the generation of hybridomas, for example by fusion with a myeloma cell, transformation with Epstein Barr Virus; expression of the signal transducer of activation and transcription (STAT), activation via CD40 and IL4 receptor signaling, and/or expression of Bcl6 (Shvarts et al. 2002. Genes Dev 16: 681-686).

In a separate step the mouse endogenous Kappa and Lambda light chain loci are rendered essentially non-functional such that at least the majority of B cells in the transgenic mice bear Ig receptors that contain the transgenic human VL region. Inactivation of the endogenous mouse immunoglobulin loci is achieved by targeted disruption of the appropriate loci by homologous recombination in mouse embryonic stem cells. Said targeted disruption comprises alteration of the genomic sequence such that substantially no functional endogenous mouse immunoglobulin Kappa and/or Lambda light chain is produced. The term "substantially no functional endogenous mouse immunoglobulin" indicates that the endogenous Kappa and/or Lambda light chain loci are functionally silenced such that the level of functional protein expression of the endogenous Kappa and/or Lambda light chain loci, preferably the endogenous Kappa light chain locus, is reduced to about 20% of the level of expression in a reference mouse, more preferred to about 10%, more preferred to about 5%, more preferred to about 2% and more preferred to about 1%. In a most preferred embodiment, the level of functional protein expression of the endogenous Kappa and/or Lambda light chain loci is reduced to 0%. The level of functional protein expression can be determined by means known to the skilled person, including western blotting and pairing with a mouse heavy chain. Said reference mouse is a mouse in which the endogenous Kappa and/or Lambda light chain loci is not disrupted. Said alteration comprises mutation and/or deletion of gene sequences that are required for functional expression of the endogenous immunoglobulin genes. Alternatively, said alteration comprises insertion of a nucleic acid into the endogenous mouse immunoglobulin Kappa and/or Lambda light chain loci such that the functional expression of the endogenous immunoglobulin genes is reduced. In one aspect, said nucleic acid comprises a silencing element resulting in transcriptional silencing of the endogenous immunoglobulin gene. In a further aspect, or in addition, said nucleic acid comprises a sequence that disrupts splicing and/or translation of the endogenous immunoglobulin gene, for example by introducing an exon that renders a frame shift in the coding sequence, or that comprises a premature stop codon. In each case chimeric animals are generated which are derived in part from the modified embryonic stem cells and are capable of transmitting the genetic modifications through the germ line. The mating of mouse strains with human immunoglobulin loci to strains with inactivated mouse loci yields animals which produce antibodies comprising essentially only human light chains.

A construct for homologous recombination is prepared by means known in the art and any undesirable sequences are removed, e.g., procaryotic sequences. Any convenient technique for introducing a construct for homologous recombination into a target cell may be employed. These techniques include spheroplast fusion, lipofection, electroporation, calcium phosphate-mediated DNA transfer or direct microinjection. After transformation or transfection of the target cells, target cells are selected by means of positive and/or negative markers, for example by neomycin resistance and/or acyclovir and/or gancyclovir resistance. Those cells which show the desired phenotype may then be further analyzed by restriction analysis, electrophoresis, Southern analysis, PCR, or the like. By identifying fragments which show the presence of the lesion(s) at the target locus, cells in which homologous recombination has occurred to inactivate a copy of the target locus are identified.

Furthermore, it is shown that upon immunization, the murine and human VH regions in the afore-mentioned transgenic mice but not the VL regions are capable of undergoing somatic hypermutations to generate high affinity antibodies. Advantageously, these antibodies encoded by germline VL regions are predicted to contribute to lower immunogenicity when applied in humans and result in more stable antibodies that are less prone to aggregation and thus safer for therapeutic use in humans.

MAbs derived from the afore-mentioned murine transgenic hosts or cells all share the same identical human VL regions. It has been described that mAbs that share the same identical VL region may be co-expressed in a single clonal cell for the production of mixtures of recombinant antibodies with functional binding sites (see WO04106375 and WO05068622). Thus, the invention describes a platform for the generation of specific and high affinity mAbs that constitute the basis for mixtures of mAbs produced by clonal cells.

It is preferred that mAbs derived from the afore-mentioned murine transgenic hosts or cells are directed against cellular targets. Preferred targets are human surface-expressed or soluble proteins or carbohydrate molecules. Further preferred targets are surface-expressed proteins or carbohydrate molecules that are expressed on the surface of bacteria, viruses, and other pathogens, especially of humans.

More specifically, preferred targets include cytokines and chemokines, including but not limited to InterLeukin 1beta (IL1beta), IL2, IL4, IL5, IL7, IL8, IL12, IL13, IL15, IL18, IL21, IL23 and chemokines such as, for example, CXC chemokines, CC chemokines, C chemokines (or γ chemokines) such as XCL1 (lymphotactin-α) and XCL2 (lymphotactin-β), and CX3C chemokines. Further included as preferred targets are receptor molecules of the cytokines and chemokines, including type I cytokine receptors such as, for example, the IL-2 receptor, type II cytokine receptors such as, for example interferon receptors, immunoglobulin (Ig) superfamily receptors, tumor necrosis factor receptor family including receptors for CD40, CD27 and CD30, serine/threonine-protein kinase receptors such as TGF beta receptors, G-protein coupled receptors such as CXCR1-CXCR7, and tyrosine kinase receptors such as fibroblast growth factor receptor (FGFR) family members, EGF receptor family members including erbB 1 (EGF-R; HER1), erbB2, (HER2), erbB3 (HER3), and erbB4 (HER4), insulin receptor family members including IGF-R1 and IGF-RII, PDGF receptor family members, Hepatocyte growth factor receptor family members including c-Met (HGF-R), Trk receptor family members, AXL receptor family members, LTK receptor family members, TIE receptor family members, ROR receptor family members, DDR receptor family members, KLG receptor family members, RYK receptor family members, MuSK receptor family members, and vascular endothelial growth factor receptor (VEGFR) family members.

Further preferred targets are targets that are over-expressed or selectively expressed in tumors such as, for example, VEGF, CD20, CD38, CD33, CEA, EpCAM, PSMA, CD54, Lewis Y, CD52, CD40, CD22, CD51/CD61, CD74, MUC-1, CD38, CD 19, CD262 (TRAIL-R2), RANKL, CTLA4, and CD30; targets that are involved in chronic inflammation such as, for example, CD25, CD11a, TNF, CD4, CD80, CD23, CD3, CD14, IFNgamma, CD40L, CD50, CD122, TGFbeta and TGFalpha.

Preferred surface-expressed proteins or carbohydrate molecules that are expressed on the surface of bacteria, viruses, and other parasitic pathogens, especially of humans, include surface markers of influenza A and B viruses such as hemagglutinin (HA) and neuraminidase (NA), filoviruses such as Ebola virus, rabies, measles, rubella, mumps, flaviviruses such as Dengue virus types 1-4, tick-borne encephalitis virus, West Nile virus, Japanese encephalitis virus, and Yellow fever virus, Paramyxoviruses including Paramyxovirus such as Parainfluenza 1, 3, Rubulavirus such as Mumpsvirus and Parainfluenza 2, 4, Morbillivirus, and Pneumovirus such as Respiratory syncytial virus, Vaccinia, small pox, coronaviruses, including Severe Acute Respiratory Syndrome (SARS) virus, hepatitis virus A, B and C, Human Immunodeficiency Virus, Herpes viruses, including cytomegalovirus, Epstein Barr virus, Herpes simplex virus, and Varicella zoster virus, parvoviruses such as, for example, B19; Legionella pneumophila; Listeria monocytogenes; Campylobacter jejuni; Staphylococcus aureus; E. coli O157:H7; Borrelia burgdorferi; Helicobacter pylori; Ehrlichia chaffeensis; Clostridium difficile; Vibrio cholera; Salmonella enterica Serotype Typhimurium; Bartonella henselae; Streptococcus pyogenes (Group A Strep); Streptococcus agalactiae (Group B Strep); Multiple drug resistant S. aureus (e.g. MRSA); Chlamydia pneumoniae; Clostridium botulinum; Vibrio vulnificus; Parachlamydia pneumonia; Corynebacterium amycolatum; Klebsiella pneumonia; Linezolid-resistant enterococci (E. faecalis and E. faecium); and Multiple drug resistant Acinetobacter baumannii.

Most preferred targets are IL-6 and its receptor, IL-6Ralpha, glycoprotein-denominated gp130, RSV, especially the surface proteins F, G and SH and non-structural proteins such as N and M, and receptor tyrosine kinases, in particular erbB1 (EGF-R; HER1), erbB2, (HER2), erbB3 (HER3), erbB4 (HER4), IGF-R1 and IGF-RII, c-Met (HGF-R).

Therefore, the invention describes a platform for the generation of specific and high affinity mAbs against the above mentioned targets that constitute the basis for mixtures of mAbs produced by clonal cells. Said specific and high affinity mAbs comprise mAbs that are directed against different epitopes on at least one of the targets. Said specific and high affinity mAbs comprise mAbs that are directed against different targets, such as, for example, one or more members of the EGF-receptor family, including erbB1 (EGF-R; HER1), erbB2, (HER2), erbB3 (HER3) and erbB4 (HER4).

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (3rd edition, Cold Spring Harbor Laboratory Press, Cold Spring Harbor, N.Y. (2001)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

### Figure legends

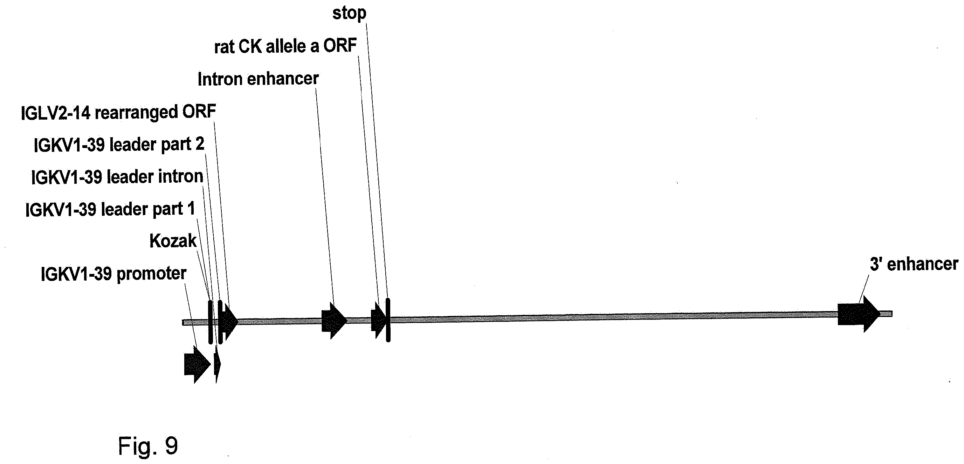

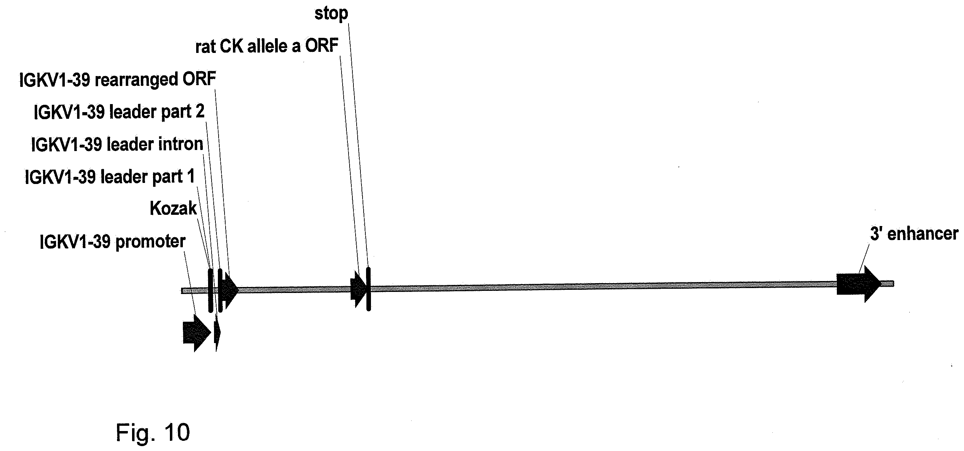

Figure 1
   A topology map of the annealing locations of mouse specific VH primers and the position of required restriction sites that are introduced by overhanging sequences at the 3' end of primers.
Figure 2
   PCR amplification steps (Amplification, Intermediate and Site introduction). The location and names of the mouse VH amplification primers (and mixtures of primers) are indicated per step.
Figure 3
   Topology of the MV1043 vector. This vector is used for the cloning of human or murine VH fragments. 012 (IGKV1-39) is indicated as the VL gene. Products of this vector in combination with helper phages in E. coli cells allow the generation of phages that display Fab fragments on the surface of the phage particles as a fusion product to the g3 protein and presence of the vector in the phage as the genetic content (F1 ORI).
Figure 4
   The topology of the mouse Ckappa locus downstream of the J-segments. Both enhancers and Ckappa region are indicated. The lower arrow indicates the region that is removed in order to silence the locus.
Figure 5
   The topology of the mouse C-lambda locus. All three active V-regions are indicated (Igl-V1, V2 and V3) as are the J-segments (Igl-J1, Igl-J2, Igl-J3, Igl-J4 and the pseudo segment Igl-J3p) and constant regions (Igl-C1, Igl-C2, Igl-C3 and Igl-C4). The regions that are deleted in order to silence the locus are indicated by deletion markers. These deletions include all active V genes (1, 2 and 3) and the intergenic segment between V2 and V3.
Figure 6
   Construct topology of IGKV1-39/J-Ck with an intron located in the leader open reading frame (ORF).
Figure 7
   Construct topology of IGLV2-14/J-Ck with an intron located in the leader open reading frame (ORF).
Figure 8
   Construct topology of VkP-IGKV1-39/J-Ck (VkP-O12). The promoter originates from the IGKV1-39 gene and is placed directly in front of the required elements for efficient transcription and translation. Intergenic sequences (including the enhancers) are derived from mice and obtained from BAC clones. The C-kappa sequence codes for the kappa constant region of rat.
Figure 9
   Construct topology of VkP-IGLV2-14/J-Ck (VkP-2a2). The promoter originates from the IGKV1-39 gene and is placed directly in front of the required elements for efficient transcription and translation. Intergenic sequences (including the enhancers) are derived from mice and obtained from BAC clones. The C-kappa sequence codes for the kappa constant region of rat.
Figure 10
   Construct topology of VkP-IGKV1-39/J-Ck-Δ1 (VkP-O12-del1) is identical to VkP-IGKV1-39/J-Ck from figure 9 except that the intron enhancer region is removed.
Figure 11
   Construct topology of VkP-IGKV1-39/J-Ck-Δ2 VkP-O12-del2) is identical to VkP-IGKV1-39/J-Ck-Δ1 from figure 10 except that a large piece of the intergenic region between the Ck gene and 3'enhancer is deleted. In addition, the 3' enhancer is reduced in size from 809 bp to 125 bp.
Figure 12
   Overview of the sequences used or referred to in this application.
Figure 13
   Generation of Rosa26-IgVk1-39 KI allele. (A) Schematic drawing of the pCAGGS-IgVK1-39 targeting vector. (B) Nucleotide sequence of the pCAGGS-IgVK1-39 targeting vector. (C) Targeting strategy.
Figure 14
   (A) Southern blot analysis of genomic DNA of ES clones comprising an insertion of the pCAGGS-IgVK1-39 targeting vector. Genomic DNA of 4 independent clones was digested with AseI and probed with 5e1 indicating the 5'-border of the targeting vector. All clones comprise a correct insertion of the targeting vector at the 5' end.
   (B) Southern blot analysis of genomic DNA of ES clones comprising an insertion of the pCAGGS-IgVK1-39 targeting vector. Genomic DNA of 4 independent clones was digested with MscI and probed with 3e1 indicating the 3'-border of the targeting vector. All clones comprise a correct insertion of the targeting vector at the 3' end.
   (C) Southern blot analysis of genomic DNA of ES clones comprising an insertion of the pCAGGS-IgVK1-39 targeting vector. Genomic DNA of 4 independent clones was digested with BamHI and probed with an internal Neo probe indicating the 5'-border of the targeting vector. All clones comprise a correct, single insertion of the targeting vector.
Figure 15
   Generation of Rosa26-IgVl2-14 KI allele. (A) Schematic drawing of the pCAGGS-IgVL2-14 targeting vector. (B) Nucleotide sequence of the pCAGGS-IgVL2-14 targeting vector containing the CAGGS expression insert based on the rearranged germline IGLV2-14/J V lambda region (IGLV2-14/J-Ck). (C) Targeting strategy.
Figure 16
   Epibase® profile of IGKV1-39 residues 1-107. Sub-figure A displays the binding strength for DRB1 allotypes, while C displays the binding strength for DRB3/4/5, DQ and DP allotypes. The values in the figure represent dissociation constants (Kd's) and are plotted on a logarithmic scale in the range 0.01 µM - 0.1 µM (very strong binders may have run off the plot). For medium binding peptides, qualitative values are given only, and weak and non-binders are not shown. Values are plotted on the first residue of the peptide in the target sequence (the peptide itself extends by another 9 residues). Importantly, only the strongest binding receptor for each peptide is shown: cross-reacting allotypes with lower affinity are not visible in this plot. The strongest binding receptor is indicated by its serotypic name. Finally, any germline-filtered peptides are plotted with a lighter color in the epitope map (in this case, no non-self epitopes were found). Sub-figure B shows the HLA binding promiscuity for every decameric peptide (Y-axis: the number of HLA allotypes recognizing critical epitopes in each of the peptides starting at the indicated residue shown on the X-axis). The promiscuity is measured as the number of allotypes out of the total of 47 for which the peptide is a critical binder. White columns refer to self-peptides, and black columns (absent here) to non-self peptides.
Figure 17
   Epitope map of IGKV1-39 showing the presence of peptide binders predicted in the sequence of IGKV1-39 by serotype in the 15-mer format. Each 15-mer is numbered as indicated in the top of the figure. The full sequence of the corresponding 15-mer is listed in Table 7. Black boxes indicate the presence of one or more critical self-epitopes in the 15-mer for the serotype listed on the left. Critical epitopes are operationally defined as strong or medium DRB1 binders and strong DRB3/4/5 or DP or DQ binders.
Figure 18
   Constitutive knock-out (KO) of the Ig kappa locus. (A) Targeting strategy. (B) Schematic drawing of the pIgKappa targeting vector.
Figure 19
   Constitutive KO of the Ig lambda locus. (A) First step of the targeting strategy. (B) Second step of the targeting strategy.
Figure 20
   Schematic drawing of targeting vectors. (A) pVkP-O12 (VkP-IGKV1-39/J-Ck); (B) pVkP-O12-del1 (VkP-IGKV1-39/J-Ck-Δ1); (C) pVkP-O12-del2 (VkP-IGKV1-39/J-Ck-Δ2).
Figure 21
   Targeting strategies for insertion of transgene into the Rosa26 locus by targeted transgenesis using RMCE. (A) VkP-O12 (VkP-IGKV1-39/J-Ck); (B) VkP-O12-del1 (VkP-IGKV1-39/J-Ck-Δ1); (C) VkP-O12-del2 (VkP-IGKV1-39/J-Ck-Δ2).
Figure 22
   Topology of the MV1057 vector. Replacing the indicated stuffer fragment with a VH fragment yields an expression vector that can be transfected to eukaryotic cells for the production of IgG1 antibodies with light chains containing an 012 (IGKV1-39) VL gene.
Figure 23
   Lack of transgenic human Vk1 light chain expression in non-B cell populations of the spleen.
Figure 24
   Transgenic human Vk1 light chain is expressed in all B cell populations of the spleen.
Figure 25
   Transgenic human Vk1 light chain is expressed in B1 cells of the peritoneal cavity.
Figure 26
   Transgenic human Vk1 light chain is not expressed in pro- and pre-B cells but in the immature and recirculating populations B cells in the bone marrow. (A) Gating of bone marrow cells. (B) Histograms of transgene expression with overlay from one WT control.
Figure 27
   Transgenic human Vk1 light chain is directly correlated with endogenous light chain and IgM expression in circulating B cells in the blood.

### Examples.

### Example 1

### Human light chain V-gene clones

This example describes the rationale behind the choice of two human light chain V- genes, one gene of the kappa type and one gene of the lambda type, that are used as a proof of concept for light chain expressing transgenic mice. De Wildt et al. 1999 (de Wildt et al. (1999) J. Mol. Biol. 285(3):895) analysed the expression of human light chains in peripheral IgG-positive B-cells. Based on these data, IGKV1-39 (012) and IGLV2-14 (2a2) were chosen as light chains as they were well represented in the B-cell repertoire. The J-segment sequence of the light chains has been chosen based upon sequences as presented in GenBank ABA26122 for IGKV1-39 (Rabquer,B.J., Smithson,S.L., Shriner,A.K. and Westerink,M.A.J.) and GenBank AAF20450 for IGLV2-14 (Ignatovich,O., Tomlinson,I.M., Popov,A.V., Bruggemann,M. and Winter,G. J. Mol. Biol. 294 (2), 457-465 (1999)).

All framework segments are converted into germline amino acid sequences to provide the lowest immunogenicity possible in potential clinical applications.

### Example 2

### Obtaining mouse heavy chain V-genes that pair with human IGKV1-39 gene segment to form functional antibody binding sites

This example describes the identification of mouse heavy chain V-genes that are capable of pairing with a single, rearranged human germline IGKV1-39/J region. A spleen VH repertoire from mice that were immunized with tetanus toxoid was cloned in a phage display Fab vector with a single human IGKV1-39-C kappa light chain and subjected to panning against tetanus toxoid. Clones obtained after a single round of panning were analyzed for their binding specificity. The murine VH genes encoding tetanus toxoid-specific Fab fragments were subjected to sequence analysis to identify unique clones and assign VH, DH and JH utilization.

Many of the protocols described here are standard protocols for the construction of phage display libraries and the panning of phages for binding to an antigen of interest and described in Antibody Phage Display: Methods and Protocols (editor(s): Philippa M. O'Brien, Robert Aitken).

### Immunizations

BALB/c mice received one immunization with tetanus toxoid and were boosted after 6 weeks with tetanus toxoid.

### Splenocyte isolation

Preparation of spleen cell suspension. After dissection, the spleen was washed with PBS and transferred to a 60 mm petridish with 20 ml PBS. A syringe capped with 20 ml PBS and a G20 needle was used to repeatedly flush the spleen. After washing the flushed cells with PBS, the cells were carefully brought into suspension using 20 ml PBS and left on a bench for 5 minutes to separate the splenocytes from the debris and cell clusters. The splenocytes suspension was transferred on top of a Ficoll-Paque™ PLUS-filled tube and processed according to the manufacturer's procedures for lymphocyte isolation (Amersham Biosciences).

### RNA isolation and cDNA synthesis

After isolation and pelleting of lymphocytes, the cells were suspended in TRIzol LS Reagent (Invitrogen) for the isolation of total RNA according to the accompanying manufacturer's protocol and subjected to reverse transcription reaction using 1 microgram of RNA, Superscript III RT in combination with dT20 according to manufacturer's procedures (Invitrogen).

### PCR amplification of cDNA

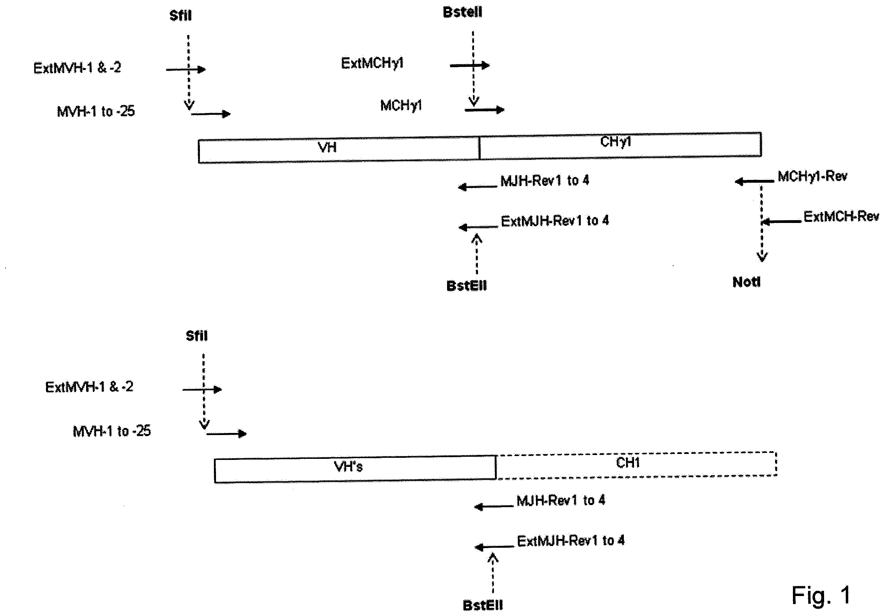

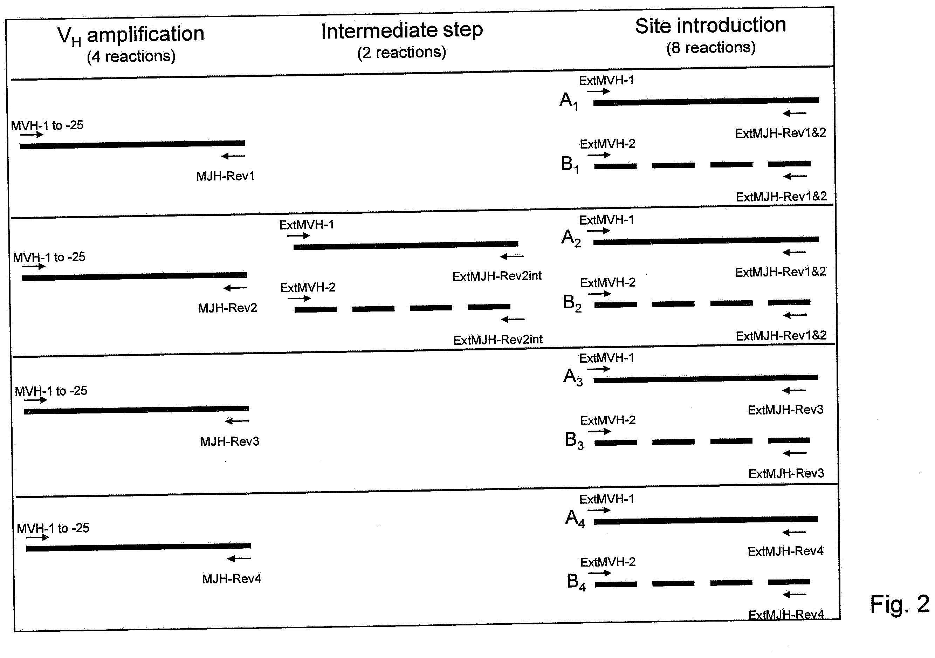

The cDNA was amplified in a PCR reaction using primer combinations that allow the amplification of approximately 110 different murine V-genes belonging to 15 VH families (table 1; RefSeq NG_005838; Thiebe et al., 1999. European Journal of Immunology 29: 2072 - 2081). In the first round, primer combinations that bind to the 5' end of the V-genes and 3' end of the J regions were used. In the second round, PCR products that were generated with the MJH-Rev2 primer were amplified in order to introduce modifications in the 3' region to enable efficient cloning of the products. In the last round of amplification, all PCR products were amplified using primers that introduce a SfiI restriction site at the 5' end and a BstEII restriction site at the 3' end (see figures 1 and 2, and table 1).

Reaction conditions for 1st round PCR: 4 different reactions combining all 25 forward primers (MVH1 to MVH25, table 1 and figure 2) and 1 reverse primer per reaction (MJH-Rev1, MJH-Rev2, MJH-Rev3 or MJH-Rev4; see table 1 and figure 2). 50 microliter PCR volumes were composed of 2 microliter cDNA (from RT reactions), 10 microliter 5* Phusion polymerase HF buffer, 40 nM of each of the 25 forward primers (total concentration of 1 micromolar), 1 micromolar reverse primer, 1 microliter 10 mM dNTP stock, 1.25 unit Phusion polymerase and sterile MQ water. The thermocycler program consisted of a touch down program: 1 cycle 98°C for 30 seconds, 30 cycles 98°C for 10 seconds, 58°C decreasing 0.2°C per cycle 10 seconds, 72°C 20 seconds and 1 cycle 72°C for 3 minutes. The second round PCR program was set up only for the products of the 1st PCR that contain the MJH-Rev2 primer: 2 different reactions combining either the ExtMVH-1 or ExtMVH-2 primers (table 1 and figure 2) in combination with the reverse primer ExtMJH-Rev2int (table 1 and figure 2). 50 microliter PCR volumes were composed of 50 ng PCR product (from first PCR round), 10 microliter 5* Phusion polymerase HF buffer, 500 nM of each forward primer, 1 micromolar reverse primer, 1 microliter 10 mM dNTP stock, 1.25 unit Phusion polymerase and sterile MQ water. The thermocycler program consisted of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. The third round PCR program was setup as described in figure 2. 50 microliter PCR volumes were composed of 50 ng PCR product (from earlier PCR rounds, figure 2), 10 microliter 5* Phusion polymerase HF buffer, 1 micromolar forward primer (table 1 and figure 2), 1 micromolar reverse primer, 1 microliter 10 mM dNTP stock, 1.25 unit Phusion polymerase and sterile MQ water. The program consists of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. After PCR amplifications, all PCR products were gel purified using Qiaex II according to the manufacturer's protocols.

### Restriction enzyme digestions

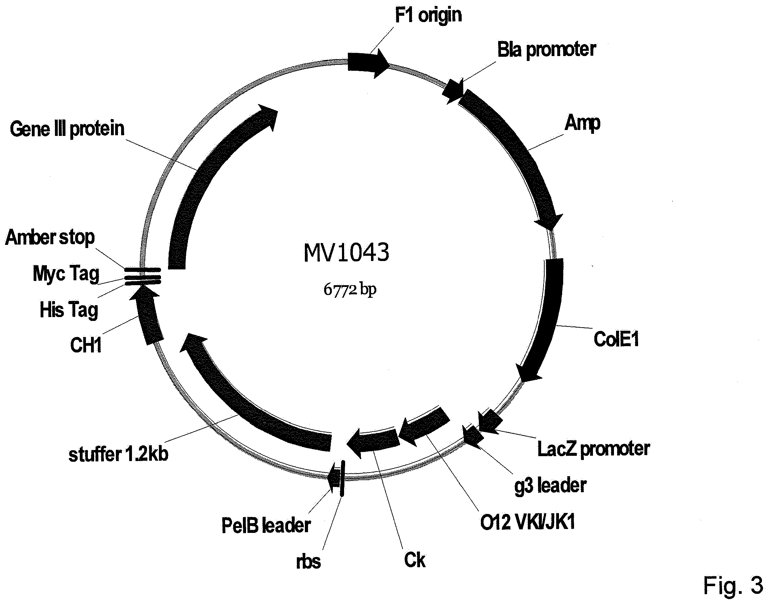

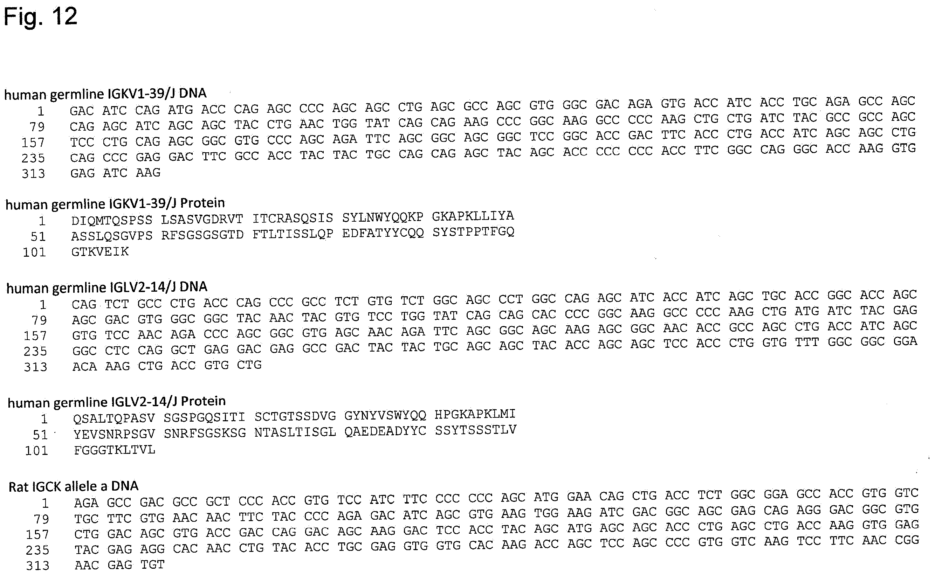

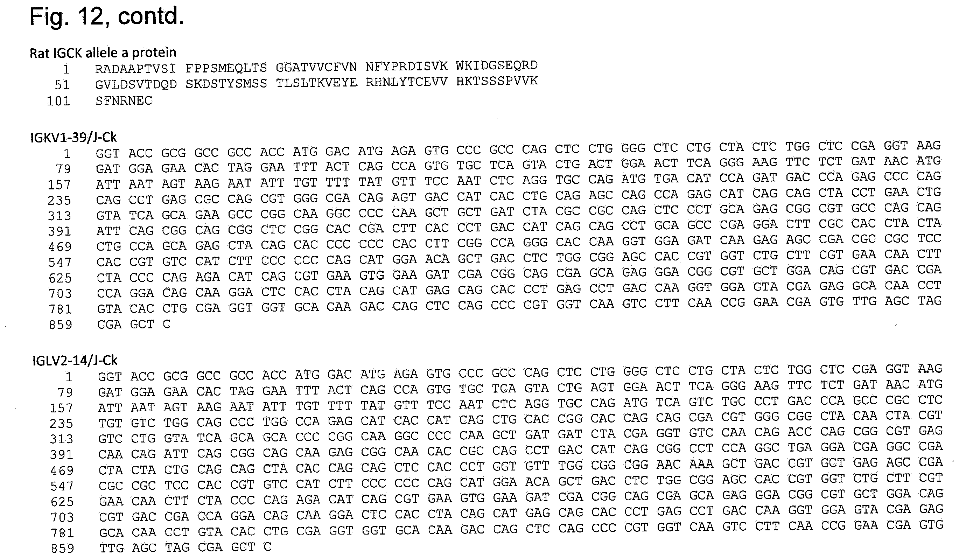

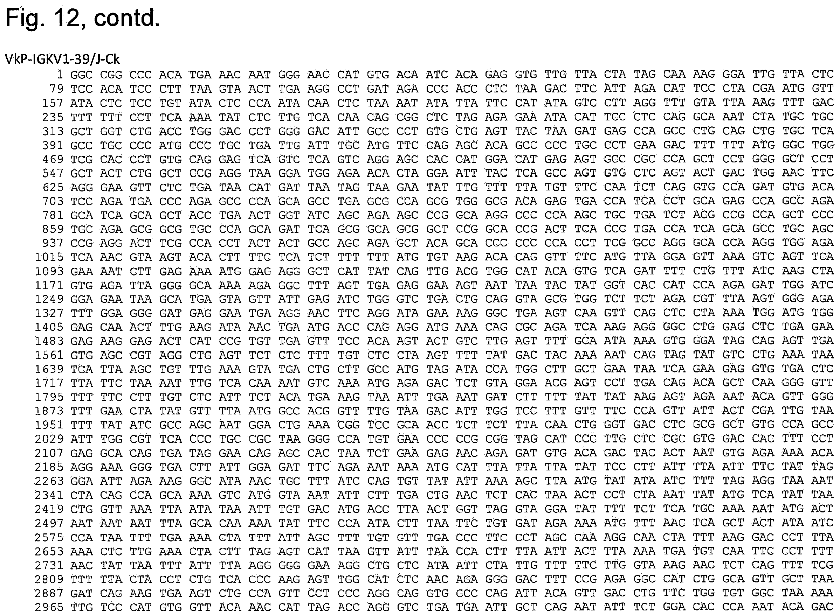

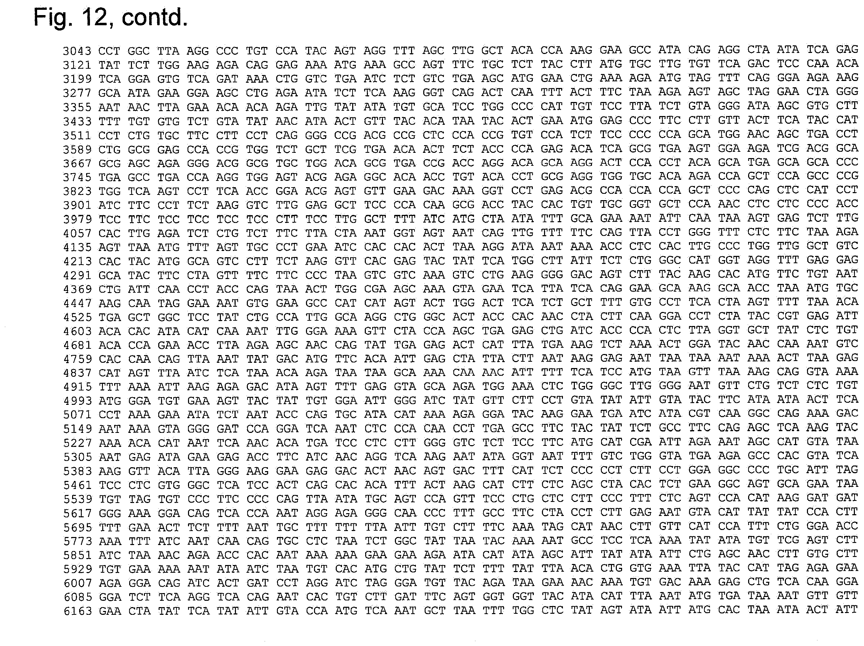

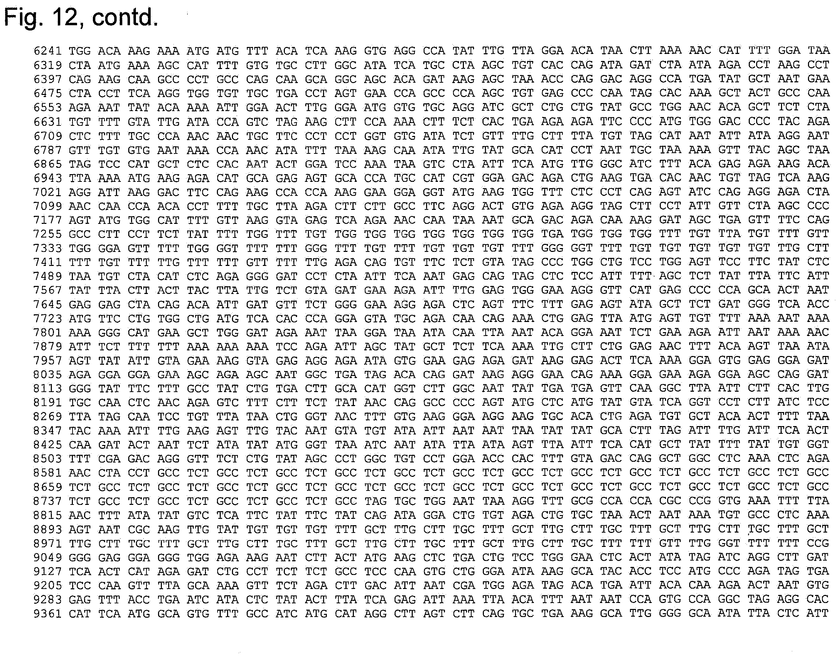

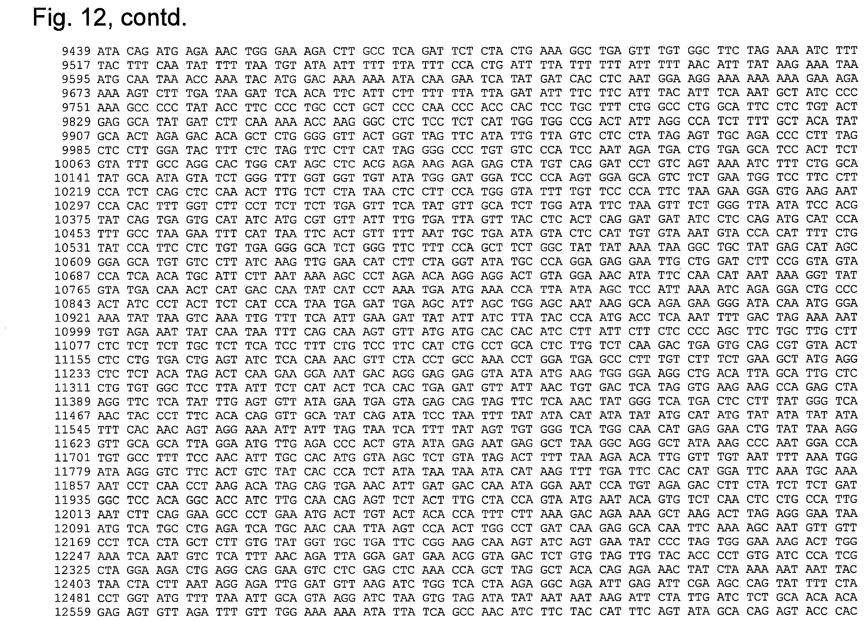

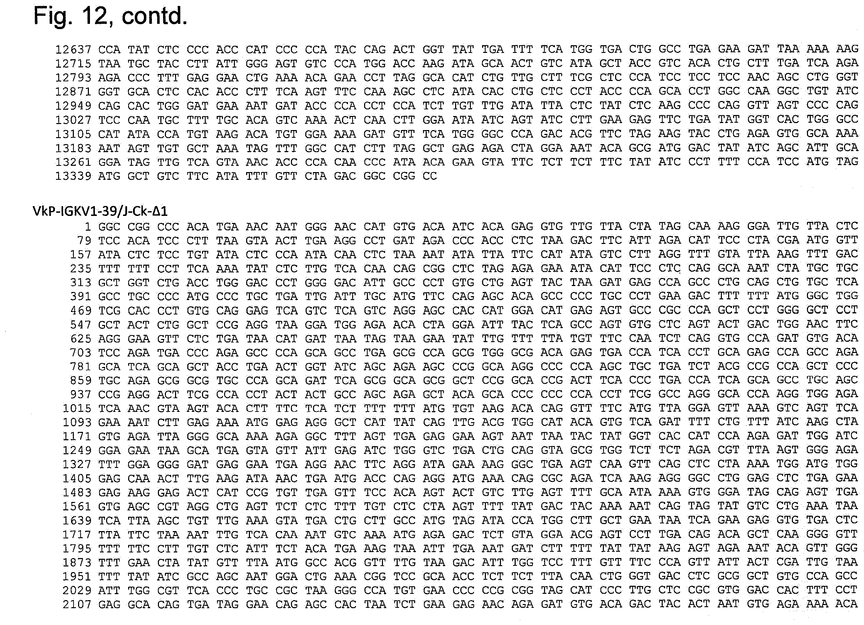

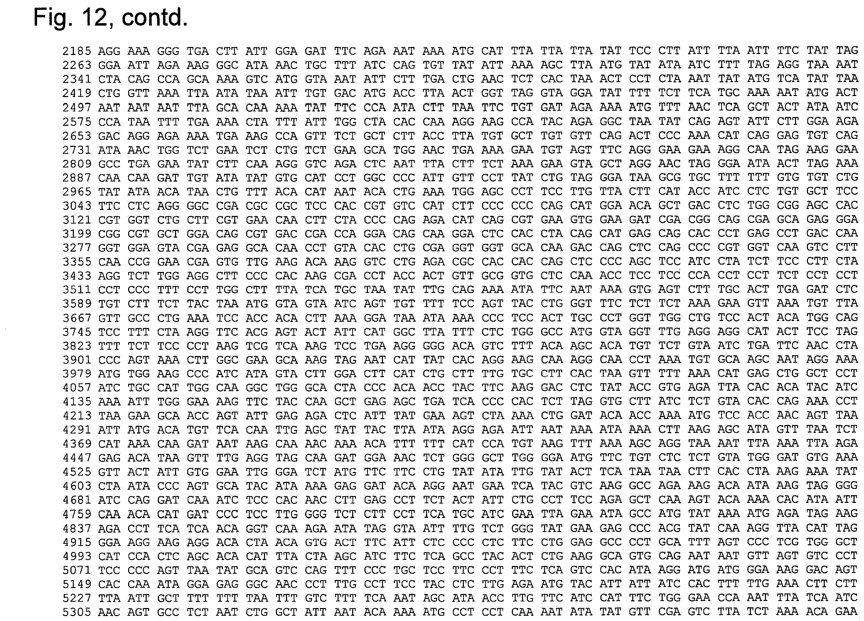

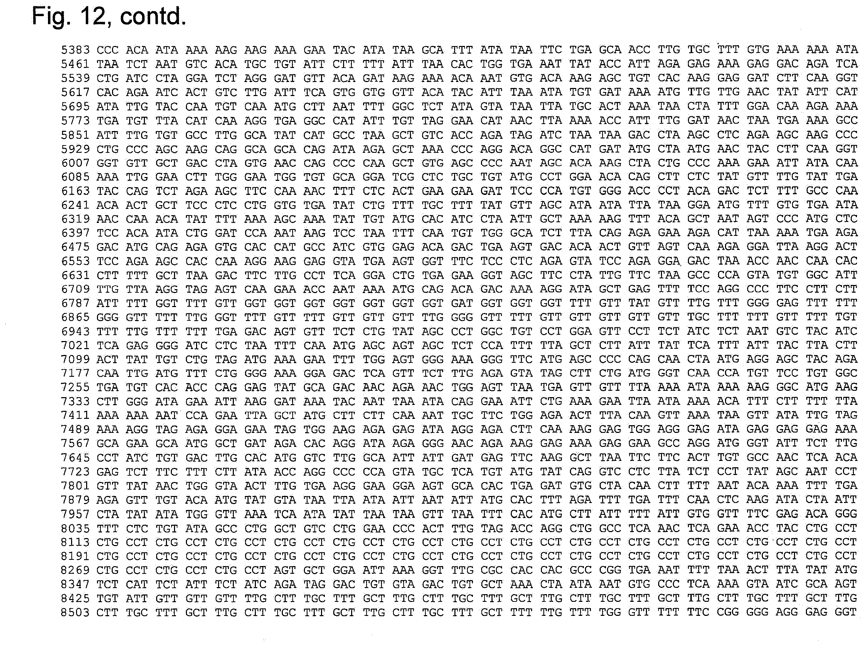

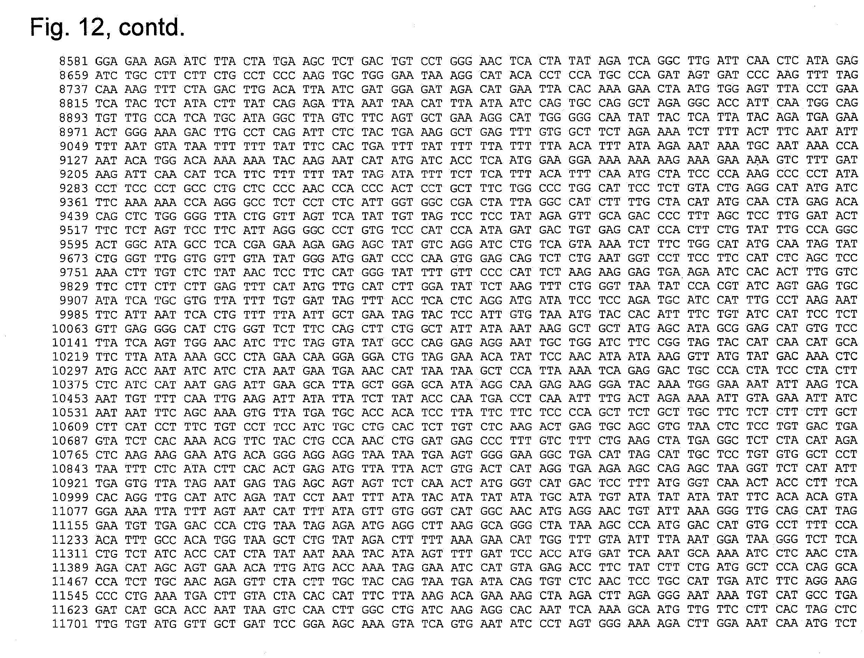

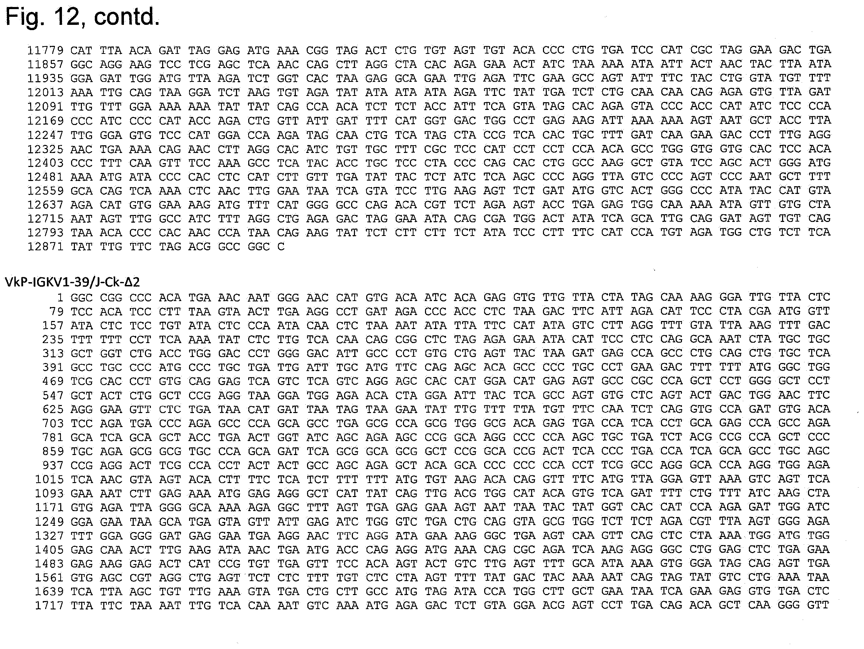

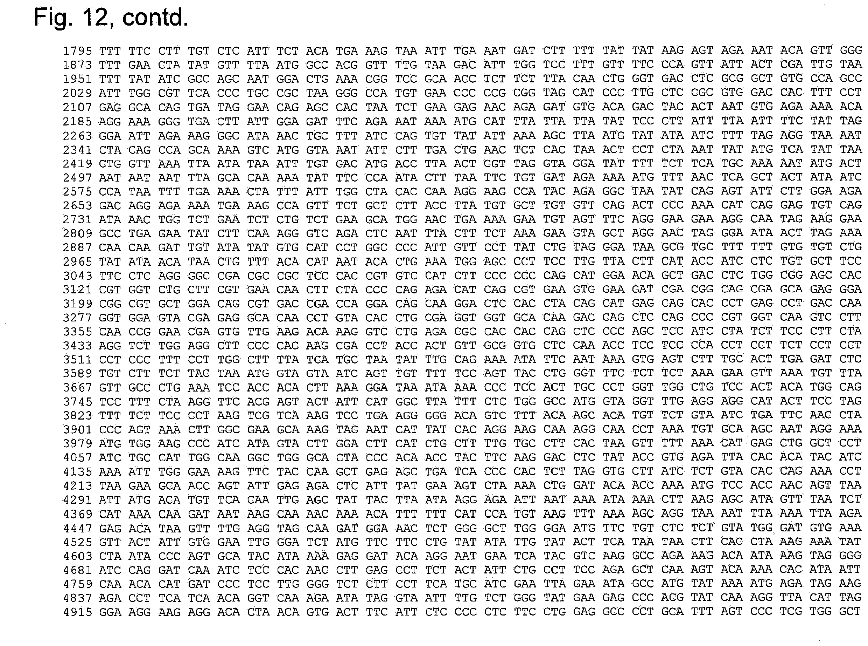

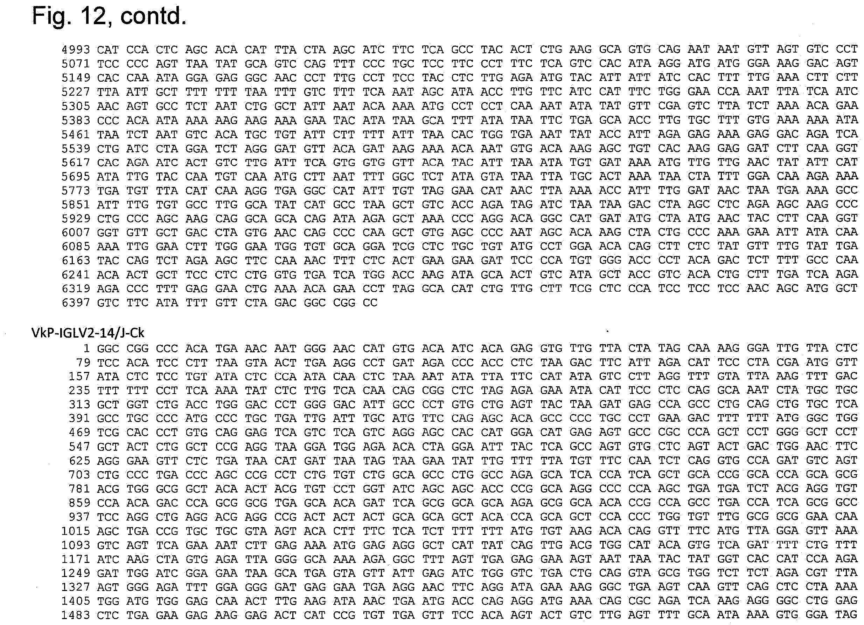

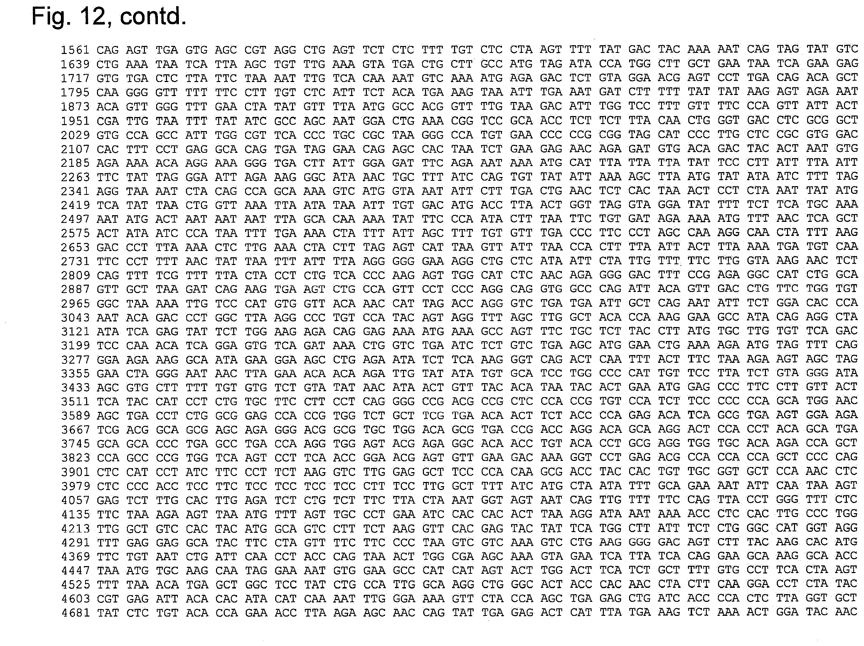

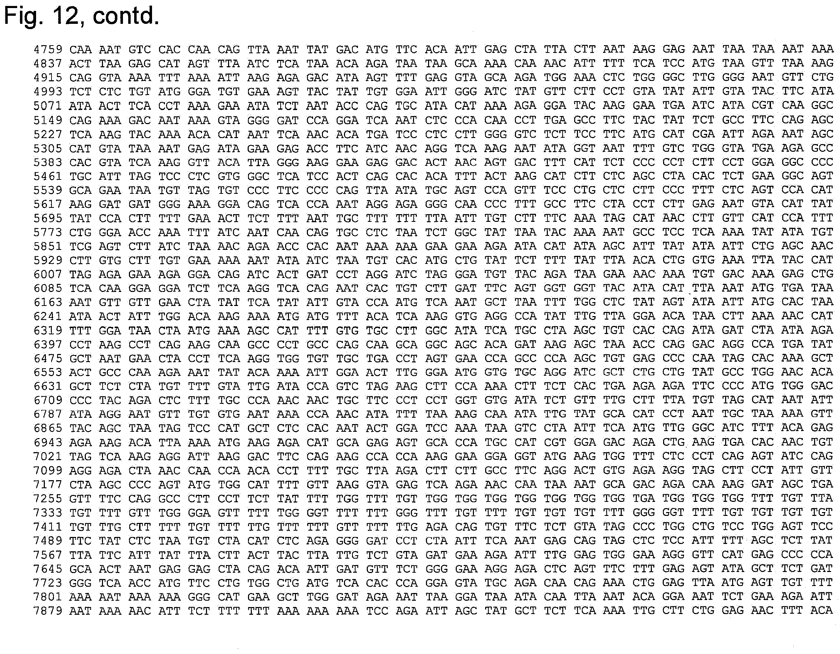

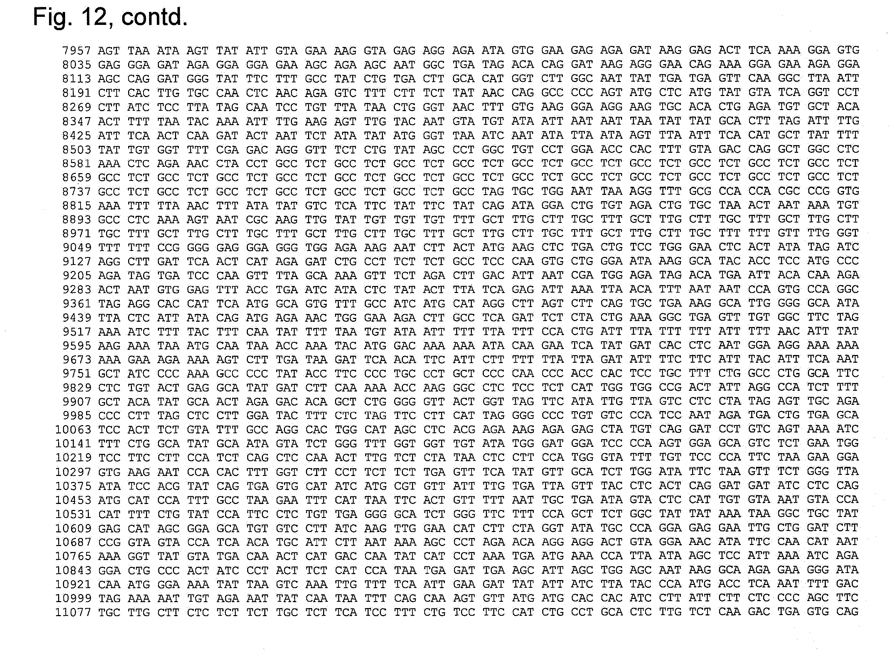

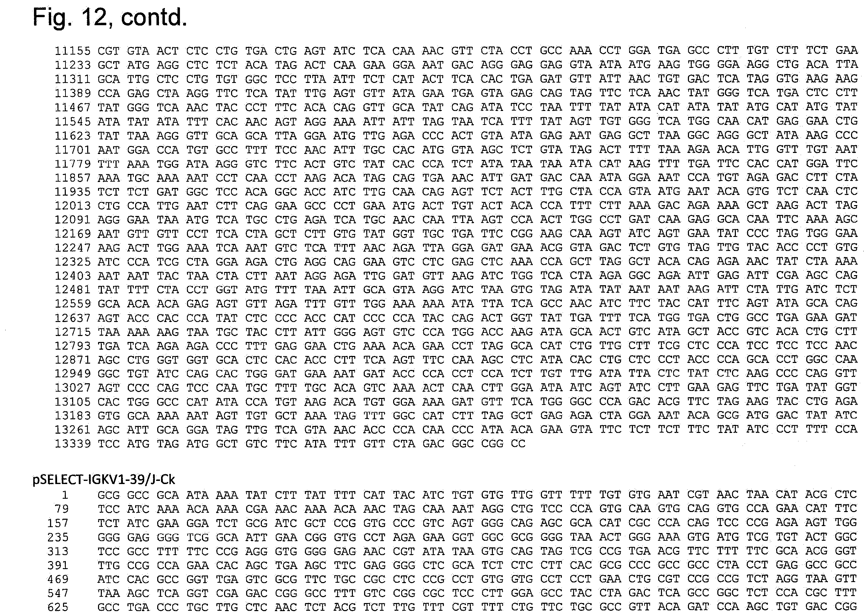

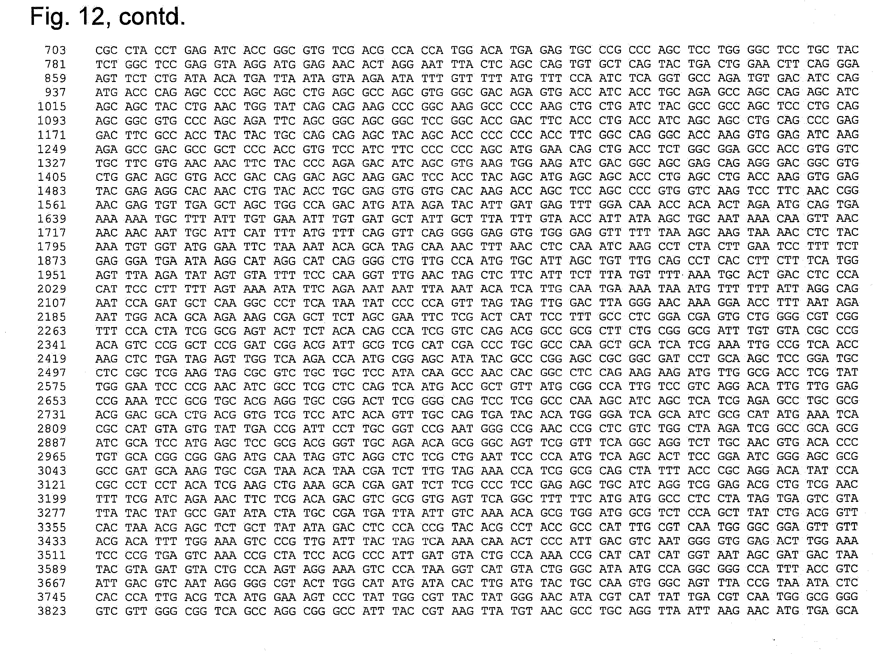

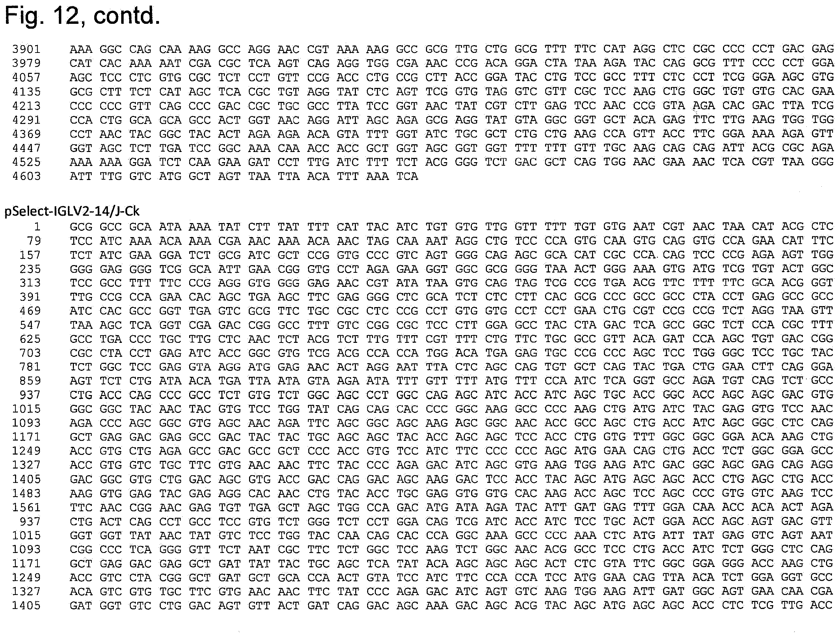

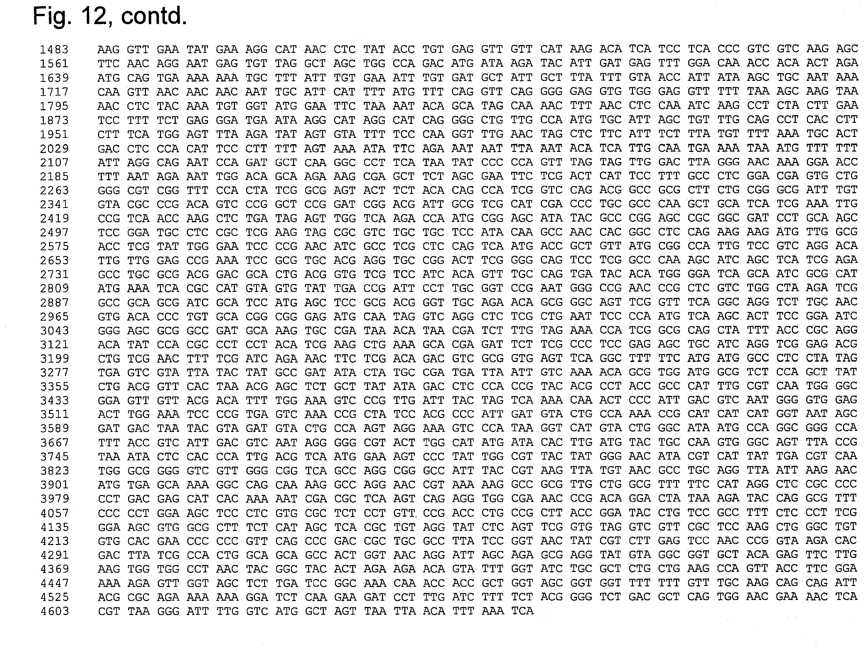

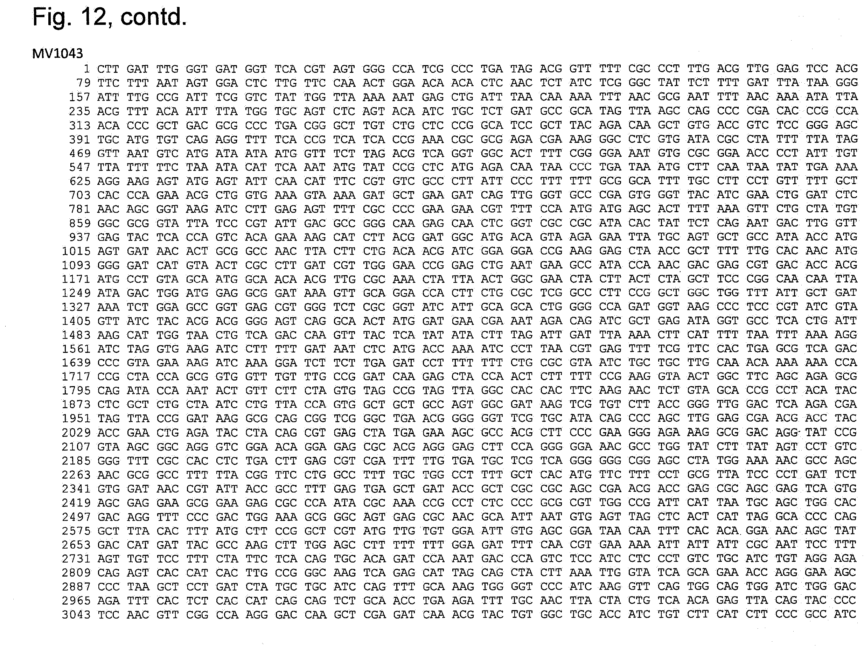

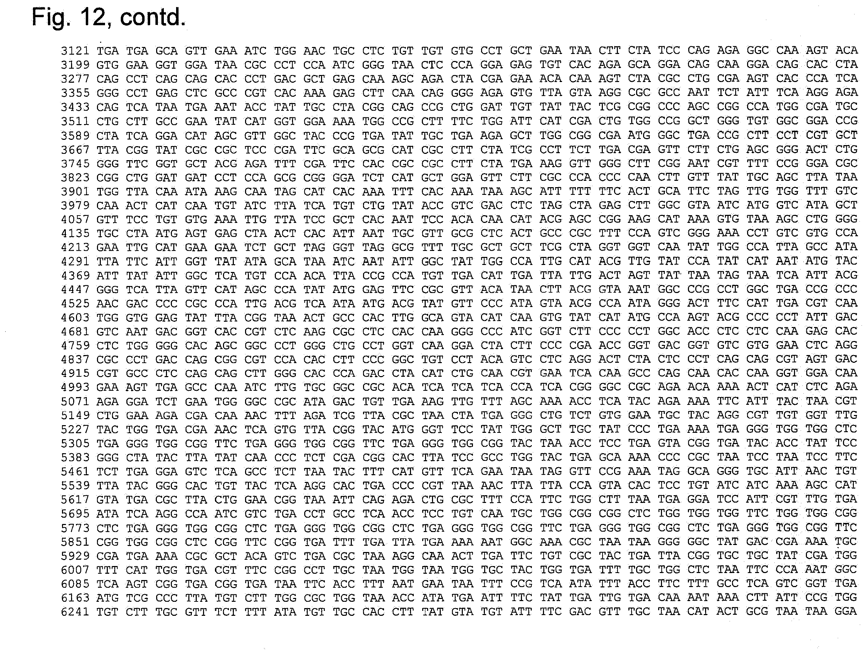

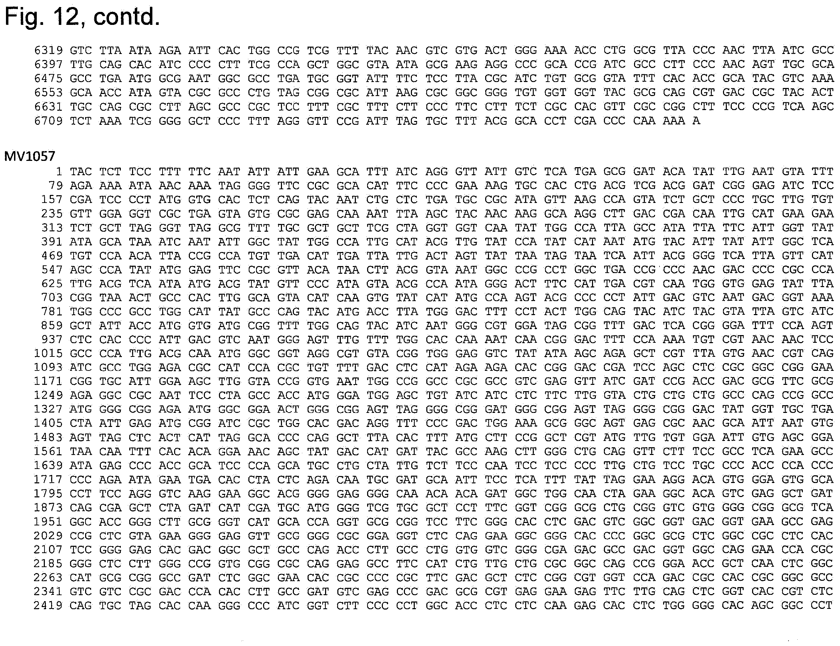

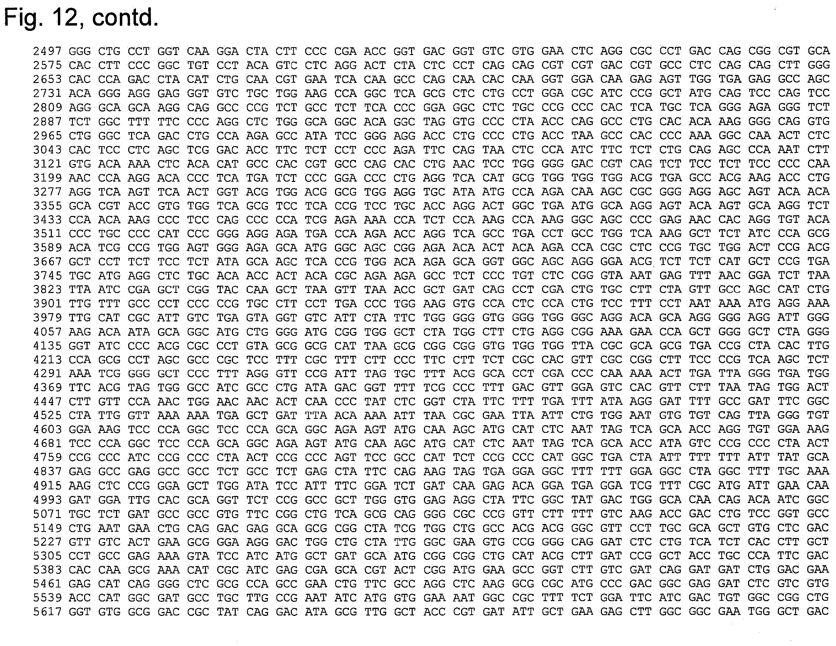

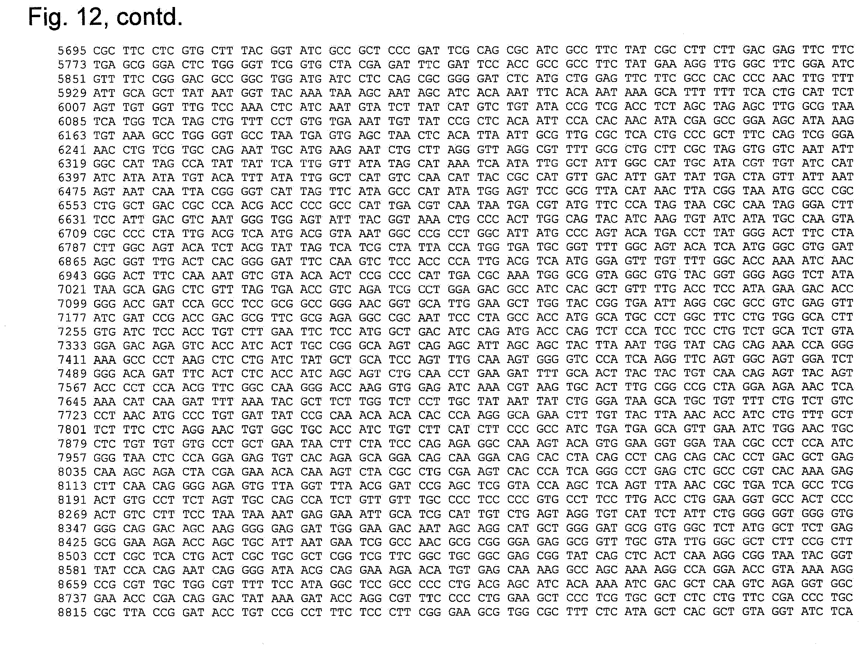

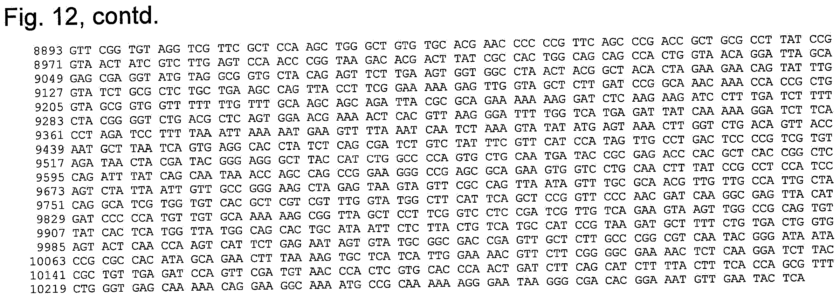

Purified products were digested with BstEII and SfiI in two steps. First 1 microgram of DNA was digested in 100 microliter reactions consisting of 10 microliters of 10* NEB buffer 3 (New England Biolabs), 1 microliter 100* BSA, 12.5 unit BstEII and sterile water for 6 hours at 60°C in a stove. The products were purified using Qiaquick PCR Purification kit from Qiagen according to the manual instructions and eluted in 40 microliter water. Next all products were further digested with SfiI in 100 microliter reactions consisting of 10 microliters of 10* NEB buffer 2 (New England Biolabs), 1 microliter 100* BSA, 12.5 unit SfiI and sterile water for 12 hours at 50°C in a stove. The digested fragments were purified by Qiaquick Gel Extraction kit following gel separation on a 20 cm 1.5% agarose TBE plus ethidium bromide gel at 80 V. 100 microgram of the acceptor vector (MV1043, figures 3 and 12) was digested with 50 units Eco91I in 600 microliter under standard conditions (Tango buffer) and next purified on a 0.9% agarose gel. After a second digestion step under prescribed conditions with 400 units SfiI in 500 microliter for 12 hours, 100 units BsrGI were added for 3 hours at 50°C.

### Ligations

Each PCR product was ligated separately according to the following scheme: 70 ng digested PCR products, 300 ng digested acceptor vector, 100 units T4 Ligase (NEB), 1* ligase buffer in 30 microliters for 16 hours at 12°C. The ligation reactions were purified with phenol/chloroform/isoamyl alcohol extractions followed by glycogen precipitations (Sigma-Aldrich #G1767) according to the manufacturer's protocol and finally dissolved in 25 microliter sterile water.

### Transformations and library storage

The purified ligation products were transformed by electroporation using 1200 microliter TG1 electrocompetent bacteria (Stratagene #200123) per ligation batch and plated on LB carbenicillin plates containing 4% glucose. Libraries were harvested by scraping the bacteria in 50 ml LB carbenicillin. After centrifugation at 2000g for 20 minutes at 4°C, the bacterial pellets were resuspended carefully in 2 ml ice cold 2*TY/30% glycerol on ice water and frozen on dry-ice/ethanol before storage at -80°C.

### Library amplification

Libraries were grown and harvested according to procedures as described by Kramer et al. 2003 (Kramer et al. 2003. Nucleic Acids Res. 31(11): e59) using VCSM13 (Stratagene) as helper phage strain.

### Selection of phages on coated immunotubes

Tetanus toxoid was dissolved in PBS in a concentration of 2 µg/ml and coated to MaxiSorp Nunc-Immuno Tube (Nunc 444474) overnight at 4°C. After discarding the coating solution, the tubes were blocked with 2% skim milk (ELK) in PBS (blocking buffer) for 1 hour at RT. In parallel, 0.5 ml of the phage library was mixed with 1 ml blocking buffer and incubated for 20 minutes at room temperature. After blocking the phages, the phage solution was added to the tetanus toxoid coated tubes and incubated for 2 hours at RT on a slowly rotating platform to allow binding. Next, the tubes were washed 10 times with PBS/0.05% Tween-20 followed by phage elution by an incubation with 1 ml 50 mM glycine-HCl pH 2.2 10 min at RT on rotating wheel and directly followed by neutralization of the harvested eluent with 0.5 ml 1 M Tris-HCl pH 7.5.

Harvesting phage clones 5 ml XL1-Blue MRF (Stratagene) culture at O.D. 0.4 was added to the harvested phage solution and incubated for 30 minutes at 37°C without shaking to allow infection of the phages. Bacteria were plated on Carbenicillin/Tetracycline 4% glucose 2*TY plates and grown overnight at 37°C.

### Phage production

Phages were grown and processed as described by Kramer et al. 2003 (Kramer et al. 2003. Nucleic Acids Res. 31(11): e59) using VCSM13 as helper phage strain.

### Phage ELISA

ELISA plates were coated with 100 microliter tetanus toxoid per well at a concentration of 2 microgram/ml in PBS overnight at 4°C. Plates coated with 100 microliter thyroglobulin at a concentration of 2 microgram/ml in PBS were used as a negative control. Wells were emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution were added and incubated for 1 hour at RT. Next 5 washing steps with PBS-0.05% Tween-20 removed unbound phages. Bound phages were detected by incubating the wells with 100 microliter anti-M13-HRP antibody conjugate (diluted 1/5000 in blocking buffer) for 1 hour at room temperature. Free antibody was removed by repeating the washing steps as described above, followed by TMB substrate incubation until color development was visible. The reaction was stopped by adding 100 microliter of 2 M H₂SO₄ per well and analyzed on an ELISA reader at 450 nm emission wavelength (Table 2). Higher numbers indicate stronger signals and thus higher incidence of specific binding of the phage-Fab complex.

### Sequencing

Clones that gave signals at least 3 times above the background signal (Table 2) were propagated, used for DNA miniprep procedures (see procedures Qiagen miniPrep manual) and subjected to nucleotide sequence analysis. Sequencing was performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). Mouse VH sequences of 28 tetanus toxoid binding clones are depicted in Table 3. The results show that the selected murine VH genes belong to different gene families, and different individual members from these gene families are able to pair with the rearranged human IGKV1-39/J VH region to form functional tetanus toxoid-specific antibody binding sites. From the sequence analyses, it was concluded that the murine VH regions utilize a diversity of DH and JH gene segments.

### Example 3

### Silencing of the mouse kappa light chain locus

This example describes the silencing of the mouse endogenous kappa light chain locus. The endogenous kappa locus is modified by homologous recombination in ES cells, followed by the introduction of genetically modified ES cells in mouse embryos to obtain genetically adapted offspring.

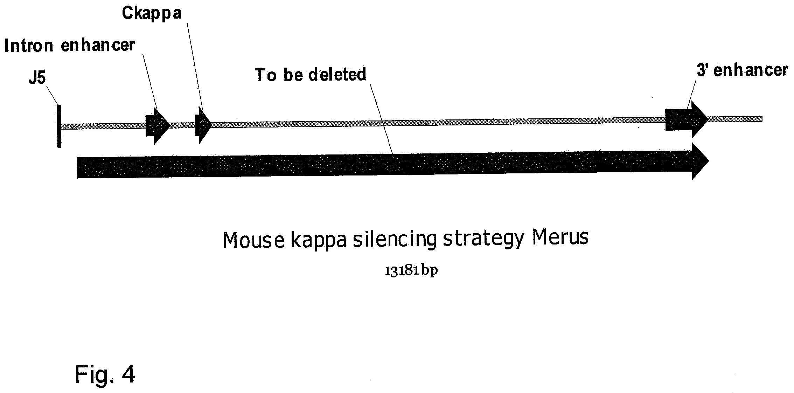

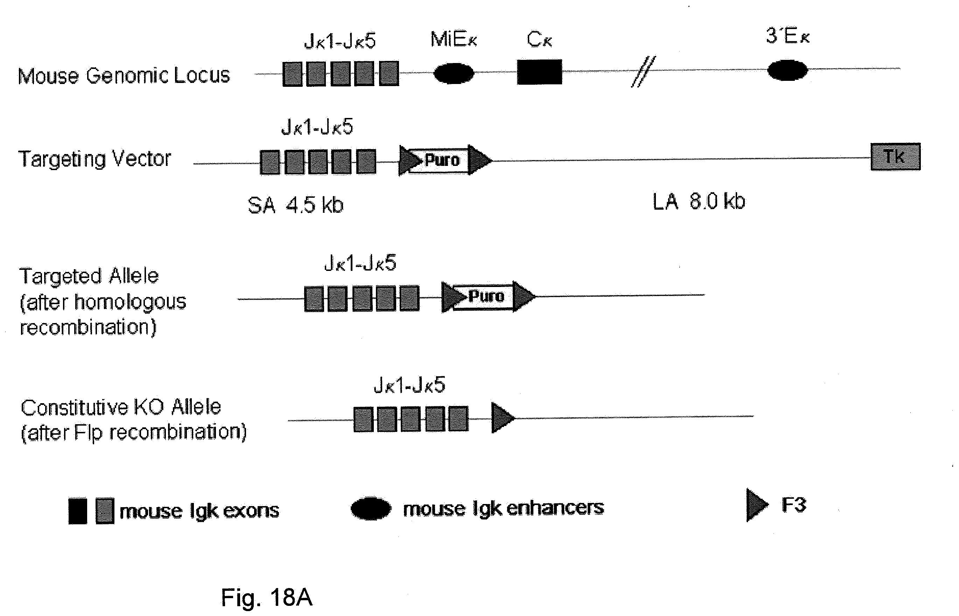

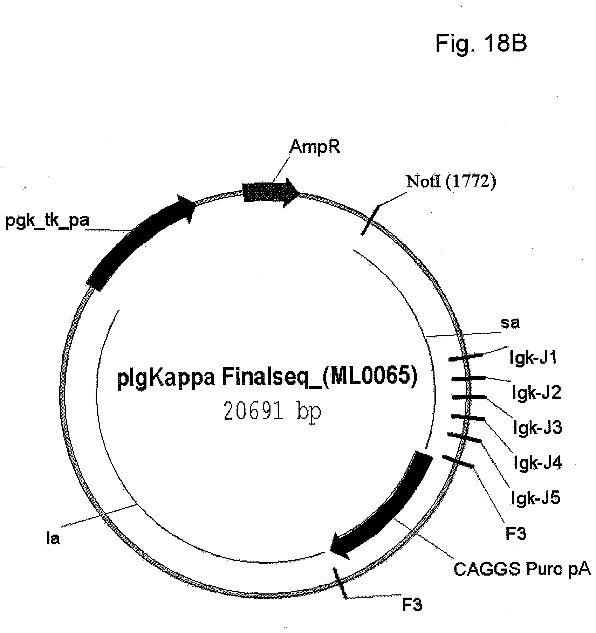

A vector that contains an assembled nucleotide sequence consisting of a part comprising the J-region to 338 bp downstream of the J5 gene segment fused to a sequence ending 3' of the 3' CK enhancer is used for homologous recombination in ES cells. The assembled sequence is used to delete a genomic DNA fragment spanning from 3' of the JK region to just 3' of the 3' CK enhancer. As a consequence of this procedure, the CK constant gene, the 3' enhancer and some intergenic regions are removed (see figures 4 and 18).

### Construction of the targeting vector

A vector that received 4.5-8 kb flanking arms on the 3' and 5' end fused to the deletion segment was used for targeted homologous recombination in an ES cell line. Both arms were obtained by PCR means ensuring maximum homology. The targeting strategy allows generation of constitutive KO allele. The mouse genomic sequence encompassing the Igk intronic enhancer, Igk constant region and the Igk 3' enhancer was replaced with a PuroR cassette, which was flanked by F3 sites and inserted downstream of the Jk elements. Flp-mediated removal of the selection marker resulted in a constitutive KO allele. The replacement of the Igk MiEk-Igk C-Igk 3'E genomic region (approx. 10 kb) with a F3-Puro cassette (approx. 3 kb) was likely to decrease the efficiency of homologous recombination. Therefore, the arms of homology were extended accordingly and more ES cell colonies were analysed after transfection in order to identify homologous recombinant clones.

### Generation of ES cells bearing the deleted kappa fragment

The generation of genetically modified ES cells was essentially performed as described (Seibler et al. Nucleic Acids Res. 2003 Feb 15;31(4):e12). See also example 14 for a detailed description.

Generation of ES mice by tetraploid embryo complementation.

The production of mice by tetraploid embryo complementation using genetically modified ES cells was essentially performed as described (Eggan et al., PNAS 98, 6209-6214; Seibler J, et al. Nucleic Acids Res. 2003 Feb 15;31(4):e12; Hogan et al., (Summary of mouse development. Manipulating the Mouse Embryo, (1994) Cold Spring Harbor Laboratory Press, Cold Spring Harbor NY.), pp. 253-289.)).

### Example 4

### Silencing of the mouse lambda light chain locus

This example describes the silencing of the mouse endogenous lambda light chain locus. The endogenous lambda locus is modified by homologous recombination in ES cells followed by the introduction of genetically modified ES cells in mouse embryos to obtain genetically adapted offspring.

Two regions of the murine lambda locus that together contain all functional lambda V regions are subject to deletion.

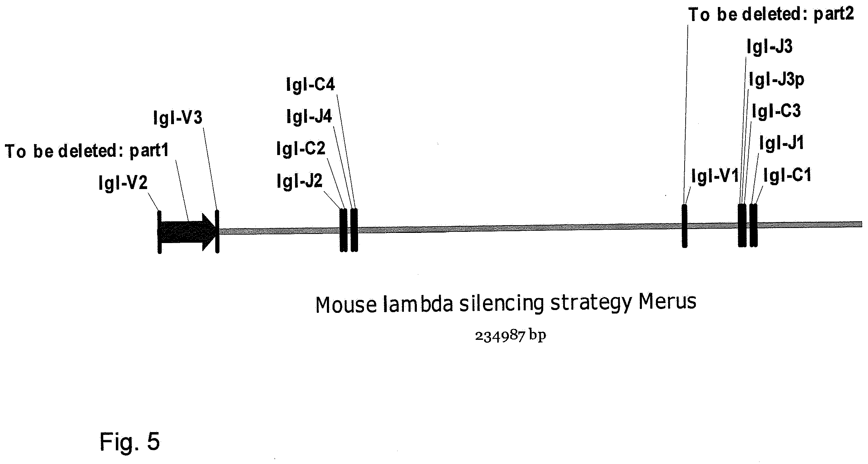

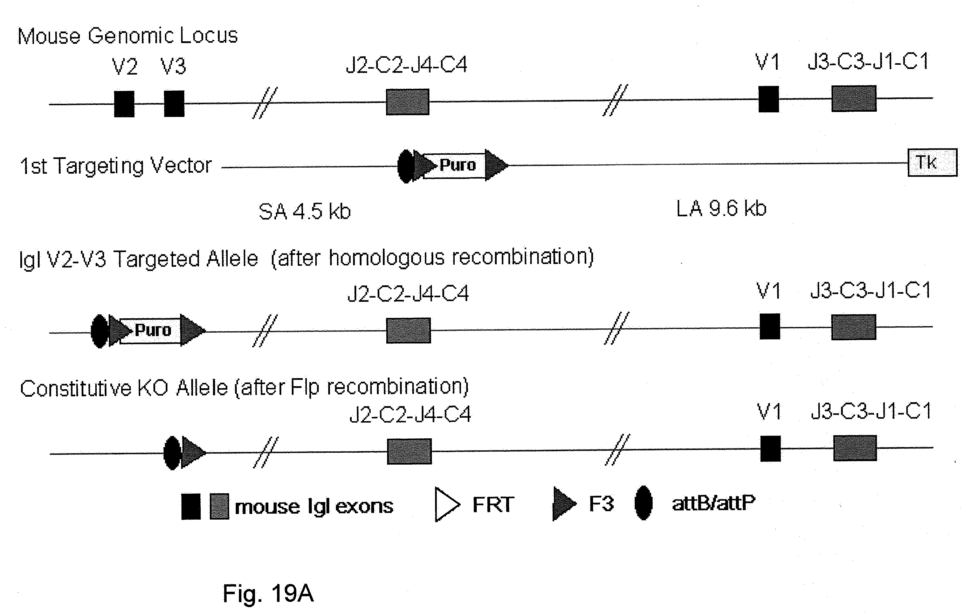

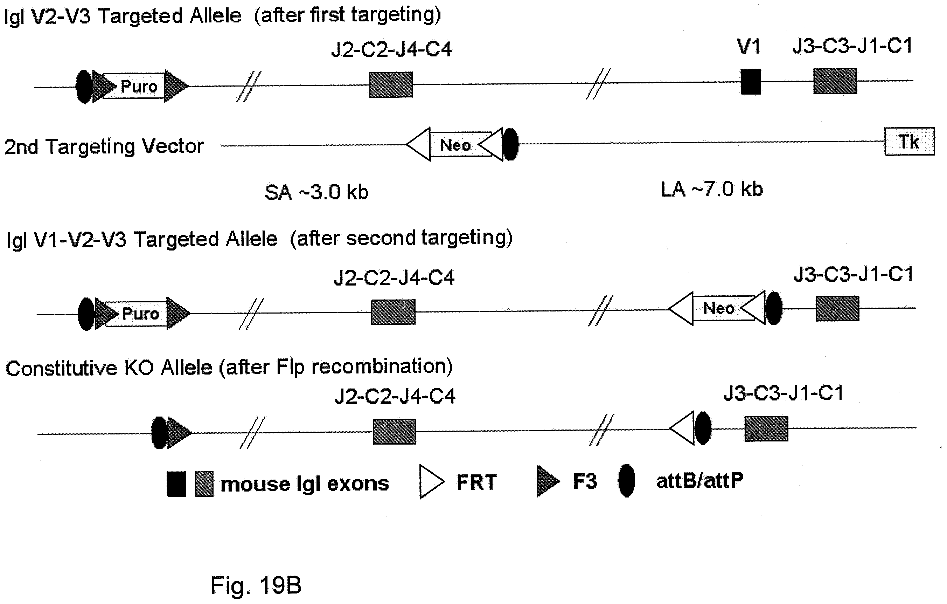

The first region targeted for homologous recombination-based deletion is a region that is located 408 bp upstream of the start site of the IGLV2 gene segment and ends 215 bp downstream of IGLV3 gene segment, including the intergenic sequence stretch between these IGLV gene segments. The second region that is subject to a deletion involves the IGLV1 gene segment consisting of a fragment spanning from 392 bp upstream to 171 bp downstream of the IGLV1 gene segment. As a consequence of these two deletion steps, all functional V-lambda genes segments are deleted, rendering the locus functionally inactive (figures 5 and 19).

### Construction of the targeting vectors

Vectors that received 3-9.6 kb flanking arms on the 3' and 5' end fused to the deletion segment were used for targeted homologous recombination in an ES cell line. Both arms were obtained by PCR means ensuring maximum homology. In a first step, the mouse genomic sequence encompassing the Igl V2-V3 regions were replaced with a PuroR cassette flanked by F3 sites, which yields a constitutive KO allele after Flp-mediated removal of selection marker (see figure 19A). In a second step, the mouse genomic sequence encompassing the Igl V1 region was replaced with a Neo cassette in ES cell clones which already carried a deletion of the Igl V2-V3 regions (see figure 19B). The selection marker (NeoR) was flanked by FRT sites. A constitutive KO allele was obtained after Flp-mediated removal of selection markers.

### Generation of ES cells bearing the deleted lambda fragment

The generation of genetically modified ES cells was essentially performed as described (Seibler J, Zevnik B, Küter-Luks B, Andreas S, Kern H, Hennek T, Rode A, Heimann C, Faust N, Kauselmann G, Schoor M, Jaenisch R, Rajewsky K, Kühn R, Schwenk F. Nucleic Acids Res. 2003 Feb 15;31(4):e12). See also example 14 for a detailed description. To show that both targeting events occurred on the same chromosome several double targeted clones were selected for the in vitro deletion with pCMV C31deltaCpG. The clones were expanded under antibiotic pressure on a mitotically inactivated feeder layer comprised of mouse embryonic fibroblasts in DMEM High Glucose medium containing 20% FCS (PAN) and 1200 u/mL Leukemia Inhibitory Factor (Millipore ESG 1107). 1x10⁷ cells from each clone were electroporated with 20 µg of circular pCMV C31deltaCpG at 240 V and 500 µF and plated on four 10 cm dishes each. 2 - 3 days after electroporation cells were harvested and analysed by PCR. Primers used were:
2005_5: CCCTTTCCAATCTTTATGGG
2005_7: AGGTGGATTGGTGTCTTTTTCTC
2005_9: GTCATGTCGGCGACCCTACGCC

PCR reactions were performed in mixtures comprising 5 µl PCR Buffer 10x (Invitrogen), 2 µl MgCl₂ (50 mM), 1 µl dNTPs (10 mM), 1 µl first primer (5 µM), 1 µl second primer (5 µM), 0,4 µl Taq (5 U/ul, Invitrogen), 37,6µl H₂O, and 2 µl DNA. The program used was 95°C 5'; followed by 35 cycles of 95°C 30"; 60°C 30"; 72°C 1'; followed by 72°C 10'.

### Generation of ES mice by tetraploid embryo complementation.

The production of mice by tetraploid embryo complementation using genetically modified ES cells was essentially performed as described (Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kühn R, Schwenk F. Nucleic Acids Res. 2003 Feb 15;31(4):e12; Hogan et al., (Cold Spring Harbor Laboratory Press, Cold Spring Harbor NY.), pp. 253-289).

### Example 5

### Construction of the CAGGS expression insert based on a rearranged human germline IGKV1-39/J-Ck gene (IGKV1-39/J-Ck)

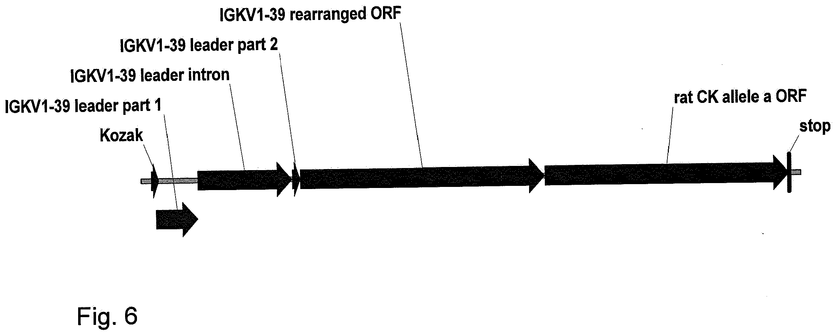

This example describes the construction of a CAGGS expression cassette incorporating the rearranged human germline IGKV1-39/J region. This insert expression cassette encompasses cloning sites, a Kozak sequence, a leader sequence containing an intron, an open reading frame of the rearranged IGKV1-39 region, a rat CK constant region from allele a and a translational stop sequence (IGKV1-39/J-Ck; figure 6). The primary construct consists of naturally occurring sequences and has been analyzed and optimized by removing undesired cis acting elements like internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions is optimized for expression in mice. The intron sequence is unchanged and thus represents the sequence identical to the coding part of the human IGKV1-39 leader intron.

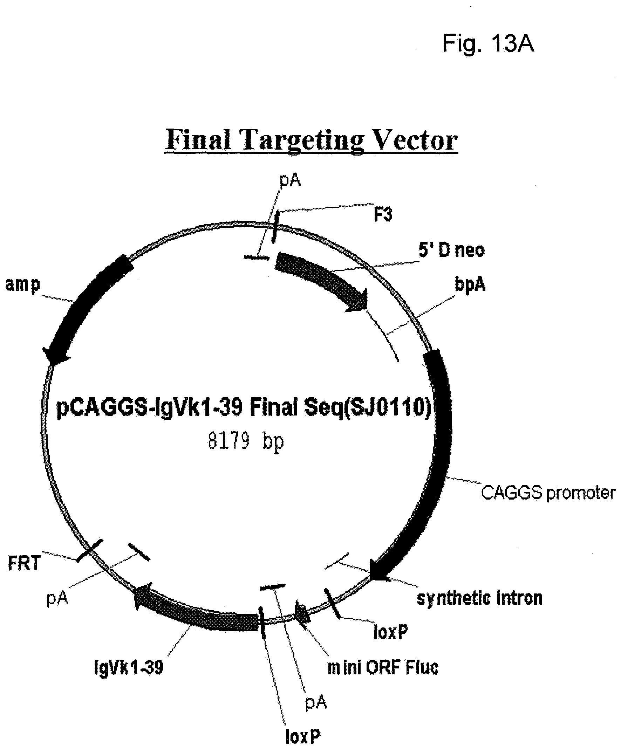

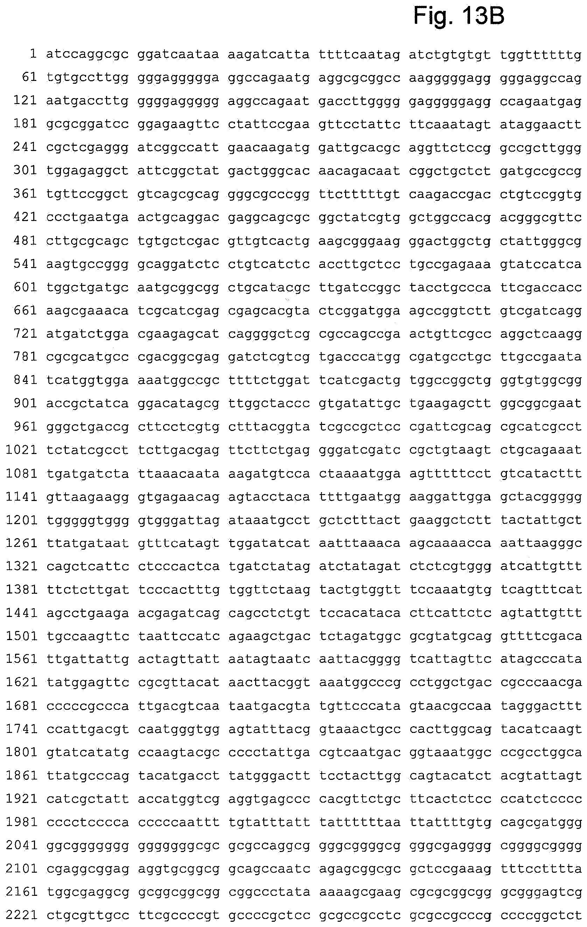

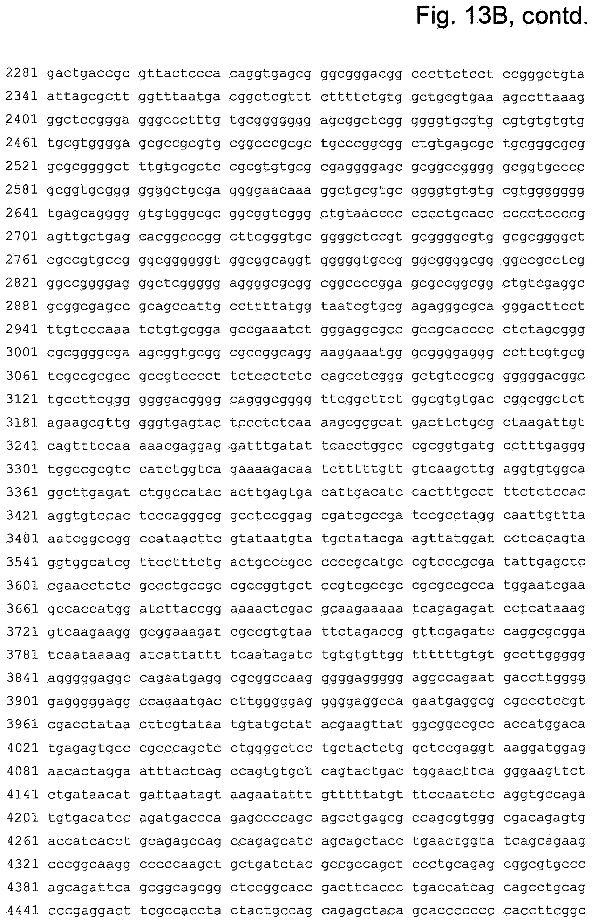

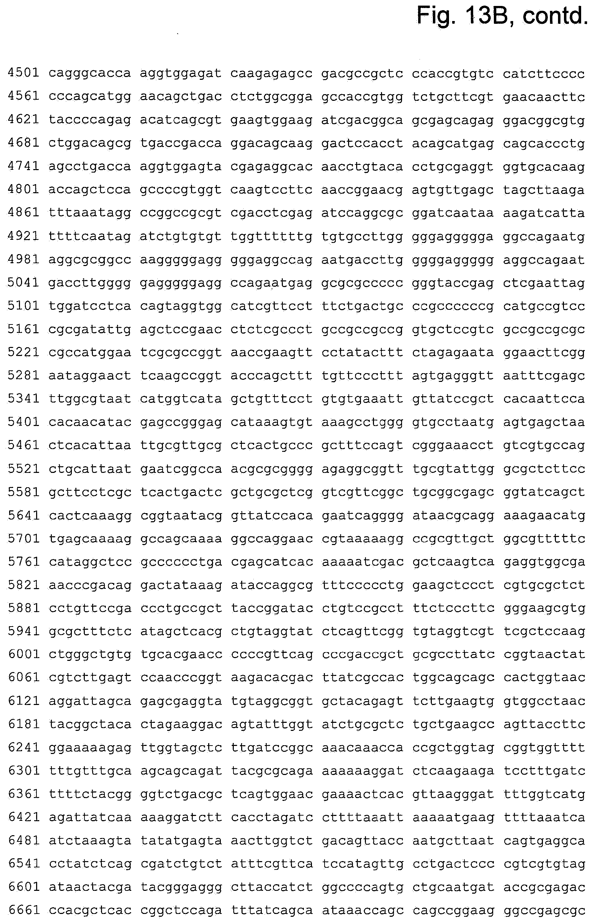

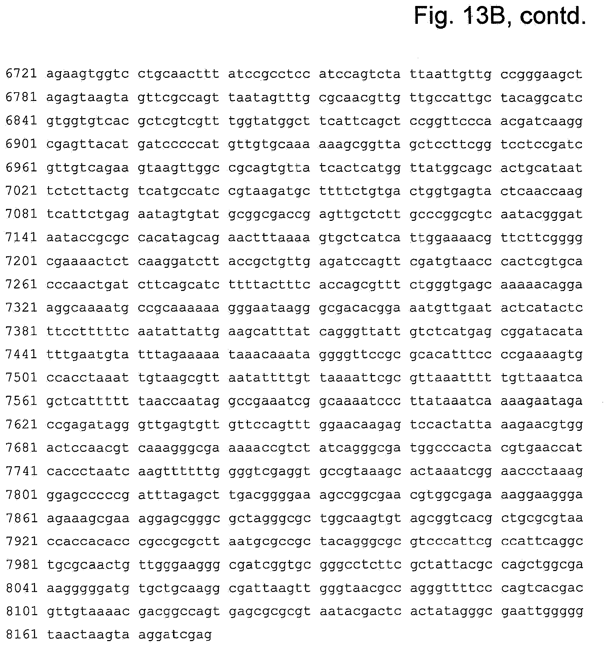

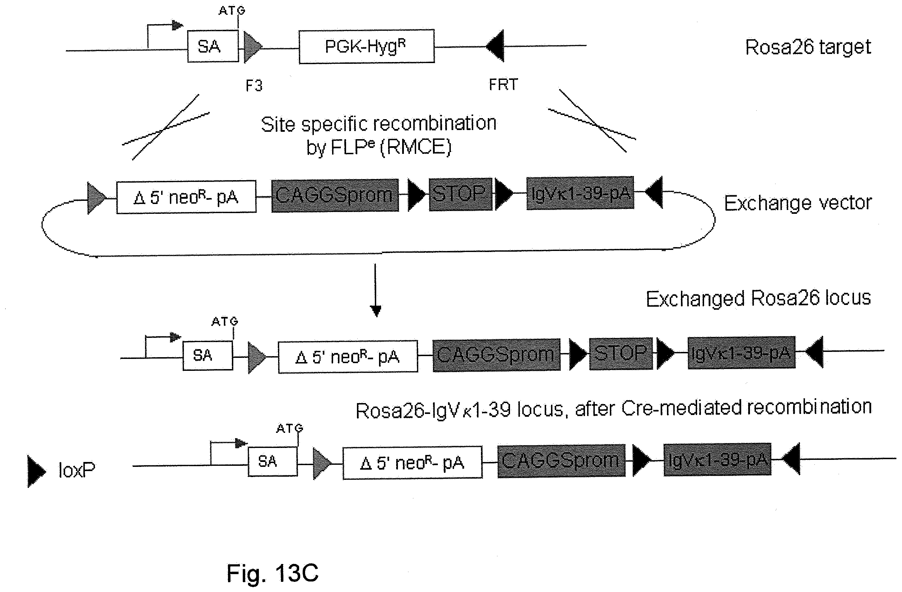

At the 5' end of the expression cassette, a NotI site was introduced and on the 3' site a NheI site. Both sites are used for cloning in the CAGGS expression module. After gene assembly according to methods used by GeneArt, the insert is digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. Promoter and/or cDNA fragments were amplified by PCR, confirmed by sequencing and/or cloned directly from delivered plasmids into an RMCE exchange vector harbouring the indicated features. A schematic drawing and the confirmed sequence of the final targeting vector pCAGGS-IgVK1-39 are shown in figure 13A and 13B. The targeting strategy is depicted in figure 13C.

### Example 6

### CAGGS expression insert based on the rearranged germline IGLV2-14/J V lambda region (IGLV2-14/J-Ck)

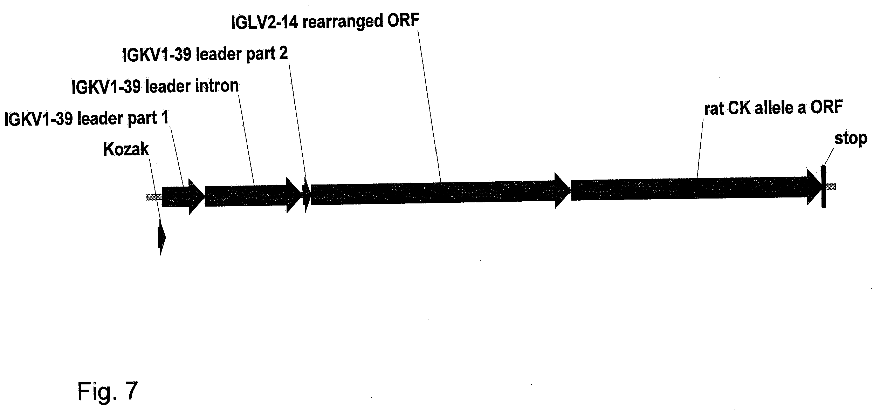

This example describes the sequence and insertion of an expression cassette incorporating the rearranged germline IGLV2-14/J V lambda region. This insert encompasses cloning sites, a Kozak sequence, a leader sequence containing an intron, an open reading frame of the rearranged IGLV2-14/J region, a rat CK constant region from allele a and a translational stop sequence (IGLV2-14/J-Ck; figure 7). The primary construct consists of naturally-occurring sequences and has been analyzed and optimized by removing undesired cis acting elements like: internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions was optimized for expression in mice. The intron sequence is unchanged and thus represents the sequence identical to the human IGKV1-39 leader intron.

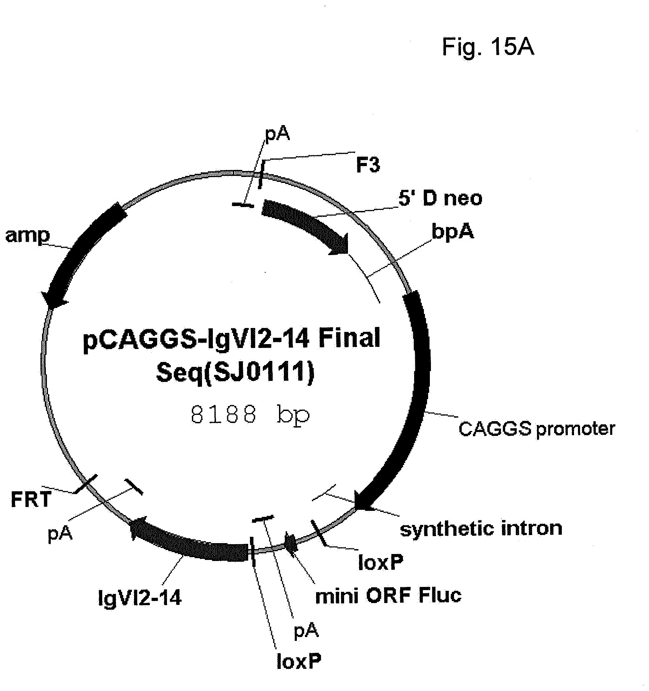

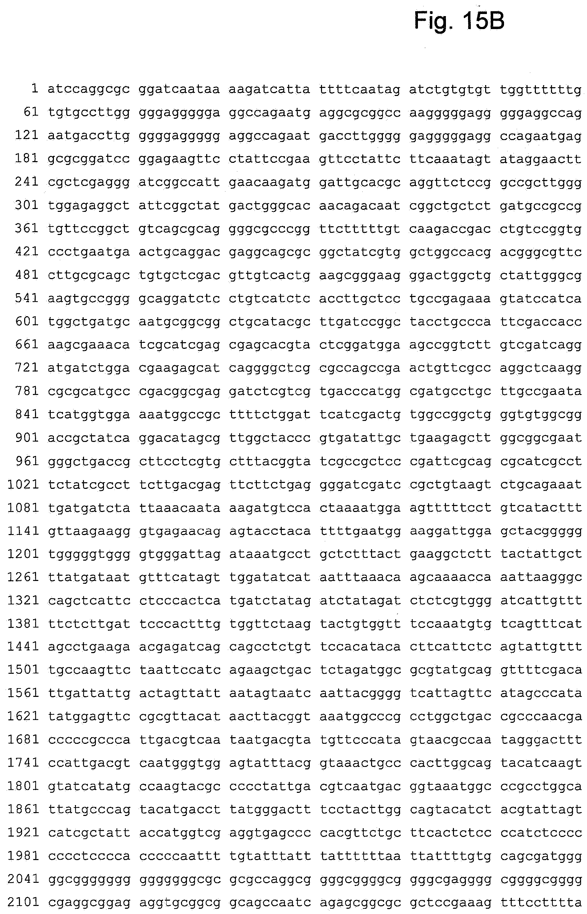

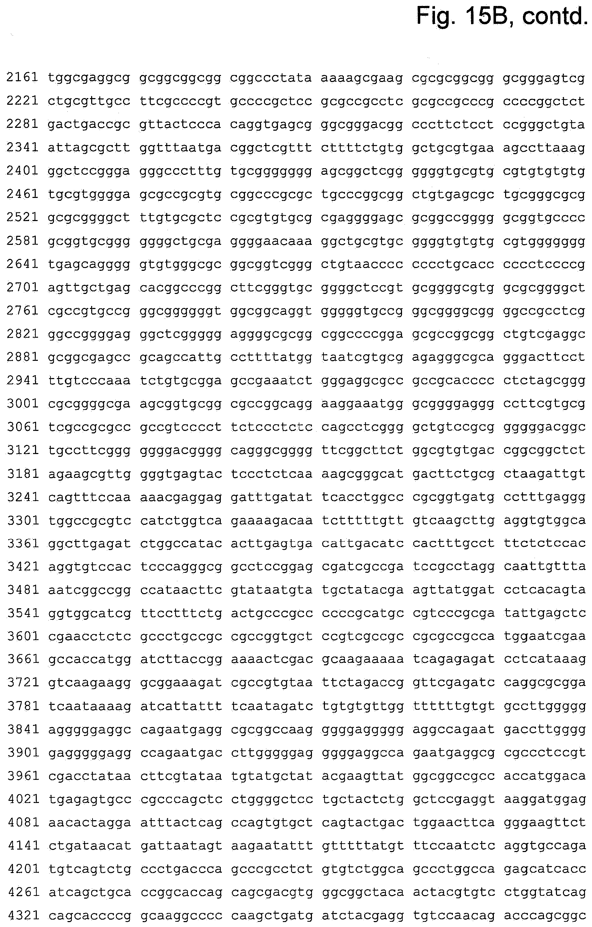

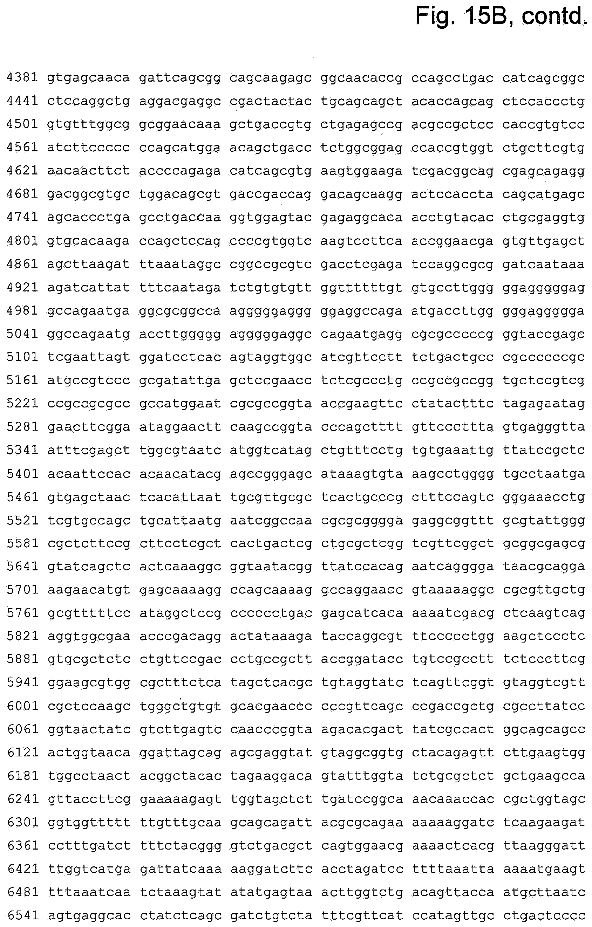

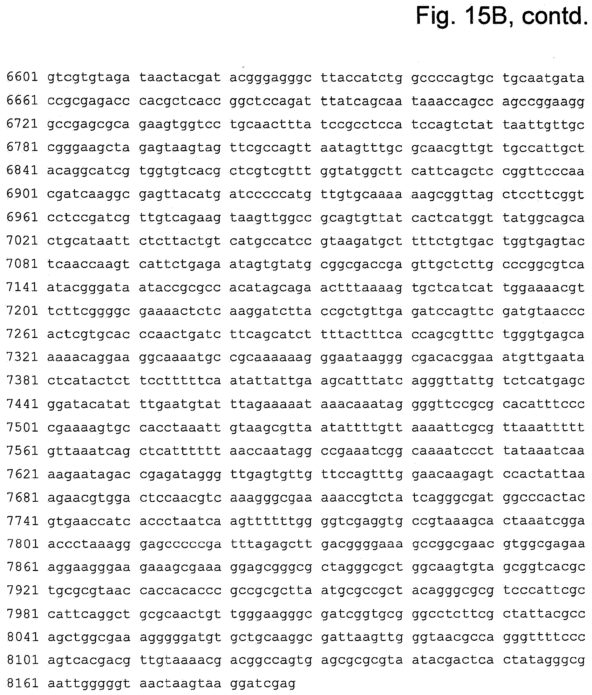

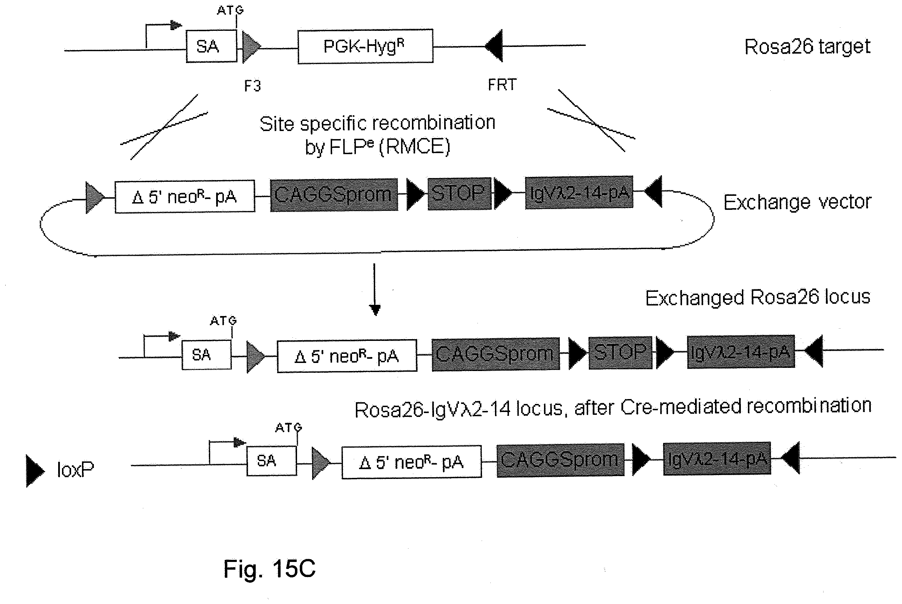

At the 5' end of the expression cassette, a NotI site was introduced and on the 3' site a NheI site. Both sites are used for cloning in the CAGGS expression module as described by TaconicArtemis. After gene assembly according to methods used by GeneArt, the insert was digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. To construct the final ROSA26 RMCE targeting vector, promoter and/or cDNA fragments were amplified by PCR. Amplified products were confirmed by sequencing and/or cloned directly from delivered plasmids into an RMCE exchange vector harbouring the indicated features. A schematic drawing and the confirmed sequence of the final targeting vector pCAGGS-IgVL2-14 is shown in figure 15A and 15B. The targeting strategy is depicted in figure 15C.

### Example 7

### Expression of IGKV1-39/J-Ck in HEK293 cell lines (pSELECT-IGKV1-39/J-Ck)

This example describes a method to verify that the IGKV1-39/J-Ck constructs described in example 5 enable expression and detection of the IGKV1-39/J-Ck L chain in HEK293 cells. The IGKV1-39/J insert (figure 6) was modified at the 5' end by changing the NotI site into a SalI site. This change is required for cloning of the product into the expression cassette plasmid pSELECT-hygro (InvivoGen). The CAGGS expression insert IGKV1-39/J-Ck and pSELECT-hygro were digested with SalI and NheI, ligated and used to transform competent XL1-Blue cells using standard techniques. Colonies were picked and DNA purified using Qiagen Midi-prep columns according to the manufacturer's procedures. The resulting light chain (LC) expressing vector named 0817676_pSELECT_0815426 was used to transfect HEK293 cells with Fugene6 (Roche) according to the manufacturer's protocols. Supernatants were screened for the presence of IGKV1-39/J-Ck light chains by ELISA and western blot using anti-rat-Ck antibodies (Beckton Dickinson #550336 and 553871) and protocols used in the art.

The VH of anti-tetanus toxoid (TT) IgG MG1494 was cloned into IgG expression vector MV1056 using restriction sites SfiI and BstEII. The resulting clone was sequence verified. HEK293T cells were transfected with five different vector combinations as shown in Table 4 (see example 8 for details of vector 0817678_pSELECT_0815427). Supernatants were harvested and IgG concentrations determined (see Table 4). No IgG could be detected for supernatants A and B containing light chain only as expected (detection antibody recognized Fc part of IgG). IgG concentration in supernatants C and D was comparable to that of positive control supernatant E, indicating correct expression of the light chain constructs.

Binding to TT was analyzed by ELISA to check functionality of the produced antibodies, using hemoglobin as negative control antigen. No TT-specific binding could be detected for supernatants A and B containing light chain only, as expected. TT-specific binding for supernatants C and D was at least as good as for positive control supernatant E, confirming correct expression of the light chain constructs and functional assembly with heavy chain. Antibodies were detected not only using an anti-human IgG secondary antibody, but also an anti-rat Ckappa light chain secondary antibody. The results confirm that the anti-rat Ckappa antibody (BD Pharmingen #553871, clone MRK-1) recognizes the light chain expressed by the pSELECT vectors.

Supernatants were analyzed by non-reducing SDS-PAGE and Western blot (not shown). Detection using an anti-human IgG heavy chain antibody did not show bands for supernatants A and B containing light chain only, as expected. Results for supernatants C and D were comparable to positive control supernatant E, with a band close to the 170 kD marker as expected for intact IgG. Additional lower molecular weight bands were observed as well for supernatants C, D and E, which might represent degradation products, IgG fragments resulting from (partial) reduction and/or irrelevant protein bands due to non-specific binding of the detection antibody.

Detection using an anti-rat Ckappa light chain antibody showed a band close to the 26 kD marker for supernatants A and B, as expected for light chain only. This band was much more intense for A compared to B, indicating that the free IGKV1-39 light chain may be better expressed and/or more stable than the free IGLV2-14 light chain. No bands were detected for control supernatant E as expected, since the expressed IgG contains a human Ckappa light chain. For supernatants C and D, expected bands close to the 170 kD marker were observed; lower molecular weight bands were also observed, but to a lesser extent than above using the anti-human IgG antibody.

In conclusion, transfection of the light chain expression constructs combined with the heavy chain of anti-tetanus toxoid (TT) IgG MG1494 resulted in IgG production comparable to the positive control construct for both the pSELECT kappa and lambda light chain constructs. Both IgG productions yielded ELISA signals in a TT ELISA that were better than or comparable to the control IgG. SDS-PAGE and Western blot analysis confirmed the presence of intact IgG. The tested anti-rat Ckappa antibody worked efficiently in both ELISA and Western blot. Culture supernatant from cells transfected with light chain constructs only did not result in detectable IgG production nor in detectable TT-specific binding, while free light chain was detected on Western blot.

### Example 8

### Expression of IGLV2-14/J-Ck in HEK293 cell lines (pSELECT-IGLV2-14/J-Ck)

This example describes a method to verify that the IGLV2-14/J constructs described in example 6 enable expression and detection of the IGLV2-14/J-Ck L chain in HEK293 cells. The IGLV2-14/J-Ck insert (figure 7) was modified at the 5' end by changing the NotI site into a SalI site. This change is required for cloning of the product into the expression cassette plasmid pSELECT-hygro (InvivoGen). The CAGGS expression insert IGLV2-14/J-Ck and pSELECT-hygro were digested with SalI and NheI ligated and used to transform competentXL1-Blue cells using standard techniques. Colonies were picked and DNA purified using Qiagen Midi-prep columns according to the manufacturer's procedures. The resulting light chain (LC) expressing vector named 0817678_pSELECT_0815427 was used to transfect HEK293 cells with Fugene6 (Roche) according to the manufacturer's protocols. Supernatants were screened for the presence of IGLV2-14/J-Ck light chains by ELISA and western blot using anti-rat-Ck antibodies (Becton Dickinson #550336 and 553871) and protocols used in the art. See example 7 for details and results.

### Example 9

### Construction of a VK promoter-driven expression construct containing an IGKV1-39/J insert and multiple enhancer elements derived from the murine CK locus (VkP-IGKV1-39/J-Ck; VkP-O12)

This example describes the construction of an expression cassette that contains relevant elements to enable B-cell and developmental/differentiation stage-specific expression of the rearranged human IGKV1-39 VK region, based on the IGKV1-39 VK promoter region, leader containing an intron, germline V-gene, CDR3, IGKJ segment, mouse intergenic region located between Jk and CK, rat Ck allele a open reading frame, and a mouse intergenic fragment from the 3' end of the mouse CK gene ending just 3' of the 3'CK enhancer.

Optimized open reading frames of the leader, IGKV1-39 rearranged gene, and rat CK allele a gene, as described in example 5, was used for the construction of the expression cassette. The VK promoter region was obtained by gene synthesis procedures (GeneArt, GmbH) and is almost identical to the sequence of the human IGKV1-39 region between -500 bp and the ATG (start site) of the gene. The only deviation from the natural sequence is the introduction of a GCCACCATGG Kozak sequence at the ATG (start) site in order to promote translation. A genomic fragment from a mouse BAC clone (TaconicArtemis) is used as the basis for the introduction of individual elements. This fragment is identical to the sequence of the mouse VK locus starting with the intron donor site located directly 3' of the JK5 region and ending just 3' of the 3' CK enhancer and covers approximately 12.5 kb.

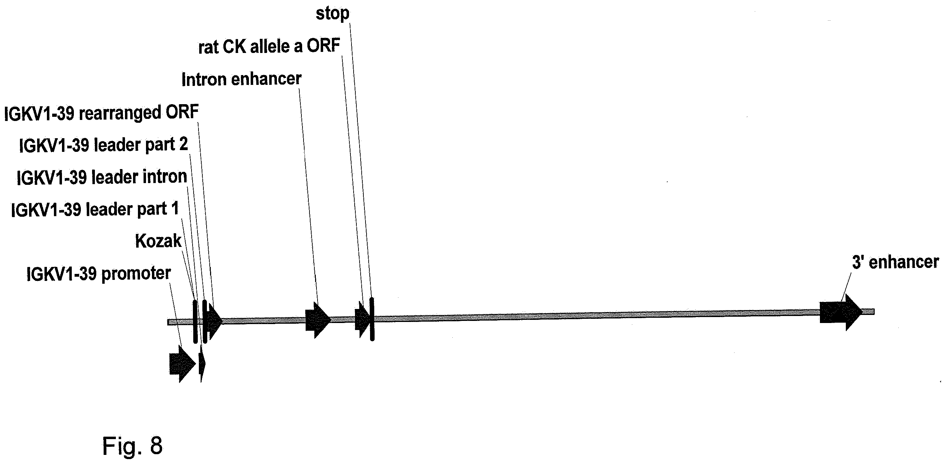

The final construct contains from 5' to 3' end the following elements: human genomic IGKV1-39 promoter (500 bp), a Kozak sequence, a human IGKV1-39 leader part 1 (optimized), a human IGKV1-39 leader intron, a human IGKV1-39 leader part 2 (optimized), a human IGKV1-39 germline gene (optimized), a human J-region (optimized), a mouse intergenic region including the intron enhancer element, a rat (Rattus norvegicus) kappa constant region (optimized), and a mouse intergenic region including the 3' kappa enhancer. The elements of this expression cassette are shown in figure 8 and named VkP-IGKV1-39/J-Ck (VkP-O12). An outline of the pVkP-O12 vector and the targeting strategy is depicted in figure 20A and 21A. The vector was introduced into ES cells following standard procedures (see example 14).

### Example 10

Construction of a VK promoter-driven expression construct containing an IGLV2-14/J clone and multiple CK locus-derived enhancer elements (VkP-IGLVL2-14/J-Ck; VkP-2a2).

This example describes the same construct as described in example 9, except that the IGKV1-39 gene and J-region are replaced by the optimized human IGLV2-14 germline gene including a unique V-J region (VkP-IGLV2-14/J-Ck; VkP-2a2; figure 9).

### Example 11

### Construction of a VK promoter-driven expression construct containing an IGKV1-39 clone lacking the CK intron enhancer element (VkP-IGKV1-39/J-Ck-Δ1: VkP-O12-del1)

The construct described in example 9 was modified by removing the CK intron enhancer element, located in the intergenic region between the human J region and the rat CK region by standard PCR modification and DNA cloning methodologies (GeneArt, GmBH). The resulting expression cassette is shown in figure 10 and named VkP-IGKV1-39/J-Ck-Δ1 (VkP-012-dell).

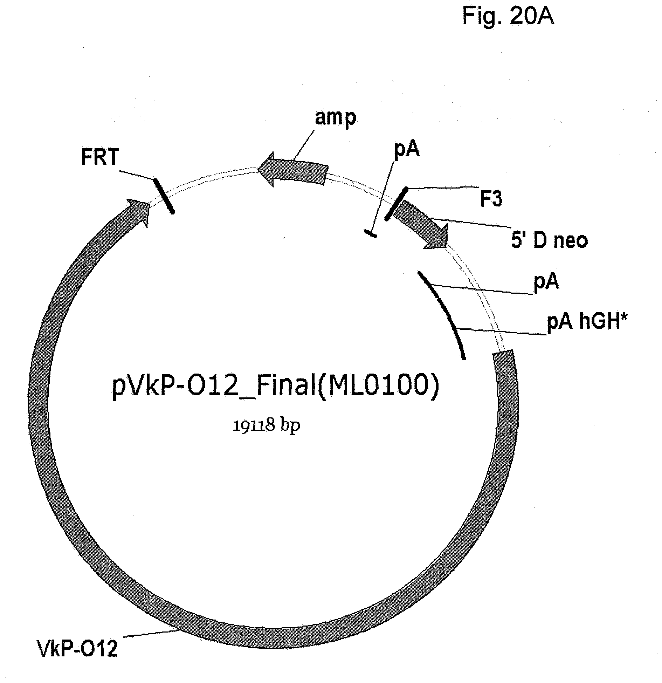

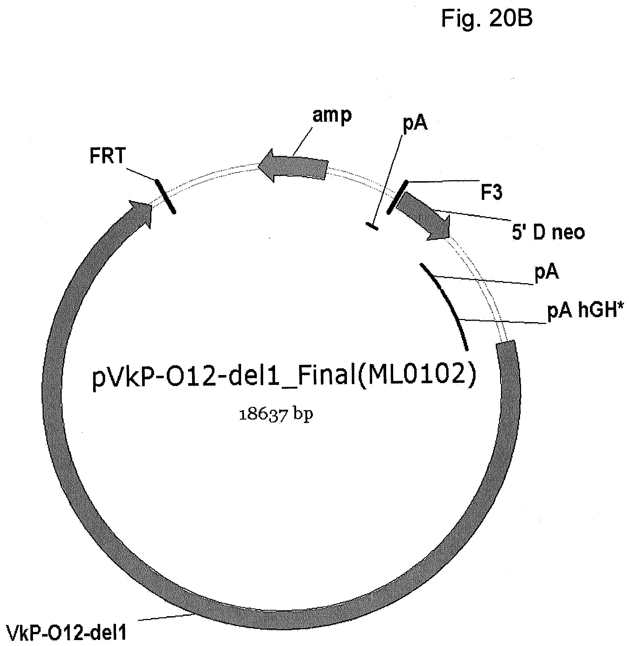

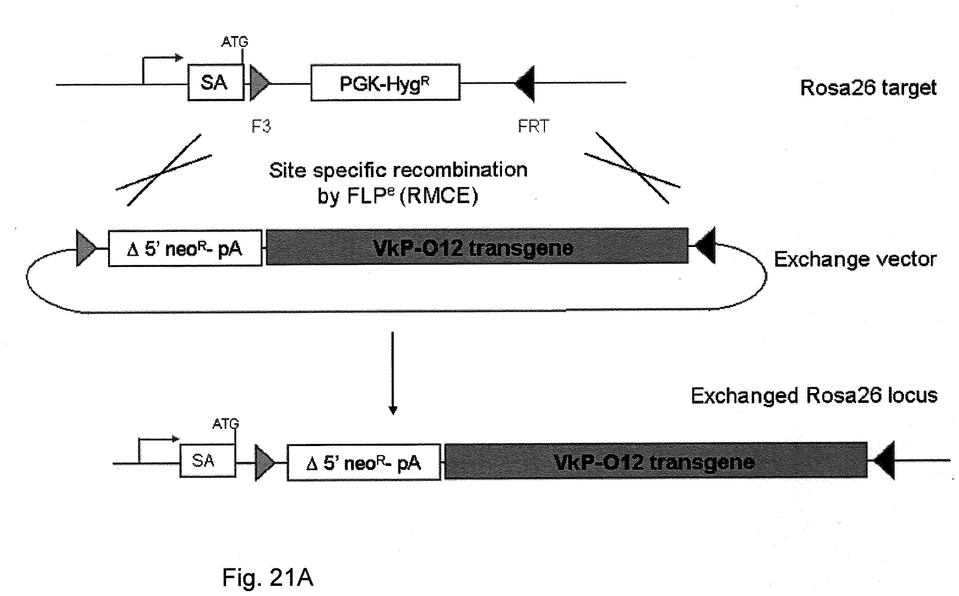

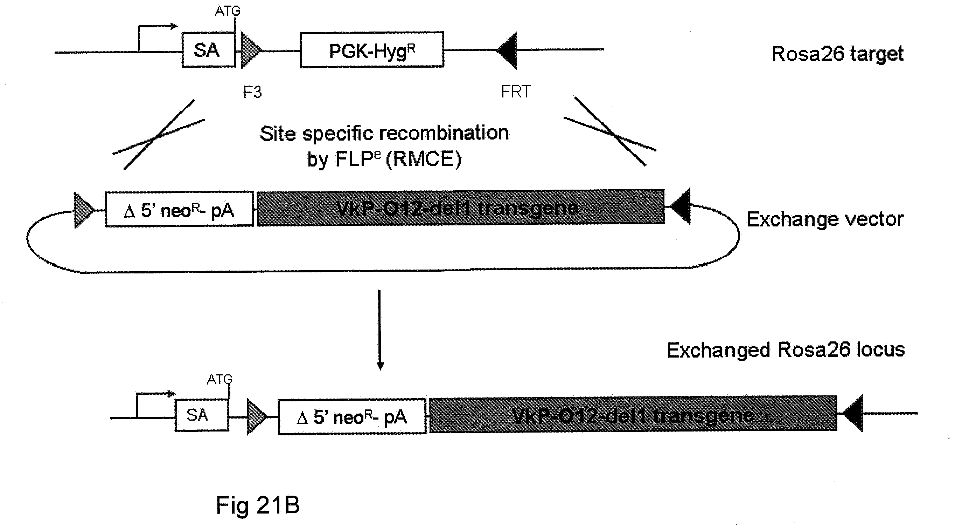

An outline of the pVkP-O12-del1 vector and the targeting strategy is depicted in figure 20B and 21B. The vector was introduced into ES cells following standard procedures (see example 14).

### Example 12

### Construction of a VK promoter-driven expression construct containing an IGKV1-39 clone lacking the CK intron enhancer element and a truncated 3' CK enhancer element (VkP-IGKV1-39/J-Ck-Δ2; VkP-O12-del2)

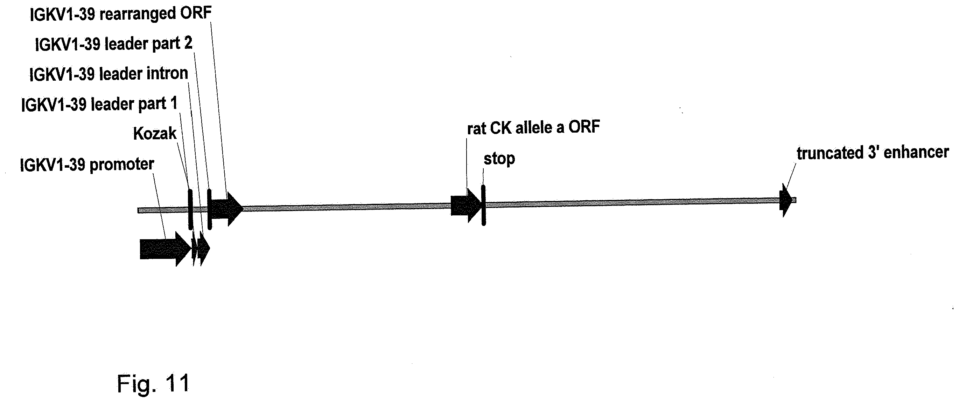

The construct described in example 11 was modified by truncating the 3' CK enhancer element and deleting part of the intergenic region 3' of the rat Ck gene, to remove potential inhibitory elements. This was achieved by removing the intergenic sequence between an EcoRV site (located 3' of the rat Ck gene) and the NcoI site present in the 3' enhancer (5993 bp) and further removing the sequence between the 3' enhancer BstXI site and the BstXI site 3' of the 3'enhancer (474 bp) using standard methods. The resulting expression cassette is shown in figure 11 and named VkP-IGKV1-39/J-Ck-Δ2 (VkP-O12-del2).

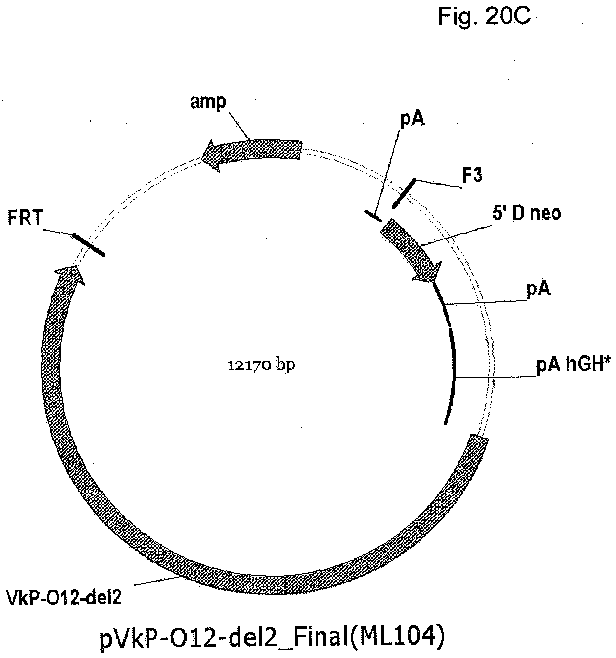

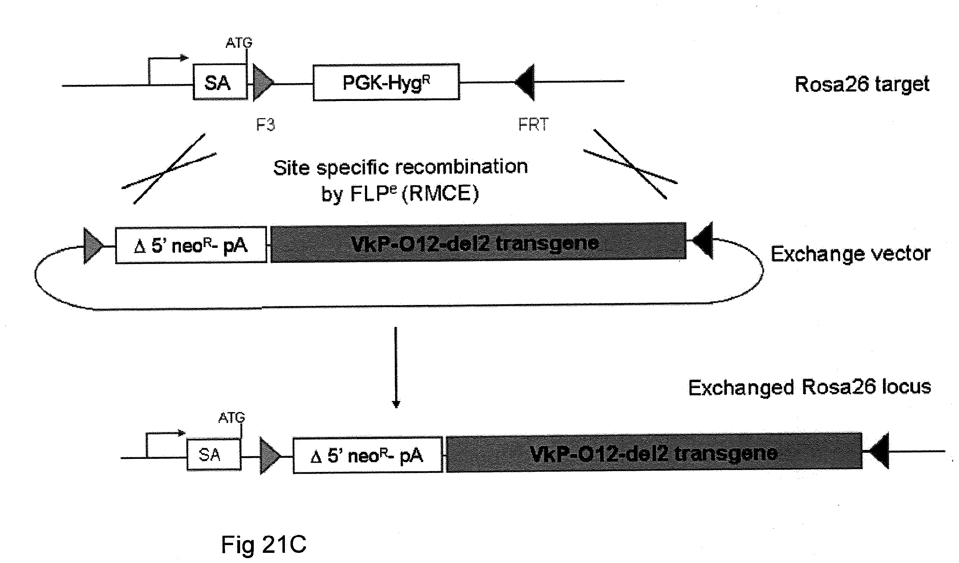

An outline of the pVkP-O12-del2 vector and the targeting strategy is depicted in figure 20C and 21C. The vector was introduced into ES cells following standard procedures (see example 14).

### Example 13

### Expression of Vk constructs in cell lines

The constructs described in example 9-12 are tested for their ability to produce light chain proteins in the myeloma cell lines MPC11 (ATCC CCL167), B-cell lymphoma WEHI231 (ATCC CRL-1702), the T-cell lymphoma EL4 (ATCC TIB-39) and in HEK293 (ATCC CRL1573). The enhancer and promoter elements in the construct enable expression in the B-cell lines but not in cell lines derived form other tissues. After transfection of the cell lines using purified linearized DNA and Fugene6 (Roche) cells are cultured for transient expression. Cells and supernatant are harvested and subjected to SDS-PAGE analysis followed by western blotting using a specific anti-rat-C-kappa antibody. Supernatants are analyzed in ELISA for secreted L chains using the anti-rat CK antibody (Beckton Dickinson #550336).

### Example 14

### Generation of transgenic ES lines

All constructs as described in examples 3, 4, 5, 6, 9, 10, 11 and 12 were used to generate individual stable transgenic ES lines by means of homologous recombination. The methods for generation of transgenic ES lines via homologous recombination are known in the field (e.g. Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kühn R, Schwenk F. Nucleic Acids Res. 2003 Feb 15;31(4):e12; Hogan et al. (Cold Spring Harbor Laboratory Press, Cold Spring Harbor NY.), pp. 253-289.).

For all constructs described in examples 5-6, and examples 9-12, the RMCE ES cell line (derived from mouse strain 129S6B6F1-Gt(ROSA)26Sortm10Arte) was grown on a mitotically inactivated feeder layer comprised of mouse embryonic fibroblasts (MEF) in DMEM High Glucose medium containing 15% FBS (PAN 1302-P220821). Leukemia Inhibitory Factor (Chemicon ESG 1107) was added to the medium at a concentration of 900 U/mL. For manipulation, 2x10⁵ ES-cells were plated on 3,5 cm dishes in 2 ml medium. Directly before transfection, 2 ml fresh medium was added to the cells. 3 ul Fugene6 Reagent (Roche; Catalog No. 1 814 443) was mixed with 100 ul serum free medium (OptiMEM I with Glutamax I; Invitrogen; Catalog No. 51985-035) and incubated for 5 min. 100 ul of the Fugene/OptiMEM solution was added to 2 ug circular vector and 2 ug CAGGS-Flp and incubated for 20 min. This transfection complex was added dropwise to the cells and mixed. Fresh medium was added to the cells the following day. From day 2 onwards the medium was replaced daily with medium containing 250 ug/mL G418 (Geneticin; Invitrogen; Catalog No. 10131-019). Seven days after transfection, single clones were isolated, expanded, and molecular analyzed by Southern blotting according to standard procedures.

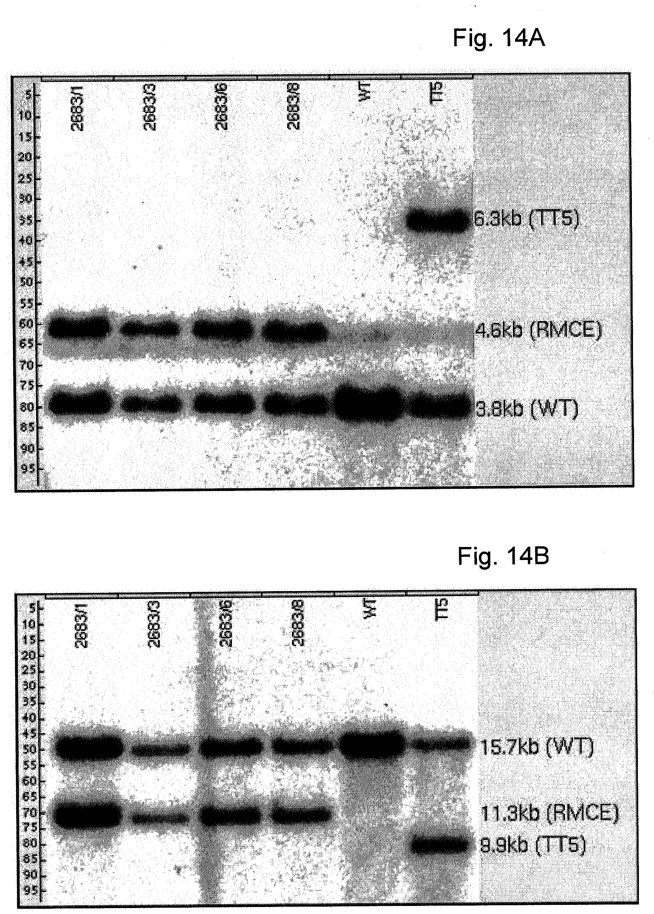

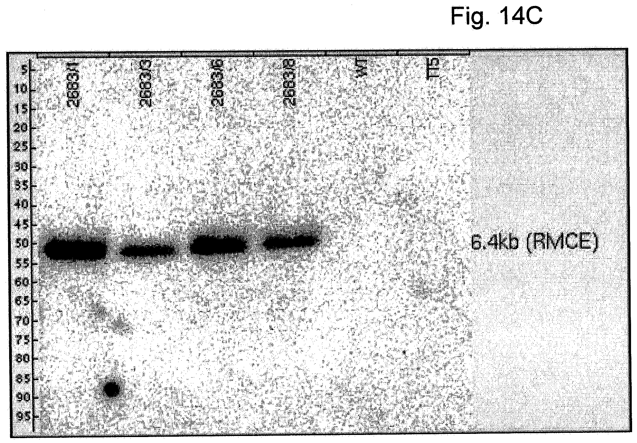

For each construct, analysis of multiple clones by restriction enzyme digestion of genomic DNA of single clones followed by hybridization with 5' probes, 3' probes, and internal probes resulted in clones that comprised a correct, single insertion at the correct position in the Rosa26 locus. An example is provided in figure 14.

### Example 15

### Generation of transgenic mouse strains

All ES cell lines that were generated and verified for their modifications as described in example 14 were used to generate stable transgenic mice by means of tetraploid recombination. The methods are known in the field. In general, after administration of hormones, superovulated Balb/c females were mated with Balb/c males. Blastocysts were isolated from the uterus at dpc 3.5. For microinjection, blastocysts were placed in a drop of DMEM with 15% FCS under mineral oil. A flat tip, piezo actuated microinjection-pipette with an internal diameter of 12 - 15 micrometer was used to inject 10-15 targeted C57BL/6 N.tac ES cells into each blastocyst. After recovery, injected blastocysts were transferred to each uterine horn of 2.5 days post coitum, pseudopregnant NMRI females. Chimerism was measured in chimeras (G0) by coat colour contribution of ES cells to the Balb/c host (black/white). Highly chimeric mice were bred to strain C57BL/6 females. Depending on the project requirements, the C57BL/6 mating partners are non-mutant (W) or mutant for the presence of a recombinase gene (Flp-Deleter or Cre-deleter or CreER inducible deleter or combination of Flp-deleter/CreER). Germline transmission was identified by the presence of black, strain C57BL/6, offspring (G1).

For example, ESC clone IgVK1-39 2683 8 (see examples 5 and 14) was injected in a total of 62 blastocysts in 3 independent experiments. 3 litters were obtained with a total of 6 pups. All pups were chimaeric. 3 heterozygous offspring pups were obtained that were used for further crossing.

ESC Clone Kappa 2692 A-C10 (see examples 3 and 14) was injected in a total of 54 blastocysts in 3 independent experiments. 3 litters were obtained with a total of 11 pups, of which 10 were chimaeric. 8 heterozygous offspring pups were obtained that were used for further crossing.

ESC Clone Kappa 2692 B-C1 (see examples 3 and 14) was injected in a total of 51 blastocysts in 3 independent experiments. 2 litters were obtained with a total of 6 pups, of which 4 were chimaeric. 3 heterozygous offspring pups were obtained that were used for further crossing.

### Example 16

### Breeding

This example describes the breeding for obtaining mice that contain transgenic expression cassettes as described example 14 and knock out mice in which the endogenous lambda and kappa loci have been silenced. The localization of V-lambda on chromosome 16 and CD19 on chromosome 7 allow standard breeding procedures. The breeding of the co-localized Vk locus and Rosa26 locus on chromosome 6 with a distance of about 24 cM requires special attention during the screening as only a percentage of the offspring shows crossover in a way that both modifications are brought together on one chromosome.

All four loci have to be combined in a single mouse strain that is homo- or heterozygous for CD19-cre (not described) and modified Rosa26 transgene and homozygous for the other loci. Breeding is performed by standard breeding and screening techniques as appropriate and offered by commercial breeding companies (e.g. TaconicArtemis).

### Example 17

### Immunizations of mice

Primary and booster immunization of mice are performed using standard protocols.

To validate the transgenic expression of human rearranged Vκ 012 (IGKV1-39) - rat Cκ light chains (see example 5, 14-16) in B cells from CD19-HuVκ1 mice and to assess its impact on VH repertoire size, diversity of VH family usage and V(D)J recombination after immunization, the CD19-HuVκ1 transgenic mice are immunized with tetanus toxin vaccine (TT vaccine) and VH sequence diversity of randomly-picked clones from CD19-HuVκ1 mice are compared with TT-immunized wt mice and CD19-Cre HuVkl negative littermates. Data on the SHM frequency of the human Vκ 012 transgene in the immunized mice are obtained. A diverse collection of at least 40 TT-specific, clonally-unrelated mAbs containing the human Vκ 012 are recovered from CD19-HuVκ1 mice by phage display.

For this, three adult CD19-HuVκ1 mice are vaccinated with TT vaccine using standard immunization procedures. After immunization, serum titers are measured using TT specific ELISA (TT: Statens Serum Institute, Art. no. 2674) and spleen suspensions subjected to cell sorting by the FACS procedure after staining with a rat Cκ-specific monoclonal antibody to isolate transgenic B cells (clone RG7/9.1; BD Pharmingen# 553901, Lot# 06548). RNA from rat Cκ-positive B cells are extracted and the resulting cDNA material used for library building and SHM analysis.

The standard monoclonal mouse anti-rat Cκ antibody (clone RG7/9.1; BD Pharmingen# 553901, Lot# 06548) is used in FACS analysis of transgene expressing B cells (Meyer et al., 1996, Int. Immunol., 8: 1561). The clone RG7/9.1 antibody reacts with a monotypic (common) kappa chain determinant. This anti-rat Cκ antibody (clone RG7/9.1 (BD Pharmingen# 553901, Lot# 06548) is labeled with R-phycoerythrin (PE) using the LYNX rapid conjugation kit according to the manufacturer's instructions for FACS analysis and sorting. The labeled antibody is firstly tested by flow cytometry for binding to rat Cκ-containing functional light chain proteins produced into transiently transfected HEK-293T cells; the un-conjugated antibody serves as a positive control. Two other antibodies shown to bind to rat Cκ by ELISA and Western-blot (see example 7) are tested as well by flow cytometry.

Fab-phage display library building is carried out with a set of optimized degenerate PCR primers designed to amplify C57BL/6 VH genes; the minimal library size is 10⁶ clones, and minimal insert frequency is 80%. The vector used, MV1043 (figures 3 and 12), contains the human Vκ 012 fused to a human Cκ region. The rat Cκ is therefore exchanged for the human counterpart in the library generation process.

Before selection, VH sequencing of 96 randomly picked clones is performed to validate VH repertoire diversity that is compared to diversity obtained from an unselected library previously generated using the same procedures from BALB/c mice immunized with TT. A library from C57Bl/6 wt mice that are immunized in the same way allows diversity comparison between two preselected libraries sharing the same vaccine and the same genetic background.

Several independent selections are performed on TT coated in immunotubes. Variables that may be included are selections using biotinylated antigens in solution or selections on captured TT. Based on the number and diversity of ELISA-positive clones obtained in the first selections, decisions on additional rounds of selection are made. Clones are considered positive when > 3x positive over a negative control clone. Positive clones are analyzed by ELISA against a panel of negative control antigens to verify antigen specificity. The aim is to identify at least 40 unique VH regions, as based on unique CDR3 sequences and V_{H}DJ_{H} rearrangements.

Amplification of the cDNA material from rat Cκ-positive sorted B cells is performed with a PCR forward primer specific to the human leader sequence and a PCR reverse primer specific to the rat Cκ sequence, in a region not redundant with the mouse Cκ sequence, as reported in a recent study (Brady et al., 2006, JIM, 315: 61). Primer combinations and annealing temperatures are firstly tested on cDNA from HEK-293T cells transfected with 0817676_pSELECT_0815426 = pSELECT vector with IGKV1-39 DNA cassette (see example 7).

The amplification products is cloned in pJET-1 vector and after XL1-blue transformation, 96 colonies are sequenced for assessing VL SHM frequency by direct comparison to the Vκ 012 (IGKV1-39) germline sequence. The R/S ratio method, as described in our study on human TT-specific antibodies (de Kruif et al., 2009, J. Mol. Biol., 387: 548) allows discrimination between random mutations and antigen-driven mutations that occurred on VL sequences.

### Example 18

Immunofluorescent analysis of B cell populations in transgenic mouse lines.

This example describes the use of antibodies and flow cytometry to analyze B cell populations in primary (bone marrow) and secondary (spleen, peritoneal) lymphoid organs and blood. Methods and reagents are described in Middendorp et al. (2002) J. Immunol. 168:2695 and Middendorp et al. (2004) J. Immunol. 172:1371. For analysis of early B cell development in bone marrow, cells were surface stained with combinations of antibodies (Becton Dickinson) specific for B220, CD19, CD25, IgM, IgD, mouse Ckappa, mouse Clambda and rat Ckappa to detect pro-B cells, pre-B cells, large pre-B cells, early and late immature B cells and recirculating B cell populations expressing the transgene on their surface. DAPI staining (Invitrogen) was included to exclude dead cells from the analysis and FC block (Becton Dickinson) to inhibit antibody interaction with Fc receptors on myeloid cells. For analysis of surface transgene expression on B cell populations in peripheral lymphoid organs and blood, cells were stained with combinations of antibodies (Becton Dickinson) specific for B220, CD5, CD19, CD21, CD23, IgM, IgD, mouse Ckappa, mouse Clambda and rat Ckappa. DAPI staining was included to exclude dead cells from the analysis and FC block to inhibit antibody interaction with Fc receptors on myeloid cells. In addition, combinations of antibodies (Becton Dickinson) specific for CD3, CD4, CD11b, CD11c and NK1.1 were included to determine if transgene expression occurred in cell types outside of the B cell compartment.

Three mice heterozygous for the human IGKV1-39/rat Ckappa transgene and heterozygous for the CD19-Cre transgene on a C57BL6 background (HuVk1/CD19-Cre) were analysed. As controls for the FACS analysis, three littermate mice wild type for the human IGKV1-39/rat Ckappa transgene and heterozygous for the CD19-Cre transgene on a C57BL6 background (CD19-Cre) and two C57BL6/NTac mice (Wt) were included. All animals were allowed to acclimatize in the animal facility for 1 week before analysis and all mice were male and 6 weeks of age. Lymphocytes were isolated from the femurs, spleens, peritoneal cavity and blood of mice using conventional techniques as previously described (Middendorp et al. (2002) J. Immunol. 168:2695 and Middendorp et al. (2004) J. Immunol. 172:1371). Antibodies were pre-combined as shown in Table 10 and staining was carried out in 96 well plates. Incubation with the PE-conjugated anti-rat C kappa (described above) was carried out before staining with the rat anti-murine antibodies to avoid non-specific binding. After completion of cell staining, labeled cells were analysed on a Becton Dickinson LSR II FACS machine and the acquired data analysed with FlowJo software (v6.4.7).

Transgenic mice were similar in weight, appearance and activity to wild type mice. No gross anatomical alterations were observed during the harvesting of tissues. No difference was observed in the numbers of B cells in the bone marrow (BM) and spleen (Table 11) or in the numbers of B cells, T cells and myeloid cells in peripheral organs between transgenic and wild type mice. In addition, the frequency or proportion of the cells in the different lymphocyte developmental pathways was not altered in transgenic mice when compared to wild type mice. Thus in the double transgenic (HuVk1/CD19-Cre) and transgenic (CD19-Cre) mice lymphoid and most importantly B cell development was indistinguishable from wild type mice.

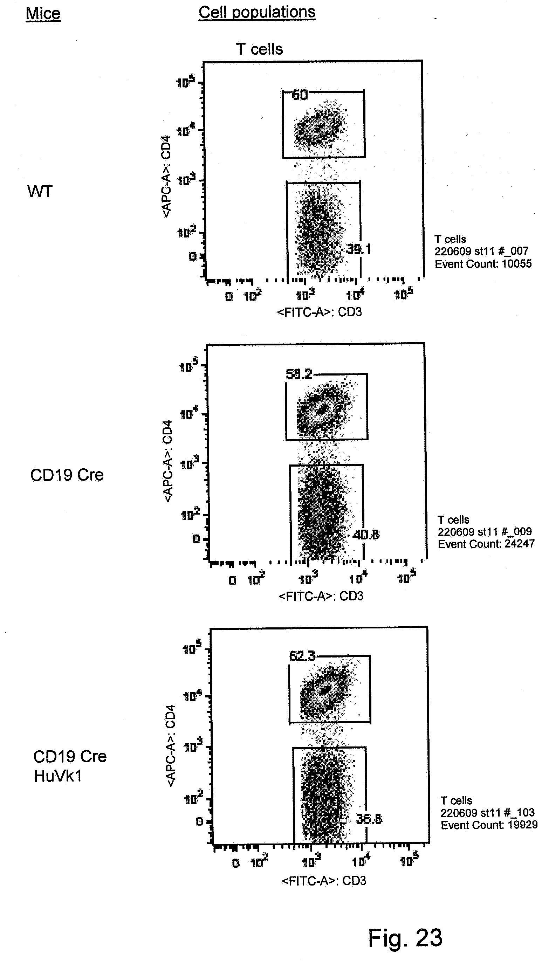

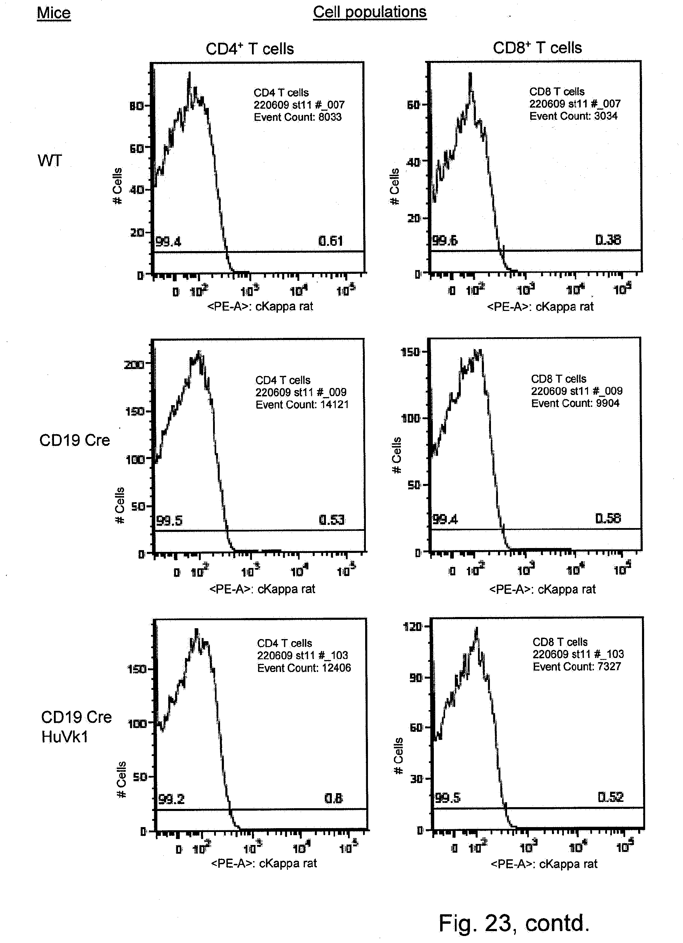

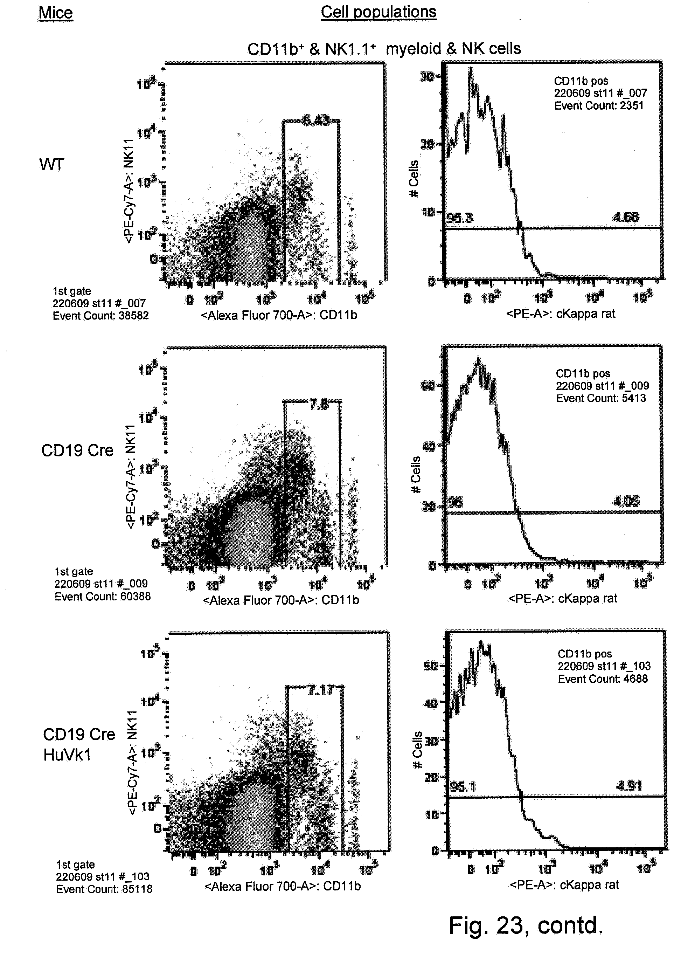

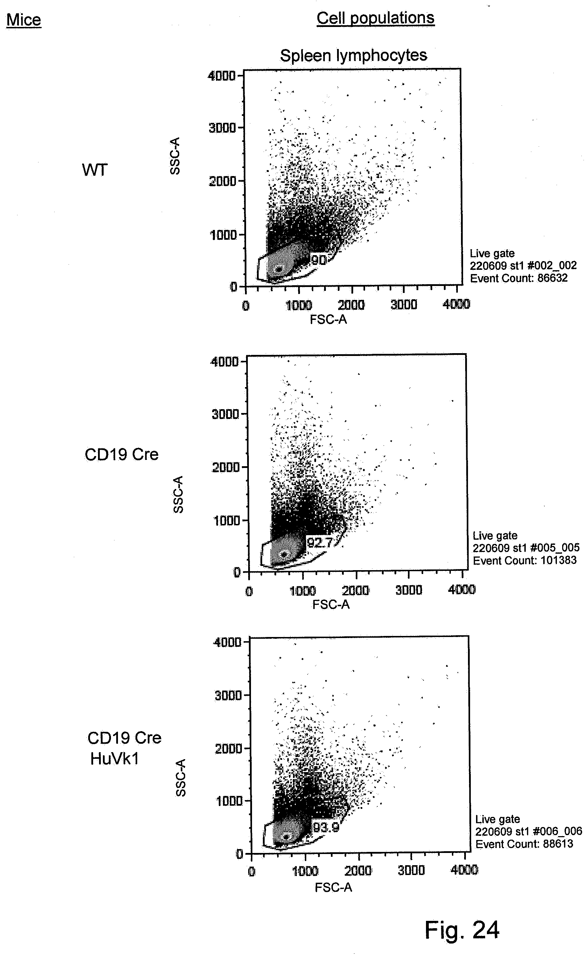

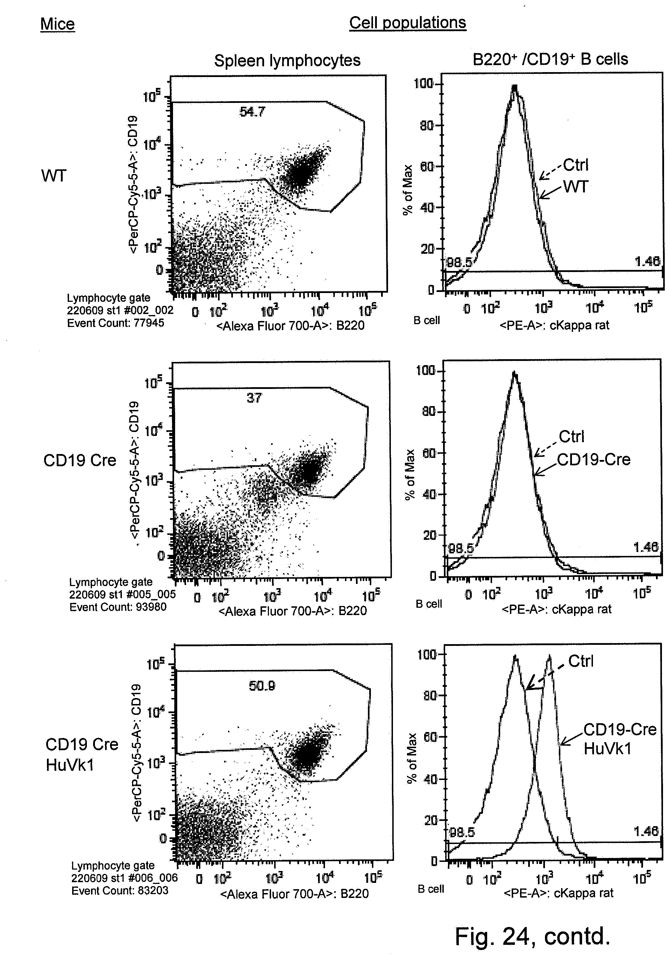

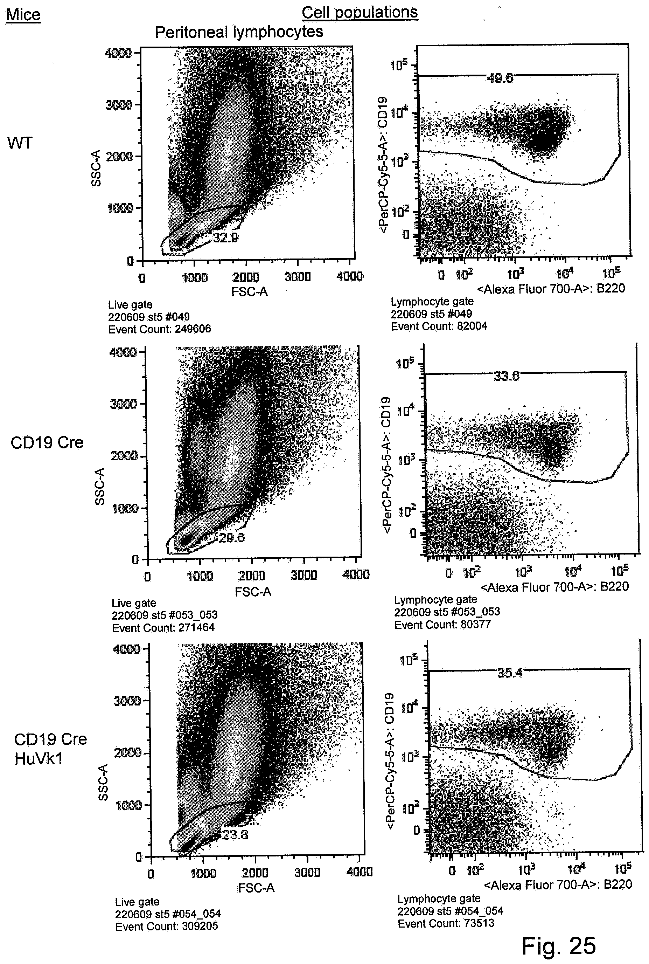

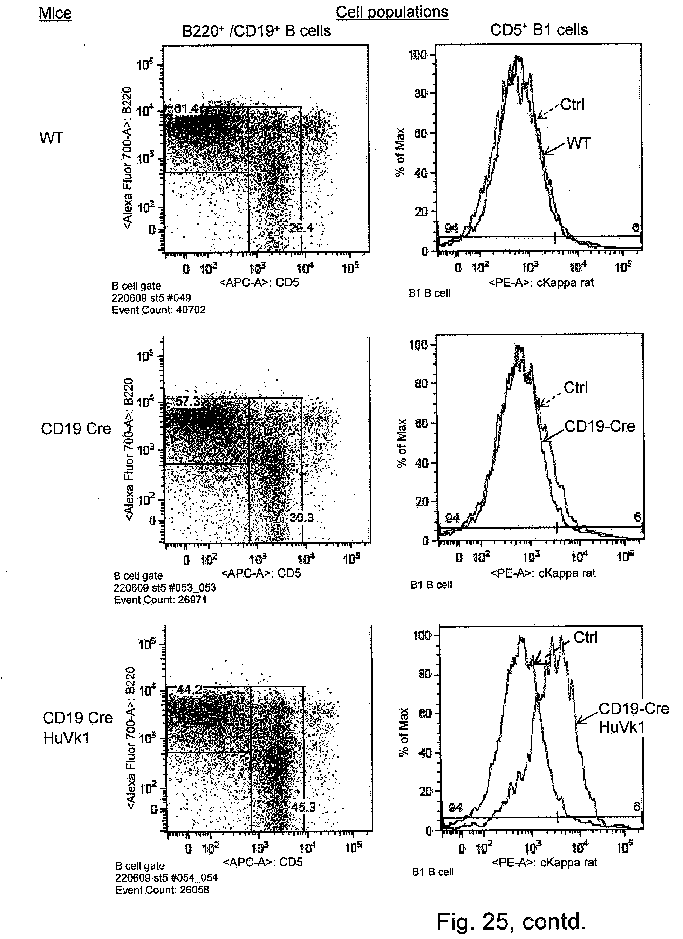

In the peripheral lymphoid organs, staining with the transgene specific antibody (anti-ratCkappa-PE) was only observed in the B cell populations. T cell, myeloid cell and NK cell populations were all negative for surface expression of the transgene in the spleen (Figure 23). In contrast, in cells stained with the pan B cell markers B220 and CD19 all cells were shifted to the right in the FACS plot indicating cell surface expression of the transgene (Figure 24). A similar transgene-specific staining was measured in CD5⁺ B1 cells of the peritoneum, a developmentally distinct population of B cells (Figure 25).

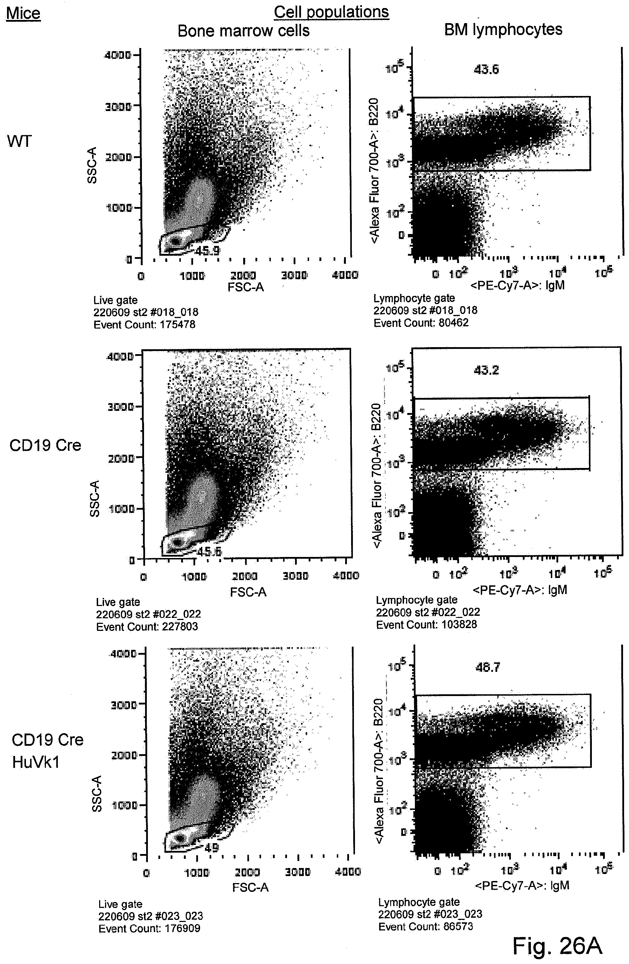

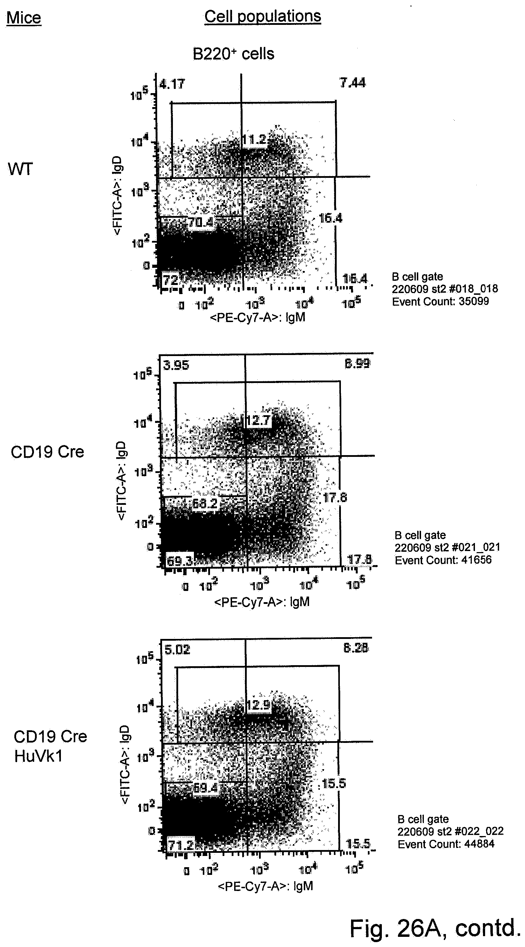

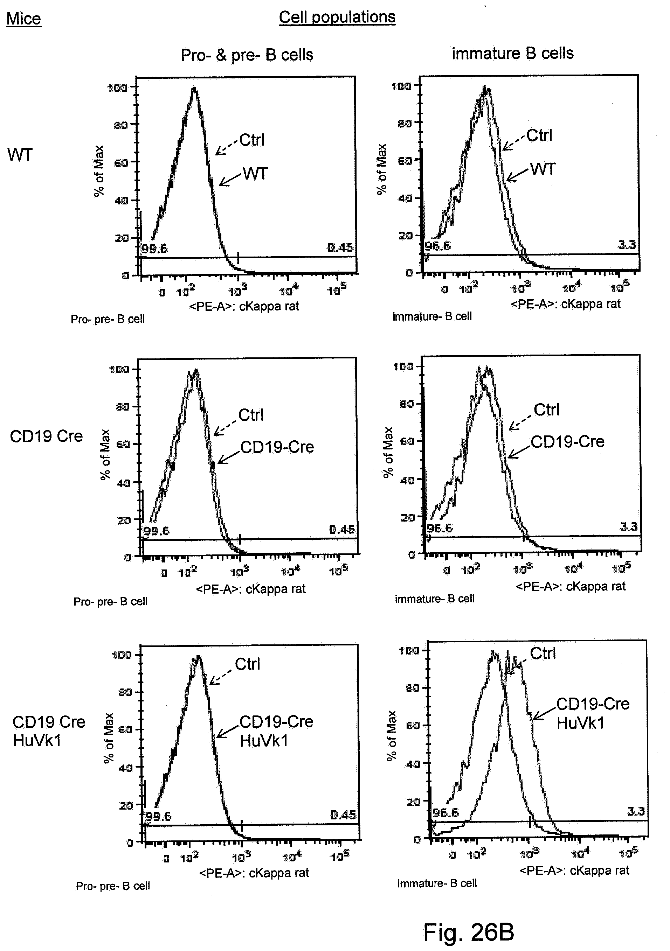

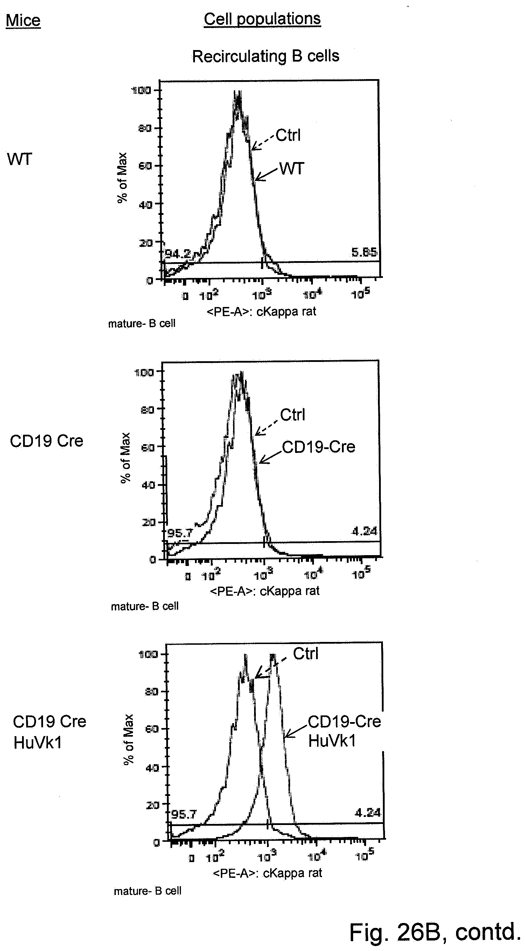

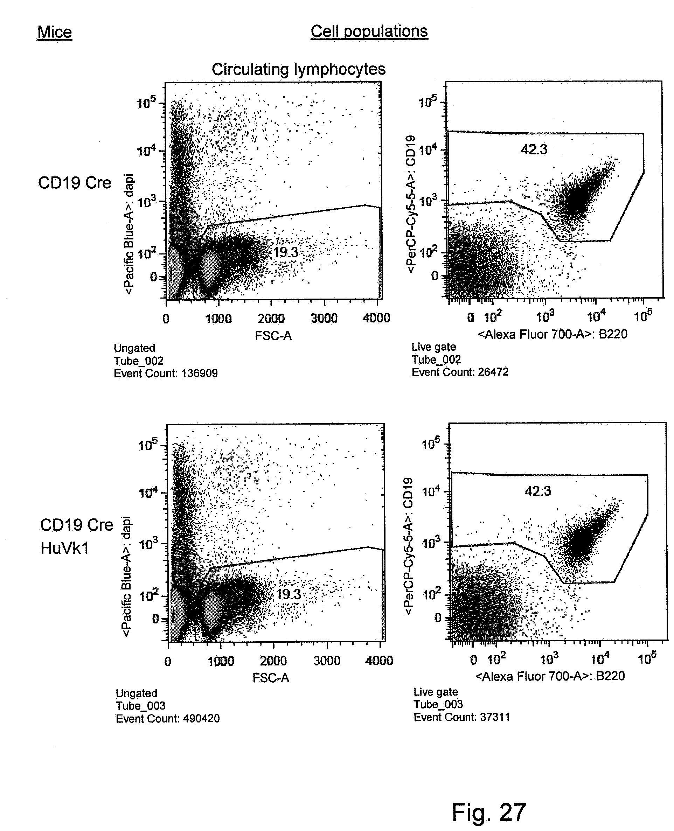

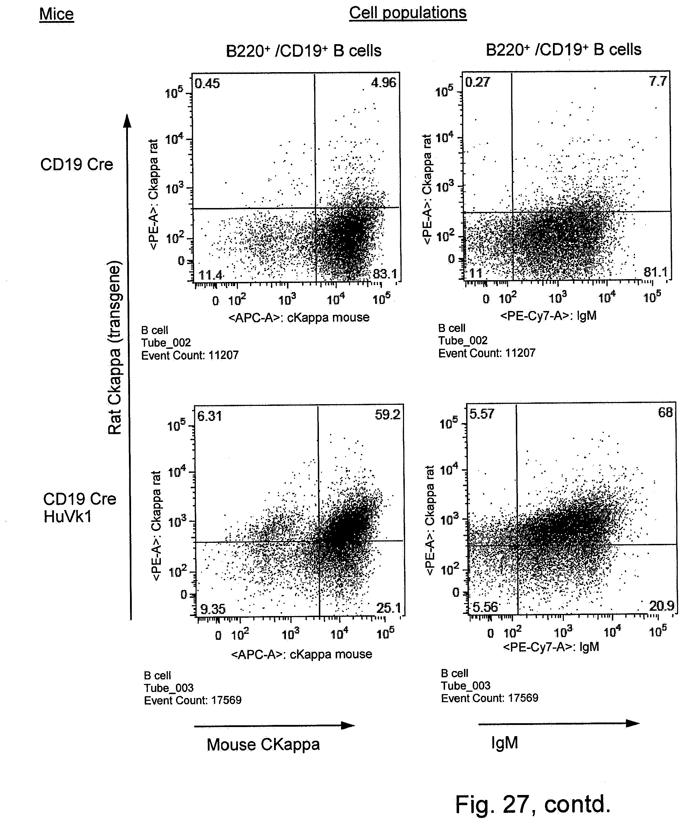

Differentiation of B cells from multilineage precursors to mature B cells occurs in the bone marrow. In the lymphocytes analyzed from the bone marrow, extracellular and transgene expression was not detectable in the earliest B cell progenitors the pro- and pre-B cell consistent with the pattern of normal light chain expression (Figure 26). Transgene expression first becomes detectable in immature B cells, the developmental stage at which the germline murine light chain undergoes rearrangement and is expressed at the cell surface in the context of the preselected heavy chain (Figure 26). Consistent with the staining in the spleen transgenic light chain expression is also detected on mature recirculating B cells (Figure 26). Thus the CD19-Cre driven expression of the transgene is consistent with the normal pattern of light chain expression. The staining with the endogenous light chain- specific antibody is more intense than that of the transgene-specific light chain antibody. This may indicate a higher expression level of the endogenous light chain, a more sensitive staining with the endogenous light chain-specific antibody or a combination of both. Importantly, the intensity of the surface expression of the transgenic light chain is correlated with both endogenous light chain and IgM surface expression as observed in staining of circulating B cells in the blood (Figure 27).

Thus overall this analysis demonstrates that expression of the human IGKV1-39/Ckappa transgene is restricted to the B cell compartment and the temporal regulation of its expression is similar to the endogenous kappa and lambda light chains resulting in normal development of all B cell populations. The apparent lower level of expression of the transgene could be explained by the strength of the promoter in comparison to the promoter and enhancers present on endogenous light chain genes or by a delay in transgene expression that gives the endogenous light chains a competitive advantage in pairing with the rearranged heavy chain. This is consistent with the observation that as B cells mature the relative intensity of transgene staining increases compared to the endogenous light chains. In addition, the observation that B cells numbers are normal and that every surface Ig+ B cell co-expresses an endogenous and transgenic light chain supports the conclusion that the IGKV1-39 variable region is capable of pairing with a normal repertoire of different murine heavy chain variable regions. We conclude from this analysis that insertion of the IGKV1-39/rat Ckappa transgene driven by the CD19-Cre activated CAGGS promoter in the Rosa locus facilitates timely and B cell-specific expression of the transgene and that the transgene is capable of pairing with a normal repertoire of murine heavy chains.

### Example 19

### Epibase® T-cell epitope profile for IGKV1-39.

The protein sequence of IGKV1-39 (figure 12, human germline IGKV1-39/J Protein) was scanned for the presence of putative HLA class II restricted epitopes, also known as T_{H}-epitopes. For this, Algonomics' Epibase® platform was applied to IGKV1-39. In short, the platform analyzes the HLA binding specificities of all possible 10-mer peptides derived from a target sequence (Desmet et al. Nature 1992, 356:539-542; Desmet et al. FASEB J. 1997, 11:164-172; Desmet et al. Proteins 2002, 48:31-43; Desmet et al. Proteins 2005, 58:53-69). Profiling is done at the allotype level for 20 DRB1, 7 DRB3/4/5, 13 DQ and 7 DP, i.e. 47 HLA class II receptors in total (see Table 5). Epibase® calculates a quantitative estimate of the free energy of binding ΔG_{bind} of a peptide for each of the 47 HLA class II receptors. These data were then further processed as follows:
- Free energies were converted into Kd-values through ΔG_{bind} = RT ln(Kd).
- Peptides were classified as strong (S), medium (M), weak and non (N) binders. The following cutoffs were applied:
   S: strong binder: Kd < 0.1 µM.
   M: medium binder: 0.1 µM ≤ Kd < 0.8 µM.
   N: weak and non-binder: 0.8 µM ≤ Kd.
- Peptides corresponding to self-peptides were treated separately. The list of self-peptides was taken from 293 antibody germline sequences. They are referred to as 'germline-filtered' peptides.

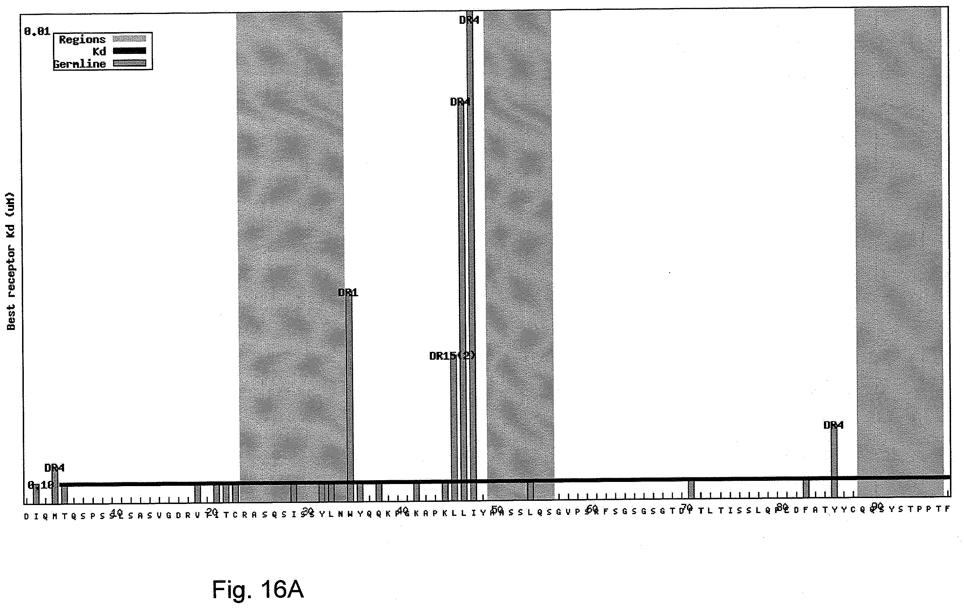

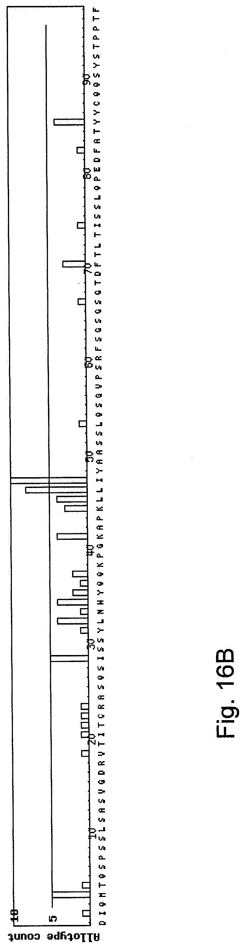

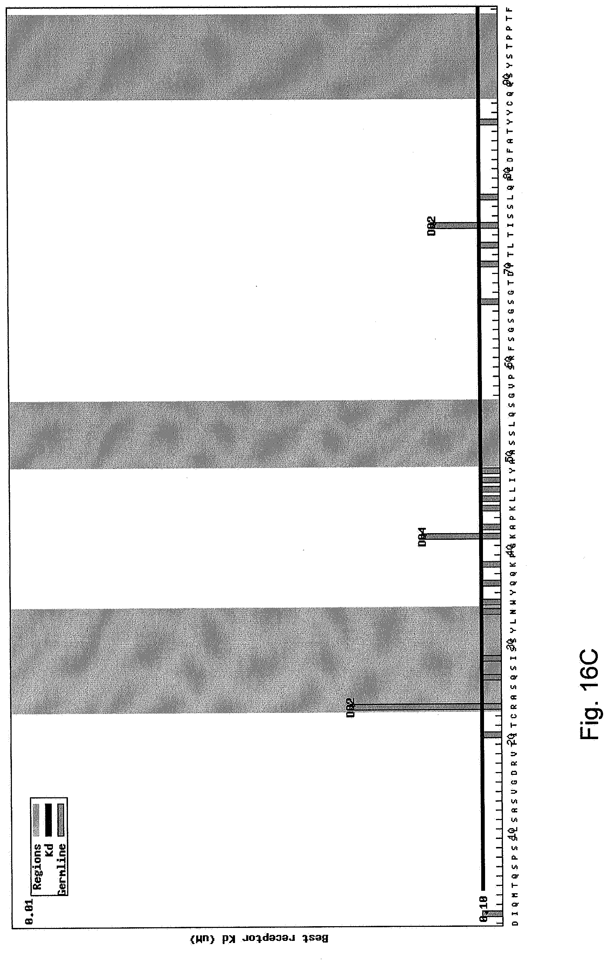

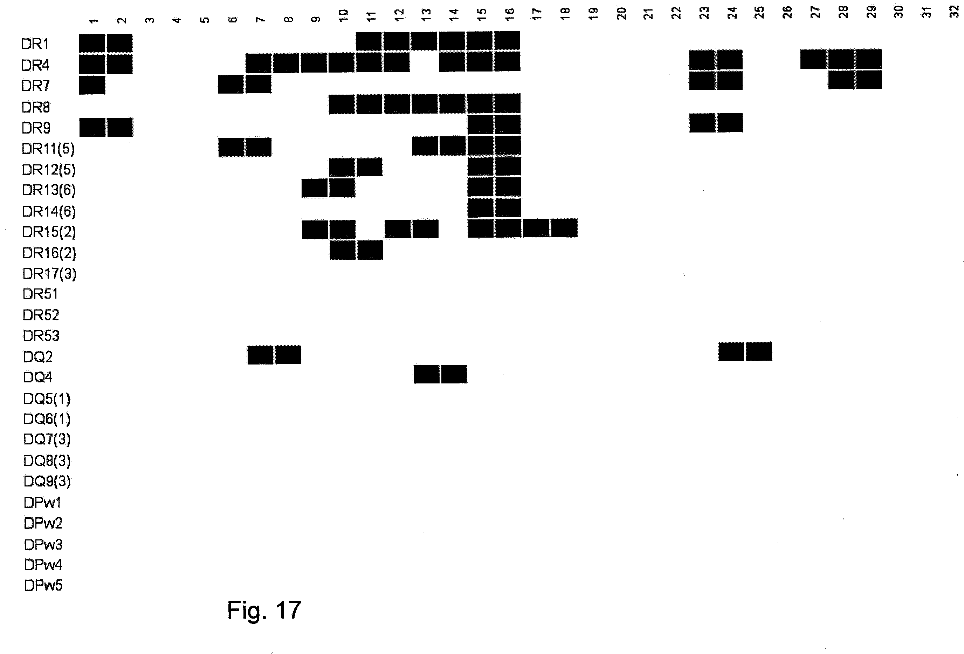

S- and M-peptides are mapped onto the target sequence in so-called epitope maps; S-affinities are plotted quantitatively; M-values are presented qualitatively. As a general overview of the results, Table 6 lists the number of strong and medium binders in the analyzed proteins, for the groups of HLA class II receptors corresponding to the DRB1, DQ, DP and DRB3/4/5 genes. Counting was done separately for strong and medium affinity binders. Peptides binding to multiple allotypes of the same group were counted as one. Values between brackets refer to germline-filtered peptides. In Table 7, the sequence is shown in a format suitable for experimental work. The sequence is broken down in consecutive 15-mers overlapping by 12 residues. For each 15mer, the promiscuity is listed (the number of allotypes out of a total of 47 for which the 15-mer contains a critical binder), as well as the implied serotypes. The Epibase® profile and epitope maps are shown in figure 16 and 17.

It was concluded that IGKV1-39 contains no strong non-self DRB1 binders. Typically, significantly more binders were found for DRB1 than for other HLA genes. This is in agreement with experimental evidence that allotypes belonging to the DRB1 group are more potent peptide binders. Medium strength epitopes for DRB1 allotypes are expected to contribute to the population response, and cannot be disregarded. Again, no non-self DRB1 binders were found in IGKV1-39.

In the humoral response raised against an antigen, the observed T_{H} cell activation/proliferation is generally interpreted in terms of the DRB1 specificity. However, one cannot ignore the possible contribution of the DRB3/4/5, DQ and DP genes. Given the lower expression levels of these genes as compared to DRB1, the focus was on the class of strong epitopes for DRB3/4/5, DQ and DP. 'Critical epitopes' are those epitopes that are strong binders for any DRB1, DRB3/4/5, DQ or DP allotype or are medium binders for DRB1. IGKV1-39 contains no strong or medium non-self binders for DRB3/4/5, DQ, or DP.

A number of peptides are also present in germline sequences (values between brackets in Table 6). Such peptides may very well bind to HLA but they are assumed to be self and, hence, non-immunogenic. In total, 6 strong and 16 medium germline-filtered DRB1 binders were found in IGKV1-39. Framework region 1 up to framework region 3 is an exact match for germline V-segment VKI 2-1-(1) 012 (VBase), a.k.a. IGKV1-39*01 (IMGT). Framework region 4 is an exact match for germline J-segment JK1 (V-base) a.k.a. IGKJ1*01(IMGT). It is hardly surprising that these segments do not contain any non-self epitopes.

### Example 20

### Production characteristics of IGKV1-39

There is a great demand for antibody discovery platforms that yield therapeutic antibodies that are thermodynamically stable and give good expression yields. These characteristics are important in ensuring the stability of the drug substance during production and after injection of the drug product into the patient. In addition good expression yields impact directly on the cost of drug manufacture and thus pricing, patient access and profitability. Virtually all therapeutic antibodies in clinical use today are composed of human IgG1 and kappa constant regions but use different heavy and light chain variable regions that confer specificity. Human variable heavy and light chain domains can be divided into families that have greater than 80% sequence divergence. When rearranged examples of these families in germline configuration are combined and compared for stability and yield it is clear that the gene families are not equal in terms of biophysical properties. In particular V_{H}3, V_{H}1 and V_{H}5 have favourable stability for the heavy chains and Vk1 and Vk3 have the best stability and yield of light chains. In addition when mutations are introduced as part of the somatic hypermutation process they can interfere with V_{H}/V_{L} pairing. To assess the effect that different light chain genes with different rates of mutation have on the production characteristics of a fixed V_{H} chain, a Fab phage display library was built of light chains (kappa and lambda) from six naive healthy donors combined with a panel of 44 TT binding heavy chains from immunized donors. After one round of selection TT binding Fab clones were isolated. Several of these shared the same V_{H} gene as the TT clone PG1433 in combination with different light chains. The Fab light chain fragments were recloned into a kappa expression vector and transfected in combination with DNA encoding the heavy chain of PG1433 into 293 cells and specific IgG production measured by ELISA. As demonstrated in table 8 the selected clones containing PG1433 V_{H} combined with different light chains had between 5 and 10 fold lower protein expression PG1433 V_{H} combined with IGKV1-39. Note that all of the light chains contained amino acid mutations within their coding regions that might disrupt V_{H} paring and reduce production stability. Thus, in addition to reducing the chances of unwanted immunogenicity, it is expected that the use of the light chain IGKV1-39 without mutations contributes to improved production stability and yields of various specificity-contributing V_{H} genes. Indeed stable clones generated by the transfection of different V_{H} genes all paired with IGKV1-39 are able to be passaged extensively and still retain robust production characteristics as shown in table 9.

### Example 21

Generation of mice expressing fully human VH and VL regions.

Transgenic mice according to the invention are crossed with mice that already contain a human VH locus. Examples of appropriate mice comprising a human VH locus are disclosed in Taylor et al. (1992). Nucleic Acids Res 20: 6287-95; Lonberg et al. (1994). Nature 368: 856-9; Green et al. (1994). Nat Genet 7: 13-21; Dechiara et al. (2009). Methods Mol Biol 530: 311-24.).

After crossing and selecting for mice that are at least heterozygous for the IGKV1-39 transgene and the human VH locus, selected mice are immunized with a target. VH genes are harvested as described hereinabove. This method has the advantage that the VH genes are already fully human and thus do not require humanization.

### Example 22

### Isolation, characterization, Oligoclonics formatting and production of antibodies targeting human IL6 for treatment of chronic inflammatory diseases such as rheumatoid arthritis

A spleen VH repertoire from transgenic mice that are immunized with human recombinant IL6 is cloned in a phage display Fab vector with a single human IGKV1-39-C kappa light chain (identical to the mouse transgene) and subjected to panning against the immunogen human IL6. Clones that are obtained after two to four rounds of panning are analyzed for their binding specificity. VH genes encoding IL6-specific Fab fragments are subjected to sequence analysis to identify unique clones and assign VH, DH and JH utilization. The Fab fragments are reformatted as IgG1 molecules and transiently expressed. Unique clones are then grouped based on non-competition in binding assays and subjected to affinity and functional analysis. The most potent anti-IL6 IgG1 mAbs are subsequently expressed as combinations of two, three, four or five heavy chains comprising different VH-regions in the Oligoclonics format, together with one IGKV1-39-C-based kappa light chain and tested in vitro for complex formation with IL-6. The Oligoclonics are also tested *in vivo* for clearance of human IL-6 from mice. An Oligoclonic with the most potent clearance activity is chosen and the murine VH genes humanized according to conventional methods. The humanized IgG1 are transfected into a mammalian cell line to generate a stable clone. An optimal subclone is selected for the generation of a master cell bank and the generation of clinical trial material.

Many of the protocols described here are standard protocols for the construction of phage display libraries and the panning of phages for binding to an antigen of interest and are described, for example, in Antibody Phage Display: Methods and Protocols. 2002. Editor(s): Philippa M. O'Brien, Robert Aitken. Humana Press, Totowa, New Jersey, USA.

### Immunizations

Transgenic mice receive three immunizations with human IL6 every two weeks using the adjuvant Sigma titerMax according to manufacturer's instructions.

### RNA isolation and cDNA synthesis

Three days after the last immunization, spleens and lymphnodes from the mice are removed and passed through a 70 micron filter into a tube containing PBS pH 7.4 to generate a single cell suspension. After washing and pelleting of lymphocytes, cells are suspended in TRIzol LS Reagent (Invitrogen) for the isolation of total RNA according to the manufacturer's protocol and subjected to reverse transcription reaction using 1 microgram of RNA, Superscript III RT in combination with dT20 according to manufacturer's procedures (Invitrogen).

The generation of Fab phage display libraries is carried out as described in Example 2.

### Selection of phages on coated immunotubes

Human recombinant IL6 is dissolved in PBS in a concentration of 5 µg/ml and coated to MaxiSorp Nunc-Immuno Tube (Nunc 444474) overnight at 4°C. After discarding the coating solution, the tubes are blocked with 2% skim milk (ELK) in PBS (blocking buffer) for 1 hour at Room Temperature (RT). In parallel, 0.5 ml of the phage library is mixed with 1 ml blocking buffer and incubated for 20 minutes at room temperature. After blocking the phages, the phage solution is added to the IL6 coated tubes and incubated for 2 hours at RT on a slowly rotating platform to allow binding. Next, the tubes are washed 10 times with PBS/0.05% Tween-20 followed by phage elution by incubating with 1 ml 50 mM glycine-HCl pH 2.2 10 min at RT on rotating wheel and directly followed by neutralization of the harvested eluent with 0.5 ml 1 M Tris-HCl pH 7.5.

### Harvesting phage clones

A 5 ml XL1-Blue MRF (Stratagene) culture at O.D. 0.4 is added to the harvested phage solution and incubated for 30 minutes at 37°C without shaking to allow infection of the phages. Bacteria are plated on Carbenicillin/Tetracycline 4% glucose 2*TY plates and grown overnight at 37°C.

### Phage production

Phages are grown and processed as described by Kramer et al. 2003 (Kramer et al. 2003. Nucleic Acids Res. 31(11): e59) using VCSM13 as helper phage strain.

### Phage ELISA

ELISA plates are coated with 100 microliter human recombinant IL6 per well at a concentration of 2.5 microgram/ml in PBS overnight at 4°C. Plates coated with 100 microliter thyroglobulin at a concentration of 2 microgram/ml in PBS are used as a negative control. Wells are emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution are added and incubated for 1 hour at RT. Unbound phages are subsequently removed by 5 washing steps with PBS-0.05% Tween-20. Bound phages are detected by incubating the wells with 100 microliter anti-M13-HRP antibody conjugate (diluted 1/5000 in blocking buffer) for 1 hour at room temperature. Free antibody is removed by repeating the washing steps as described above, followed by TMB substrate incubation until color development was visible. The reaction is stopped by adding 100 microliter of 2 M H2SO4 per well and analyzed on an ELISA reader at 450 nm emission wavelength.

### Sequencing

Clones that give signals at least 3 times above the background signal are propagated, used for DNA miniprep procedures (see procedures Qiagen miniPrep manual) and subjected to nucleotide sequence analysis. Sequencing is performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). The sequences of the murine VH regions are analyzed for diversity of DH and JH gene segments.

### Construction and expression of chimeric IgG1

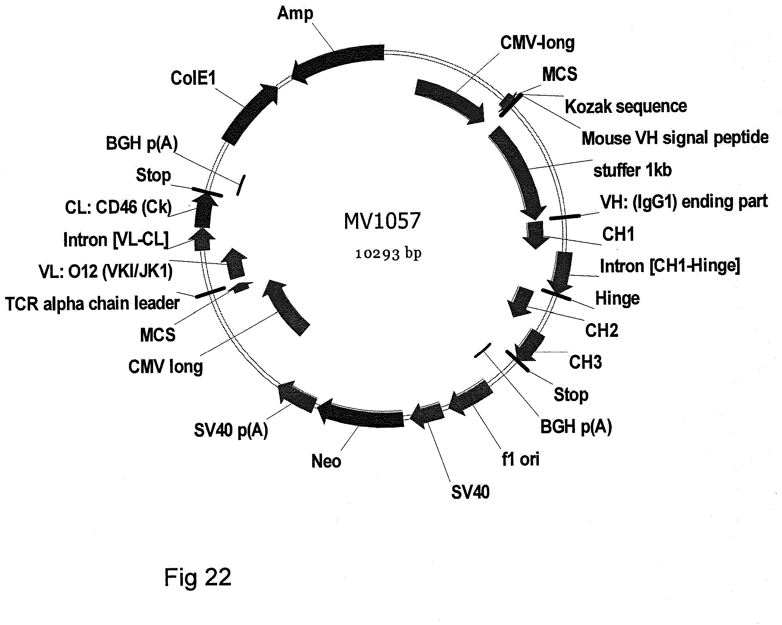

Vector MV1057 (figures 12 and 22) was generated by cloning the transgene (IGKV1-39) L chain fragment into a derivative of vector pcDNA3000Neo (Crucell, Leiden, The Netherlands) that contains the human IgG1- and kappa constant regions. VH regions are cloned into MV1057 and nucleotide sequences for all constructs are verified according to standard techniques. The resulting constructs are transiently expressed in HEK293T cells and supernatants containing chimeric IgG1 are obtained and purified using standard procedures as described before (Throsby, M. 2006. J Virol 80: 6982-92).

### IgG1 binding and competition analysis

IgG1 antibodies are titrated in ELISA using IL6 coated plates as described above and an anti-human IgG peroxidase conjugate. Competition ELISAs to group antibodies based on epitope recognition are performed by incubating Fab phages together with IgG1 or with commercial antibodies against IL6 (e.g. Abcam cat. no. ab9324) in IL6 coated plates, followed by detection of bound Fab phage using an anti-M13 peroxidase conjugate.

### IgG1 affinity measurements

The affinities of the antibodies to IL6 are determined with the Quantitative kinetic protocol on the Octet (ForteBio). Antibodies are captured onto an Anti-Human IgG Fc Capture biosensor and exposed to free IL6 and analyzed using proprietary software to calculate the Kd of each antibody.

### Functional activity of IL6 antibodies

To test the ability of the selected antibodies to inhibit binding between IL6 and IL6 receptor (IL6R), an ELISA based assay is used. Various concentrations of antibody are mixed with a fixed concentration (10 ng/ml) of biotinylated IL6 as described by Naoko et al. 2007, Can. Res. 67: 817-875. The IL6-antibody immune complex is added to immobilized IL6R. The binding of biotinylated IL6 to IL6R is detected with horseradish peroxidase-conjugated streptavidin. The reduction of ELISA signal is a measurement of inhibition. As positive control for inhibition of binding between IL6 and IL6R either anti-IL6R antibody (Abcam cat. no. ab34351; clone B-R6) or anti IL6 antibody (Abcam cat. no. ab9324) is used.

*In vitro* blocking activity of the selected anti-IL6 antibodies is measured in a proliferation assay using the IL6 dependent cell line 7TD1. Briefly, cells are incubated with different concentrations of human IL6 with or without the anti-IL6 antibody. The available amount of IL6 determines the degree of proliferation. Thus if an added antibody blocks IL6 binding the proliferation readout is reduced compared to a non binding antibody control. Proliferation is measured by the incorporation of 5-bromo-2'-deoxyuridine (BrdU) into the DNA using the BrdU proliferation kit (Roche cat. no. 11444611001) according to the manufacturer's instructions.

### Generation of anti-IL6 Oligoclonics

The most potent anti-IL6 antibodies are selected from each epitope group. The expression constructs expressing these antibodies are transfected into HEK293T cells in non-competing groups of three in different ratios (1:1:1; 3:1:1; 1:3:1; 1:1:3; 3:3:1; 1:3:3; 3:1:3; 10:1:1; 1:10:1; 1:1:10; 10:10:1; 1:10:10; 10:1:10; 3:10:1; 10:3:1; 1:10:3; 3:1:10; 10:1:3; 1:3:10). Antibody containing supernatants are harvested and purified and characterized as above.

### Complex formation and in vivo clearance of anti-IL6 Oligoclonics

To measure the ability of anti-IL6 Oligoclonics to form immune complexes and to analyze these complexes Size Exclusion Chromatography (SEC) is used according to the approach disclosed by Min-Soo Kim et al. (2007) JMB 374: 1374-1388 to characterize the immune-complexes formed with different antibodies to TNFα. Different molar ratios of the anti-IL6 Oligoclonics are mixed with human IL6 and incubated for 20 hours at 4°C or 25°C. The mixture is analyzed on an HPLC system fitted with a size exclusion column; different elution times are correlated to molecular weight using a molecular weight standards.

The ability of antibodies to form complexes with IL6 is correlated with their ability to rapidly clear the cytokine from the circulation in vivo. This is confirmed by measuring the clearance of radiolabelled IL6 from mice. Briefly, female, 6- to 8-week-old Balb/c mice are obtained and 18 hrs before the experiment, the animals are injected intravenously (IV) via the lateral tail vein with different doses of purified anti-IL6 Oligoclonics. On day 0 the mice are
injected IV with 50 microliters of radiolabeled IL-6 (1x10E7 cpm/mL) under the same conditions. Blood samples (approximately 50 microliters) are collected at several time intervals and stored at 4°C. The samples are centrifuged for 5 minutes at 4000 xg and the radioactivity of the serum determined. All pharmacokinetic experiments are performed simultaneously with three animals for each treatment.

### Generation of anti-IL6 Oligoclonics stable clones and preclinical development

A lead anti-IL6 Oligoclonic is selected based on the *in vitro* and *in vivo* potency as determined above. The murine VH genes are humanized according to standard methods and combined with the fully human IGKV1-39 light chain in an expression vector as described above. Examples of humanization methods include those based on paradigms such as resurfacing (Padlan, E. A., et al., (1991). Mol. Immunol., 28, 489), superhumanization (Tan, P., D. A., et al., (2002) J. Immunol., 169, 1119) and human string content optimization (Lazar, G. A., et al., (2007). Mol. Immunol., 44, 1986). The three constructs are transfected into PER.C6 cells at the predetermined optimal ratio (described above) under the selective pressure of G418 according to standard methods. A stable high producing anti-IL6 Oligoclonic clone is selected and a working and qualified master cell bank generated.

**Table 1 List of primers**

| DO- | **Primer** | **Sequence** |
|---|---|---|
| 0012 | **CH1_Rev1** | TGCCAGGGGGAAGACCGATG |
| 0656 | **MVH-1** | GCCGGCCATGGCC**GAGGTRMAGCTTCAGGAGTCAGGAC** |
| 0657 | **MVH-2** | GCCGGCCATGGCC**GAGGTSCAGCTKCAGCAGTCAGGAC** |
| 0658 | **MVH-3** | GCCGGCCATGGCC**CAGGTGCAGCTGAAGSASTCAGG** |
| 0659 | **MVH-4** | GCCGGCCATGGCC**GAGGTGCAGCTTCAGGAGTCSGGAC** |
| 0660 | **MVH-5** | GCCGGCCATGGCC**GARGTCCAGCTGCAACAGTCYGGAC** |
| 0661 | **MVH-6** | GCCGGCCATGGCC**CAGGTCCAGCTKCAGCAATCTGG** |
| 0662 | **MVH-7** | GCCGGCCATGGCC**CAGSTBCAGCTGCAGCAGTCTGG** |
| 0663 | **MVH-8** | GCCGGCCATGGCC**CAGGTYCAGCTGCAGCAGTCTGGRC** |
| 0664 | **MVH-9** | GCCGGCCATGGCC**CAGGTYCAGCTYCAGCAGTCTGG** |
| 0665 | **MVH-10** | GCCGGCCATGGCC**GAGGTCCARCTGCAACAATCTGGACC** |
| 0666 | **MVH-11** | GCCGGCCATGGCC**CAGGTCCACGTGAAGCAGTCTGGG** |
| 0667 | **MVH-12** | GCCGGCCATGGCC**GAGGTGAASSTGGTGGAATCTG** |
| 0668 | **MVH-13** | GCCGGCCATGGCC**GAVGTGAAGYTGGTGGAGTCTG** |
| 0669 | **MVH-14** | GCCGGCCATGGCC**GAGGTGCAGSKGGTGGAGTCTGGGG** |
| 0670 | **MVH-15** | GCCGGCCATGGCC**GAKGTGCAMCTGGTGGAGTCTGGG** |
| 0671 | **MVH-16** | GCCGGCCATGGCC**GAGGTGAAGCTGATGGARTCTGG** |
| 0672 | **MVH-17** | GCCGGCCATGGCC**GAGGTGCARCTTGTTGAGTCTGGTG** |
| 0673 | **MVH-18** | GCCGGCCATGGCC**GARGTRAAGCTTCTCGAGTCTGGA** |
| 0674 | **MVH-19** | GCCGGCCATGGCC**GAAGTGAARSTTGAGGAGTCTGG** |
| 0675 | **MVH-20** | GCCGGCCATGGCC**GAAGTGATGCTGGTGGAGTCTGGG** |
| 0676 | **MVH-21** | GCCGGCCATGGCC**CAGGTTACTCTRAAAGWGTSTGGCC** |
| 0677 | **MVH-22** | GCCGGCCATGGCC**CAGGTCCAACTVCAGCARCCTGG** |
| 0678 | **MVH-23** | GCCGGCCATGGCC**CAGGTYCARCTGCAGCAGTCTG** |
| 0679 | **MVH-24** | GCCGGCCATGGCC**GATGTGAACTTGGAAGTGTCTGG** |
| 0680 | **MVH-25** | GCCGGCCATGGCC**GAGGTGAAGGTCATCGAGTCTGG** |
| 0681 | **ExtMVH-1** | CAGTCACAGATCCTCGCGAATT*GGCCCA****GCCGGCC*ATGGCCSANG** |
| 0682 | **ExtMVH-2** | CAGTCACAGATCCTCGCGAATT*GGCCCA****GCCGGCCA*TGGCCSANC** |
| 0683 | **MJH-Rev1** | GGGGGTGTCG**TTTTGGCTGAGGAGAC *GGTGACC* GTGG** |
| 0684 | **MJH-Rev2** | GGGGGTGTCG**TTTTGGCTGAGGAGAC *TGTGAGA* GTGG** |
| 0685 | **MJH-Rev3** | GGGGGTGTCG**TTTTGGCTGCAGAGAC *AGTGACC* AGAG** |
| 0686 | **MJH-Rev4** | GGGGGTGTCG**TTTTGGCTGAGGAGAC *GGTGACT* GAGG** |
| 0687 | **ExtMJH-** | GGGGGTGTCG**TTTTGGCTGAGGAGAC *GGTGACC* GTGG** |
| 0688 | **ExtMJH-** | GGGGGTGTCG**TTTTGGCTGAGGAGAC *GGTGACA* GTGG** |
| 0690 | **ExtMJH-** | GGGGGTGTCG**TTTTGGCTGAGGAGAC *GGTGACC* AGAG** |
| 0691 | **ExtMJH-** | GGGGGTGTCG**TTTTGGCTGAGGAGAC *GGTGACC* GAGG** |

**Table 2**

| Phage ELISA signal levels as measured at 450nm. TT-coated plates represent plates that were coated with tetanus toxoid. Thyroglobulin coated plates are used as negative controls. 10/10 and 15/15 indicate the number of wash steps with PBS-Tween during panning procedures. The 10/10 tetanus toxoid and 10/10 thyroglobulin plates and the 15/15 tetanus toxoid and 15/15 thyroglobulin plates are duplicates from each other except for the coating agent. OD values higher than 3 times the background are assumed specific. | | | | | | | | | | | | |
|---|---|---|---|---|---|---|---|---|---|---|---|---|
| **TT-coated plate 10/10 washings** | | | | | | | | | | | | |
| | **1** | **2** | **3** | **4** | **5** | **6** | **7** | **8** | **9** | **10** | **11** | **12** |
| A | 0.139 | 0.093 | 0.089 | 0.121 | 0.117 | 0.598 | 0.146 | 0.115 | 0.18 | 0.155 | 0.543 | **0.601** |
| B | 0.136 | 0.404 | 0.159 | 0.187 | 0.489 | 0.134 | 0.216 | 0.092 | 0.222 | 0.108 | 0.181 | **0.484** |
| C | 0.197 | 0.526 | 0.09 | 0.213 | 0.395 | 0.155 | 0.108 | 0.12 | 0.183 | 0.136 | 0.092 | **0.866** |
| D | 0.143 | 0.258 | 0.101 | 0.422 | 0.088 | 0.243 | 0.485 | 0.251 | 0.304 | 0.198 | 0.478 | **0.091** |
| E | 0.445 | 0.169 | 0.526 | 0.481 | 0.206 | 0.285 | 0.111 | 0.119 | 0.128 | 0.2 | 0.118 | **0.098** |
| F | 0.237 | 0.291 | 0.594 | 0.139 | 0.206 | 0.565 | 0.543 | 0.091 | 0.136 | 0.227 | 0.228 | **0.099** |
| G | 0.459 | 0.102 | 0.152 | 0.659 | 0.203 | 0.452 | 0.152 | 0.133 | 0.094 | 0.102 | 0.375 | 0.098 |
| H | 0.341 | 0.623 | 0.745 | 0.415 | 0.682 | 0.527 | 0.655 | 0.114 | 0.258 | 0.284 | 0.685 | 0.113 |
| **TT-coated plate 15/15 washings** | | | | | | | | | | | | |

| | **1** | **2** | **3** | **4** | **5** | **6** | **7** | **8** | **9** | **10** | **11** | **12** |
|---|---|---|---|---|---|---|---|---|---|---|---|---|
| A | 0.247 | 0.582 | 0.421 | 0.428 | 0.133 | 0.082 | 0.262 | 0.079 | 0.343 | 0.414 | 0.095 | **0.292** |
| B | 0.065 | 0.364 | 0.073 | 0.042 | 0.049 | 0.071 | 0.046 | 0.103 | 0.078 | 0.057 | 0.048 | **0.155** |
| C | 0.081 | 0.044 | 0.066 | 0.082 | 0.225 | 0.444 | 0.203 | 0.362 | 0.122 | 0.047 | 0.052 | **0.309** |
| D | 0.092 | 0.11 | 0.59 | 0.22 | 0.33 | 0.544 | 0.058 | 0.159 | 0.047 | 0.174 | 0.086 | **0.05** |
| E | 0.469 | 0.577 | 0.206 | 0.304 | 0.13 | 0.749 | 0.431 | 0.062 | 0.167 | 0.049 | 0.056 | **0.049** |
| F | 0.846 | 0.07 | 0.561 | 0.656 | 0.882 | 0.094 | 0.383 | 0.13 | 0.152 | 0.098 | 0.134 | **0.048** |
| G | 0.537 | 0.052 | 0.49 | 0.105 | 0.337 | 0.193 | 0.514 | 0.294 | 0.068 | 0.35 | 0.525 | 0.05 |
| H | 0.061 | 0.306 | 0.157 | 0.853 | 0.054 | 0.534 | 0.102 | 0.235 | 0.441 | 0.412 | 0.565 | 0.061 |

| **Thyroglobulin-coated plate 10/10 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| A | 0.047 | 0.051 | 0.045 | 0.043 | 0.051 | 0.044 | 0.046 | 0.042 | 0.047 | 0.048 | 0.049 | **0.05** |
| B | 0.042 | 0.042 | 0.042 | 0.042 | 0.043 | 0.041 | 0.041 | 0.042 | 0.043 | 0.045 | 0.042 | **0.046** |
| C | 0.044 | 0.043 | 0.043 | 0.044 | 0.043 | 0.044 | 0.043 | 0.042 | 0.043 | 0.041 | 0.044 | **0.046** |
| D | 0.045 | 0.044 | 0.044 | 0.044 | 0.045 | 0.046 | 0.045 | 0.056 | 0.045 | 0.049 | 0.048 | **0.73** |
| E | 0.046 | 0.045 | 0.046 | 0.044 | 0.045 | 0.044 | 0.044 | 0.044 | 0.047 | 0.046 | 0.047 | **0.926** |
| F | 0.048 | 0.045 | 0.044 | 0.046 | 0.044 | 0.043 | 0.044 | 0.046 | 0.046 | 0.046 | 0.046 | **0.792** |
| G | 0.051 | 0.048 | 0.045 | 0.045 | 0.044 | 0.043 | 0.048 | 0.045 | 0.048 | 0.051 | 0.045 | 0.053 |
| H | 0.064 | 0.05 | 0.049 | 0.047 | 0.05 | 0.051 | 0.047 | 0.046 | 0.047 | 0.047 | 0.047 | 0.056 |

| **Thyroglobulin-coated plate 15/15 washings** | | | | | | | | | | | | |
|---|---|---|---|---|---|---|---|---|---|---|---|---|
| | 1 | **2** | **3** | **4** | **5** | **6** | **7** | **8** | **9** | **10** | **11** | **12** |
| A | 0.036 | 0.049 | 0.045 | 0.044 | 0.046 | 0.047 | 0.046 | 0.042 | 0.042 | 0.043 | 0.042 | **0.041** |
| B | 0.045 | 0.042 | 0.041 | 0.043 | 0.043 | 0.043 | 0.045 | 0.045 | 0.047 | 0.048 | 0.044 | **0.045** |
| C | 0.049 | 0.047 | 0.047 | 0.046 | 0.046 | 0.046 | 0.045 | 0.047 | 0.046 | 0.045 | 0.045 | **0.052** |
| D | 0.047 | 0.049 | 0.048 | 0.048 | 0.048 | 0.048 | 0.047 | 0.052 | 0.048 | 0.046 | 0.048 | **0.456** |
| E | 0.049 | 0.047 | 0.047 | 0.047 | 0.047 | 0.049 | 0.047 | 0.048 | 0.047 | 0.046 | 0.048 | **0.412** |
| F | 0.05 | 0.047 | 0.046 | 0.046 | 0.046 | 0.046 | 0.046 | 0.046 | 0.046 | 0.047 | 0.048 | **0.528** |
| G | 0.05 | 0.048 | 0.045 | 0.045 | 0.046 | 0.049 | 0.048 | 0.046 | 0.053 | 0.049 | 0.05 | 0.057 |
| H | 0.057 | 0.05 | 0.046 | 0.045 | 0.047 | 0.049 | 0.047 | 0.047 | 0.046 | 0.047 | 0.053 | 0.048 |

**Table 3**

| Protein sequence analysis of ELISA positive tetanus toxoid binders. CDR3 sequence, CDR3 length, VH family members and specific name, JH origin and DH origin of the clones is indicated. | | | | | |
|---|---|---|---|---|---|
| **CDR3** | **CDR3 length** | **VH** | **DH** | **JH** | **V Gene family** |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAFYTYDEKPWFAY | 15 | musIGHV192 | IGHD2-14*01 | JH3 mouse | VH7183 |
| HISYYRYDEEVSFAY | 15 | musIGHV192 | IGHD2-14*01 | JH3 mouse | VH7183 |
| HISYYRYDEEVSFAY | 15 | musIGHV192 | IGHD2-14*01 | JH3 mouse | VH7183 |
| GWRAFAY | 7 | musIGHV131 | DSP2.9 | JH3 mouse | VH7183 |
| GWRAFAY | 7 | musIGHV131 | DSP2.9 | JH3 mouse | VH7183 |
| GWRAFAY | 7 | musIGHV131 | DSP2.9 | JH3 mouse | VH7183 |
| DRGNYYGMDY | 10 | musIGHV178 | DSP2.1 | JH4 mouse | VH7183 |
| LGDYYVDWFFAV | 12 | musIGHV165 | DFL16.1 | JH1 mouse | VH7183 |
| NFPAWFAF | 8 | musIGHV547 | DST4.3inv | JH3 mouse | VJH558 |
| NFPAWFAY | 8 | musIGHV547 | DSP2.1 | JH3 mouse | VJH558 |
| NFPAWFVY | 8 | musIGHV547 | DSP2.1 | JH3 mouse | VJH558 |
| SFTPVPFYYGYDWYFDV | 17 | musIGHV532 | DSP2.3 | JH1 mouse | VJH558 |
| SFTPVPFYYGYDWYFDV | 17 | musIGHV532 | DSP2.3 | JH1 mouse | VJH558 |
| SDYDWYFDV | 9 | musIGHV286 | DSP2.2 | JH1 mouse | VJH558 |
| SDYDWYFDV | 9 | musIGHV286 | DSP2.2 | JH1 mouse | VJH558 |
| DSKWAYYFDY | 10 | musIGHV532 | DST4.3 | JH2 mouse | VJH558 |
| GDYTGYGMDY | 10 | musIGHV125 | DSP2.13 | JH4 mouse | VHSM7 |
| GDYTGYGMDY | 10 | musIGHV125 | DSP2.13 | JH4 mouse | VHSM7 |
| GGYDGYWFPY | 10 | musIGHV125 | DSP2.9 | JH3 mouse | VHSM7 |

**Table 4**

| Vector combinations that were transfected to HEK293T. | | | | | |
|---|---|---|---|---|---|
| **Code** | **HC vector** | **LC vector** | **Combined vector** | **Prep name** | **Cone. (µg/ml)** |
| A | x | 0817676_pSELECT_ 0815426 (IGKV1-39) | x | PIGKV1-39/ P1 | - |
| B | x | 0817678_pSELECT_ 0815427 (IGLV2-14) | x | PIGLV2-14/ P1 | - |
| C | MV1110 | 0817676_pSELECT_ 0815426 (IGKV1-39) | x | PMV1110/ IGKV1-39/P1 | 11.0 |
| D | MV1110 | 0817678_pSELECT_ 0815427 (IGLV2-14) | x | PMV1110/ IGLV2-14/P1 | 15.4 |
| E | x | x | MG1494 | MG1494/P2 | 16.1 |

**Table 5. HLA allotypes considered in T_{H}-epitope profiling. The corresponding serotypes are shown, as well as allotype frequencies in the Caucasian population (Klitz et al. Tissue Antigens 2003, 62:296-307; Gjertson and Terasake (eds) in: HLA 1997; Gjertson and Terasake (eds) in: HLA 1998; Castelli et al. J. Immunol. 2002, 169:6928-6934). Frequencies can add up to more than 100% since each individual has 2 alleles for each gene. If all allele frequencies of a single gene were known, they would add up to slightly less than 200% due to homozygous individuals.**

| HLA type | Serotype | Population % | HLA type | Serotype | Population % |
|---|---|---|---|---|---|
| DRB1*0101 | DR1 | 17.4 | DRB4*0103 | DR53 | 21 |
| DRB1*0102 | DR1 | 4.9 | DRB5*0101 | DR51 | 15.8 |
| DRB1*0301 | DR17(3) | 21.2 | DRB5*0202 | DR-51 | 5.7 |
| DRB1*0401 | DR4 | 11.5 | DQA1*0101/DQB1*0501 | DQ5(1) | 20.5 |
| DRB1*0402 | DR4 | 3.1 | DQA1*0102/DQB1*0502 | DQ5(1) | 2.6 |
| DRB1*0404 | DR4 | 5.5 | DQA1*0102/DQB1*0602 | DQ6(1) | 26.5 |
| DRB1*0405 | DR4 | 2.2 | DQA1*0102/DQB1*0604 | DQ6(1) | 6.7 |
| DRB1*0407 | DR4 | <2 | DQA1*0103/DQB1*0603 | DQ6(1) | 11 |
| DRB1*0701 | DR7 | 23.4 | DQA1*104/DQB1*0503 | DQ5(1) | 4 |
| DRB1*0801 | DR8 | 3.3 | DQA1*0201/DQB1*0202 | DQ2 | 20.9 |
| DRB1*0802 | DR8 | <2 | DQA1*0201/DQB1*0303 | DQ9(3) | 7.2 |
| DRB1*0901 | DR9 | <2 | DQA1*0301/DQB1*0301 | DQ7(3) | 12.5 |
| DRB1*1101 | DR11(5) | 17 | DQA1*0301/DQB1*0302 | DQ8(3) | 18.3 |
| DRB1*1104 | DR11(5) | 5.7 | DQA1*0401/DQB1*0402 | DQ4 | 4.5 |
| DRB1*1201 | DR12(5) | 3.1 | DQA1*0501/DQB1*0201 | DQ2 | 24.6 |
| DRB1*1301 | DR13(6) | 15.4 | DQA1*0501/DQB1*0301 | DQ7(3) | 20.9 |
| DRB1*1302 | DR13(6) | 10.8 | DPA1*0103/DPB1*0201 | DPw2 | 19.9 |
| DRB1*1401 | DR14(6) | 4.2 | DPA1*0103/DPB1*0401 | DPw4 | 65.1 |
| DRB1*1501 | DR15(2) | 13.2 | DPA1*0103/DPB1*0402 | DPw4 | 24.3 |
| DRB1*1601 | DR16(2) | 5.5 | DPA1*0201/DPB1*0101 | DPw1 | 6.3 |
| DRB3*0101 | DR52 | 24.6 | DPA1*0201/DPB1*0301 | DPw3 | <2 |
| DRB3*0202 | DR52 | 43 | DP41*0201/DPB1*0501 | DPw5 | <2 |
| DRB3*0301 | DR52 | 10 | DPA1*0201/DPB1*0901 | | 2.4 |
| DRB4*0101 | DR53 | 25.5 | | | |

**Table 6. T_{H} epitope counts for IGKV1-39. Peptides binding to multiple HLAs of the same group (DRB1, DRB3/4/5, DP, DQ) are counted as one. Values between brackets refer to germline-filtered peptides.**

| | DRB1 | | DRB3/4/5 | | DQ | | DP | |
|---|---|---|---|---|---|---|---|---|
| | Strong | Medium | Strong | Medium | Strong | Medium | Strong | Medium |
| Merus IGKV1-39 | 0(+6) | 0(+16) | 0(+0) | 0(+5) | 0(+3) | 0(+9) | 0(+0) | 0(+9) |

**Table 7. Mapping of Epibase® predictions for Merus IGKV1-39 in the classical 15-mer peptide format. This table shows the allotype count of critical epitopes and implicated serotypes for each of the 15-mers spanning the Merus IGKV1-39 sequence.**

| 15mer | Start position | 15-mer sequence | Allotype count | Implicated serotypes |
|---|---|---|---|---|
| 1 | 1 | DIQMTQSPSSLSASV | 6 | DR1, DR4, DR7, DR9 |
| 2 | 4 | MTQSPSSLSASVGDR | 5 | DR1, DR4, DR9 |
| 3 | 7 | SPSSLSASVGDRVTI | 0 | |
| 4 | 10 | SLSASVGDRVTITCR | 0 | |
| 5 | 13 | ASVGDRVTITCRASQ | 0 | |
| 6 | 16 | GDRVTITCPASQSIS | 2 | DR11(5), DR7 |
| 7 | 19 | VTITCRASQSISSYL | 4 | DQ2, DR11(5), DR4, DR7 |
| 8 | 22 | TCFASQSISSYLNWY | 2 | DQ2, DR4 |
| 9 | 25 | ASQSISSYLNWYQQK | 5 | DR13(6), DR15(2), DR4 |
| 10 | 28 | SISSYLNWYQQKPGK | 8 | DR12(5), DR13(6), DR15(2), DR16(2), DR4, DR8 |
| 11 | 31 | SYLNWYQQKPGKAPK | 10 | DR1, DR12(5), DR16(2), DR4, DR51, DR8 |
| 12 | 34 | NWYQQKPGKAPKLLI | 9 | DR1, DR15(2), DR4, DR51, DR8 |
| 13 | 37 | QQKPGKAPKLLIYAA | 7 | DQ4, DR1, DR11(5), DR15(2), DR51, DR8 |
| 14 | 40 | PGKAPKLLIYAASSL | 7 | DQ4, DR1, DR11(5), DR4, DR8 DR1, DR11(5), DR12(5). DR13(6), DR14(6), DR15(2). DR4, |
| 15 | 43 | APKLLIYAASSLQSG | 15 | DR51, DR8, DR9 DR1, DR11(5), DR12(5), DR13(6), DR14(6). DR15(2), DR4, |
| 16 | 46 | LLIYAASSLQSGVPS | 15 | DR51, DR8, DR9 |
| 17 | 49 | YAASSLQSGVPSRFS | 1 | DR15(2) |
| 18 | 52 | SSLQSGVPSRFSGSG | 1 | DR15(2) |
| 19 | 55 | QSGVPSRFSGSGSGT | 0 | |
| 20 | 58 | VPSRFSGSGSGTDFT | 0 | |
| 21 | 61 | RFSGSGSGTDFTLTI | 0 | |
| 22 | 64 | GSGSGTDFTLTISSL | 1 | DR52 |
| 23 | 67 | SCTDFTLTISSLQPE | 4 | DR4, DR52, DR7, DR9 |
| 24 | 70 | DFTLTISSLQPEDFA | 4 | DQ2, DR4, DR7, DR9 |
| 25 | 73 | LTISSLOPEDFATYY | 1 | DQ2 |
| 26 | 76 | SSLQPEDFATYYCQQ | 0 | |
| 27 | 79 | QPEDFATYYCQQSYS | 1 | DR4 |
| 28 | 82 | DFATYYCQQSYSTPP | 5 | DR4, DR51, DR7 |
| 29 | 85 | TYYCQQSYSTPPTFG | 4 | DR4, DR51. DR7 |
| 30 | 88 | CQQSYSTPPTFGQGT | 0 | |
| 31 | 91 | SYSTPFTFGQGTKVE | 0 | |
| 32 | 94 | TPPTFGQGTKVEIK | 0 | |

**Table 8: The V_{H} gene from PG1433 paired with various light chain genes with differing rates of amino acid mutation were compared for production levels with the original clone containing the IGKV1-39 gene.**

| **IgG name** | **Light chain gene** | **Number of amino acid mutations** | **concentration (µB/ml)** |
|---|---|---|---|
| *PG1433* | ***1-39*** | ***0*** | *63, 45.5, 38.6 (avg* = ***49**)* |
| PG1631 | 1-12 | 4 | 10.5 |
| PG1632 | 1-27 | 7 | 9.3 |
| PG1634 | 1D-12 | 10 | 10.8 |
| PG1635 | 1D-33 | 6 | 10.2 |
| PG1642 | 1-5 | 8 | 7.1 |
| PG1644 | 1-9 | 3 | 7.8 |
| PG1650 | 1D-39 | 3 | 9.1 |
| PG1652 | 2D-28 | 3 | 7.1 |
| PG1653 | 3-15 | 14 | 7 |
| PG1654 | 3-20 | 2 | 5.2 |
| PG1674 | 1-40 | 7 | 8.2 |
| PG1678 | 2-11 | 2 | 8.1 |
| PG1680 | 2-14 | 15 | 10.8 |
| PG1682 | 3-1 | 13 | 9.9 |
| PG1683 | 6-57 | 6 | 13.9 |

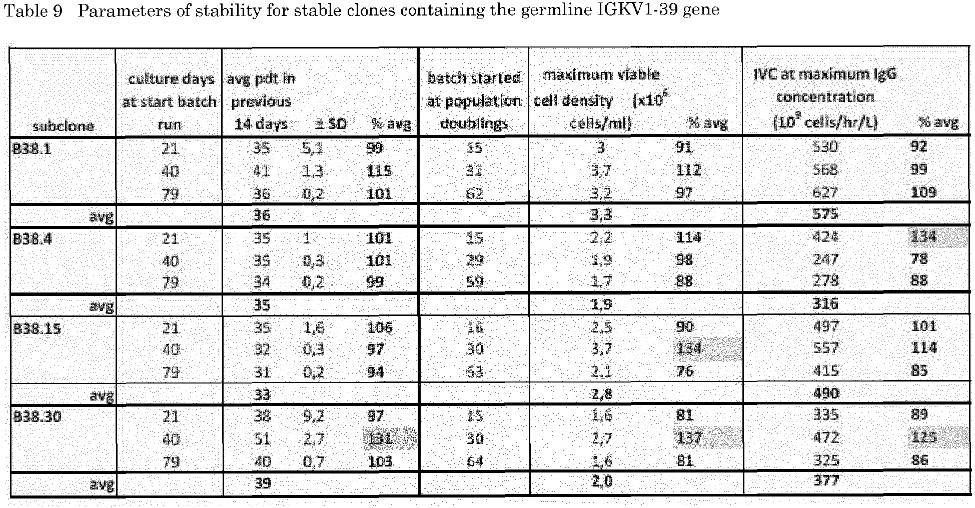

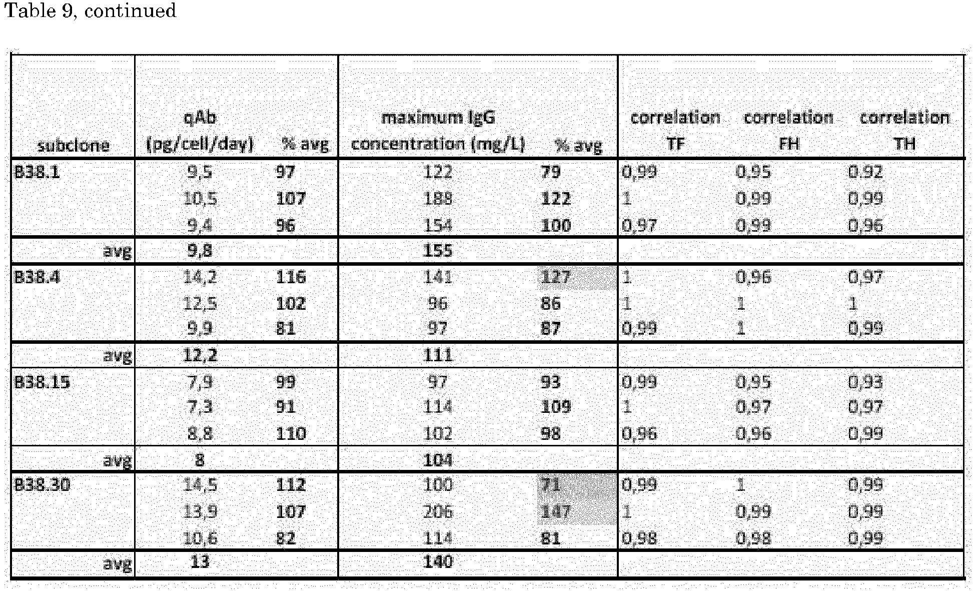

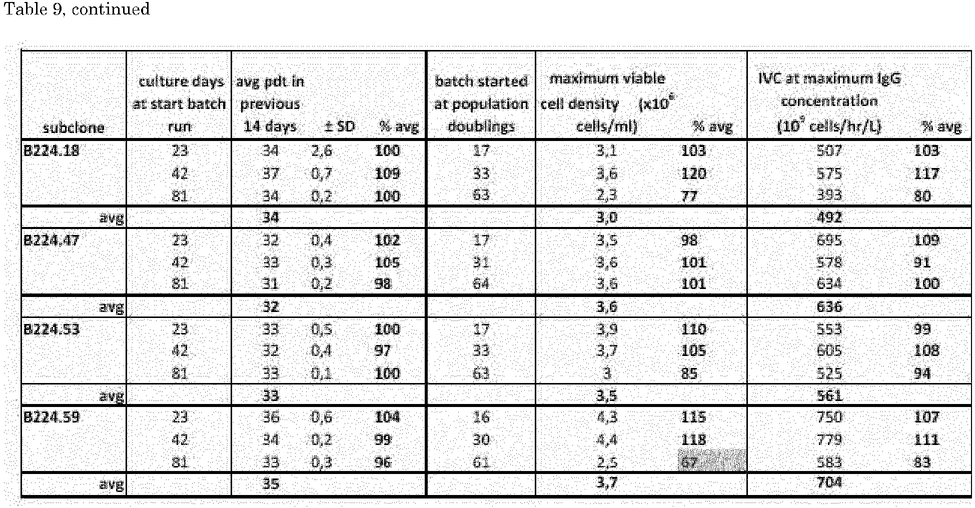

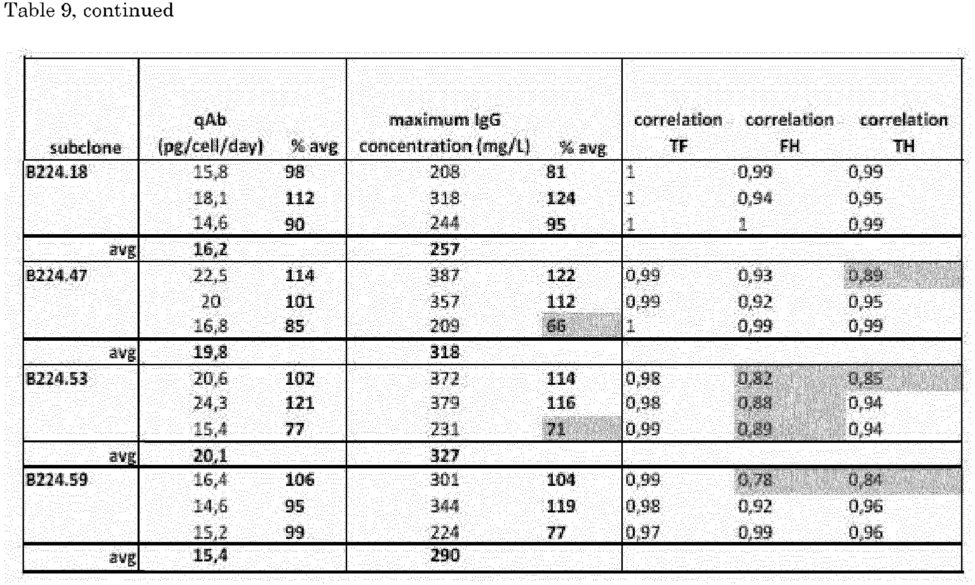

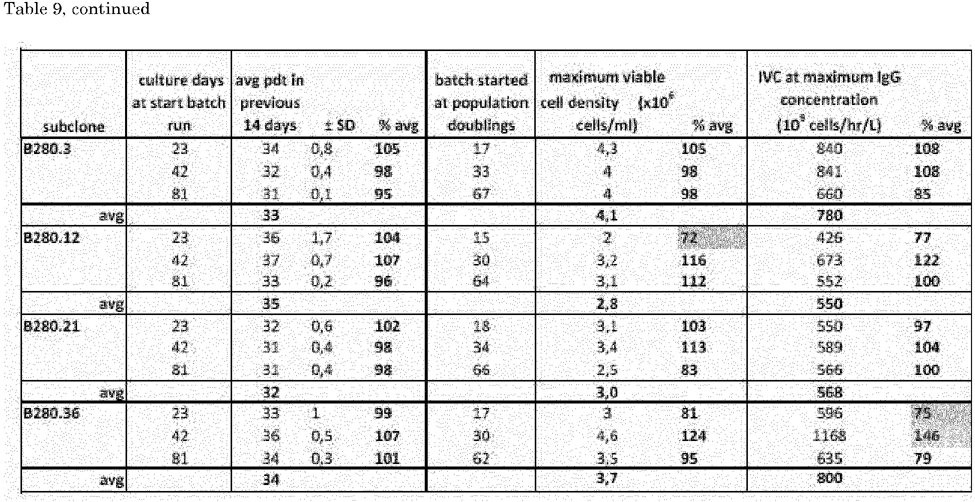

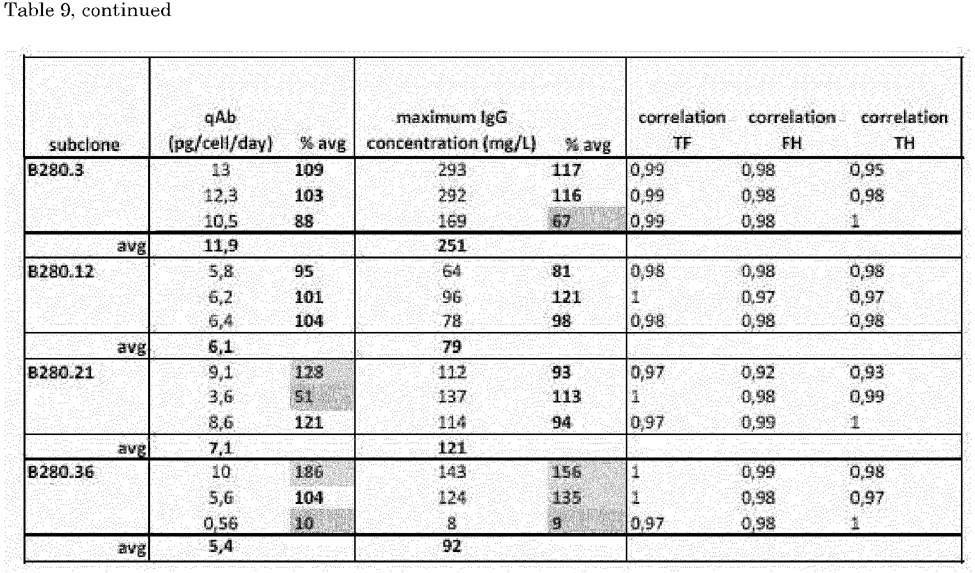

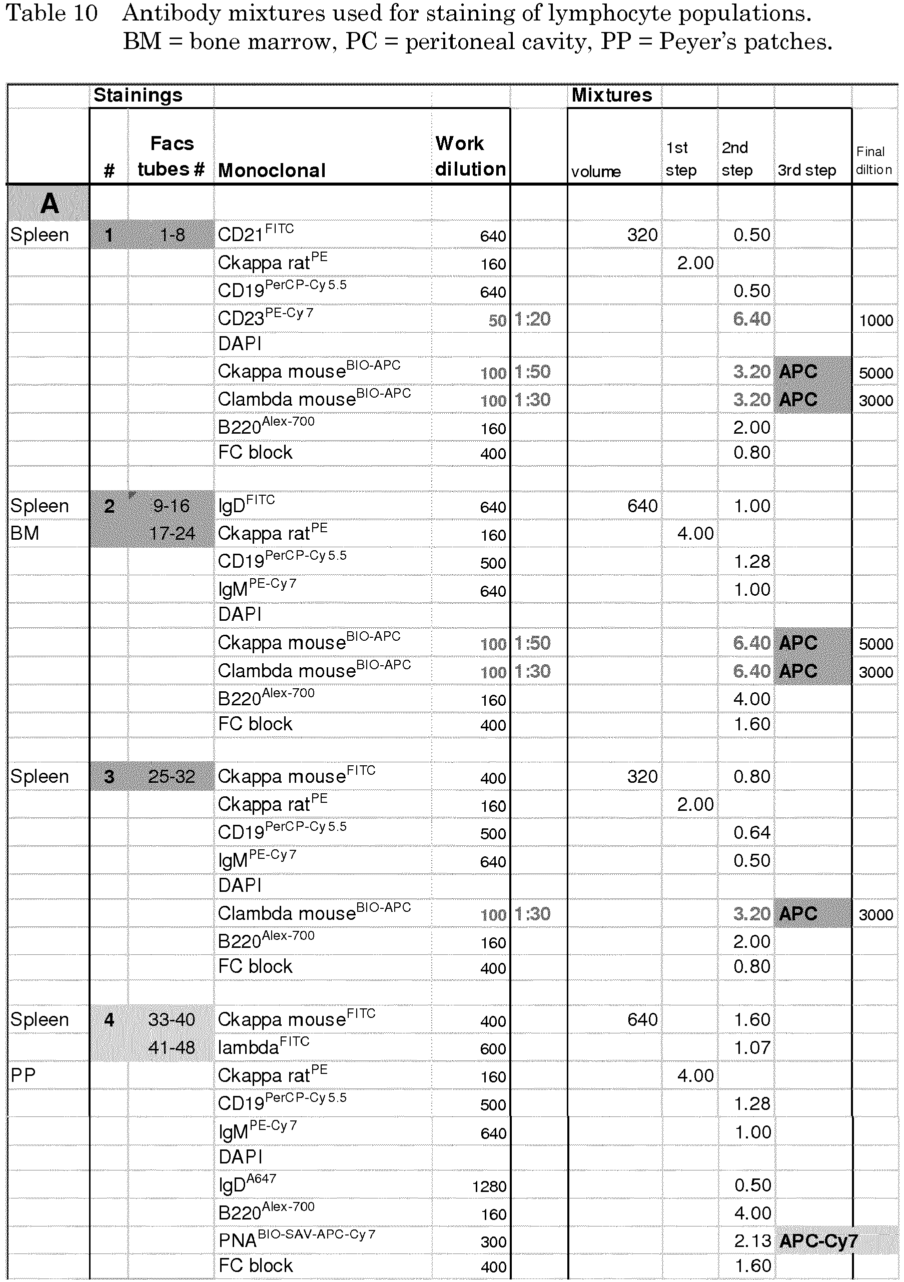

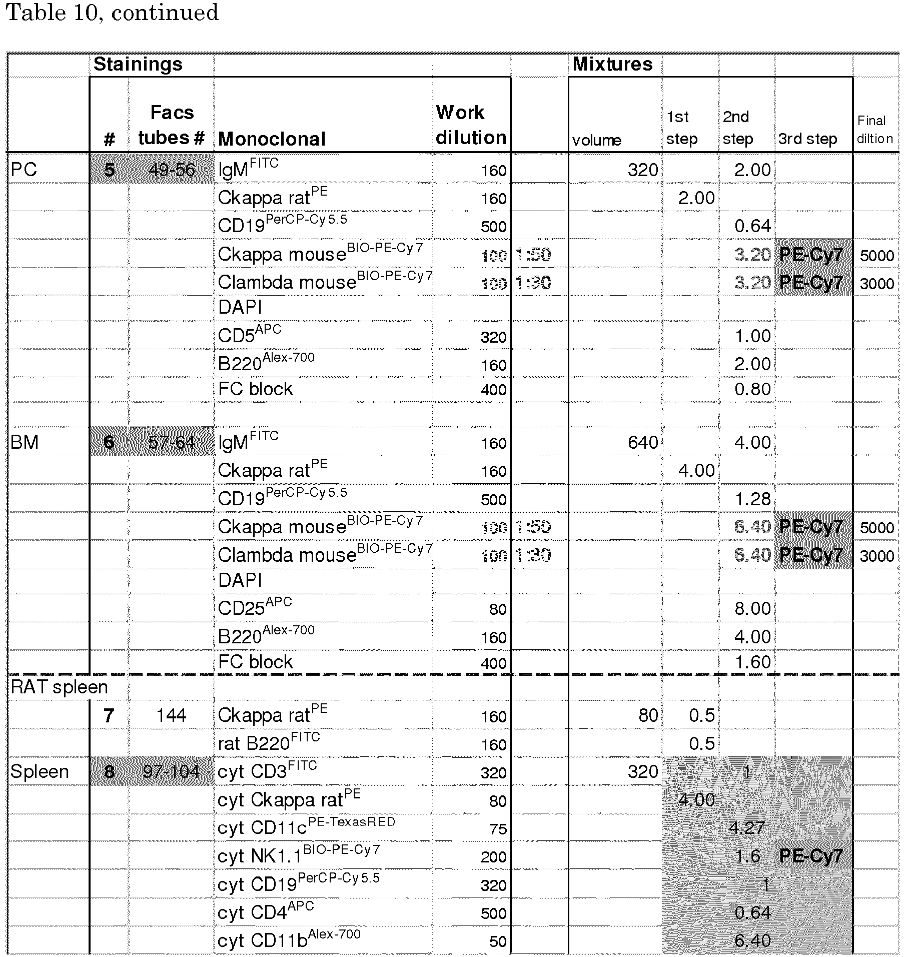

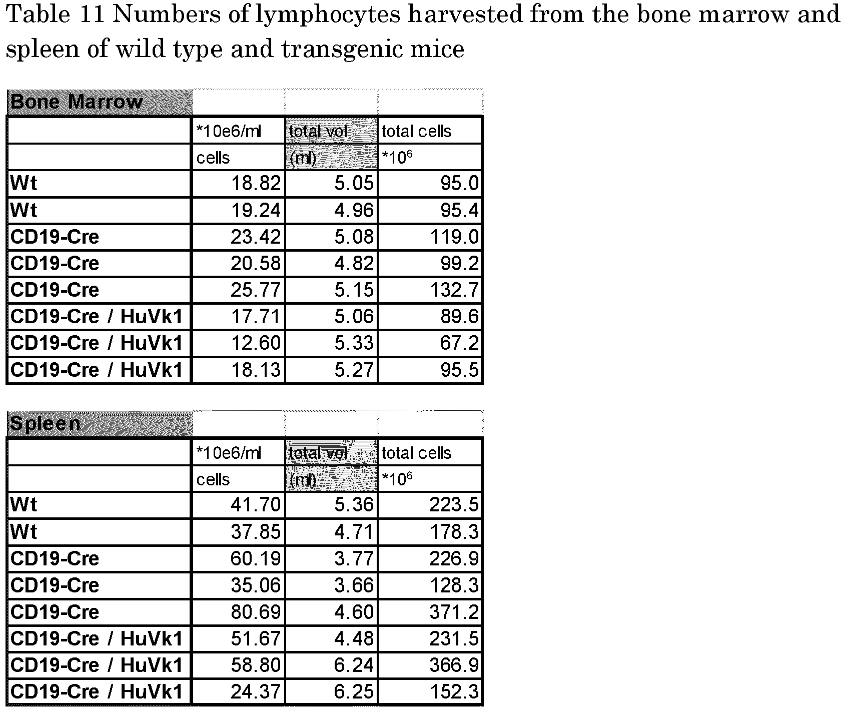

## Claims

1. A transgenic murine host capable of producing immunoglobulins comprising a human VL region, said murine host comprising integrated in its germline a rearranged nucleic acid comprising one human germline V and one human germline J gene segment encoding a rearranged human VL region and a light chain constant region of any animal species, wherein said light chain variable region is capable of pairing with at least two different heavy chains encoded by the host, such that variety in specificity of antibodies comprising said human VL region is retained through rearrangements and hypermutations in the heavy chains.

2. A transgenic host according to claim 1, wherein the host is a mouse.

3. A transgenic host according to claim 1 or 2, wherein said light chain constant region is a murine light chain constant region.

4. A transgenic host according to any one of claims 1-3, wherein the integration is in a locus that is resistant to silencing.

5. A transgenic host according to any one of claims 1-3, wherein the integration is in the Rosa-locus.

6. A transgenic host according to any one of claims 1-5, wherein the light chain variable region encoding nucleic acid is provided with a means that allows expression of said nucleic acid essentially limited to cells of B cell lineage.

7. A transgenic host according to claim 6, wherein the light chain variable region encoding nucleic acid is provided with a means that allows expression of the light chain variable region encoding nucleic acid predominantly during a certain stage of the development of B cells.

8. A transgenic host according to claim 7, wherein said means comprises a promoter selected from the group of CD19, CD20, µHC, VpreB1, VpreB2, VpreB3, λ5, Igα, Igβ, κLC, λLC, and BSAP (Pax5).

9. A transgenic host according to claim 7 or 8, wherein said means comprises a cre-lox system.

10. A transgenic host according to any one of claims 1-9, wherein said light chain variable region is capable of pairing with at least two host heavy chains.

11. A transgenic host according to any one of claims 1-9, wherein said light chain variable region is capable of pairing with at least two human heavy chains.

12. A transgenic host according to any one of claims 1-11, wherein at least one of the endogenous loci encoding an endogenous light chain is functionally silenced.

13. A transgenic host according to anyone of claims 1-12, wherein the endogenous κ light chain locus is functionally silenced.

14. A transgenic host according to any one of claims 1-13, wherein the sequence of the light chain variable region encoding nucleic acid is a human germline vκ sequence.

15. A transgenic host according to claim 14, wherein the germline vκ sequence is based on 012.

16. A transgenic host according to claim 15, wherein the germline vκ sequence is IGKV1-39*01 / IGKJ1*01.

17. A method for producing a desired antibody comprising exposing a host according to any one of claims 1-16 to an antigen such that an antibody response is induced and isolating the antibodies specific for the antigen.

18. A method for producing a desired antibody comprising exposing a host according to any one of claims 1-16 to an antigen such that an antibody response is induced and isolating cells producing such antibodies, culturing and harvesting said antibodies.

19. A method for producing a desired antibody comprising exposing a host according to any one of claims 1-16 to an antigen such that an antibody response is induced and isolating a nucleic acid encoding at least part of such an antibody, inserting said nucleic acid or a copy or a derivative thereof in an expression cassette and expressing said antibody in a host cell.

## Patentansprüche

1. Transgener muriner Wirt, der in der Lage ist, Immunglobuline herzustellen, die eine humane VL-Region umfassen, wobei der murine Wirt eine in seine Keimbahn integrierte rearrangierte Nukleinsäure umfasst, die ein humanes Keimbahn-V- und ein humanes Keimbahn-J-Gensegment umfasst, die eine rearrangierte humane VL-Region kodiert, und eine konstante Region einer leichten Kette einer beliebigen Tierart, wobei die variable Region der leichten Kette in der Lage ist, sich mit mindestens zwei verschiedenen schweren Ketten zu paaren, die vom Wirt kodiert werden, so dass die Vielfalt der Spezifität von Antikörpern, die die humane VL-Region umfassen, durch Umlagerungen ("rearrangements") und Hypermutationen in den schweren Ketten erhalten bleibt.

2. Transgener Wirt nach Anspruch 1, wobei der Wirt eine Maus ist.

3. Transgener Wirt nach Anspruch 1 oder 2, wobei die konstante Region der leichten Kette eine murine konstante Region der leichten Kette ist.

4. Transgener Wirt nach einem beliebigen der Ansprüche 1-3, wobei die Integration in einem Locus ist, der gegen Silencing resistent ist.

5. Transgener Wirt nach einem beliebigen der Ansprüche 1-3, wobei die Integration in dem Rosa-Locus ist.

6. Transgener Wirt nach einem beliebigen der Ansprüche 1-5, wobei die die variable Region der leichten Kette kodierende Nukleinsäure bereitgestellt ist mit einem Mittel, das die Expression der Nukleinsäure im Wesentlichen auf Zellen der B-Zell-Linie beschränkt erlaubt.

7. Transgener Wirt nach Anspruch 6, wobei die die variable Region der leichten Kette kodierende Nukleinsäure bereitgestellt ist mit einem Mittel, das die Expression der die variable Region der leichten Kette kodierenden Nukleinsäure, vorwiegend während eines bestimmten Stadiums der Entwicklung von B-Zellen erlaubt.

8. Transgener Wirt nach Anspruch 7, wobei das Mittel einen Promotor umfasst, der aus der Gruppe von CD19, CD20, µHC, VpreB1, VpreB2, VpreB3, λ5, Igα, Igβ, κLC, λLC und BSAP (Pax5) ausgewählt ist.

9. Transgener Wirt nach Anspruch 7 oder 8, wobei das Mittel ein cre-lox-System umfasst.

10. Transgener Wirt nach einem beliebigen der Ansprüche 1-9, wobei die variable Region der leichten Kette in der Lage ist, sich mit mindestens zwei schweren Ketten des Wirts zu paaren.

11. Transgener Wirt nach einem beliebigen der Ansprüche 1-9, wobei die variable Region der leichten Kette in der Lage ist, sich mit mindestens zwei humanen schweren Ketten zu paaren.

12. Transgener Wirt nach einem beliebigen der Ansprüche 1-11, wobei mindestens einer der endogenen Loci, die eine endogene leichte Kette kodieren, funktionell stillgelegt ("silenced") ist.

13. Transgener Wirt nach einem beliebigen der Ansprüche 1-12, wobei der endogene κ-Leichtketten-Locus funktionell stillgelegt ("silenced") ist.

14. Transgener Wirt nach einem beliebigen der Ansprüche 1-13, wobei die Sequenz der Nukleinsäure, die die variable Region der leichten Kette kodiert, eine humane Keimbahn-vκ-Sequenz ist.

15. Transgener Wirt nach Anspruch 14, wobei die Keimbahn-vκ-Sequenz auf O12 basiert.

16. Transgener Wirt nach Anspruch 15, wobei die Keimbahn-vκ-Sequenz IGKV1-39*01 / IGKJ1*01 ist.

17. Verfahren zum Herstellen eines gewünschten Antikörpers, umfassend das Aussetzen eines Wirts nach einem beliebigen der Ansprüche 1-16 gegenüber einem Antigen, so dass eine Antikörperantwort induziert wird, und Isolieren der für das Antigen spezifischen Antikörper.

18. Verfahren zum Herstellen eines gewünschten Antikörpers, umfassend das Aussetzen eines Wirts nach einem beliebigen der Ansprüche 1-16 gegenüber einem Antigen, so dass eine Antikörperantwort induziert wird, und Isolieren von Zellen, die solche Antikörper produzieren, Kultivieren und Ernten dieser Antikörper.

19. Verfahren zum Herstellen eines gewünschten Antikörpers, umfassend das Aussetzen eines Wirts nach einem beliebigen der Ansprüche 1-16 gegenüber einem Antigen, so dass eine Antikörperantwort induziert wird, und Isolieren einer Nukleinsäure, die mindestens einen Teil eines solchen Antikörpers kodiert, Einfügen dieser Nukleinsäure oder einer Kopie oder eines Derivats davon in eine Expressionskassette und Exprimieren des Antikörpers in einer Wirtszelle.

## Revendications

1. Hôte murin transgénique capable de produire des immunoglobulines comprenant une région VL humaine, ledit hôte murin comprenant, intégré dans sa lignée germinale, un acide nucléique réarrangé comprenant un segment de gène V de lignée germinale humaine et un segment de gène J de lignée germinale humaine codant pour une région VL humaine réarrangée et une région constante de chaîne légère d'une quelconque espèce animale, dans lequel ladite région variable de chaîne légère est capable de s'apparier avec au moins deux chaînes lourdes différentes codées par l'hôte, de sorte qu'une diversité, en termes de spécificité des anticorps comprenant ladite région VL humaine, soit conservée par des réarrangements et des hypermutations dans les chaînes lourdes.

2. Hôte transgénique selon la revendication 1, dans lequel l'hôte est une souris.

3. Hôte transgénique selon la revendication 1 ou 2, dans lequel ladite région constante de chaîne légère est une région constante de chaîne légère murine.

4. Hôte transgénique selon l'une quelconque des revendications 1 à 3, dans lequel l'intégration est dans un locus qui est résistant à une extinction.

5. Hôte transgénique selon l'une quelconque des revendications 1 à 3, dans lequel l'intégration est dans le locus Rosa.

6. Hôte transgénique selon l'une quelconque des revendications 1 à 5, dans lequel l'acide nucléique codant pour la région variable de chaîne légère est mis à disposition avec un moyen qui permet une expression dudit acide nucléique essentiellement limitée aux cellules de la lignée des cellules B.

7. Hôte transgénique selon la revendication 6, dans lequel l'acide nucléique codant pour la région variable de chaîne légère est mis à disposition avec un moyen qui permet l'expression de l'acide nucléique codant pour la région variable de chaîne légère principalement pendant un certain stade du développement des cellules B.

8. Hôte transgénique selon la revendication 7, dans lequel ledit moyen comprend un promoteur sélectionné dans le groupe de CD19, CD20, µHC, VpreB1, VpreB2, VpreB3, λ5, Igα, Igβ, κLC, λLC, et BSAP (Pax5).

9. Hôte transgénique selon la revendication 7 ou 8, dans lequel ledit moyen comprend système cre-lox.

10. Hôte transgénique selon l'une quelconque des revendications 1 à 9, dans lequel ladite région variable de chaîne légère est capable de s'apparier avec au moins deux chaînes lourdes de l'hôte.

11. Hôte transgénique selon l'une quelconque des revendications 1 à 9, dans lequel ladite région variable de chaîne légère est capable de s'apparier avec au moins deux chaînes lourdes humaines.

12. Hôte transgénique selon l'une quelconque des revendications 1 à 11, dans lequel au moins un des loci endogènes codant pour une chaîne légère endogène a subi une extinction fonctionnelle.

13. Hôte transgénique selon l'une quelconque des revendications 1 à 12, dans lequel le locus de chaîne légère κ endogène a subi une extinction fonctionnelle.

14. Hôte transgénique selon l'une quelconque des revendications 1 à 13, dans lequel la séquence de l'acide nucléique codant pour la région variable de chaîne légère est une séquence vκ de lignée germinale humaine.

15. Hôte transgénique selon la revendication 14, dans lequel la séquence vκ de lignée germinale est basée sur O12.

16. Hôte transgénique selon la revendication 15, dans lequel la séquence vκ de lignée germinale est IGKV1-39*01/IGKJ1*01.

17. Procédé pour produire un anticorps souhaité comprenant l'exposition d'un hôte selon l'une quelconque des revendications 1 à 16 à un antigène de sorte qu'une réponse d'anticorps soit induite et l'isolement des anticorps spécifiques pour l'antigène.

18. Procédé pour produire un anticorps souhaité comprenant l'exposition d'un hôte selon l'une quelconque des revendications 1 à 16 à un antigène de sorte qu'une réponse d'anticorps soit induite et l'isolement des cellules produisant de tels anticorps, la mise en culture et la récolte desdits anticorps.

19. Procédé pour produire un anticorps souhaité comprenant l'exposition d'un hôte selon l'une quelconque des revendications 1 à 16 à un antigène de sorte qu'une réponse d'anticorps soit induite et l'isolement d'un acide nucléique codant pour au moins une partie d'un tel anticorps, l'insertion dudit acide nucléique ou d'une copie ou d'un dérivé de celui-ci dans une cassette d'expression et l'expression dudit anticorps dans une cellule hôte.


### SEQUENCE LISTING

<110> Merus N.V.
<120> Antibody producing non-human mammals.
<130> M70121PCEPT2
<140> EP12175544.1
   <141> 2009-06-29
<150> PCT/NL2008/050430
   <151> 2008-06-27
<160> 101
<170> PatentIn version 3.3
<210> 1
   <211> 20
   <212> DNA
   <213> Artificial
<220>
   <223> primer
<400> 1
   ccctttccaa tctttatggg 20
<210> 2
   <211> 23
   <212> DNA
   <213> Artificial
<220>
   <223> primer
<400> 2
   aggtggattg gtgtcttttt ctc 23
<210> 3
   <211> 22
   <212> DNA
   <213> Artificial
<220>
   <223> primer
<400> 3
   gtcatgtcgg cgaccctacg cc 22
<210> 4
   <211> 20
   <212> DNA
   <213> Artificial
<220>
   <223> primer
<400> 4
   tgccaggggg aagaccgatg 20
<210> 5
   <211> 38
   <212> DNA
   <213> Artificial
<220>
   <223> primer
<400> 5
   gccggccatg gccgaggtrm agcttcagga gtcaggac 38
<210> 6
   <211> 38
   <212> DNA
   <213> Artificial
<220>
   <223> primer
<400> 6
   gccggccatg gccgaggtsc agctkcagca gtcaggac 38
<210> 7
   <211> 36
   <212> DNA
   <213> Artificial
<220>
   <223> primer
<400> 7
   gccggccatg gcccaggtgc agctgaagsa stcagg 36
<210> 8
   <211> 38
   <212> DNA
   <213> Artificial
<220>
   <223> primer
<400> 8
   gccggccatg gccgaggtgc agcttcagga gtcsggac 38
<210> 9
   <211> 38
   <212> DNA
   <213> Artificial
<220>
   <223> primer
<400> 9
   gccggccatg gccgargtcc agctgcaaca gtcyggac 38
<210> 10
   <211> 36
   <212> DNA
   <213> Artificial
<220>
   <223> primer
<400> 10
   gccggccatg gcccaggtcc agctkcagca atctgg 36
<210> 11
   <211> 36
   <212> DNA
   <213> Artificial
<220>
   <223> primer
<400> 11
   gccggccatg gcccagstbc agctgcagca gtctgg 36
<210> 12
   <211> 38
   <212> DNA
   <213> Artificial
<220>
   <223> primer
<400> 12
   gccggccatg gcccaggtyc agctgcagca gtctggrc 38
<210> 13
   <211> 36
   <212> DNA
   <213> Artificial
<220>
   <223> primer
<400> 13
   gccggccatg gcccaggtyc agctycagca gtctgg 36
<210> 14
   <211> 39
   <212> DNA
   <213> Artificial
<220>
   <223> primer
<400> 14
   gccggccatg gccgaggtcc arctgcaaca atctggacc 39
<210> 15
   <211> 37
   <212> DNA
   <213> Artificial
<220>
   <223> primer
<400> 15
   gccggccatg gcccaggtcc acgtgaagca gtctggg 37
<210> 16
   <211> 35
   <212> DNA
   <213> Artificial
<220>
   <223> primer
<400> 16
   gccggccatg gccgaggtga asstggtgga atctg 35
<210> 17
   <211> 35
   <212> DNA
   <213> Artificial
<220>
   <223> primer
<400> 17
   gccggccatg gccgavgtga agytggtgga gtctg 35
<210> 18
   <211> 38
   <212> DNA
   <213> Artificial
<220>
   <223> primer
<400> 18
   gccggccatg gccgaggtgc agskggtgga gtctgggg 38
<210> 19
   <211> 37
   <212> DNA
   <213> Artificial
<220>
   <223> primer
<400> 19
   gccggccatg gccgakgtgc amctggtgga gtctggg 37
<210> 20
   <211> 36
   <212> DNA
   <213> Artificial
<220>
   <223> primer
<400> 20
   gccggccatg gccgaggtga agctgatgga rtctgg 36
<210> 21
   <211> 38
   <212> DNA
   <213> Artificial
<220>
   <223> primer
<400> 21
   gccggccatg gccgaggtgc arcttgttga gtctggtg 38
<210> 22
   <211> 37
   <212> DNA
   <213> Artificial
<220>
   <223> primer
<400> 22
   gccggccatg gccgargtra agcttctcga gtctgga 37
<210> 23
   <211> 36
   <212> DNA
   <213> Artificial
<220>
   <223> primer
<400> 23
   gccggccatg gccgaagtga arsttgagga gtctgg 36
<210> 24
   <211> 37
   <212> DNA
   <213> Artificial
<220>
   <223> primer
<400> 24
   gccggccatg gccgaagtga tgctggtgga gtctggg 37
<210> 25
   <211> 38
   <212> DNA
   <213> Artificial
<220>
   <223> primer
<400> 25
   gccggccatg gcccaggtta ctctraaagw gtstggcc 38
<210> 26
   <211> 36
   <212> DNA
   <213> Artificial
<220>
   <223> primer
<400> 26
   gccggccatg gcccaggtcc aactvcagca rcctgg 36
<210> 27
   <211> 35
   <212> DNA
   <213> Artificial
<220>
   <223> primer
<400> 27
   gccggccatg gcccaggtyc arctgcagca gtctg 35
<210> 28
   <211> 36
   <212> DNA
   <213> Artificial
<220>
   <223> primer
<400> 28
   gccggccatg gccgatgtga acttggaagt gtctgg 36
<210> 29
   <211> 36
   <212> DNA
   <213> Artificial
<220>
   <223> primer
<400> 29
   gccggccatg gccgaggtga aggtcatcga gtctgg 36
<210> 30
   <211> 45
   <212> DNA
   <213> Artificial
<220>
   <223> primer
<220>
   <221> misc_feature
   <222> (44)..(44)
   <223> n is a, c, g, or t
<400> 30
   cagtcacaga tcctcgcgaa ttggcccagc cggccatggc csang 45
<210> 31
   <211> 45
   <212> DNA
   <213> Artificial
<220>
   <223> primer
<220>
   <221> misc_feature
   <222> (44)..(44)
   <223> n is a, c, g, or t
<400> 31
   cagtcacaga tcctcgcgaa ttggcccagc cggccatggc csanc 45
<210> 32
   <211> 37
   <212> DNA
   <213> Artificial
<220>
   <223> primer
<400> 32
   gggggtgtcg ttttggctga ggagacggtg accgtgg 37
<210> 33
   <211> 37
   <212> DNA
   <213> Artificial
<220>
   <223> primer
<400> 33
   gggggtgtcg ttttggctga ggagactgtg agagtgg 37
<210> 34
   <211> 37
   <212> DNA
   <213> Artificial
<220>
   <223> primer
<400> 34
   gggggtgtcg ttttggctgc agagacagtg accagag 37
<210> 35
   <211> 37
   <212> DNA
   <213> Artificial
<220>
   <223> primer
<400> 35
   gggggtgtcg ttttggctga ggagacggtg actgagg 37
<210> 36
   <211> 37
   <212> DNA
   <213> Artificial
<220>
   <223> primer
<400> 36
   gggggtgtcg ttttggctga ggagacggtg accgtgg 37
<210> 37
   <211> 37
   <212> DNA
   <213> Artificial
<220>
   <223> primer
<400> 37
   gggggtgtcg ttttggctga ggagacggtg acagtgg 37
<210> 38
   <211> 37
   <212> DNA
   <213> Artificial
<220>
   <223> primer
<400> 38
   gggggtgtcg ttttggctga ggagacggtg accagag 37
<210> 39
   <211> 37
   <212> DNA
   <213> Artificial
<220>
   <223> primer
<400> 39
   gggggtgtcg ttttggctga ggagacggtg accgagg 37
<210> 40
   <211> 15
   <212> PRT
   <213> Artificial
<220>
   <223> CDR3
<400> 40
   His Gly Ala Tyr Tyr Thr Tyr Asp Glu Lys Ala Trp Phe Ala Tyr
   1               5                   10                  15
<210> 41
   <211> 15
   <212> PRT
   <213> Artificial
<220>
   <223> CDR3
<400> 41
   His Gly Ala Phe Tyr Thr Tyr Asp Glu Lys Pro Trp Phe Ala Tyr
   1               5                   10                  15
<210> 42
   <211> 15
   <212> PRT
   <213> Artificial
<220>
   <223> CDR3
<400> 42
   His Ile Ser Tyr Tyr Arg Tyr Asp Glu Glu Val Ser Phe Ala Tyr
   1               5                   10                  15
<210> 43
   <211> 7
   <212> PRT
   <213> Artificial
<220>
   <223> CDR3
<400> 43
   Gly Trp Arg Ala Phe Ala Tyr
   1               5
<210> 44
   <211> 10
   <212> PRT
   <213> Artificial
<220>
   <223> CDR3
<400> 44
   Asp Arg Gly Asn Tyr Tyr Gly Met Asp Tyr
   1               5                   10
<210> 45
   <211> 12
   <212> PRT
   <213> Artificial
<220>
   <223> CDR3
<400> 45
   Leu Gly Asp Tyr Tyr Val Asp Trp Phe Phe Ala Val
   1               5                   10
<210> 46
   <211> 8
   <212> PRT
   <213> Artificial
<220>
   <223> CDR3
<400> 46
   Asn Phe Pro Ala Trp Phe Ala Phe
   1               5
<210> 47
   <211> 17
   <212> PRT
   <213> Artificial
<220>
   <223> CDR3
<400> 47
   Ser Phe Thr Pro Val Pro Phe Tyr Tyr Gly Tyr Asp Trp Tyr Phe Asp
   1               5                   10                  15
   Val
<210> 48
   <211> 9
   <212> PRT
   <213> Artificial
<220>
   <223> CDR3
<400> 48
   Ser Asp Tyr Asp Trp Tyr Phe Asp Val
   1               5
<210> 49
   <211> 10
   <212> PRT
   <213> Artificial
<220>
   <223> CDR3
<400> 49
   Asp Ser Lys Trp Ala Tyr Tyr Phe Asp Tyr
   1               5                   10
<210> 50
   <211> 10
   <212> PRT
   <213> Artificial
<220>
   <223> CDR3
<400> 50
   Gly Asp Tyr Thr Gly Tyr Gly Met Asp Tyr
   1               5                   10
<210> 51
   <211> 10
   <212> PRT
   <213> Artificial
<220>
   <223> CDR3
<400> 51
   Gly Gly Tyr Asp Gly Tyr Trp Phe Pro Tyr
   1 5 10
<210> 52
   <211> 15
   <212> PRT
   <213> Artificial
<220>
   <223> epitope IGKV1-39
<400> 52
   Asp Ile Gln Met Thr Gln Ser Pro Ser Ser Leu Ser Ala Ser Val
   1 5 10 15
<210> 53
   <211> 15
   <212> PRT
   <213> Artificial
<220>
   <223> epitope IGKV1-39
<400> 53
   Met Thr Gln Ser Pro Ser Ser Leu Ser Ala Ser Val Gly Asp Arg
   1 5 10 15
<210> 54
   <211> 15
   <212> PRT
   <213> Artificial
<220>
   <223> epitope IGKV1-39
<400> 54
   Ser Pro Ser Ser Leu Ser Ala Ser Val Gly Asp Arg Val Thr Ile
   1 5 10 15
<210> 55
   <211> 15
   <212> PRT
   <213> Artificial
<220>
   <223> epitope IGKV1-39
<400> 55
   Ser Leu Ser Ala Ser Val Gly Asp Arg Val Thr Ile Thr Cys Arg
   1 5 10 15
<210> 56
   <211> 15
   <212> PRT
   <213> Artificial
<220>
   <223> Epitope IGKV1-39
<400> 56
   Ala Ser Val Gly Asp Arg Val Thr Ile Thr Cys Arg Ala Ser Gln
   1 5 10 15
<210> 57
   <211> 15
   <212> PRT
   <213> Artificial
<220>
   <223> Epitope IGKV1-39
<400> 57
   Gly Asp Arg Val Thr Ile Thr Cys Arg Ala Ser Gln Ser Ile Ser
   1 5 10 15
<210> 58
   <211> 15
   <212> PRT
   <213> Artificial
<220>
   <223> Epitope IGKV1-39
<400> 58
   Val Thr Ile Thr Cys Arg Ala Ser Gln Ser Ile Ser Ser Tyr Leu
   1 5 10 15
<210> 59
   <211> 15
   <212> PRT
   <213> Artificial
<220>
   <223> Epitope IGKV1-39
<400> 59
   Thr Cys Arg Ala Ser Gln Ser Ile Ser Ser Tyr Leu Asn Trp Tyr
   1 5 10 15
<210> 60
   <211> 15
   <212> PRT
   <213> Artificial
<220>
   <223> Epitope IGKV1-39
<400> 60
   Ala Ser Gln Ser Ile Ser Ser Tyr Leu Asn Trp Tyr Gln Gln Lys
   1               5                   10                  15
<210> 61
   <211> 15
   <212> PRT
   <213> Artificial
<220>
   <223> Epitope IGKV1-39
<400> 61
   Ser Ile Ser Ser Tyr Leu Asn Trp Tyr Gln Gln Lys Pro Gly Lys
   1               5                   10                  15
<210> 62
   <211> 15
   <212> PRT
   <213> Artificial
<220>
   <223> Epitope IGKV1-39
<400> 62
   Ser Tyr Leu Asn Trp Tyr Gln Gln Lys Pro Gly Lys Ala Pro Lys
   1               5                   10                  15
<210> 63
   <211> 15
   <212> PRT
   <213> Artificial
<220>
   <223> Epitope IGKV1-39
<400> 63
   Asn Trp Tyr Gln Gln Lys Pro Gly Lys Ala Pro Lys Leu Leu Ile
   1               5                   10                  15
<210> 64
   <211> 15
   <212> PRT
   <213> Artificial
<220>
   <223> Epitope IGKV1-39
<400> 64
   Gln Gln Lys Pro Gly Lys Ala Pro Lys Leu Leu Ile Tyr Ala Ala
   1 5 10 15
<210> 65
   <211> 15
   <212> PRT
   <213> Artificial
<220>
   <223> Epitope IGKV1-39
<400> 65
   Pro Gly Lys Ala Pro Lys Leu Leu Ile Tyr Ala Ala Ser Ser Leu
   1 5 10 15
<210> 66
   <211> 15
   <212> PRT
   <213> Artificial
<220>
   <223> Epitope IGKV1-39
<400> 66
   Ala Pro Lys Leu Leu Ile Tyr Ala Ala Ser Ser Leu Gln Ser Gly
   1 5 10 15
<210> 67
   <211> 15
   <212> PRT
   <213> Artificial
<220>
   <223> Epitope IGKV1-39
<400> 67
   Leu Leu Ile Tyr Ala Ala Ser Ser Leu Gln Ser Gly Val Pro Ser
   1 5 10 15
<210> 68
   <211> 15
   <212> PRT
   <213> Artificial
<220>
   <223> Epitope IGKV1-39
<400> 68
   Tyr Ala Ala Ser Ser Leu Gln Ser Gly Val Pro Ser Arg Phe Ser
   1 5 10 15
<210> 69
   <211> 15
   <212> PRT
   <213> Artificial
<220>
   <223> Epitope IGKV1-39
<400> 69
   Ser Ser Leu Gln Ser Gly Val Pro Ser Arg Phe Ser Gly Ser Gly
   1 5 10 15
<210> 70
   <211> 15
   <212> PRT
   <213> Artificial
<220>
   <223> Epitope IGKV1-39
<400> 70
   Gln Ser Gly Val Pro Ser Arg Phe Ser Gly Ser Gly Ser Gly Thr
   1 5 10 15
<210> 71
   <211> 15
   <212> PRT
   <213> Artificial
<220>
   <223> Epitope IGKV1-39
<400> 71
   Val Pro Ser Arg Phe Ser Gly Ser Gly Ser Gly Thr Asp Phe Thr
   1 5 10 15
<210> 72
   <211> 15
   <212> PRT
   <213> Artificial
<220>
   <223> Epitope IGKV1-39
<400> 72
   Arg Phe Ser Gly Ser Gly Ser Gly Thr Asp Phe Thr Leu Thr Ile
   1 5 10 15
<210> 73
   <211> 15
   <212> PRT
   <213> Artificial
<220>
   <223> Epitope IGKV1-39
<400> 73
   Gly Ser Gly Ser Gly Thr Asp Phe Thr Leu Thr Ile Ser Ser Leu
   1 5 10 15
<210> 74
   <211> 15
   <212> PRT
   <213> Artificial
<220>
   <223> Epitope IGKV1-39
<400> 74
   Ser Gly Thr Asp Phe Thr Leu Thr Ile Ser Ser Leu Gln Pro Glu
   1 5 10 15
<210> 75
   <211> 15
   <212> PRT
   <213> Artificial
<220>
   <223> Epitope IGKV1-39
<400> 75
   Asp Phe Thr Leu Thr Ile Ser Ser Leu Gln Pro Glu Asp Phe Ala
   1 5 10 15
<210> 76
   <211> 15
   <212> PRT
   <213> Artificial
<220>
   <223> Epitope IGKV1-39
<400> 76
   Leu Thr Ile Ser Ser Leu Gln Pro Glu Asp Phe Ala Thr Tyr Tyr
   1 5 10 15
<210> 77
   <211> 15
   <212> PRT
   <213> Artificial
<220>
   <223> Epitope IGKV1-39
<400> 77
   Ser Ser Leu Gln Pro Glu Asp Phe Ala Thr Tyr Tyr Cys Gln Gln
   1 5 10 15
<210> 78
   <211> 15
   <212> PRT
   <213> Artificial
<220>
   <223> Epitope IGKV1-39
<400> 78
   Gln Pro Glu Asp Phe Ala Thr Tyr Tyr Cys Gln Gln Ser Tyr Ser
   1               5                   10                  15
<210> 79
   <211> 15
   <212> PRT
   <213> Artificial
<220>
   <223> Epitope IGKV1-39
<400> 79
   Asp Phe Ala Thr Tyr Tyr Cys Gln Gln Ser Tyr Ser Thr Pro Pro
   1               5                   10                  15
<210> 80
   <211> 15
   <212> PRT
   <213> Artificial
<220>
   <223> Epitope IGKV1-39
<400> 80
   Thr Tyr Tyr Cys Gln Gln Ser Tyr Ser Thr Pro Pro Thr Phe Gly
   1               5                   10                  15
<210> 81
   <211> 15
   <212> PRT
   <213> Artificial
<220>
   <223> Epitope IGKV1-39
<400> 81
   Cys Gln Gln Ser Tyr Ser Thr Pro Pro Thr Phe Gly Gln Gly Thr
   1               5                   10                  15
<210> 82
   <211> 15
   <212> PRT
   <213> Artificial
<220>
   <223> Epitope IGKV1-39
<400> 82
   Ser Tyr Ser Thr Pro Pro Thr Phe Gly Gln Gly Thr Lys Val Glu
   1               5                   10                  15
<210> 83
   <211> 14
   <212> PRT
   <213> Artificial
<220>
   <223> Epitope IGKV1-39
<400> 83
   Thr Pro Pro Thr Phe Gly Gln Gly Thr Lys Val Glu Ile Lys
   1               5                   10
<210> 84
   <211> 321
   <212> DNA
   <213> Homo sapiens
<220>
   <221> CDS
   <222> (1)..(321)
<400> 84
   gac atc cag atg acc cag agc ccc agc agc ctg agc gcc agc gtg ggc       48
   Asp Ile Gln Met Thr Gln Ser Pro Ser Ser Leu Ser Ala Ser Val Gly
   1               5                   10                  15
   gac aga gtg acc atc acc tgc aga gcc agc cag agc atc agc agc tac       96
   Asp Arg Val Thr Ile Thr Cys Arg Ala Ser Gln Ser Ile Ser Ser Tyr
               20                  25                  30
   ctg aac tgg tat cag cag aag ccc ggc aag gcc ccc aag ctg ctg atc      144
   Leu Asn Trp Tyr Gln Gln Lys Pro Gly Lys Ala Pro Lys Leu Leu Ile
           35                  40                  45
   tac gcc gcc agc tcc ctg cag agc ggc gtg ccc agc aga ttc agc ggc      192
   Tyr Ala Ala Ser Ser Leu Gln Ser Gly Val Pro Ser Arg Phe Ser Gly
       50                  55                  60
   agc ggc tcc ggc acc gac ttc acc ctg acc atc agc agc ctg cag ccc      240
   Ser Gly Ser Gly Thr Asp Phe Thr Leu Thr Ile Ser Ser Leu Gln Pro
   65                  70                  75                  80
   gag gac ttc gcc acc tac tac tgc cag cag agc tac agc acc ccc ccc      288
   Glu Asp Phe Ala Thr Tyr Tyr Cys Gln Gln Ser Tyr Ser Thr Pro Pro
                   85                  90                  95
   acc ttc ggc cag ggc acc aag gtg gag atc aag                          321
   Thr Phe Gly Gln Gly Thr Lys Val Glu Ile Lys
               100                 105
<210> 85
   <211> 107
   <212> PRT
   <213> Homo sapiens
<400> 85
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Pro
                   85                  90                  95
   Thr Phe Gly Gln Gly Thr Lys Val Glu Ile Lys
               100                 105
<210> 86
   <211> 330
   <212> DNA
   <213> Homo sapiens
<220>
   <221> CDS
   <222> (1)..(330)
<400> 86
   cag tct gcc ctg acc cag ccc gcc tct gtg tct ggc agc cct ggc cag        48
   Gln Ser Ala Leu Thr Gln Pro Ala Ser Val Ser Gly Ser Pro Gly Gln
   1               5                   10                  15
   agc atc acc atc agc tgc acc ggc acc agc agc gac gtg ggc ggc tac        96
   Ser Ile Thr Ile Ser Cys Thr Gly Thr Ser Ser Asp Val Gly Gly Tyr
               20                  25                  30
   aac tac gtg tcc tgg tat cag cag cac ccc ggc aag gcc ccc aag ctg       144
   Asn Tyr Val Ser Trp Tyr Gln Gln His Pro Gly Lys Ala Pro Lys Leu
           35                  40                  45
   atg atc tac gag gtg tcc aac aga ccc agc ggc gtg agc aac aga ttc       192
   Met Ile Tyr Glu Val Ser Asn Arg Pro Ser Gly Val Ser Asn Arg Phe
       50                  55                  60
   agc ggc agc aag agc ggc aac acc gcc agc ctg acc atc agc ggc ctc       240
   Ser Gly Ser Lys Ser Gly Asn Thr Ala Ser Leu Thr Ile Ser Gly Leu
   65                  70                  75                      80
   cag gct gag gac gag gcc gac tac tac tgc agc agc tac acc agc agc       288
   Gln Ala Glu Asp Glu Ala Asp Tyr Tyr Cys Ser Ser Tyr Thr Ser Ser
                   85                  90                  95
   tcc acc ctg gtg ttt ggc ggc gga aca aag ctg acc gtg ctg               330
   Ser Thr Leu Val Phe Gly Gly Gly Thr Lys Leu Thr Val Leu
               100                 105                 110
<210> 87
   <211> 110
   <212> PRT
   <213> Homo sapiens
<400> 87
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Glu Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                      80
   Gln Ala Glu Asp Glu Ala Asp Tyr Tyr Cys Ser Ser Tyr Thr Ser Ser
                   85                  90                  95
   Ser Thr Leu Val Phe Gly Gly Gly Thr Lys Leu Thr Val Leu
               100                 105                 110
<210> 88
   <211> 321
   <212> DNA
   <213> Rattus norvegicus
<220>
   <221> CDS
   <222> (1)..(321)
<400> 88
   aga gcc gac gcc gct ccc acc gtg tcc atc ttc ccc ccc agc atg gaa       48
   Arg Ala Asp Ala Ala Pro Thr Val Ser Ile Phe Pro Pro Ser Met Glu
   1               5                   10                  15
   cag ctg acc tct ggc gga gcc acc gtg gtc tgc ttc gtg aac aac ttc       96
   Gln Leu Thr Ser Gly Gly Ala Thr Val Val Cys Phe Val Asn Asn Phe
               20                  25                  30
   tac ccc aga gac atc agc gtg aag tgg aag atc gac ggc agc gag cag      144
   Tyr Pro Arg Asp Ile Ser Val Lys Trp Lys Ile Asp Gly Ser Glu Gln
           35                  40                  45
   agg gac ggc gtg ctg gac agc gtg acc gac cag gac agc aag gac tcc      192
   Arg Asp Gly Val Leu Asp Ser Val Thr Asp Gln Asp Ser Lys Asp Ser
       50                  55                  60
   acc tac agc atg agc agc acc ctg agc ctg acc aag gtg gag tac gag      240
   Thr Tyr Ser Met Ser Ser Thr Leu Ser Leu Thr Lys Val Glu Tyr Glu
   65                  70                  75                  80
   agg cac aac ctg tac acc tgc gag gtg gtg cac aag acc agc tcc agc      288
   Arg His Asn Leu Tyr Thr Cys Glu Val Val His Lys Thr Ser Ser Ser
                   85                  90                  95
   ccc gtg gtc aag tcc ttc aac cgg aac gag tgt                          321
   Pro Val Val Lys Ser Phe Asn Arg Asn Glu Cys
               100                 105
<210> 89
   <211> 107
   <212> PRT
   <213> Rattus norvegicus
<400> 89
   Arg Ala Asp Ala Ala Pro Thr Val Ser Ile Phe Pro Pro Ser Met Glu
   1               5                   10                  15
   Gln Leu Thr Ser Gly Gly Ala Thr Val Val Cys Phe Val Asn Asn Phe
               20                  25                  30
   Tyr Pro Arg Asp Ile Ser Val Lys Trp Lys Ile Asp Gly Ser Glu Gln
           35                  40                  45
   Arg Asp Gly Val Leu Asp Ser Val Thr Asp Gln Asp Ser Lys Asp Ser
       50                  55                  60
   Thr Tyr Ser Met Ser Ser Thr Leu Ser Leu Thr Lys Val Glu Tyr Glu
   65                  70                  75                  80
   Arg His Asn Leu Tyr Thr Cys Glu Val Val His Lys Thr Ser Ser Ser
                   85                  90                  95
   Pro Val Val Lys Ser Phe Asn Arg Asn Glu Cys
               100                 105
<210> 90
   <211> 865
   <212> DNA
   <213> Artificial
<220>
   <223> IGKV1-39/J-Ck
<400> 90
   ggtaccgcgg ccgccaccat ggacatgaga gtgcccgccc agctcctggg gctcctgcta      60
   ctctggctcc gaggtaagga tggagaacac taggaattta ctcagccagt gtgctcagta     120
   ctgactggaa cttcagggaa gttctctgat aacatgatta atagtaagaa tatttgtttt     180
   tatgtttcca atctcaggtg ccagatgtga catccagatg acccagagcc ccagcagcct     240
   gagcgccagc gtgggcgaca gagtgaccat cacctgcaga gccagccaga gcatcagcag     300
   ctacctgaac tggtatcagc agaagcccgg caaggccccc aagctgctga tctacgccgc     360
   cagctccctg cagagcggcg tgcccagcag attcagcggc agcggctccg gcaccgactt     420
   caccctgacc atcagcagcc tgcagcccga ggacttcgcc acctactact gccagcagag     480
   ctacagcacc ccccccacct tcggccaggg caccaaggtg gagatcaaga gagccgacgc     540
   cgctcccacc gtgtccatct tcccccccag catggaacag ctgacctctg gcggagccac     600
   cgtggtctgc ttcgtgaaca acttctaccc cagagacatc agcgtgaagt ggaagatcga     660
   cggcagcgag cagagggacg gcgtgctgga cagcgtgacc gaccaggaca gcaaggactc     720
   cacctacagc atgagcagca ccctgagcct gaccaaggtg gagtacgaga ggcacaacct     780
   gtacacctgc gaggtggtgc acaagaccag ctccagcccc gtggtcaagt ccttcaaccg     840
   gaacgagtgt tgagctagcg agctc                                           865
<210> 91
   <211> 874
   <212> DNA
   <213> Artificial
<220>
   <223> IGLV2-14/J-Ck
<400> 91
   ggtaccgcgg ccgccaccat ggacatgaga gtgcccgccc agctcctggg gctcctgcta      60
   ctctggctcc gaggtaagga tggagaacac taggaattta ctcagccagt gtgctcagta     120
   ctgactggaa cttcagggaa gttctctgat aacatgatta atagtaagaa tatttgtttt     180
   tatgtttcca atctcaggtg ccagatgtca gtctgccctg acccagcccg cctctgtgtc     240
   tggcagccct ggccagagca tcaccatcag ctgcaccggc accagcagcg acgtgggcgg     300
   ctacaactac gtgtcctggt atcagcagca ccccggcaag gcccccaagc tgatgatcta     360
   cgaggtgtcc aacagaccca gcggcgtgag caacagattc agcggcagca agagcggcaa     420
   caccgccagc ctgaccatca gcggcctcca ggctgaggac gaggccgact actactgcag     480
   cagctacacc agcagctcca ccctggtgtt tggcggcgga acaaagctga ccgtgctgag     540
   agccgacgcc gctcccaccg tgtccatctt cccccccagc atggaacagc tgacctctgg     600
   cggagccacc gtggtctgct tcgtgaacaa cttctacccc agagacatca gcgtgaagtg     660
   gaagatcgac ggcagcgagc agagggacgg cgtgctggac agcgtgaccg accaggacag     720
   caaggactcc acctacagca tgagcagcac cctgagcctg accaaggtgg agtacgagag     780
   gcacaacctg tacacctgcg aggtggtgca caagaccagc tccagccccg tggtcaagtc     840
   cttcaaccgg aacgagtgtt gagctagcga gctc                                 874
<210> 92
   <211> 13373
   <212> DNA
   <213> Artificial
<220>
   <223> VkP-IGKV1-39/J-Ck
<400> 92
   ggccggccca catgaaacaa tgggaaccat gtgacaatca cagaggtgtt gttactatag     60
   caaaagggat tgttactctc cacatccctt taagtaactt gaaggcctga tagacccacc    120
   ctctaagact tcattagaca ttccctacga atggttatac tctcctgtat actcccaata    180
   caactctaaa atatattatt ccatatagtc cttaggtttg tattaaagtt tgactttttt    240
   ccttcaaaat atctcttgtc acaacagcgg ctctagagag aaatacattc cctccaggca    300
   aatctatgct gcgctggtct gacctgggac cctggggaca ttgcccctgt gctgagttac    360
   taagatgagc cagccctgca gctgtgctca gcctgcccca tgccctgctg attgatttgc    420
   atgttccaga gcacagcccc ctgccctgaa gactttttta tgggctggtc gcaccctgtg    480
   caggagtcag tctcagtcag gagccaccat ggacatgaga gtgcccgccc agctcctggg    540
   gctcctgcta ctctggctcc gaggtaagga tggagaacac taggaattta ctcagccagt 600
   gtgctcagta ctgactggaa cttcagggaa gttctctgat aacatgatta atagtaagaa 660
   tatttgtttt tatgtttcca atctcaggtg ccagatgtga catccagatg acccagagcc 720
   ccagcagcct gagcgccagc gtgggcgaca gagtgaccat cacctgcaga gccagccaga 780
   gcatcagcag ctacctgaac tggtatcagc agaagcccgg caaggccccc aagctgctga 840
   tctacgccgc cagctccctg cagagcggcg tgcccagcag attcagcggc agcggctccg 900
   gcaccgactt caccctgacc atcagcagcc tgcagcccga ggacttcgcc acctactact 960
   gccagcagag ctacagcacc ccccccacct tcggccaggg caccaaggtg gagatcaaac 1020
   gtaagtacac ttttctcatc tttttttatg tgtaagacac aggttttcat gttaggagtt 1080
   aaagtcagtt cagaaaatct tgagaaaatg gagagggctc attatcagtt gacgtggcat 1140
   acagtgtcag attttctgtt tatcaagcta gtgagattag gggcaaaaag aggctttagt 1200
   tgagaggaaa gtaattaata ctatggtcac catccaagag attggatcgg agaataagca 1260
   tgagtagtta ttgagatctg ggtctgactg caggtagcgt ggtcttctag acgtttaagt 1320
   gggagatttg gaggggatga ggaatgaagg aacttcagga tagaaaaggg ctgaagtcaa 1380
   gttcagctcc taaaatggat gtgggagcaa actttgaaga taaactgaat gacccagagg 1440
   atgaaacagc gcagatcaaa gaggggcctg gagctctgag aagagaagga gactcatccg 1500
   tgttgagttt ccacaagtac tgtcttgagt tttgcaataa aagtgggata gcagagttga 1560
   gtgagccgta ggctgagttc tctcttttgt ctcctaagtt tttatgacta caaaaatcag 1620
   tagtatgtcc tgaaataatc attaagctgt ttgaaagtat gactgcttgc catgtagata 1680
   ccatggcttg ctgaataatc agaagaggtg tgactcttat tctaaaattt gtcacaaaat 1740
   gtcaaaatga gagactctgt aggaacgagt ccttgacaga cagctcaagg ggtttttttc 1800
   ctttgtctca tttctacatg aaagtaaatt tgaaatgatc tttttattta taagagtaga 1860
   aatacagttg ggtttgaact atatgtttta atggccacgg ttttgtaaga catttggtcc 1920
   tttgttttcc cagttattac tcgattgtaa ttttatatcg ccagcaatgg actgaaacgg 1980
   tccgcaacct cttctttaca actgggtgac ctcgcggctg tgccagccat ttggcgttca 2040
   ccctgccgct aagggccatg tgaacccccg cggtagcatc ccttgctccg cgtggaccac 2100
   tttcctgagg cacagtgata ggaacagagc cactaatctg aagagaacag agatgtgaca 2160
   gactacacta atgtgagaaa aacaaggaaa gggtgactta ttggagattt cagaaataaa 2220
   atgcatttat tattatattc ccttatttta attttctatt agggaattag aaagggcata 2280
   aactgcttta tccagtgtta tattaaaagc ttaatgtata taatctttta gaggtaaaat 2340
   ctacagccag caaaagtcat ggtaaatatt ctttgactga actctcacta aactcctcta 2400
   aattatatgt catattaact ggttaaatta atataaattt gtgacatgac cttaactggt 2460
   taggtaggat atttttcttc atgcaaaaat atgactaata ataatttagc acaaaaatat 2520
   ttcccaatac tttaattctg tgatagaaaa atgtttaact cagctactat aatcccataa 2580
   ttttgaaaac tatttattag cttttgtgtt tgacccttcc ctagccaaag gcaactattt 2640
   aaggaccctt taaaactctt gaaactactt tagagtcatt aagttattta accactttta 2700
   attactttaa aatgatgtca attccctttt aactattaat ttattttaag gggggaaagg 2760
   ctgctcataa ttctattgtt tttcttggta aagaactctc agttttcgtt tttactacct 2820
   ctgtcaccca agagttggca tctcaacaga ggggactttc cgagaggcca tctggcagtt 2880
   gcttaagatc agaagtgaag tctgccagtt cctcccaggc aggtggccca gattacagtt 2940
   gacctgttct ggtgtggcta aaaattgtcc catgtggtta caaaccatta gaccagggtc 3000
   tgatgaattg ctcagaatat ttctggacac ccaaatacag accctggctt aaggccctgt 3060
   ccatacagta ggtttagctt ggctacacca aaggaagcca tacagaggct aatatcagag 3120
   tattcttgga agagacagga gaaaatgaaa gccagtttct gctcttacct tatgtgcttg 3180
   tgttcagact cccaaacatc aggagtgtca gataaactgg tctgaatctc tgtctgaagc 3240
   atggaactga aaagaatgta gtttcaggga agaaaggcaa tagaaggaag cctgagaata 3300
   tcttcaaagg gtcagactca atttactttc taaagaagta gctaggaact agggaataac 3360
   ttagaaacaa caagattgta tatatgtgca tcctggcccc attgttcctt atctgtaggg 3420
   ataagcgtgc ttttttgtgt gtctgtatat aacataactg tttacacata atacactgaa 3480
   atggagccct tccttgttac ttcataccat cctctgtgct tccttcctca ggggccgacg 3540
   ccgctcccac cgtgtccatc ttccccccca gcatggaaca gctgacctct ggcggagcca 3600
   ccgtggtctg cttcgtgaac aacttctacc ccagagacat cagcgtgaag tggaagatcg 3660
   acggcagcga gcagagggac ggcgtgctgg acagcgtgac cgaccaggac agcaaggact 3720
   ccacctacag catgagcagc accctgagcc tgaccaaggt ggagtacgag aggcacaacc 3780
   tgtacacctg cgaggtggtg cacaagacca gctccagccc cgtggtcaag tccttcaacc 3840
   ggaacgagtg ttgaagacaa aggtcctgag acgccaccac cagctcccca gctccatcct 3900
   atcttccctt ctaaggtctt ggaggcttcc ccacaagcga cctaccactg ttgcggtgct 3960
   ccaaacctcc tccccacctc cttctcctcc tcctcccttt ccttggcttt tatcatgcta 4020
   atatttgcag aaaatattca ataaagtgag tctttgcact tgagatctct gtctttctta 4080
   ctaaatggta gtaatcagtt gtttttccag ttacctgggt ttctcttcta aagaagttaa 4140
   atgtttagtt gccctgaaat ccaccacact taaaggataa ataaaaccct ccacttgccc 4200
   tggttggctg tccactacat ggcagtcctt tctaaggttc acgagtacta ttcatggctt 4260
   atttctctgg gccatggtag gtttgaggag gcatacttcc tagttttctt cccctaagtc 4320
   gtcaaagtcc tgaaggggga cagtctttac aagcacatgt tctgtaatct gattcaacct 4380
   acccagtaaa cttggcgaag caaagtagaa tcattatcac aggaagcaaa ggcaacctaa 4440
   atgtgcaagc aataggaaaa tgtggaagcc catcatagta cttggacttc atctgctttt 4500
   gtgccttcac taagttttta aacatgagct ggctcctatc tgccattggc aaggctgggc 4560
   actacccaca acctacttca aggacctcta taccgtgaga ttacacacat acatcaaaat 4620
   ttgggaaaag ttctaccaag ctgagagctg atcaccccac tcttaggtgc ttatctctgt 4680
   acaccagaaa ccttaagaag caaccagtat tgagagactc atttatgaaa gtctaaaact 4740
   ggatacaacc aaaatgtcca ccaacagtta aattatgaca tgttcacaat tgagctatta 4800
   cttaataagg agaattaata aaataaaact taagagcata gtttaatctc ataaacaaga 4860
   taataagcaa aacaaaacat tttttcatcc atgtaagttt aaaagcaggt aaaatttaaa 4920
   attaagagag acataagttt tgaggtagca agatggaaac tctggggctt ggggaatgtt 4980
   ctgtctctct gtatgggatg tgaaagttac tattgtggaa ttgggatcta tgttcttcct 5040
   gtatatattg tatacttcat aataacttca cctaaagaaa tatctaatac ccagtgcata 5100
   cataaaagag gatacaagga atgaatcata cgtcaaggcc agaaagacaa taaagtaggg 5160
   gatccaggat caaatctccc acaaccttga gccttctact attctgcctt ccagagctca 5220
   aagtacaaaa cacataattc aaacacatga tccctccttg gggtctcttc cttcatgcat 5280
   cgaattagaa atagccatgt ataaaatgag atagaagaga ccttcatcaa caggtcaaag 5340
   aatataggta attttgtctg ggtatgaaga gcccacgtat caaaggttac attagggaag 5400
   gaagaggaca ctaacagtga ctttcattct ccccctcttc ctggaggccc ctgcatttag 5460
   tccctcgtgg gctcatccac tcagcacaca tttactaagc atcttctcag cctacactct 5520
   gaaggcagtg cagaataatg ttagtgtccc ttcccccagt taatatgcag tccagtttcc 5580
   ctgctccttc cctttctcag tccacataag gatgatggga aaggacagtc accaaatagg 5640
   agagggcaac cctttgcctt cctacctctt gagaatgtac attattatcc actttttgaa 5700
   acttctttta attgcttttt tttaatttgt cttttcaaat agcataacct tgttcatcca 5760
   tttctgggaa ccaaatttat caatcaacag tgcctctaat ctggctatta atacaaaaat 5820
   gcctcctcaa aatatatatg ttcgagtctt atctaaaaca gaacccacaa taaaaaagaa 5880
   gaaagaatac atataagcat ttatataatt ctgagcaacc ttgtgctttg tgaaaaaaat 5940
   ataatctaat gtcacatgct gtattctttt tatttaacac tggtgaaatt ataccattag 6000
   agagaaagag gacagatcac tgatcctagg atctagggat gttacagata agaaaacaaa 6060
   tgtgacaaag agctgtcaca aggaggatct tcaaggtcac agaatcactg tcttgatttc 6120
   agtggtggtt acatacattt aaatatgtga taaaatgttg ttgaactata ttcatatatt 6180
   gtaccaatgt caaatgctta attttggctc tatagtataa ttatgcacta aataactatt 6240
   tggacaaaga aaatgatgtt tacatcaaag gtgaggccat atttgttagg aacataactt 6300
   aaaaaccatt ttggataact aatgaaaagc cattttgtgt gccttggcat atcatgccta 6360
   agctgtcacc agatagatct aataagacct aagcctcaga agcaagcccc tgcccagcaa 6420
   gcaggcagca cagataagag ctaaacccag gacaggccat gatatgctaa tgaactacct 6480
   tcaaggtggt gttgctgacc tagtgaacca gccccaagct gtgagcccca atagcacaaa 6540
   gctactgccc aaagaaatta tacaaaaatt ggaactttgg gaatggtgtg caggatcgct 6600
   ctgctgtatg cctggaacac agcttctcta tgttttgtat tgataccagt ctagaagctt 6660
   ccaaaacttt ctcactgaag aagattcccc atgtgggacc cctacagact cttttgccca 6720
   aacaactgct tccctcctgg tgtgatatct gttttgcttt tatgttagca taatattata 6780
   aggaatgttt gtgtgaataa accaaacata ttttaaaagc aaatattgta tgcacatcct 6840
   aattgctaaa aagtttacag ctaatagtcc catgctctcc acaatactgg atccaaataa 6900
   gtcctaattt caatgttggg catctttaca gagagaaaga cattaaaaat gaagagacat 6960
   gcagagagtg caccatgcca tcgtggagac agactgaagt gacacaactg ttagtcaaag 7020
   aggattaagg acttccagaa gccaccaaag gaaggaggta tgaagtggtt tctccctcag 7080
   agtatccaga ggagactaaa ccaaccaaca cctttttgct taagacttct tgccttcagg 7140
   actgtgagaa ggtagcttcc tattgttcta agccccagta tgtggcattt tgttaaggta 7200
   gagtcaagaa accaataaaa tgcagacaga caaaaggata gctgagtttt ccaggccctt 7260
   ccttcttatt tttggttttg ttggtggtgg tggtggtggt ggtgatggtg gtggttttgt 7320
   ttatgttttg tttggggagt tttttggggt ttttttgggt tttgtttttg ttgttgtttt 7380
   gggggttttt gttgttgttg ttgtttgctt ttttgttttt tgttttttgt ttttttgaga 7440
   cagtgtttct ctgtatagcc ctggctgtcc tggagttcct tctatctcta atgtctacat 7500
   ctcagagggg atcctctaat ttcaaatgag cagtagctct ccatttttag ctcttattta 7560
   ttcatttatt tacttactta cttattgtct gtagatgaaa gaattttgga gtgggaaagg 7620
   gttcatgagc ccccagcaac taatgaggag ctacagacaa ttgatgtttc tggggaaagg 7680
   agactcagtt tctttgagag tatagcttct gatgggtcaa ccatgttcct gtggctgatg 7740
   tcacacccag gagtatgcag acaacagaaa ctggagttaa tgagttgttt taaaaataaa 7800
   aaagggcatg aagcttggga tagaaattaa ggataaatac aattaaatac aggaaattct 7860
   gaaagaatta ataaaaacat ttcttttttt aaaaaaaaat ccagaattag ctatgcttct 7920
   tcaaaattgc ttctggagaa ctttacaagt taaataagtt atattgtaga aaaggtagag 7980
   aggagaatag tggaagagag agataaggag acttcaaaag gagtggaggg agatagagga 8040
   ggagaaagca gaagcaatgg ctgatagaca caggataaga gggaacagaa aggagaaaga 8100
   ggaagccagg atgggtattt ctttgcctat ctgtgacttg cacatggtct tggcaattat 8160
   tgatgagttc aaggcttaat tcttcacttg tgccaactca acagagtctt tctttcttat 8220
   aaccaggccc ccagtatgct catgtatgta tcaggtcctc ttatctcctt atagcaatcc 8280
   tgtttataac tgggtaactt tgtgaaggga aggaagtgca cactgagatg tgctacaact 8340
   ttttaataca aaattttgaa gagtttgtac aatgtatgta taattaataa ttaatattat 8400
   gcactttaga ttttgatttc aactcaagat actaattcta tatatatggg ttaaatcaat 8460
   atattaataa gtttaatttc acatgcttat ttttattgtg gttttcgaga cagggtttct 8520
   ctgtatagcc ctggctgtcc tggaacccac tttgtagacc aggctggcct caaactcaga 8580
   aacctacctg cctctgcctc tgcctctgcc tctgcctctg cctctgcctc tgcctctgcc 8640
   tctgcctctg cctctgcctc tgcctctgcc tctgcctctg cctctgcctc tgcctctgcc 8700
   tctgcctctg cctctgcctc tgcctctgcc tctgcctctg cctctgcctc tgcctctgcc 8760
   tctgcctagt gctggaatta aaggtttgcg ccaccacgcc cggtgaaatt tttaaacttt 8820
   atatatgtct cattctattt ctatcagata ggactgtgta gactgtgcta aactaataaa 8880
   tgtgccctca aaagtaatcg caagttgtat tgttgttgtt ttgctttgct ttgctttgct 8940
   ttgctttgct ttgctttgct ttgctttgct ttgctttgct ttgctttgct ttgctttgct 9000
   ttgctttgct ttgctttgct ttgctttttt gttttgggtt tttttccggg ggagggaggg 9060
   tggagaaaga atcttactat gaagctctga ctgtcctggg aactcactat atagatcagg 9120
   cttgattcaa ctcatagaga tctgccttct tctgcctccc aagtgctggg aataaaggca 9180
   tacacctcca tgcccagata gtgatcccaa gttttagcaa aagtttctag acttgacatt 9240
   aatcgatgga gatagacatg aattacacaa agaactaatg tggagtttac ctgaatcata 9300
   ctctatactt tatcagagat taaattaaca tttaataatc cagtgccagg ctagaggcac 9360
   cattcaatgg cagtgtttgc catcatgcat aggcttagtc ttcagtgctg aaaggcattg 9420
   ggggcaatat tactcattat acagatgaga aactgggaaa gacttgcctc agattctcta 9480
   ctgaaaggct gagtttgtgg cttctagaaa atcttttact ttcaatattt ttaatgtata 9540
   atttttttat ttccactgat tttatttttt atttttaaca tttataagaa ataaatgcaa 9600
   taaaccaaat acatggacaa aaaaatacaa gaatcatatg atcacctcaa tggaaggaaa 9660
   aaaaaagaaa gaaaaagtct ttgataagat tcaacattca ttcttttttt attagatatt 9720
   ttcttcattt acatttcaaa tgctatcccc aaagccccct ataccttccc ctgccctgct 9780
   ccccaaccca cccactcctg ctttctggcc ctggcattcc tctgtactga ggcatatgat 9840
   cttcaaaaaa ccaagggcct ctcctctcat tggtggccga ctattaggcc atcttttgct 9900
   acatatgcaa ctagagacac agctctgggg gttactggtt agttcatatt gttagtcctc 9960
   ctatagagtt gcagacccct ttagctcctt ggatactttc tctagttcct tcattagggg 10020
   ccctgtgtcc catccaatag atgactgtga gcatccactt ctgtatttgc caggcactgg 10080
   catagcctca cgagaaagag agagctatgt caggatcctg tcagtaaaat ctttctggca 10140
   tatgcaatag tatctgggtt tggtggttgt atatgggatg gatccccaag tggagcagtc 10200
   tctgaatggt ccttccttcc atctcagctc caaactttgt ctctataact ccttccatgg 10260
   gtattttgtt ccccattcta agaaggagtg aagaatccac actttggtct tccttcttct 10320
   tgagtttcat atgttgcatc ttggatattc taagtttctg ggttaatatc cacgtatcag 10380
   tgagtgcata tcatgcgtgt tattttgtga ttagtttacc tcactcagga tgatatcctc 10440
   cagatgcatc catttgccta agaatttcat taattcactg tttttaattg ctgaatagta 10500
   ctccattgtg taaatgtacc acattttctg tatccattcc tctgttgagg ggcatctggg 10560
   ttctttccag cttctggcta ttataaataa ggctgctatg agcatagcgg agcatgtgtc 10620
   cttatcaagt tggaacatct tctaggtata tgcccaggag aggaattgct ggatcttccg 10680
   gtagtaccat caacatgcat tcttaataaa agccctagaa caaggaggac tgtaggaaac 10740
   atattccaac ataataaagg ttatgtatga caaactcatg accaatatca tcctaaatga 10800
   atgaaaccat taataagctc cattaaaatc agaggactgc ccactatccc tacttctcat 10860
   ccataatgag attgaagcat tagctggagc aataaggcaa gagaagggat acaaatggga 10920
   aaatattaag tcaaattgtt ttcaattgaa gattatatta tcttataccc aatgacctca 10980
   aattttgact agaaaaattg tagaaattat caataatttc agcaaagtgt tatgatgcac 11040
   cacatcctta ttcttctccc cagcttctgc ttgcttctct cttcttgctc ttcatccttt 11100
   ctgtccttcc atctgcctgc actcttgtct caagactgag tgcagcgtgt aactctcctg 11160
   tgactgagta tctcacaaaa cgttctacct gccaaacctg gatgagccct ttgtctttct 11220
   gaagctatga ggctctctac atagactcaa gaaggaaatg acagggagga ggtaataatg 11280
   aagtggggaa ggctgacatt agcattgctc ctgtgtggct ccttaatttc tcatacttca 11340
   cactgagatg ttattaactg tgactcatag gtgaagaagc cagagctaag gttctcatat 11400
   ttgagtgtta tagaatgagt agagcagtag ttctcaaact atgggtcatg actcctttat 11460
   gggtcaaact accctttcac acaggttgca tatcagatat cctaatttta tatacatata 11520
   tatatgcata tgtatatata tatatttcac aacagtagga aaattattta gtaatcattt 11580
   tatagttgtg ggtcatggca acatgaggaa ctgtattaaa gggttgcagc attaggaatg 11640
   ttgagaccca ctgtaataga gaatgaggct taaggcaggg ctataaagcc caatggacca 11700
   tgtgcctttt ccaacatttg ccacatggta agctctgtat agacttttta aagaacattg 11760
   gtttgtaatt ttaaatggat aagggtcttc actgtctatc acccatctat ataataaata 11820
   cataagtttt gattccacca tggattcaaa tgcaaaaatc ctcaacctaa gacatagcag 11880
   tgaaacattg atgaccaaat aggaaatcca tgtagagacc ttctatcttc tgatggctcc 11940
   acaggcacca tcttgcaaca gagttctact ttgctaccag taatgaatac agtgtctcaa 12000
   ctcctgccat tgaatcttca ggaagcccct gaaatgactt gtactacacc atttcttaaa 12060
   gacagaaaag ctaagactta gagggaataa atgtcatgcc tgagatcatg caaccaatta 12120
   agtccaactt ggcctgatca agaggcacaa ttcaaaagca atgttgttcc ttcactagct 12180
   cttgtgtatg gttgctgatt ccggaagcaa agtatcagtg aatatcccta gtgggaaaag 12240
   acttggaaat caaatgtctc atttaacaga ttaggagatg aaacggtaga ctctgtgtag 12300
   ttgtacaccc ctgtgatccc atcgctagga agactgaggc aggaagtcct cgagctcaaa 12360
   ccagcttagg ctacacagag aaactatcta aaaaataatt actaactact taataggaga 12420
   ttggatgtta agatctggtc actaagaggc agaattgaga ttcgaagcca gtattttcta 12480
   cctggtatgt tttaaattgc agtaaggatc taagtgtaga tatataataa taagattcta 12540
   ttgatctctg caacaacaga gagtgttaga tttgtttgga aaaaaatatt atcagccaac 12600
   atcttctacc atttcagtat agcacagagt acccacccat atctccccac ccatccccca 12660
   taccagactg gttattgatt ttcatggtga ctggcctgag aagattaaaa aaagtaatgc 12720
   taccttattg ggagtgtccc atggaccaag atagcaactg tcatagctac cgtcacactg 12780
   ctttgatcaa gaagaccctt tgaggaactg aaaacagaac cttaggcaca tctgttgctt 12840
   tcgctcccat cctcctccaa cagcctgggt ggtgcactcc acaccctttc aagtttccaa 12900
   agcctcatac acctgctccc taccccagca cctggccaag gctgtatcca gcactgggat 12960
   gaaaatgata ccccacctcc atcttgtttg atattactct atctcaagcc ccaggttagt 13020
   ccccagtccc aatgcttttg cacagtcaaa actcaacttg gaataatcag tatccttgaa 13080
   gagttctgat atggtcactg ggcccatata ccatgtaaga catgtggaaa agatgtttca 13140
   tggggcccag acacgttcta gaagtacctg agagtggcaa aaaatagttg tgctaaatag 13200
   tttggccatc tttaggctga gagactagga aatacagcga tggactatat cagcattgca 13260
   ggatagttgt cagtaaacac cccacaaccc ataacagaag tattctcttc tttctatatc 13320
   ccttttccat ccatgtagat ggctgtcttc atatttgttc tagacggccg gcc 13373
<210> 93
   <211> 12892
   <212> DNA
   <213> Artificial
<220>
   <223> VkP-IGKVl-39/J-Ck-deltal
<400> 93
   ggccggccca catgaaacaa tgggaaccat gtgacaatca cagaggtgtt gttactatag 60
   caaaagggat tgttactctc cacatccctt taagtaactt gaaggcctga tagacccacc 120
   ctctaagact tcattagaca ttccctacga atggttatac tctcctgtat actcccaata 180
   caactctaaa atatattatt ccatatagtc cttaggtttg tattaaagtt tgactttttt 240
   ccttcaaaat atctcttgtc acaacagcgg ctctagagag aaatacattc cctccaggca 300
   aatctatgct gcgctggtct gacctgggac cctggggaca ttgcccctgt gctgagttac 360
   taagatgagc cagccctgca gctgtgctca gcctgcccca tgccctgctg attgatttgc 420
   atgttccaga gcacagcccc ctgccctgaa gactttttta tgggctggtc gcaccctgtg 480
   caggagtcag tctcagtcag gagccaccat ggacatgaga gtgcccgccc agctcctggg 540
   gctcctgcta ctctggctcc gaggtaagga tggagaacac taggaattta ctcagccagt 600
   gtgctcagta ctgactggaa cttcagggaa gttctctgat aacatgatta atagtaagaa 660
   tatttgtttt tatgtttcca atctcaggtg ccagatgtga catccagatg acccagagcc 720
   ccagcagcct gagcgccagc gtgggcgaca gagtgaccat cacctgcaga gccagccaga 780
   gcatcagcag ctacctgaac tggtatcagc agaagcccgg caaggccccc aagctgctga 840
   tctacgccgc cagctccctg cagagcggcg tgcccagcag attcagcggc agcggctccg 900
   gcaccgactt caccctgacc atcagcagcc tgcagcccga ggacttcgcc acctactact 960
   gccagcagag ctacagcacc ccccccacct tcggccaggg caccaaggtg gagatcaaac 1020
   gtaagtacac ttttctcatc tttttttatg tgtaagacac aggttttcat gttaggagtt 1080
   aaagtcagtt cagaaaatct tgagaaaatg gagagggctc attatcagtt gacgtggcat 1140
   acagtgtcag attttctgtt tatcaagcta gtgagattag gggcaaaaag aggctttagt 1200
   tgagaggaaa gtaattaata ctatggtcac catccaagag attggatcgg agaataagca 1260
   tgagtagtta ttgagatctg ggtctgactg caggtagcgt ggtcttctag acgtttaagt 1320
   gggagatttg gaggggatga ggaatgaagg aacttcagga tagaaaaggg ctgaagtcaa 1380
   gttcagctcc taaaatggat gtgggagcaa actttgaaga taaactgaat gacccagagg 1440
   atgaaacagc gcagatcaaa gaggggcctg gagctctgag aagagaagga gactcatccg 1500
   tgttgagttt ccacaagtac tgtcttgagt tttgcaataa aagtgggata gcagagttga 1560
   gtgagccgta ggctgagttc tctcttttgt ctcctaagtt tttatgacta caaaaatcag 1620
   tagtatgtcc tgaaataatc attaagctgt ttgaaagtat gactgcttgc catgtagata 1680
   ccatggcttg ctgaataatc agaagaggtg tgactcttat tctaaaattt gtcacaaaat 1740
   gtcaaaatga gagactctgt aggaacgagt ccttgacaga cagctcaagg ggtttttttc 1800
   ctttgtctca tttctacatg aaagtaaatt tgaaatgatc tttttattta taagagtaga 1860
   aatacagttg ggtttgaact atatgtttta atggccacgg ttttgtaaga catttggtcc 1920
   tttgttttcc cagttattac tcgattgtaa ttttatatcg ccagcaatgg actgaaacgg 1980
   tccgcaacct cttctttaca actgggtgac ctcgcggctg tgccagccat ttggcgttca 2040
   ccctgccgct aagggccatg tgaacccccg cggtagcatc ccttgctccg cgtggaccac 2100
   tttcctgagg cacagtgata ggaacagagc cactaatctg aagagaacag agatgtgaca 2160
   gactacacta atgtgagaaa aacaaggaaa gggtgactta ttggagattt cagaaataaa 2220
   atgcatttat tattatattc ccttatttta attttctatt agggaattag aaagggcata 2280
   aactgcttta tccagtgtta tattaaaagc ttaatgtata taatctttta gaggtaaaat 2340
   ctacagccag caaaagtcat ggtaaatatt ctttgactga actctcacta aactcctcta 2400
   aattatatgt catattaact ggttaaatta atataaattt gtgacatgac cttaactggt 2460
   taggtaggat atttttcttc atgcaaaaat atgactaata ataatttagc acaaaaatat 2520
   ttcccaatac tttaattctg tgatagaaaa atgtttaact cagctactat aatcccataa 2580
   ttttgaaaac tatttatttg gctacaccaa aggaagccat acagaggcta atatcagagt 2640
   attcttggaa gagacaggag aaaatgaaag ccagtttctg ctcttacctt atgtgcttgt 2700
   gttcagactc ccaaacatca ggagtgtcag ataaactggt ctgaatctct gtctgaagca 2760
   tggaactgaa aagaatgtag tttcagggaa gaaaggcaat agaaggaagc ctgagaatat 2820
   cttcaaaggg tcagactcaa tttactttct aaagaagtag ctaggaacta gggaataact 2880
   tagaaacaac aagattgtat atatgtgcat cctggcccca ttgttcctta tctgtaggga 2940
   taagcgtgct tttttgtgtg tctgtatata acataactgt ttacacataa tacactgaaa 3000
   tggagccctt ccttgttact tcataccatc ctctgtgctt ccttcctcag gggccgacgc 3060
   cgctcccacc gtgtccatct tcccccccag catggaacag ctgacctctg gcggagccac 3120
   cgtggtctgc ttcgtgaaca acttctaccc cagagacatc agcgtgaagt ggaagatcga 3180
   cggcagcgag cagagggacg gcgtgctgga cagcgtgacc gaccaggaca gcaaggactc 3240
   cacctacagc atgagcagca ccctgagcct gaccaaggtg gagtacgaga ggcacaacct 3300
   gtacacctgc gaggtggtgc acaagaccag ctccagcccc gtggtcaagt ccttcaaccg 3360
   gaacgagtgt tgaagacaaa ggtcctgaga cgccaccacc agctccccag ctccatccta 3420
   tcttcccttc taaggtcttg gaggcttccc cacaagcgac ctaccactgt tgcggtgctc 3480
   caaacctcct ccccacctcc ttctcctcct cctccctttc cttggctttt atcatgctaa 3540
   tatttgcaga aaatattcaa taaagtgagt ctttgcactt gagatctctg tctttcttac 3600
   taaatggtag taatcagttg tttttccagt tacctgggtt tctcttctaa agaagttaaa 3660
   tgtttagttg ccctgaaatc caccacactt aaaggataaa taaaaccctc cacttgccct 3720
   ggttggctgt ccactacatg gcagtccttt ctaaggttca cgagtactat tcatggctta 3780
   tttctctggg ccatggtagg tttgaggagg catacttcct agttttcttc ccctaagtcg 3840
   tcaaagtcct gaagggggac agtctttaca agcacatgtt ctgtaatctg attcaaccta 3900
   cccagtaaac ttggcgaagc aaagtagaat cattatcaca ggaagcaaag gcaacctaaa 3960
   tgtgcaagca ataggaaaat gtggaagccc atcatagtac ttggacttca tctgcttttg 4020
   tgccttcact aagtttttaa acatgagctg gctcctatct gccattggca aggctgggca 4080
   ctacccacaa cctacttcaa ggacctctat accgtgagat tacacacata catcaaaatt 4140
   tgggaaaagt tctaccaagc tgagagctga tcaccccact cttaggtgct tatctctgta 4200
   caccagaaac cttaagaagc aaccagtatt gagagactca tttatgaaag tctaaaactg 4260
   gatacaacca aaatgtccac caacagttaa attatgacat gttcacaatt gagctattac 4320
   ttaataagga gaattaataa aataaaactt aagagcatag tttaatctca taaacaagat 4380
   aataagcaaa acaaaacatt ttttcatcca tgtaagttta aaagcaggta aaatttaaaa 4440
   ttaagagaga cataagtttt gaggtagcaa gatggaaact ctggggcttg gggaatgttc 4500
   tgtctctctg tatgggatgt gaaagttact attgtggaat tgggatctat gttcttcctg 4560
   tatatattgt atacttcata ataacttcac ctaaagaaat atctaatacc cagtgcatac 4620
   ataaaagagg atacaaggaa tgaatcatac gtcaaggcca gaaagacaat aaagtagggg 4680
   atccaggatc aaatctccca caaccttgag ccttctacta ttctgccttc cagagctcaa 4740
   agtacaaaac acataattca aacacatgat ccctccttgg ggtctcttcc ttcatgcatc 4800
   gaattagaaa tagccatgta taaaatgaga tagaagagac cttcatcaac aggtcaaaga 4860
   atataggtaa ttttgtctgg gtatgaagag cccacgtatc aaaggttaca ttagggaagg 4920
   aagaggacac taacagtgac tttcattctc cccctcttcc tggaggcccc tgcatttagt 4980
   ccctcgtggg ctcatccact cagcacacat ttactaagca tcttctcagc ctacactctg 5040
   aaggcagtgc agaataatgt tagtgtccct tcccccagtt aatatgcagt ccagtttccc 5100
   tgctccttcc ctttctcagt ccacataagg atgatgggaa aggacagtca ccaaatagga 5160
   gagggcaacc ctttgccttc ctacctcttg agaatgtaca ttattatcca ctttttgaaa 5220
   cttcttttaa ttgctttttt ttaatttgtc ttttcaaata gcataacctt gttcatccat 5280
   ttctgggaac caaatttatc aatcaacagt gcctctaatc tggctattaa tacaaaaatg 5340
   cctcctcaaa atatatatgt tcgagtctta tctaaaacag aacccacaat aaaaaagaag 5400
   aaagaataca tataagcatt tatataattc tgagcaacct tgtgctttgt gaaaaaaata 5460
   taatctaatg tcacatgctg tattcttttt atttaacact ggtgaaatta taccattaga 5520
   gagaaagagg acagatcact gatcctagga tctagggatg ttacagataa gaaaacaaat 5580
   gtgacaaaga gctgtcacaa ggaggatctt caaggtcaca gaatcactgt cttgatttca 5640
   gtggtggtta catacattta aatatgtgat aaaatgttgt tgaactatat tcatatattg 5700
   taccaatgtc aaatgcttaa ttttggctct atagtataat tatgcactaa ataactattt 5760
   ggacaaagaa aatgatgttt acatcaaagg tgaggccata tttgttagga acataactta 5820
   aaaaccattt tggataacta atgaaaagcc attttgtgtg ccttggcata tcatgcctaa 5880
   gctgtcacca gatagatcta ataagaccta agcctcagaa gcaagcccct gcccagcaag 5940
   caggcagcac agataagagc taaacccagg acaggccatg atatgctaat gaactacctt 6000
   caaggtggtg ttgctgacct agtgaaccag ccccaagctg tgagccccaa tagcacaaag 6060
   ctactgccca aagaaattat acaaaaattg gaactttggg aatggtgtgc aggatcgctc 6120
   tgctgtatgc ctggaacaca gcttctctat gttttgtatt gataccagtc tagaagcttc 6180
   caaaactttc tcactgaaga agattcccca tgtgggaccc ctacagactc ttttgcccaa 6240
   acaactgctt ccctcctggt gtgatatctg ttttgctttt atgttagcat aatattataa 6300
   ggaatgtttg tgtgaataaa ccaaacatat tttaaaagca aatattgtat gcacatccta 6360
   attgctaaaa agtttacagc taatagtccc atgctctcca caatactgga tccaaataag 6420
   tcctaatttc aatgttgggc atctttacag agagaaagac attaaaaatg aagagacatg 6480
   cagagagtgc accatgccat cgtggagaca gactgaagtg acacaactgt tagtcaaaga 6540
   ggattaagga cttccagaag ccaccaaagg aaggaggtat gaagtggttt ctccctcaga 6600
   gtatccagag gagactaaac caaccaacac ctttttgctt aagacttctt gccttcagga 6660
   ctgtgagaag gtagcttcct attgttctaa gccccagtat gtggcatttt gttaaggtag 6720
   agtcaagaaa ccaataaaat gcagacagac aaaaggatag ctgagttttc caggcccttc 6780
   cttcttattt ttggttttgt tggtggtggt ggtggtggtg gtgatggtgg tggttttgtt 6840
   tatgttttgt ttggggagtt ttttggggtt tttttgggtt ttgtttttgt tgttgttttg 6900
   ggggtttttg ttgttgttgt tgtttgcttt tttgtttttt gttttttgtt tttttgagac 6960
   agtgtttctc tgtatagccc tggctgtcct ggagttcctt ctatctctaa tgtctacatc 7020
   tcagagggga tcctctaatt tcaaatgagc agtagctctc catttttagc tcttatttat 7080
   tcatttattt acttacttac ttattgtctg tagatgaaag aattttggag tgggaaaggg 7140
   ttcatgagcc cccagcaact aatgaggagc tacagacaat tgatgtttct ggggaaagga 7200
   gactcagttt ctttgagagt atagcttctg atgggtcaac catgttcctg tggctgatgt 7260
   cacacccagg agtatgcaga caacagaaac tggagttaat gagttgtttt aaaaataaaa 7320
   aagggcatga agcttgggat agaaattaag gataaataca attaaataca ggaaattctg 7380
   aaagaattaa taaaaacatt tcttttttta aaaaaaaatc cagaattagc tatgcttctt 7440
   caaaattgct tctggagaac tttacaagtt aaataagtta tattgtagaa aaggtagaga 7500
   ggagaatagt ggaagagaga gataaggaga cttcaaaagg agtggaggga gatagaggag 7560
   gagaaagcag aagcaatggc tgatagacac aggataagag ggaacagaaa ggagaaagag 7620
   gaagccagga tgggtatttc tttgcctatc tgtgacttgc acatggtctt ggcaattatt 7680
   gatgagttca aggcttaatt cttcacttgt gccaactcaa cagagtcttt ctttcttata 7740
   accaggcccc cagtatgctc atgtatgtat caggtcctct tatctcctta tagcaatcct 7800
   gtttataact gggtaacttt gtgaagggaa ggaagtgcac actgagatgt gctacaactt 7860
   tttaatacaa aattttgaag agtttgtaca atgtatgtat aattaataat taatattatg 7920
   cactttagat tttgatttca actcaagata ctaattctat atatatgggt taaatcaata 7980
   tattaataag tttaatttca catgcttatt tttattgtgg ttttcgagac agggtttctc 8040
   tgtatagccc tggctgtcct ggaacccact ttgtagacca ggctggcctc aaactcagaa 8100
   acctacctgc ctctgcctct gcctctgcct ctgcctctgc ctctgcctct gcctctgcct 8160
   ctgcctctgc ctctgcctct gcctctgcct ctgcctctgc ctctgcctct gcctctgcct 8220
   ctgcctctgc ctctgcctct gcctctgcct ctgcctctgc ctctgcctct gcctctgcct 8280
   ctgcctagtg ctggaattaa aggtttgcgc caccacgccc ggtgaaattt ttaaacttta 8340
   tatatgtctc attctatttc tatcagatag gactgtgtag actgtgctaa actaataaat 8400
   gtgccctcaa aagtaatcgc aagttgtatt gttgttgttt tgctttgctt tgctttgctt 8460
   tgctttgctt tgctttgctt tgctttgctt tgctttgctt tgctttgctt tgctttgctt 8520
   tgctttgctt tgctttgctt tgcttttttg ttttgggttt ttttccgggg gagggagggt 8580
   ggagaaagaa tcttactatg aagctctgac tgtcctggga actcactata tagatcaggc 8640
   ttgattcaac tcatagagat ctgccttctt ctgcctccca agtgctggga ataaaggcat 8700
   acacctccat gcccagatag tgatcccaag ttttagcaaa agtttctaga cttgacatta 8760
   atcgatggag atagacatga attacacaaa gaactaatgt ggagtttacc tgaatcatac 8820
   tctatacttt atcagagatt aaattaacat ttaataatcc agtgccaggc tagaggcacc 8880
   attcaatggc agtgtttgcc atcatgcata ggcttagtct tcagtgctga aaggcattgg 8940
   gggcaatatt actcattata cagatgagaa actgggaaag acttgcctca gattctctac 9000
   tgaaaggctg agtttgtggc ttctagaaaa tcttttactt tcaatatttt taatgtataa 9060
   tttttttatt tccactgatt ttatttttta tttttaacat ttataagaaa taaatgcaat 9120
   aaaccaaata catggacaaa aaaatacaag aatcatatga tcacctcaat ggaaggaaaa 9180
   aaaaagaaag aaaaagtctt tgataagatt caacattcat tcttttttta ttagatattt 9240
   tcttcattta catttcaaat gctatcccca aagcccccta taccttcccc tgccctgctc 9300
   cccaacccac ccactcctgc tttctggccc tggcattcct ctgtactgag gcatatgatc 9360
   ttcaaaaaac caagggcctc tcctctcatt ggtggccgac tattaggcca tcttttgcta 9420
   catatgcaac tagagacaca gctctggggg ttactggtta gttcatattg ttagtcctcc 9480
   tatagagttg cagacccctt tagctccttg gatactttct ctagttcctt cattaggggc 9540
   cctgtgtccc atccaataga tgactgtgag catccacttc tgtatttgcc aggcactggc 9600
   atagcctcac gagaaagaga gagctatgtc aggatcctgt cagtaaaatc tttctggcat 9660
   atgcaatagt atctgggttt ggtggttgta tatgggatgg atccccaagt ggagcagtct 9720
   ctgaatggtc cttccttcca tctcagctcc aaactttgtc tctataactc cttccatggg 9780
   tattttgttc cccattctaa gaaggagtga agaatccaca ctttggtctt ccttcttctt 9840
   gagtttcata tgttgcatct tggatattct aagtttctgg gttaatatcc acgtatcagt 9900
   gagtgcatat catgcgtgtt attttgtgat tagtttacct cactcaggat gatatcctcc 9960
   agatgcatcc atttgcctaa gaatttcatt aattcactgt ttttaattgc tgaatagtac 10020
   tccattgtgt aaatgtacca cattttctgt atccattcct ctgttgaggg gcatctgggt 10080
   tctttccagc ttctggctat tataaataag gctgctatga gcatagcgga gcatgtgtcc 10140
   ttatcaagtt ggaacatctt ctaggtatat gcccaggaga ggaattgctg gatcttccgg 10200
   tagtaccatc aacatgcatt cttaataaaa gccctagaac aaggaggact gtaggaaaca 10260
   tattccaaca taataaaggt tatgtatgac aaactcatga ccaatatcat cctaaatgaa 10320
   tgaaaccatt aataagctcc attaaaatca gaggactgcc cactatccct acttctcatc 10380
   cataatgaga ttgaagcatt agctggagca ataaggcaag agaagggata caaatgggaa 10440
   aatattaagt caaattgttt tcaattgaag attatattat cttataccca atgacctcaa 10500
   attttgacta gaaaaattgt agaaattatc aataatttca gcaaagtgtt atgatgcacc 10560
   acatccttat tcttctcccc agcttctgct tgcttctctc ttcttgctct tcatcctttc 10620
   tgtccttcca tctgcctgca ctcttgtctc aagactgagt gcagcgtgta actctcctgt 10680
   gactgagtat ctcacaaaac gttctacctg ccaaacctgg atgagccctt tgtctttctg 10740
   aagctatgag gctctctaca tagactcaag aaggaaatga cagggaggag gtaataatga 10800
   agtggggaag gctgacatta gcattgctcc tgtgtggctc cttaatttct catacttcac 10860
   actgagatgt tattaactgt gactcatagg tgaagaagcc agagctaagg ttctcatatt 10920
   tgagtgttat agaatgagta gagcagtagt tctcaaacta tgggtcatga ctcctttatg 10980
   ggtcaaacta ccctttcaca caggttgcat atcagatatc ctaattttat atacatatat 11040
   atatgcatat gtatatatat atatttcaca acagtaggaa aattatttag taatcatttt 11100
   atagttgtgg gtcatggcaa catgaggaac tgtattaaag ggttgcagca ttaggaatgt 11160
   tgagacccac tgtaatagag aatgaggctt aaggcagggc tataaagccc aatggaccat 11220
   gtgccttttc caacatttgc cacatggtaa gctctgtata gactttttaa agaacattgg 11280
   tttgtaattt taaatggata agggtcttca ctgtctatca cccatctata taataaatac 11340
   ataagttttg attccaccat ggattcaaat gcaaaaatcc tcaacctaag acatagcagt 11400
   gaaacattga tgaccaaata ggaaatccat gtagagacct tctatcttct gatggctcca 11460
   caggcaccat cttgcaacag agttctactt tgctaccagt aatgaataca gtgtctcaac 11520
   tcctgccatt gaatcttcag gaagcccctg aaatgacttg tactacacca tttcttaaag 11580
   acagaaaagc taagacttag agggaataaa tgtcatgcct gagatcatgc aaccaattaa 11640
   gtccaacttg gcctgatcaa gaggcacaat tcaaaagcaa tgttgttcct tcactagctc 11700
   ttgtgtatgg ttgctgattc cggaagcaaa gtatcagtga atatccctag tgggaaaaga 11760
   cttggaaatc aaatgtctca tttaacagat taggagatga aacggtagac tctgtgtagt 11820
   tgtacacccc tgtgatccca tcgctaggaa gactgaggca ggaagtcctc gagctcaaac 11880
   cagcttaggc tacacagaga aactatctaa aaaataatta ctaactactt aataggagat 11940
   tggatgttaa gatctggtca ctaagaggca gaattgagat tcgaagccag tattttctac 12000
   ctggtatgtt ttaaattgca gtaaggatct aagtgtagat atataataat aagattctat 12060
   tgatctctgc aacaacagag agtgttagat ttgtttggaa aaaaatatta tcagccaaca 12120
   tcttctacca tttcagtata gcacagagta cccacccata tctccccacc catcccccat 12180
   accagactgg ttattgattt tcatggtgac tggcctgaga agattaaaaa aagtaatgct 12240
   accttattgg gagtgtccca tggaccaaga tagcaactgt catagctacc gtcacactgc 12300
   tttgatcaag aagacccttt gaggaactga aaacagaacc ttaggcacat ctgttgcttt 12360
   cgctcccatc ctcctccaac agcctgggtg gtgcactcca caccctttca agtttccaaa 12420
   gcctcataca cctgctccct accccagcac ctggccaagg ctgtatccag cactgggatg 12480
   aaaatgatac cccacctcca tcttgtttga tattactcta tctcaagccc caggttagtc 12540
   cccagtccca atgcttttgc acagtcaaaa ctcaacttgg aataatcagt atccttgaag 12600
   agttctgata tggtcactgg gcccatatac catgtaagac atgtggaaaa gatgtttcat 12660
   ggggcccaga cacgttctag aagtacctga gagtggcaaa aaatagttgt gctaaatagt 12720
   ttggccatct ttaggctgag agactaggaa atacagcgat ggactatatc agcattgcag 12780
   gatagttgtc agtaaacacc ccacaaccca taacagaagt attctcttct ttctatatcc 12840
   cttttccatc catgtagatg gctgtcttca tatttgttct agacggccgg cc 12892
<210> 94
   <211> 6425
   <212> DNA
   <213> VkP-IGKVl-39/J-Ck-delta2
<400> 94
   ggccggccca catgaaacaa tgggaaccat gtgacaatca cagaggtgtt gttactatag     60
   caaaagggat tgttactctc cacatccctt taagtaactt gaaggcctga tagacccacc    120
   ctctaagact tcattagaca ttccctacga atggttatac tctcctgtat actcccaata    180
   caactctaaa atatattatt ccatatagtc cttaggtttg tattaaagtt tgactttttt 240
   ccttcaaaat atctcttgtc acaacagcgg ctctagagag aaatacattc cctccaggca 300
   aatctatgct gcgctggtct gacctgggac cctggggaca ttgcccctgt gctgagttac 360
   taagatgagc cagccctgca gctgtgctca gcctgcccca tgccctgctg attgatttgc 420
   atgttccaga gcacagcccc ctgccctgaa gactttttta tgggctggtc gcaccctgtg 480
   caggagtcag tctcagtcag gagccaccat ggacatgaga gtgcccgccc agctcctggg 540
   gctcctgcta ctctggctcc gaggtaagga tggagaacac taggaattta ctcagccagt 600
   gtgctcagta ctgactggaa cttcagggaa gttctctgat aacatgatta atagtaagaa 660
   tatttgtttt tatgtttcca atctcaggtg ccagatgtga catccagatg acccagagcc 720
   ccagcagcct gagcgccagc gtgggcgaca gagtgaccat cacctgcaga gccagccaga 780
   gcatcagcag ctacctgaac tggtatcagc agaagcccgg caaggccccc aagctgctga 840
   tctacgccgc cagctccctg cagagcggcg tgcccagcag attcagcggc agcggctccg 900
   gcaccgactt caccctgacc atcagcagcc tgcagcccga ggacttcgcc acctactact 960
   gccagcagag ctacagcacc ccccccacct tcggccaggg caccaaggtg gagatcaaac 1020
   gtaagtacac ttttctcatc tttttttatg tgtaagacac aggttttcat gttaggagtt 1080
   aaagtcagtt cagaaaatct tgagaaaatg gagagggctc attatcagtt gacgtggcat 1140
   acagtgtcag attttctgtt tatcaagcta gtgagattag gggcaaaaag aggctttagt 1200
   tgagaggaaa gtaattaata ctatggtcac catccaagag attggatcgg agaataagca 1260
   tgagtagtta ttgagatctg ggtctgactg caggtagcgt ggtcttctag acgtttaagt 1320
   gggagatttg gaggggatga ggaatgaagg aacttcagga tagaaaaggg ctgaagtcaa 1380
   gttcagctcc taaaatggat gtgggagcaa actttgaaga taaactgaat gacccagagg 1440
   atgaaacagc gcagatcaaa gaggggcctg gagctctgag aagagaagga gactcatccg 1500
   tgttgagttt ccacaagtac tgtcttgagt tttgcaataa aagtgggata gcagagttga 1560
   gtgagccgta ggctgagttc tctcttttgt ctcctaagtt tttatgacta caaaaatcag 1620
   tagtatgtcc tgaaataatc attaagctgt ttgaaagtat gactgcttgc catgtagata 1680
   ccatggcttg ctgaataatc agaagaggtg tgactcttat tctaaaattt gtcacaaaat 1740
   gtcaaaatga gagactctgt aggaacgagt ccttgacaga cagctcaagg ggtttttttc 1800
   ctttgtctca tttctacatg aaagtaaatt tgaaatgatc ttttttatta taagagtaga 1860
   aatacagttg ggtttgaact atatgtttta atggccacgg ttttgtaaga catttggtcc 1920
   tttgttttcc cagttattac tcgattgtaa ttttatatcg ccagcaatgg actgaaacgg 1980
   tccgcaacct cttctttaca actgggtgac ctcgcggctg tgccagccat ttggcgttca 2040
   ccctgccgct aagggccatg tgaacccccg cggtagcatc ccttgctccg cgtggaccac 2100
   tttcctgagg cacagtgata ggaacagagc cactaatctg aagagaacag agatgtgaca 2160
   gactacacta atgtgagaaa aacaaggaaa gggtgactta ttggagattt cagaaataaa 2220
   atgcatttat tattatattc ccttatttta attttctatt agggaattag aaagggcata 2280
   aactgcttta tccagtgtta tattaaaagc ttaatgtata taatctttta gaggtaaaat 2340
   ctacagccag caaaagtcat ggtaaatatt ctttgactga actctcacta aactcctcta 2400
   aattatatgt catattaact ggttaaatta atataaattt gtgacatgac cttaactggt 2460
   taggtaggat atttttcttc atgcaaaaat atgactaata ataatttagc acaaaaatat 2520
   ttcccaatac tttaattctg tgatagaaaa atgtttaact cagctactat aatcccataa 2580
   ttttgaaaac tatttatttg gctacaccaa aggaagccat acagaggcta atatcagagt 2640
   attcttggaa gagacaggag aaaatgaaag ccagtttctg ctcttacctt atgtgcttgt 2700
   gttcagactc ccaaacatca ggagtgtcag ataaactggt ctgaatctct gtctgaagca 2760
   tggaactgaa aagaatgtag tttcagggaa gaaaggcaat agaaggaagc ctgagaatat 2820
   cttcaaaggg tcagactcaa tttactttct aaagaagtag ctaggaacta gggaataact 2880
   tagaaacaac aagattgtat atatgtgcat cctggcccca ttgttcctta tctgtaggga 2940
   taagcgtgct tttttgtgtg tctgtatata acataactgt ttacacataa tacactgaaa 3000
   tggagccctt ccttgttact tcataccatc ctctgtgctt ccttcctcag gggccgacgc 3060
   cgctcccacc gtgtccatct tcccccccag catggaacag ctgacctctg gcggagccac 3120
   cgtggtctgc ttcgtgaaca acttctaccc cagagacatc agcgtgaagt ggaagatcga 3180
   cggcagcgag cagagggacg gcgtgctgga cagcgtgacc gaccaggaca gcaaggactc 3240
   cacctacagc atgagcagca ccctgagcct gaccaaggtg gagtacgaga ggcacaacct 3300
   gtacacctgc gaggtggtgc acaagaccag ctccagcccc gtggtcaagt ccttcaaccg 3360
   gaacgagtgt tgaagacaaa ggtcctgaga cgccaccacc agctccccag ctccatccta 3420
   tcttcccttc taaggtcttg gaggcttccc cacaagcgac ctaccactgt tgcggtgctc 3480
   caaacctcct ccccacctcc ttctcctcct cctccctttc cttggctttt atcatgctaa 3540
   tatttgcaga aaatattcaa taaagtgagt ctttgcactt gagatctctg tctttcttac 3600
   taaatggtag taatcagttg tttttccagt tacctgggtt tctcttctaa agaagttaaa 3660
   tgtttagttg ccctgaaatc caccacactt aaaggataaa taaaaccctc cacttgccct 3720
   ggttggctgt ccactacatg gcagtccttt ctaaggttca cgagtactat tcatggctta 3780
   tttctctggg ccatggtagg tttgaggagg catacttcct agttttcttc ccctaagtcg 3840
   tcaaagtcct gaagggggac agtctttaca agcacatgtt ctgtaatctg attcaaccta 3900
   cccagtaaac ttggcgaagc aaagtagaat cattatcaca ggaagcaaag gcaacctaaa 3960
   tgtgcaagca ataggaaaat gtggaagccc atcatagtac ttggacttca tctgcttttg 4020
   tgccttcact aagtttttaa acatgagctg gctcctatct gccattggca aggctgggca 4080
   ctacccacaa cctacttcaa ggacctctat accgtgagat tacacacata catcaaaatt 4140
   tgggaaaagt tctaccaagc tgagagctga tcaccccact cttaggtgct tatctctgta 4200
   caccagaaac cttaagaagc aaccagtatt gagagactca tttatgaaag tctaaaactg 4260
   gatacaacca aaatgtccac caacagttaa attatgacat gttcacaatt gagctattac 4320
   ttaataagga gaattaataa aataaaactt aagagcatag tttaatctca taaacaagat 4380
   aataagcaaa acaaaacatt ttttcatcca tgtaagttta aaagcaggta aaatttaaaa 4440
   ttaagagaga cataagtttt gaggtagcaa gatggaaact ctggggcttg gggaatgttc 4500
   tgtctctctg tatgggatgt gaaagttact attgtggaat tgggatctat gttcttcctg 4560
   tatatattgt atacttcata ataacttcac ctaaagaaat atctaatacc cagtgcatac 4620
   ataaaagagg atacaaggaa tgaatcatac gtcaaggcca gaaagacaat aaagtagggg 4680
   atccaggatc aaatctccca caaccttgag ccttctacta ttctgccttc cagagctcaa 4740
   agtacaaaac acataattca aacacatgat ccctccttgg ggtctcttcc ttcatgcatc 4800
   gaattagaaa tagccatgta taaaatgaga tagaagagac cttcatcaac aggtcaaaga 4860
   atataggtaa ttttgtctgg gtatgaagag cccacgtatc aaaggttaca ttagggaagg 4920
   aagaggacac taacagtgac tttcattctc cccctcttcc tggaggcccc tgcatttagt 4980
   ccctcgtggg ctcatccact cagcacacat ttactaagca tcttctcagc ctacactctg 5040
   aaggcagtgc agaataatgt tagtgtccct tcccccagtt aatatgcagt ccagtttccc 5100
   tgctccttcc ctttctcagt ccacataagg atgatgggaa aggacagtca ccaaatagga 5160
   gagggcaacc ctttgccttc ctacctcttg agaatgtaca ttattatcca ctttttgaaa 5220
   cttcttttaa ttgctttttt ttaatttgtc ttttcaaata gcataacctt gttcatccat 5280
   ttctgggaac caaatttatc aatcaacagt gcctctaatc tggctattaa tacaaaaatg 5340
   cctcctcaaa atatatatgt tcgagtctta tctaaaacag aacccacaat aaaaaagaag 5400
   aaagaataca tataagcatt tatataattc tgagcaacct tgtgctttgt gaaaaaaata 5460
   taatctaatg tcacatgctg tattcttttt atttaacact ggtgaaatta taccattaga 5520
   gagaaagagg acagatcact gatcctagga tctagggatg ttacagataa gaaaacaaat 5580
   gtgacaaaga gctgtcacaa ggaggatctt caaggtcaca gaatcactgt cttgatttca 5640
   gtggtggtta catacattta aatatgtgat aaaatgttgt tgaactatat tcatatattg 5700
   taccaatgtc aaatgcttaa ttttggctct atagtataat tatgcactaa ataactattt 5760
   ggacaaagaa aatgatgttt acatcaaagg tgaggccata tttgttagga acataactta 5820
   aaaaccattt tggataacta atgaaaagcc attttgtgtg ccttggcata tcatgcctaa 5880
   gctgtcacca gatagatcta ataagaccta agcctcagaa gcaagcccct gcccagcaag 5940
   caggcagcac agataagagc taaacccagg acaggccatg atatgctaat gaactacctt 6000
   caaggtggtg ttgctgacct agtgaaccag ccccaagctg tgagccccaa tagcacaaag 6060
   ctactgccca aagaaattat acaaaaattg gaactttggg aatggtgtgc aggatcgctc 6120
   tgctgtatgc ctggaacaca gcttctctat gttttgtatt gataccagtc tagaagcttc 6180
   caaaactttc tcactgaaga agattcccca tgtgggaccc ctacagactc ttttgcccaa 6240
   acaactgctt ccctcctggt gtgatcatgg accaagatag caactgtcat agctaccgtc 6300
   acactgcttt gatcaagaag accctttgag gaactgaaaa cagaacctta ggcacatctg 6360
   ttgctttcgc tcccatcctc ctccaacagc atggctgtct tcatatttgt tctagacggc 6420
   cggcc 6425
<210> 95
   <211> 13382
   <212> DNA
   <213> Artificial
<220>
   <223> VkP-IGLV2-14/J-Ck
<400> 95
   ggccggccca catgaaacaa tgggaaccat gtgacaatca cagaggtgtt gttactatag      60
   caaaagggat tgttactctc cacatccctt taagtaactt gaaggcctga tagacccacc     120
   ctctaagact tcattagaca ttccctacga atggttatac tctcctgtat actcccaata     180
   caactctaaa atatattatt ccatatagtc cttaggtttg tattaaagtt tgactttttt     240
   ccttcaaaat atctcttgtc acaacagcgg ctctagagag aaatacattc cctccaggca     300
   aatctatgct gcgctggtct gacctgggac cctggggaca ttgcccctgt gctgagttac     360
   taagatgagc cagccctgca gctgtgctca gcctgcccca tgccctgctg attgatttgc     420
   atgttccaga gcacagcccc ctgccctgaa gactttttta tgggctggtc gcaccctgtg    480
   caggagtcag tctcagtcag gagccaccat ggacatgaga gtgcccgccc agctcctggg     540
   gctcctgcta ctctggctcc gaggtaagga tggagaacac taggaattta ctcagccagt     600
   gtgctcagta ctgactggaa cttcagggaa gttctctgat aacatgatta atagtaagaa     660
   tatttgtttt tatgtttcca atctcaggtg ccagatgtca gtctgccctg acccagcccg     720
   cctctgtgtc tggcagccct ggccagagca tcaccatcag ctgcaccggc accagcagcg     780
   acgtgggcgg ctacaactac gtgtcctggt atcagcagca ccccggcaag gcccccaagc     840
   tgatgatcta cgaggtgtcc aacagaccca gcggcgtgag caacagattc agcggcagca     900
   agagcggcaa caccgccagc ctgaccatca gcggcctcca ggctgaggac gaggccgact     960
   actactgcag cagctacacc agcagctcca ccctggtgtt tggcggcgga acaaagctga 1020
   ccgtgctgcg taagtacact tttctcatct ttttttatgt gtaagacaca ggttttcatg 1080
   ttaggagtta aagtcagttc agaaaatctt gagaaaatgg agagggctca ttatcagttg 1140
   acgtggcata cagtgtcaga ttttctgttt atcaagctag tgagattagg ggcaaaaaga 1200
   ggctttagtt gagaggaaag taattaatac tatggtcacc atccaagaga ttggatcgga 1260
   gaataagcat gagtagttat tgagatctgg gtctgactgc aggtagcgtg gtcttctaga 1320
   cgtttaagtg ggagatttgg aggggatgag gaatgaagga acttcaggat agaaaagggc 1380
   tgaagtcaag ttcagctcct aaaatggatg tgggagcaaa ctttgaagat aaactgaatg 1440
   acccagagga tgaaacagcg cagatcaaag aggggcctgg agctctgaga agagaaggag 1500
   actcatccgt gttgagtttc cacaagtact gtcttgagtt ttgcaataaa agtgggatag 1560
   cagagttgag tgagccgtag gctgagttct ctcttttgtc tcctaagttt ttatgactac 1620
   aaaaatcagt agtatgtcct gaaataatca ttaagctgtt tgaaagtatg actgcttgcc 1680
   atgtagatac catggcttgc tgaataatca gaagaggtgt gactcttatt ctaaaatttg 1740
   tcacaaaatg tcaaaatgag agactctgta ggaacgagtc cttgacagac agctcaaggg 1800
   gtttttttcc tttgtctcat ttctacatga aagtaaattt gaaatgatct tttttattat 1860
   aagagtagaa atacagttgg gtttgaacta tatgttttaa tggccacggt tttgtaagac 1920
   atttggtcct ttgttttccc agttattact cgattgtaat tttatatcgc cagcaatgga 1980
   ctgaaacggt ccgcaacctc ttctttacaa ctgggtgacc tcgcggctgt gccagccatt 2040
   tggcgttcac cctgccgcta agggccatgt gaacccccgc ggtagcatcc cttgctccgc 2100
   gtggaccact ttcctgaggc acagtgatag gaacagagcc actaatctga agagaacaga 2160
   gatgtgacag actacactaa tgtgagaaaa acaaggaaag ggtgacttat tggagatttc 2220
   agaaataaaa tgcatttatt attatattcc cttattttaa ttttctatta gggaattaga 2280
   aagggcataa actgctttat ccagtgttat attaaaagct taatgtatat aatcttttag 2340
   aggtaaaatc tacagccagc aaaagtcatg gtaaatattc tttgactgaa ctctcactaa 2400
   actcctctaa attatatgtc atattaactg gttaaattaa tataaatttg tgacatgacc 2460
   ttaactggtt aggtaggata tttttcttca tgcaaaaata tgactaataa taatttagca 2520
   caaaaatatt tcccaatact ttaattctgt gatagaaaaa tgtttaactc agctactata 2580
   atcccataat tttgaaaact atttattagc ttttgtgttt gacccttccc tagccaaagg 2640
   caactattta aggacccttt aaaactcttg aaactacttt agagtcatta agttatttaa 2700
   ccacttttaa ttactttaaa atgatgtcaa ttccctttta actattaatt tattttaagg 2760
   ggggaaaggc tgctcataat tctattgttt ttcttggtaa agaactctca gttttcgttt 2820
   ttactacctc tgtcacccaa gagttggcat ctcaacagag gggactttcc gagaggccat 2880
   ctggcagttg cttaagatca gaagtgaagt ctgccagttc ctcccaggca ggtggcccag 2940
   attacagttg acctgttctg gtgtggctaa aaattgtccc atgtggttac aaaccattag 3000
   accagggtct gatgaattgc tcagaatatt tctggacacc caaatacaga ccctggctta 3060
   aggccctgtc catacagtag gtttagcttg gctacaccaa aggaagccat acagaggcta 3120
   atatcagagt attcttggaa gagacaggag aaaatgaaag ccagtttctg ctcttacctt 3180
   atgtgcttgt gttcagactc ccaaacatca ggagtgtcag ataaactggt ctgaatctct 3240
   gtctgaagca tggaactgaa aagaatgtag tttcagggaa gaaaggcaat agaaggaagc 3300
   ctgagaatat cttcaaaggg tcagactcaa tttactttct aaagaagtag ctaggaacta 3360
   gggaataact tagaaacaac aagattgtat atatgtgcat cctggcccca ttgttcctta 3420
   tctgtaggga taagcgtgct tttttgtgtg tctgtatata acataactgt ttacacataa 3480
   tacactgaaa tggagccctt ccttgttact tcataccatc ctctgtgctt ccttcctcag 3540
   gggccgacgc cgctcccacc gtgtccatct tcccccccag catggaacag ctgacctctg 3600
   gcggagccac cgtggtctgc ttcgtgaaca acttctaccc cagagacatc agcgtgaagt 3660
   ggaagatcga cggcagcgag cagagggacg gcgtgctgga cagcgtgacc gaccaggaca 3720
   gcaaggactc cacctacagc atgagcagca ccctgagcct gaccaaggtg gagtacgaga 3780
   ggcacaacct gtacacctgc gaggtggtgc acaagaccag ctccagcccc gtggtcaagt 3840
   ccttcaaccg gaacgagtgt tgaagacaaa ggtcctgaga cgccaccacc agctccccag 3900
   ctccatccta tcttcccttc taaggtcttg gaggcttccc cacaagcgac ctaccactgt 3960
   tgcggtgctc caaacctcct ccccacctcc ttctcctcct cctccctttc cttggctttt 4020
   atcatgctaa tatttgcaga aaatattcaa taaagtgagt ctttgcactt gagatctctg 4080
   tctttcttac taaatggtag taatcagttg tttttccagt tacctgggtt tctcttctaa 4140
   agaagttaaa tgtttagttg ccctgaaatc caccacactt aaaggataaa taaaaccctc 4200
   cacttgccct ggttggctgt ccactacatg gcagtccttt ctaaggttca cgagtactat 4260
   tcatggctta tttctctggg ccatggtagg tttgaggagg catacttcct agttttcttc 4320
   ccctaagtcg tcaaagtcct gaagggggac agtctttaca agcacatgtt ctgtaatctg 4380
   attcaaccta cccagtaaac ttggcgaagc aaagtagaat cattatcaca ggaagcaaag 4440
   gcaacctaaa tgtgcaagca ataggaaaat gtggaagccc atcatagtac ttggacttca 4500
   tctgcttttg tgccttcact aagtttttaa acatgagctg gctcctatct gccattggca 4560
   aggctgggca ctacccacaa cctacttcaa ggacctctat accgtgagat tacacacata 4620
   catcaaaatt tgggaaaagt tctaccaagc tgagagctga tcaccccact cttaggtgct 4680
   tatctctgta caccagaaac cttaagaagc aaccagtatt gagagactca tttatgaaag 4740
   tctaaaactg gatacaacca aaatgtccac caacagttaa attatgacat gttcacaatt   4800
   gagctattac ttaataagga gaattaataa aataaaactt aagagcatag tttaatctca   4860
   taaacaagat aataagcaaa acaaaacatt ttttcatcca tgtaagttta aaagcaggta   4920
   aaatttaaaa ttaagagaga cataagtttt gaggtagcaa gatggaaact ctggggcttg   4980
   gggaatgttc tgtctctctg tatgggatgt gaaagttact attgtggaat tgggatctat   5040
   gttcttcctg tatatattgt atacttcata ataacttcac ctaaagaaat atctaatacc   5100
   cagtgcatac ataaaagagg atacaaggaa tgaatcatac gtcaaggcca gaaagacaat   5160
   aaagtagggg atccaggatc aaatctccca caaccttgag ccttctacta ttctgccttc   5220
   cagagctcaa agtacaaaac acataattca aacacatgat ccctccttgg ggtctcttcc   5280
   ttcatgcatc gaattagaaa tagccatgta taaaatgaga tagaagagac cttcatcaac   5340
   aggtcaaaga atataggtaa ttttgtctgg gtatgaagag cccacgtatc aaaggttaca   5400
   ttagggaagg aagaggacac taacagtgac tttcattctc cccctcttcc tggaggcccc   5460
   tgcatttagt ccctcgtggg ctcatccact cagcacacat ttactaagca tcttctcagc   5520
   ctacactctg aaggcagtgc agaataatgt tagtgtccct tcccccagtt aatatgcagt   5580
   ccagtttccc tgctccttcc ctttctcagt ccacataagg atgatgggaa aggacagtca   5640
   ccaaatagga gagggcaacc ctttgccttc ctacctcttg agaatgtaca ttattatcca   5700
   ctttttgaaa cttcttttaa ttgctttttt ttaatttgtc ttttcaaata gcataacctt   5760
   gttcatccat ttctgggaac caaatttatc aatcaacagt gcctctaatc tggctattaa   5820
   tacaaaaatg cctcctcaaa atatatatgt tcgagtctta tctaaaacag aacccacaat   5880
   aaaaaagaag aaagaataca tataagcatt tatataattc tgagcaacct tgtgctttgt   5940
   gaaaaaaata taatctaatg tcacatgctg tattcttttt atttaacact ggtgaaatta   6000
   taccattaga gagaaagagg acagatcact gatcctagga tctagggatg ttacagataa   6060
   gaaaacaaat gtgacaaaga gctgtcacaa ggaggatctt caaggtcaca gaatcactgt   6120
   cttgatttca gtggtggtta catacattta aatatgtgat aaaatgttgt tgaactatat   6180
   tcatatattg taccaatgtc aaatgcttaa ttttggctct atagtataat tatgcactaa   6240
   ataactattt ggacaaagaa aatgatgttt acatcaaagg tgaggccata tttgttagga   6300
   acataactta aaaaccattt tggataacta atgaaaagcc attttgtgtg ccttggcata   6360
   tcatgcctaa gctgtcacca gatagatcta ataagaccta agcctcagaa gcaagcccct   6420
   gcccagcaag caggcagcac agataagagc taaacccagg acaggccatg atatgctaat   6480
   gaactacctt caaggtggtg ttgctgacct agtgaaccag ccccaagctg tgagccccaa   6540
   tagcacaaag ctactgccca aagaaattat acaaaaattg gaactttggg aatggtgtgc   6600
   aggatcgctc tgctgtatgc ctggaacaca gcttctctat gttttgtatt gataccagtc   6660
   tagaagcttc caaaactttc tcactgaaga agattcccca tgtgggaccc ctacagactc 6720
   ttttgcccaa acaactgctt ccctcctggt gtgatatctg ttttgctttt atgttagcat 6780
   aatattataa ggaatgtttg tgtgaataaa ccaaacatat tttaaaagca aatattgtat 6840
   gcacatccta attgctaaaa agtttacagc taatagtccc atgctctcca caatactgga 6900
   tccaaataag tcctaatttc aatgttgggc atctttacag agagaaagac attaaaaatg 6960
   aagagacatg cagagagtgc accatgccat cgtggagaca gactgaagtg acacaactgt 7020
   tagtcaaaga ggattaagga cttccagaag ccaccaaagg aaggaggtat gaagtggttt 7080
   ctccctcaga gtatccagag gagactaaac caaccaacac ctttttgctt aagacttctt 7140
   gccttcagga ctgtgagaag gtagcttcct attgttctaa gccccagtat gtggcatttt 7200
   gttaaggtag agtcaagaaa ccaataaaat gcagacagac aaaaggatag ctgagttttc 7260
   caggcccttc cttcttattt ttggttttgt tggtggtggt ggtggtggtg gtgatggtgg 7320
   tggttttgtt tatgttttgt ttggggagtt ttttggggtt tttttgggtt ttgtttttgt 7380
   tgttgttttg ggggtttttg ttgttgttgt tgtttgcttt tttgtttttt gttttttgtt 7440
   tttttgagac agtgtttctc tgtatagccc tggctgtcct ggagttcctt ctatctctaa 7500
   tgtctacatc tcagagggga tcctctaatt tcaaatgagc agtagctctc catttttagc 7560
   tcttatttat tcatttattt acttacttac ttattgtctg tagatgaaag aattttggag 7620
   tgggaaaggg ttcatgagcc cccagcaact aatgaggagc tacagacaat tgatgtttct 7680
   ggggaaagga gactcagttt ctttgagagt atagcttctg atgggtcaac catgttcctg 7740
   tggctgatgt cacacccagg agtatgcaga caacagaaac tggagttaat gagttgtttt 7800
   aaaaataaaa aagggcatga agcttgggat agaaattaag gataaataca attaaataca 7860
   ggaaattctg aaagaattaa taaaaacatt tcttttttta aaaaaaaatc cagaattagc 7920
   tatgcttctt caaaattgct tctggagaac tttacaagtt aaataagtta tattgtagaa 7980
   aaggtagaga ggagaatagt ggaagagaga gataaggaga cttcaaaagg agtggaggga 8040
   gatagaggag gagaaagcag aagcaatggc tgatagacac aggataagag ggaacagaaa 8100
   ggagaaagag gaagccagga tgggtatttc tttgcctatc tgtgacttgc acatggtctt 8160
   ggcaattatt gatgagttca aggcttaatt cttcacttgt gccaactcaa cagagtcttt 8220
   ctttcttata accaggcccc cagtatgctc atgtatgtat caggtcctct tatctcctta 8280
   tagcaatcct gtttataact gggtaacttt gtgaagggaa ggaagtgcac actgagatgt 8340
   gctacaactt tttaatacaa aattttgaag agtttgtaca atgtatgtat aattaataat 8400
   taatattatg cactttagat tttgatttca actcaagata ctaattctat atatatgggt 8460
   taaatcaata tattaataag tttaatttca catgcttatt tttattgtgg ttttcgagac 8520
   agggtttctc tgtatagccc tggctgtcct ggaacccact ttgtagacca ggctggcctc 8580
   aaactcagaa acctacctgc ctctgcctct gcctctgcct ctgcctctgc ctctgcctct 8640
   gcctctgcct ctgcctctgc ctctgcctct gcctctgcct ctgcctctgc ctctgcctct 8700
   gcctctgcct ctgcctctgc ctctgcctct gcctctgcct ctgcctctgc ctctgcctct 8760
   gcctctgcct ctgcctagtg ctggaattaa aggtttgcgc caccacgccc ggtgaaattt 8820
   ttaaacttta tatatgtctc attctatttc tatcagatag gactgtgtag actgtgctaa 8880
   actaataaat gtgccctcaa aagtaatcgc aagttgtatt gttgttgttt tgctttgctt 8940
   tgctttgctt tgctttgctt tgctttgctt tgctttgctt tgctttgctt tgctttgctt 9000
   tgctttgctt tgctttgctt tgctttgctt tgcttttttg ttttgggttt ttttccgggg 9060
   gagggagggt ggagaaagaa tcttactatg aagctctgac tgtcctggga actcactata 9120
   tagatcaggc ttgattcaac tcatagagat ctgccttctt ctgcctccca agtgctggga 9180
   ataaaggcat acacctccat gcccagatag tgatcccaag ttttagcaaa agtttctaga 9240
   cttgacatta atcgatggag atagacatga attacacaaa gaactaatgt ggagtttacc 9300
   tgaatcatac tctatacttt atcagagatt aaattaacat ttaataatcc agtgccaggc 9360
   tagaggcacc attcaatggc agtgtttgcc atcatgcata ggcttagtct tcagtgctga 9420
   aaggcattgg gggcaatatt actcattata cagatgagaa actgggaaag acttgcctca 9480
   gattctctac tgaaaggctg agtttgtggc ttctagaaaa tcttttactt tcaatatttt 9540
   taatgtataa tttttttatt tccactgatt ttatttttta tttttaacat ttataagaaa 9600
   taaatgcaat aaaccaaata catggacaaa aaaatacaag aatcatatga tcacctcaat 9660
   ggaaggaaaa aaaaagaaag aaaaagtctt tgataagatt caacattcat tcttttttta 9720
   ttagatattt tcttcattta catttcaaat gctatcccca aagcccccta taccttcccc 9780
   tgccctgctc cccaacccac ccactcctgc tttctggccc tggcattcct ctgtactgag 9840
   gcatatgatc ttcaaaaaac caagggcctc tcctctcatt ggtggccgac tattaggcca 9900
   tcttttgcta catatgcaac tagagacaca gctctggggg ttactggtta gttcatattg 9960
   ttagtcctcc tatagagttg cagacccctt tagctccttg gatactttct ctagttcctt 10020
   cattaggggc cctgtgtccc atccaataga tgactgtgag catccacttc tgtatttgcc 10080
   aggcactggc atagcctcac gagaaagaga gagctatgtc aggatcctgt cagtaaaatc 10140
   tttctggcat atgcaatagt atctgggttt ggtggttgta tatgggatgg atccccaagt 10200
   ggagcagtct ctgaatggtc cttccttcca tctcagctcc aaactttgtc tctataactc 10260
   cttccatggg tattttgttc cccattctaa gaaggagtga agaatccaca ctttggtctt 10320
   ccttcttctt gagtttcata tgttgcatct tggatattct aagtttctgg gttaatatcc 10380
   acgtatcagt gagtgcatat catgcgtgtt attttgtgat tagtttacct cactcaggat 10440
   gatatcctcc agatgcatcc atttgcctaa gaatttcatt aattcactgt ttttaattgc 10500
   tgaatagtac tccattgtgt aaatgtacca cattttctgt atccattcct ctgttgaggg 10560
   gcatctgggt tctttccagc ttctggctat tataaataag gctgctatga gcatagcgga 10620
   gcatgtgtcc ttatcaagtt ggaacatctt ctaggtatat gcccaggaga ggaattgctg 10680
   gatcttccgg tagtaccatc aacatgcatt cttaataaaa gccctagaac aaggaggact 10740
   gtaggaaaca tattccaaca taataaaggt tatgtatgac aaactcatga ccaatatcat 10800
   cctaaatgaa tgaaaccatt aataagctcc attaaaatca gaggactgcc cactatccct 10860
   acttctcatc cataatgaga ttgaagcatt agctggagca ataaggcaag agaagggata 10920
   caaatgggaa aatattaagt caaattgttt tcaattgaag attatattat cttataccca 10980
   atgacctcaa attttgacta gaaaaattgt agaaattatc aataatttca gcaaagtgtt 11040
   atgatgcacc acatccttat tcttctcccc agcttctgct tgcttctctc ttcttgctct 11100
   tcatcctttc tgtccttcca tctgcctgca ctcttgtctc aagactgagt gcagcgtgta 11160
   actctcctgt gactgagtat ctcacaaaac gttctacctg ccaaacctgg atgagccctt 11220
   tgtctttctg aagctatgag gctctctaca tagactcaag aaggaaatga cagggaggag 11280
   gtaataatga agtggggaag gctgacatta gcattgctcc tgtgtggctc cttaatttct 11340
   catacttcac actgagatgt tattaactgt gactcatagg tgaagaagcc agagctaagg 11400
   ttctcatatt tgagtgttat agaatgagta gagcagtagt tctcaaacta tgggtcatga 11460
   ctcctttatg ggtcaaacta ccctttcaca caggttgcat atcagatatc ctaattttat 11520
   atacatatat atatgcatat gtatatatat atatttcaca acagtaggaa aattatttag 11580
   taatcatttt atagttgtgg gtcatggcaa catgaggaac tgtattaaag ggttgcagca 11640
   ttaggaatgt tgagacccac tgtaatagag aatgaggctt aaggcagggc tataaagccc 11700
   aatggaccat gtgccttttc caacatttgc cacatggtaa gctctgtata gactttttaa 11760
   agaacattgg tttgtaattt taaatggata agggtcttca ctgtctatca cccatctata 11820
   taataaatac ataagttttg attccaccat ggattcaaat gcaaaaatcc tcaacctaag 11880
   acatagcagt gaaacattga tgaccaaata ggaaatccat gtagagacct tctatcttct 11940
   gatggctcca caggcaccat cttgcaacag agttctactt tgctaccagt aatgaataca 12000
   gtgtctcaac tcctgccatt gaatcttcag gaagcccctg aaatgacttg tactacacca 12060
   tttcttaaag acagaaaagc taagacttag agggaataaa tgtcatgcct gagatcatgc 12120
   aaccaattaa gtccaacttg gcctgatcaa gaggcacaat tcaaaagcaa tgttgttcct 12180
   tcactagctc ttgtgtatgg ttgctgattc cggaagcaaa gtatcagtga atatccctag 12240
   tgggaaaaga cttggaaatc aaatgtctca tttaacagat taggagatga aacggtagac 12300
   tctgtgtagt tgtacacccc tgtgatccca tcgctaggaa gactgaggca ggaagtcctc 12360
   gagctcaaac cagcttaggc tacacagaga aactatctaa aaaataatta ctaactactt 12420
   aataggagat tggatgttaa gatctggtca ctaagaggca gaattgagat tcgaagccag 12480
   tattttctac ctggtatgtt ttaaattgca gtaaggatct aagtgtagat atataataat 12540
   aagattctat tgatctctgc aacaacagag agtgttagat ttgtttggaa aaaaatatta 12600
   tcagccaaca tcttctacca tttcagtata gcacagagta cccacccata tctccccacc 12660
   catcccccat accagactgg ttattgattt tcatggtgac tggcctgaga agattaaaaa 12720
   aagtaatgct accttattgg gagtgtccca tggaccaaga tagcaactgt catagctacc 12780
   gtcacactgc tttgatcaag aagacccttt gaggaactga aaacagaacc ttaggcacat 12840
   ctgttgcttt cgctcccatc ctcctccaac agcctgggtg gtgcactcca caccctttca 12900
   agtttccaaa gcctcataca cctgctccct accccagcac ctggccaagg ctgtatccag 12960
   cactgggatg aaaatgatac cccacctcca tcttgtttga tattactcta tctcaagccc 13020
   caggttagtc cccagtccca atgcttttgc acagtcaaaa ctcaacttgg aataatcagt 13080
   atccttgaag agttctgata tggtcactgg gcccatatac catgtaagac atgtggaaaa 13140
   gatgtttcat ggggcccaga cacgttctag aagtacctga gagtggcaaa aaatagttgt 13200
   gctaaatagt ttggccatct ttaggctgag agactaggaa atacagcgat ggactatatc 13260
   agcattgcag gatagttgtc agtaaacacc ccacaaccca taacagaagt attctcttct 13320
   ttctatatcc cttttccatc catgtagatg gctgtcttca tatttgttct agacggccgg 13380
   cc 13382
<210> 96
   <211> 4638
   <212> DNA
   <213> Artificial
<220>
   <223> pSELECT-IGKV1-39/J-Ck
<400> 96
   gcggccgcaa taaaatatct ttattttcat tacatctgtg tgttggtttt ttgtgtgaat      60
   cgtaactaac atacgctctc catcaaaaca aaacgaaaca aaacaaacta gcaaaatagg     120
   ctgtccccag tgcaagtgca ggtgccagaa catttctcta tcgaaggatc tgcgatcgct     180
   ccggtgcccg tcagtgggca gagcgcacat cgcccacagt ccccgagaag ttggggggag     240
   gggtcggcaa ttgaacgggt gcctagagaa ggtggcgcgg ggtaaactgg gaaagtgatg     300
   tcgtgtactg gctccgcctt tttcccgagg gtgggggaga accgtatata agtgcagtag     360
   tcgccgtgaa cgttcttttt cgcaacgggt ttgccgccag aacacagctg aagcttcgag     420
   gggctcgcat ctctccttca cgcgcccgcc gccctacctg aggccgccat ccacgccggt     480
   tgagtcgcgt tctgccgcct cccgcctgtg gtgcctcctg aactgcgtcc gccgtctagg 540
   taagtttaaa gctcaggtcg agaccgggcc tttgtccggc gctcccttgg agcctaccta 600
   gactcagccg gctctccacg ctttgcctga ccctgcttgc tcaactctac gtctttgttt 660
   cgttttctgt tctgcgccgt tacagatcca agctgtgacc ggcgcctacc tgagatcacc 720
   ggcgtgtcga cgccaccatg gacatgagag tgcccgccca gctcctgggg ctcctgctac 780
   tctggctccg aggtaaggat ggagaacact aggaatttac tcagccagtg tgctcagtac 840
   tgactggaac ttcagggaag ttctctgata acatgattaa tagtaagaat atttgttttt 900
   atgtttccaa tctcaggtgc cagatgtgac atccagatga cccagagccc cagcagcctg 960
   agcgccagcg tgggcgacag agtgaccatc acctgcagag ccagccagag catcagcagc 1020
   tacctgaact ggtatcagca gaagcccggc aaggccccca agctgctgat ctacgccgcc 1080
   agctccctgc agagcggcgt gcccagcaga ttcagcggca gcggctccgg caccgacttc 1140
   accctgacca tcagcagcct gcagcccgag gacttcgcca cctactactg ccagcagagc 1200
   tacagcaccc cccccacctt cggccagggc accaaggtgg agatcaagag agccgacgcc 1260
   gctcccaccg tgtccatctt cccccccagc atggaacagc tgacctctgg cggagccacc 1320
   gtggtctgct tcgtgaacaa cttctacccc agagacatca gcgtgaagtg gaagatcgac 1380
   ggcagcgagc agagggacgg cgtgctggac agcgtgaccg accaggacag caaggactcc 1440
   acctacagca tgagcagcac cctgagcctg accaaggtgg agtacgagag gcacaacctg 1500
   tacacctgcg aggtggtgca caagaccagc tccagccccg tggtcaagtc cttcaaccgg 1560
   aacgagtgtt gagctagctg gccagacatg ataagataca ttgatgagtt tggacaaacc 1620
   acaactagaa tgcagtgaaa aaaatgcttt atttgtgaaa tttgtgatgc tattgcttta 1680
   tttgtaacca ttataagctg caataaacaa gttaacaaca acaattgcat tcattttatg 1740
   tttcaggttc agggggaggt gtgggaggtt ttttaaagca agtaaaacct ctacaaatgt 1800
   ggtatggaat tctaaaatac agcatagcaa aactttaacc tccaaatcaa gcctctactt 1860
   gaatcctttt ctgagggatg aataaggcat aggcatcagg ggctgttgcc aatgtgcatt 1920
   agctgtttgc agcctcacct tctttcatgg agtttaagat atagtgtatt ttcccaaggt 1980
   ttgaactagc tcttcatttc tttatgtttt aaatgcactg acctcccaca ttcccttttt 2040
   agtaaaatat tcagaaataa tttaaataca tcattgcaat gaaaataaat gttttttatt 2100
   aggcagaatc cagatgctca aggcccttca taatatcccc cagtttagta gttggactta 2160
   gggaacaaag gaacctttaa tagaaattgg acagcaagaa agcgagcttc tagcgaattc 2220
   tcgactcatt cctttgccct cggacgagtg ctggggcgtc ggtttccact atcggcgagt 2280
   acttctacac agccatcggt ccagacggcc gcgcttctgc gggcgatttg tgtacgcccg 2340
   acagtcccgg ctccggatcg gacgattgcg tcgcatcgac cctgcgccca agctgcatca 2400
   tcgaaattgc cgtcaaccaa gctctgatag agttggtcaa gaccaatgcg gagcatatac 2460
   gcccggagcc gcggcgatcc tgcaagctcc ggatgcctcc gctcgaagta gcgcgtctgc 2520
   tgctccatac aagccaacca cggcctccag aagaagatgt tggcgacctc gtattgggaa 2580
   tccccgaaca tcgcctcgct ccagtcaatg accgctgtta tgcggccatt gtccgtcagg 2640
   acattgttgg agccgaaatc cgcgtgcacg aggtgccgga cttcggggca gtcctcggcc 2700
   caaagcatca gctcatcgag agcctgcgcg acggacgcac tgacggtgtc gtccatcaca 2760
   gtttgccagt gatacacatg gggatcagca atcgcgcata tgaaatcacg ccatgtagtg 2820
   tattgaccga ttccttgcgg tccgaatggg ccgaacccgc tcgtctggct aagatcggcc 2880
   gcagcgatcg catccatgag ctccgcgacg ggttgcagaa cagcgggcag ttcggtttca 2940
   ggcaggtctt gcaacgtgac accctgtgca cggcgggaga tgcaataggt caggctctcg 3000
   ctgaattccc caatgtcaag cacttccgga atcgggagcg cggccgatgc aaagtgccga 3060
   taaacataac gatctttgta gaaaccatcg gcgcagctat ttacccgcag gacatatcca 3120
   cgccctccta catcgaagct gaaagcacga gattcttcgc cctccgagag ctgcatcagg 3180
   tcggagacgc tgtcgaactt ttcgatcaga aacttctcga cagacgtcgc ggtgagttca 3240
   ggctttttca tgatggccct cctatagtga gtcgtattat actatgccga tatactatgc 3300
   cgatgattaa ttgtcaaaac agcgtggatg gcgtctccag cttatctgac ggttcactaa 3360
   acgagctctg cttatataga cctcccaccg tacacgccta ccgcccattt gcgtcaatgg 3420
   ggcggagttg ttacgacatt ttggaaagtc ccgttgattt actagtcaaa acaaactccc 3480
   attgacgtca atggggtgga gacttggaaa tccccgtgag tcaaaccgct atccacgccc 3540
   attgatgtac tgccaaaacc gcatcatcat ggtaatagcg atgactaata cgtagatgta 3600
   ctgccaagta ggaaagtccc ataaggtcat gtactgggca taatgccagg cgggccattt 3660
   accgtcattg acgtcaatag ggggcgtact tggcatatga tacacttgat gtactgccaa 3720
   gtgggcagtt taccgtaaat actccaccca ttgacgtcaa tggaaagtcc ctattggcgt 3780
   tactatggga acatacgtca ttattgacgt caatgggcgg gggtcgttgg gcggtcagcc 3840
   aggcgggcca tttaccgtaa gttatgtaac gcctgcaggt taattaagaa catgtgagca 3900
   aaaggccagc aaaaggccag gaaccgtaaa aaggccgcgt tgctggcgtt tttccatagg 3960
   ctccgccccc ctgacgagca tcacaaaaat cgacgctcaa gtcagaggtg gcgaaacccg 4020
   acaggactat aaagatacca ggcgtttccc cctggaagct ccctcgtgcg ctctcctgtt 4080
   ccgaccctgc cgcttaccgg atacctgtcc gcctttctcc cttcgggaag cgtggcgctt 4140
   tctcatagct cacgctgtag gtatctcagt tcggtgtagg tcgttcgctc caagctgggc 4200
   tgtgtgcacg aaccccccgt tcagcccgac cgctgcgcct tatccggtaa ctatcgtctt 4260
   gagtccaacc cggtaagaca cgacttatcg ccactggcag cagccactgg taacaggatt    4320
   agcagagcga ggtatgtagg cggtgctaca gagttcttga agtggtggcc taactacggc    4380
   tacactagaa gaacagtatt tggtatctgc gctctgctga agccagttac cttcggaaaa    4440
   agagttggta gctcttgatc cggcaaacaa accaccgctg gtagcggtgg tttttttgtt    4500
   tgcaagcagc agattacgcg cagaaaaaaa ggatctcaag aagatccttt gatcttttct    4560
   acggggtctg acgctcagtg gaacgaaaac tcacgttaag ggattttggt catggctagt    4620
   taattaacat ttaaatca                                                  4638
<210> 97
   <211> 5349
   <212> DNA
   <213> Artificial
<220>
   <223> pSelect-IGVL2-14/J-Ck
<400> 97
   gcggccgcaa taaaatatct ttattttcat tacatctgtg tgttggtttt ttgtgtgaat      60
   cgtaactaac atacgctctc catcaaaaca aaacgaaaca aaacaaacta gcaaaatagg     120
   ctgtccccag tgcaagtgca ggtgccagaa catttctcta tcgaaggatc tgcgatcgct     180
   ccggtgcccg tcagtgggca gagcgcacat cgcccacagt ccccgagaag ttggggggag     240
   gggtcggcaa ttgaacgggt gcctagagaa ggtggcgcgg ggtaaactgg gaaagtgatg     300
   tcgtgtactg gctccgcctt tttcccgagg gtgggggaga accgtatata agtgcagtag     360
   tcgccgtgaa cgttcttttt cgcaacgggt ttgccgccag aacacagctg aagcttcgag     420
   gggctcgcat ctctccttca cgcgcccgcc gccctacctg aggccgccat ccacgccggt     480
   tgagtcgcgt tctgccgcct cccgcctgtg gtgcctcctg aactgcgtcc gccgtctagg     540
   taagtttaaa gctcaggtcg agaccgggcc tttgtccggc gctcccttgg agcctaccta     600
   gactcagccg gctctccacg ctttgcctga ccctgcttgc tcaactctac gtctttgttt     660
   cgttttctgt tctgcgccgt tacagatcca agctgtgacc ggcgcctacc tgagatcacc     720
   ggcgtgtcga cgccaccatg gacatgagag tgcccgccca gctcctgggg ctcctgctac     780
   tctggctccg aggtaaggat ggagaacact aggaatttac tcagccagtg tgctcagtac     840
   tgactggaac ttcagggaag ttctctgata acatgattaa tagtaagaat atttgttttt     900
   atgtttccaa tctcaggtgc cagatgtcag tctgccctga cccagcccgc ctctgtgtct     960
   ggcagccctg gccagagcat caccatcagc tgcaccggca ccagcagcga cgtgggcggc    1020
   tacaactacg tgtcctggta tcagcagcac cccggcaagg cccccaagct gatgatctac    1080
   gaggtgtcca acagacccag cggcgtgagc aacagattca gcggcagcaa gagcggcaac    1140
   accgccagcc tgaccatcag cggcctccag gctgaggacg aggccgacta ctactgcagc 1200
   agctacacca gcagctccac cctggtgttt ggcggcggaa caaagctgac cgtgctgaga 1260
   gccgacgccg ctcccaccgt gtccatcttc ccccccagca tggaacagct gacctctggc 1320
   ggagccaccg tggtctgctt cgtgaacaac ttctacccca gagacatcag cgtgaagtgg 1380
   aagatcgacg gcagcgagca gagggacggc gtgctggaca gcgtgaccga ccaggacagc 1440
   aaggactcca cctacagcat gagcagcacc ctgagcctga ccaaggtgga gtacgagagg 1500
   cacaacctgt acacctgcga ggtggtgcac aagaccagct ccagccccgt ggtcaagtcc 1560
   ttcaaccgga acgagtgttg agctagctgg ccagacatga taagatacat tgatgagttt 1620
   ggacaaacca caactagact gactcagcct gcctccgtgt ctgggtctcc tggacagtcg 1680
   atcaccatct cctgcactgg aaccagcagt gacgttggtg gttataacta tgtctcctgg 1740
   taccaacagc acccaggcaa agcccccaaa ctcatgattt atgaggtcag taatcggccc 1800
   tcaggggttt ctaatcgctt ctctggctcc aagtctggca acacggcctc cctgaccatc 1860
   tctgggctcc aggctgagga cgaggctgat tattactgca gctcatatac aagcagcagc 1920
   actctcgtat tcggcggagg gaccaagctg accgtcctac gggctgatgc tgcaccaact 1980
   gtatccatct tcccaccatc catggaacag ttaacatctg gaggtgccac agtcgtgtgc 2040
   ttcgtgaaca acttctatcc cagagacatc agtgtcaagt ggaagattga tggcagtgaa 2100
   caacgagatg gtgtcctgga cagtgttact gatcaggaca gcaaagacag cacgtacagc 2160
   atgagcagca ccctctcgtt gaccaaggtt gaatatgaaa ggcataacct ctatacctgt 2220
   gaggttgttc ataagacatc atcctcaccc gtcgtcaaga gcttcaacag gaatgagtgt 2280
   taggctagct ggccagacat gataagatac attgatgagt ttggacaaac cacaactaga 2340
   atgcagtgaa aaaaatgctt tatttgtgaa atttgtgatg ctattgcttt atttgtaacc 2400
   attataagct gcaataaaca agttaacaac aacaattgca ttcattttat gtttcaggtt 2460
   cagggggagg tgtgggaggt tttttaaagc aagtaaaacc tctacaaatg tggtatggaa 2520
   ttctaaaata cagcatagca aaactttaac ctccaaatca agcctctact tgaatccttt 2580
   tctgagggat gaataaggca taggcatcag gggctgttgc caatgtgcat tagctgtttg 2640
   cagcctcacc ttctttcatg gagtttaaga tatagtgtat tttcccaagg tttgaactag 2700
   ctcttcattt ctttatgttt taaatgcact gacctcccac attccctttt tagtaaaata 2760
   ttcagaaata atttaaatac atcattgcaa tgaaaataaa tgttttttat taggcagaat 2820
   ccagatgctc aaggcccttc ataatatccc ccagtttagt agttggactt agggaacaaa 2880
   ggaaccttta atagaaattg gacagcaaga aagcgagctt ctagcgaatt ctcgactcat 2940
   tcctttgccc tcggacgagt gctggggcgt cggtttccac tatcggcgag tacttctaca 3000
   cagccatcgg tccagacggc cgcgcttctg cgggcgattt gtgtacgccc gacagtcccg 3060
   gctccggatc ggacgattgc gtcgcatcga ccctgcgccc aagctgcatc atcgaaattg 3120
   ccgtcaacca agctctgata gagttggtca agaccaatgc ggagcatata cgcccggagc 3180
   cgcggcgatc ctgcaagctc cggatgcctc cgctcgaagt agcgcgtctg ctgctccata 3240
   caagccaacc acggcctcca gaagaagatg ttggcgacct cgtattggga atccccgaac 3300
   atcgcctcgc tccagtcaat gaccgctgtt atgcggccat tgtccgtcag gacattgttg 3360
   gagccgaaat ccgcgtgcac gaggtgccgg acttcggggc agtcctcggc ccaaagcatc 3420
   agctcatcga gagcctgcgc gacggacgca ctgacggtgt cgtccatcac agtttgccag 3480
   tgatacacat ggggatcagc aatcgcgcat atgaaatcac gccatgtagt gtattgaccg 3540
   attccttgcg gtccgaatgg gccgaacccg ctcgtctggc taagatcggc cgcagcgatc 3600
   gcatccatga gctccgcgac gggttgcaga acagcgggca gttcggtttc aggcaggtct 3660
   tgcaacgtga caccctgtgc acggcgggag atgcaatagg tcaggctctc gctgaattcc 3720
   ccaatgtcaa gcacttccgg aatcgggagc gcggccgatg caaagtgccg ataaacataa 3780
   cgatctttgt agaaaccatc ggcgcagcta tttacccgca ggacatatcc acgccctcct 3840
   acatcgaagc tgaaagcacg agattcttcg ccctccgaga gctgcatcag gtcggagacg 3900
   ctgtcgaact tttcgatcag aaacttctcg acagacgtcg cggtgagttc aggctttttc 3960
   atgatggccc tcctatagtg agtcgtatta tactatgccg atatactatg ccgatgatta 4020
   attgtcaaaa cagcgtggat ggcgtctcca gcttatctga cggttcacta aacgagctct 4080
   gcttatatag acctcccacc gtacacgcct accgcccatt tgcgtcaatg gggcggagtt 4140
   gttacgacat tttggaaagt cccgttgatt tactagtcaa aacaaactcc cattgacgtc 4200
   aatggggtgg agacttggaa atccccgtga gtcaaaccgc tatccacgcc cattgatgta 4260
   ctgccaaaac cgcatcatca tggtaatagc gatgactaat acgtagatgt actgccaagt 4320
   aggaaagtcc cataaggtca tgtactgggc ataatgccag gcgggccatt taccgtcatt 4380
   gacgtcaata gggggcgtac ttggcatatg atacacttga tgtactgcca agtgggcagt 4440
   ttaccgtaaa tactccaccc attgacgtca atggaaagtc cctattggcg ttactatggg 4500
   aacatacgtc attattgacg tcaatgggcg ggggtcgttg ggcggtcagc caggcgggcc 4560
   atttaccgta agttatgtaa cgcctgcagg ttaattaaga acatgtgagc aaaaggccag 4620
   caaaaggcca ggaaccgtaa aaaggccgcg ttgctggcgt ttttccatag gctccgcccc 4680
   cctgacgagc atcacaaaaa tcgacgctca agtcagaggt ggcgaaaccc gacaggacta 4740
   taaagatacc aggcgtttcc ccctggaagc tccctcgtgc gctctcctgt tccgaccctg 4800
   ccgcttaccg gatacctgtc cgcctttctc ccttcgggaa gcgtggcgct ttctcatagc 4860
   tcacgctgta ggtatctcag ttcggtgtag gtcgttcgct ccaagctggg ctgtgtgcac 4920
   gaaccccccg ttcagcccga ccgctgcgcc ttatccggta actatcgtct tgagtccaac 4980
   ccggtaagac acgacttatc gccactggca gcagccactg gtaacaggat tagcagagcg 5040
   aggtatgtag gcggtgctac agagttcttg aagtggtggc ctaactacgg ctacactaga 5100
   agaacagtat ttggtatctg cgctctgctg aagccagtta ccttcggaaa aagagttggt 5160
   agctcttgat ccggcaaaca aaccaccgct ggtagcggtg gtttttttgt ttgcaagcag 5220
   cagattacgc gcagaaaaaa aggatctcaa gaagatcctt tgatcttttc tacggggtct 5280
   gacgctcagt ggaacgaaaa ctcacgttaa gggattttgg tcatggctag ttaattaaca 5340
   tttaaatca 5349
<210> 98
   <211> 6772
   <212> DNA
   <213> Artificial
<220>
   <223> MV1043
<400> 98
   cttgatttgg gtgatggttc acgtagtggg ccatcgccct gatagacggt ttttcgccct 60
   ttgacgttgg agtccacgtt ctttaatagt ggactcttgt tccaaactgg aacaacactc 120
   aactctatct cgggctattc ttttgattta taagggattt tgccgatttc ggtctattgg 180
   ttaaaaaatg agctgattta acaaaaattt aacgcgaatt ttaacaaaat attaacgttt 240
   acaattttat ggtgcagtct cagtacaatc tgctctgatg ccgcatagtt aagccagccc 300
   cgacacccgc caacacccgc tgacgcgccc tgacgggctt gtctgctccc ggcatccgct 360
   tacagacaag ctgtgaccgt ctccgggagc tgcatgtgtc agaggttttc accgtcatca 420
   ccgaaacgcg cgagacgaaa gggcctcgtg atacgcctat ttttataggt taatgtcatg 480
   ataataatgg tttcttagac gtcaggtggc acttttcggg gaaatgtgcg cggaacccct 540
   atttgtttat ttttctaaat acattcaaat atgtatccgc tcatgagaca ataaccctga 600
   taaatgcttc aataatattg aaaaaggaag agtatgagta ttcaacattt ccgtgtcgcc 660
   cttattccct tttttgcggc attttgcctt cctgtttttg ctcacccaga aacgctggtg 720
   aaagtaaaag atgctgaaga tcagttgggt gcccgagtgg gttacatcga actggatctc 780
   aacagcggta agatccttga gagttttcgc cccgaagaac gttttccaat gatgagcact 840
   tttaaagttc tgctatgtgg cgcggtatta tcccgtattg acgccgggca agagcaactc 900
   ggtcgccgca tacactattc tcagaatgac ttggttgagt actcaccagt cacagaaaag 960
   catcttacgg atggcatgac agtaagagaa ttatgcagtg ctgccataac catgagtgat 1020
   aacactgcgg ccaacttact tctgacaacg atcggaggac cgaaggagct aaccgctttt 1080
   ttgcacaaca tgggggatca tgtaactcgc cttgatcgtt gggaaccgga gctgaatgaa 1140
   gccataccaa acgacgagcg tgacaccacg atgcctgtag caatggcaac aacgttgcgc 1200
   aaactattaa ctggcgaact acttactcta gcttcccggc aacaattaat agactggatg 1260
   gaggcggata aagttgcagg accacttctg cgctcggccc ttccggctgg ctggtttatt 1320
   gctgataaat ctggagccgg tgagcgtggg tctcgcggta tcattgcagc actggggcca 1380
   gatggtaagc cctcccgtat cgtagttatc tacacgacgg ggagtcaggc aactatggat 1440
   gaacgaaata gacagatcgc tgagataggt gcctcactga ttaagcattg gtaactgtca 1500
   gaccaagttt actcatatat actttagatt gatttaaaac ttcattttta atttaaaagg 1560
   atctaggtga agatcctttt tgataatctc atgaccaaaa tcccttaacg tgagttttcg 1620
   ttccactgag cgtcagaccc cgtagaaaag atcaaaggat cttcttgaga tccttttttt 1680
   ctgcgcgtaa tctgctgctt gcaaacaaaa aaaccaccgc taccagcggt ggtttgtttg 1740
   ccggatcaag agctaccaac tctttttccg aaggtaactg gcttcagcag agcgcagata 1800
   ccaaatactg ttcttctagt gtagccgtag ttaggccacc acttcaagaa ctctgtagca 1860
   ccgcctacat acctcgctct gctaatcctg ttaccagtgg ctgctgccag tggcgataag 1920
   tcgtgtctta ccgggttgga ctcaagacga tagttaccgg ataaggcgca gcggtcgggc 1980
   tgaacggggg gttcgtgcat acagcccagc ttggagcgaa cgacctacac cgaactgaga 2040
   tacctacagc gtgagctatg agaaagcgcc acgcttcccg aagggagaaa ggcggacagg 2100
   tatccggtaa gcggcagggt cggaacagga gagcgcacga gggagcttcc agggggaaac 2160
   gcctggtatc tttatagtcc tgtcgggttt cgccacctct gacttgagcg tcgatttttg 2220
   tgatgctcgt caggggggcg gagcctatgg aaaaacgcca gcaacgcggc ctttttacgg 2280
   ttcctggcct tttgctggcc ttttgctcac atgttctttc ctgcgttatc ccctgattct 2340
   gtggataacc gtattaccgc ctttgagtga gctgataccg ctcgccgcag ccgaacgacc 2400
   gagcgcagcg agtcagtgag cgaggaagcg gaagagcgcc caatacgcaa accgcctctc 2460
   cccgcgcgtt ggccgattca ttaatgcagc tggcacgaca ggtttcccga ctggaaagcg 2520
   ggcagtgagc gcaacgcaat taatgtgagt tagctcactc attaggcacc ccaggcttta 2580
   cactttatgc ttccggctcg tatgttgtgt ggaattgtga gcggataaca atttcacaca 2640
   ggaaacagct atgaccatga ttacgccaag ctttggagcc ttttttttgg agattttcaa 2700
   cgtgaaaaaa ttattattcg caattccttt agttgttcct ttctattctc acagtgcaca 2760
   gatccaaatg acccagtctc catcctccct gtctgcatct gtaggagaca gagtcaccat 2820
   cacttgccgg gcaagtcaga gcattagcag ctacttaaat tggtatcagc agaaaccagg 2880
   gaaagcccct aagctcctga tctatgctgc atccagtttg caaagtgggg tcccatcaag 2940
   gttcagtggc agtggatctg ggacagattt cactctcacc atcagcagtc tgcaacctga 3000
   agattttgca acttactact gtcaacagag ttacagtacc cctccaacgt tcggccaagg 3060
   gaccaagctc gagatcaaac gtactgtggc tgcaccatct gtcttcatct tcccgccatc 3120
   tgatgagcag ttgaaatctg gaactgcctc tgttgtgtgc ctgctgaata acttctatcc 3180
   cagagaggcc aaagtacagt ggaaggtgga taacgccctc caatcgggta actcccagga 3240
   gagtgtcaca gagcaggaca gcaaggacag cacctacagc ctcagcagca ccctgacgct 3300
   gagcaaagca gactacgaga aacacaaagt ctacgcctgc gaagtcaccc atcagggcct 3360
   gagctcgccc gtcacaaaga gcttcaacag gggagagtgt tagtaaggcg cgccaattct 3420
   atttcaagga gacagtcata atgaaatacc tattgcctac ggcagccgct ggattgttat 3480
   tactcgcggc ccagccggcc atggcgatgc ctgcttgccg aatatcatgg tggaaaatgg 3540
   ccgcttttct ggattcatcg actgtggccg gctgggtgtg gcggaccgct atcaggacat 3600
   agcgttggct acccgtgata ttgctgaaga gcttggcggc gaatgggctg accgcttcct 3660
   cgtgctttac ggtatcgccg ctcccgattc gcagcgcatc gccttctatc gccttcttga 3720
   cgagttcttc tgagcgggac tctggggttc ggtgctacga gatttcgatt ccaccgccgc 3780
   cttctatgaa aggttgggct tcggaatcgt tttccgggac gccggctgga tgatcctcca 3840
   gcgcggggat ctcatgctgg agttcttcgc ccaccccaac ttgtttattg cagcttataa 3900
   tggttacaaa taaagcaata gcatcacaaa tttcacaaat aaagcatttt tttcactgca 3960
   ttctagttgt ggtttgtcca aactcatcaa tgtatcttat catgtctgta taccgtcgac 4020
   ctctagctag agcttggcgt aatcatggtc atagctgttt cctgtgtgaa attgttatcc 4080
   gctcacaatt ccacacaaca tacgagccgg aagcataaag tgtaaagcct ggggtgccta 4140
   atgagtgagc taactcacat taattgcgtt gcgctcactg cccgctttcc agtcgggaaa 4200
   cctgtcgtgc cagaattgca tgaagaatct gcttagggtt aggcgttttg cgctgcttcg 4260
   ctaggtggtc aatattggcc attagccata ttattcattg gttatatagc ataaatcaat 4320
   attggctatt ggccattgca tacgttgtat ccatatcata atatgtacat ttatattggc 4380
   tcatgtccaa cattaccgcc atgttgacat tgattattga ctagttatta atagtaatca 4440
   attacggggt cattagttca tagcccatat atggagttcc gcgttacata acttacggta 4500
   aatggcccgc ctggctgacc gcccaacgac ccccgcccat tgacgtcaat aatgacgtat 4560
   gttcccatag taacgccaat agggactttc cattgacgtc aatgggtgga gtatttacgg 4620
   taaactgccc acttggcagt acatcaagtg tatcatatgc caagtacgcc ccctattgac 4680
   gtcaatgacg gtcaccgtct caagcgcctc caccaagggc ccatcggtct tccccctggc 4740
   accctcctcc aagagcacct ctgggggcac agcggccctg ggctgcctgg tcaaggacta 4800
   cttccccgaa ccggtgacgg tgtcgtggaa ctcaggcgcc ctgaccagcg gcgtccacac 4860
   cttcccggct gtcctacagt cctcaggact ctactccctc agcagcgtag tgaccgtgcc 4920
   ctccagcagc ttgggcaccc agacctacat ctgcaacgtg aatcacaagc ccagcaacac 4980
   caaggtggac aagaaagttg agcccaaatc ttgtgcggcc gcacatcatc atcaccatca 5040
   cggggccgca gaacaaaaac tcatctcaga agaggatctg aatggggccg catagactgt 5100
   tgaaagttgt ttagcaaaac ctcatacaga aaattcattt actaacgtct ggaaagacga 5160
   caaaacttta gatcgttacg ctaactatga gggctgtctg tggaatgcta caggcgttgt 5220
   ggtttgtact ggtgacgaaa ctcagtgtta cggtacatgg gttcctattg ggcttgctat 5280
   ccctgaaaat gagggtggtg gctctgaggg tggcggttct gagggtggcg gttctgaggg 5340
   tggcggtact aaacctcctg agtacggtga tacacctatt ccgggctata cttatatcaa 5400
   ccctctcgac ggcacttatc cgcctggtac tgagcaaaac cccgctaatc ctaatccttc 5460
   tcttgaggag tctcagcctc ttaatacttt catgtttcag aataataggt tccgaaatag 5520
   gcagggtgca ttaactgttt atacgggcac tgttactcaa ggcactgacc ccgttaaaac 5580
   ttattaccag tacactcctg tatcatcaaa agccatgtat gacgcttact ggaacggtaa 5640
   attcagagac tgcgctttcc attctggctt taatgaggat ccattcgttt gtgaatatca 5700
   aggccaatcg tctgacctgc ctcaacctcc tgtcaatgct ggcggcggct ctggtggtgg 5760
   ttctggtggc ggctctgagg gtggcggctc tgagggtggc ggctctgagg gtggcggttc 5820
   tgagggtggc ggctctgagg gtggcggttc cggtggcggc tccggttccg gtgattttga 5880
   ttatgaaaaa atggcaaacg ctaataaggg ggctatgacc gaaaatgccg atgaaaacgc 5940
   gctacagtct gacgctaaag gcaaacttga ttctgtcgct actgattacg gtgctgctat 6000
   cgatggtttc attggtgacg tttccggcct tgctaatggt aatggtgcta ctggtgattt 6060
   tgctggctct aattcccaaa tggctcaagt cggtgacggt gataattcac ctttaatgaa 6120
   taatttccgt caatatttac cttctttgcc tcagtcggtt gaatgtcgcc cttatgtctt 6180
   tggcgctggt aaaccatatg aattttctat tgattgtgac aaaataaact tattccgtgg 6240
   tgtctttgcg tttcttttat atgttgccac ctttatgtat gtattttcga cgtttgctaa 6300
   catactgcgt aataaggagt cttaataaga attcactggc cgtcgtttta caacgtcgtg 6360
   actgggaaaa ccctggcgtt acccaactta atcgccttgc agcacatccc cctttcgcca 6420
   gctggcgtaa tagcgaagag gcccgcaccg atcgcccttc ccaacagttg cgcagcctga 6480
   atggcgaatg gcgcctgatg cggtattttc tccttacgca tctgtgcggt atttcacacc 6540
   gcatacgtca aagcaaccat agtacgcgcc ctgtagcggc gcattaagcg cggcgggtgt 6600
   ggtggttacg cgcagcgtga ccgctacact tgccagcgcc ttagcgcccg ctcctttcgc 6660
   tttcttccct tcctttctcg ccacgttcgc cggctttccc cgtcaagctc taaatcgggg 6720
   gctcccttta gggttccgat ttagtgcttt acggcacctc gaccccaaaa aa 6772
<210> 99
   <211> 10293
   <212> DNA
   <213> Artificial
<220>
   <223> MV1057
<400> 99
   tactcttcct ttttcaatat tattgaagca tttatcaggg ttattgtctc atgagcggat      60
   acatatttga atgtatttag aaaaataaac aaataggggt tccgcgcaca tttccccgaa     120
   aagtgccacc tgacgtcgac ggatcgggag atctcccgat cccctatggt gcactctcag     180
   tacaatctgc tctgatgccg catagttaag ccagtatctg ctccctgctt gtgtgttgga     240
   ggtcgctgag tagtgcgcga gcaaaattta agctacaaca aggcaaggct tgaccgacaa     300
   ttgcatgaag aatctgctta gggttaggcg ttttgcgctg cttcgctagg tggtcaatat     360
   tggccattag ccatattatt cattggttat atagcataaa tcaatattgg ctattggcca     420
   ttgcatacgt tgtatccata tcataatatg tacatttata ttggctcatg tccaacatta     480
   ccgccatgtt gacattgatt attgactagt tattaatagt aatcaattac ggggtcatta     540
   gttcatagcc catatatgga gttccgcgtt acataactta cggtaaatgg cccgcctggc     600
   tgaccgccca acgacccccg cccattgacg tcaataatga cgtatgttcc catagtaacg     660
   ccaataggga ctttccattg acgtcaatgg gtggagtatt tacggtaaac tgcccacttg     720
   gcagtacatc aagtgtatca tatgccaagt acgcccccta ttgacgtcaa tgacggtaaa     780
   tggcccgcct ggcattatgc ccagtacatg accttatggg actttcctac ttggcagtac     840
   atctacgtat tagtcatcgc tattaccatg gtgatgcggt tttggcagta catcaatggg     900
   cgtggatagc ggtttgactc acggggattt ccaagtctcc accccattga cgtcaatggg     960
   agtttgtttt ggcaccaaaa tcaacgggac tttccaaaat gtcgtaacaa ctccgcccca    1020
   ttgacgcaaa tgggcggtag gcgtgtacgg tgggaggtct atataagcag agctcgttta    1080
   gtgaaccgtc agatcgcctg gagacgccat ccacgctgtt ttgacctcca tagaagacac    1140
   cgggaccgat ccagcctccg cggccgggaa cggtgcattg gaagcttggt accggtgaat    1200
   tggccggccc gcgccgtcga ggttatcgat ccgaccgacg cgttcgcgag aggccgcaat    1260
   tccctagcca ccatgggatg gagctgtatc atcctcttct tggtactgct gctggcccag    1320
   ccggccatgg ggcggagaat gggcggaact gggcggagtt aggggcggga tgggcggagt    1380
   taggggcggg actatggttg ctgactaatt gagatgcgga tccgctggca cgacaggttt    1440
   cccgactgga aagcgggcag tgagcgcaac gcaattaatg tgagttagct cactcattag    1500
   gcaccccagg ctttacactt tatgcttccg gctcgtatgt tgtgtggaat tgtgagcgga    1560
   taacaatttc acacaggaaa cagctatgac catgattacg ccaagcttgg gctgcaggtt    1620
   ctttccgcct cagaagccat agagcccacc gcatccccag catgcctgct attgtcttcc   1680
   caatcctccc ccttgctgtc ctgccccacc ccacccccca gaatagaatg acacctactc   1740
   agacaatgcg atgcaatttc ctcattttat taggaaagga cagtgggagt ggcaccttcc   1800
   agggtcaagg aaggcacggg ggaggggcaa acaacagatg gctggcaact agaaggcaca   1860
   gtcgaggctg atcagcgagc tctagatcat cgatgcatgg ggtcgtgcgc tcctttcggt   1920
   cgggcgctgc gggtcgtggg gcgggcgtca ggcaccgggc ttgcgggtca tgcaccaggt   1980
   gcgcggtcct tcgggcacct cgacgtcggc ggtgacggtg aagccgagcc gctcgtagaa   2040
   ggggaggttg cggggcgcgg aggtctccag gaaggcgggc accccggcgc gctcggccgc   2100
   ctccactccg gggagcacga cggcgctgcc cagacccttg ccctggtggt cgggcgagac   2160
   gccgacggtg gccaggaacc acgcgggctc cttgggccgg tgcggcgcca ggaggccttc   2220
   catctgttgc tgcgcggcca gccgggaacc gctcaactcg gccatgcgcg ggccgatctc   2280
   ggcgaacacc gcccccgctt cgacgctctc cggcgtggtc cagaccgcca ccgcggcgcc   2340
   gtcgtccgcg acccacacct tgccgatgtc gagcccgacg cgcgtgagga agagttcttg   2400
   cagctcggtc accgtctcca gtgctagcac caagggccca tcggtcttcc ccctggcacc   2460
   ctcctccaag agcacctctg ggggcacagc ggccctgggc tgcctggtca aggactactt   2520
   ccccgaaccg gtgacggtgt cgtggaactc aggcgccctg accagcggcg tgcacacctt   2580
   cccggctgtc ctacagtcct caggactcta ctccctcagc agcgtcgtga ccgtgccctc   2640
   cagcagcttg ggcacccaga cctacatctg caacgtgaat cacaagccca gcaacaccaa   2700
   ggtggacaag agagttggtg agaggccagc acagggaggg agggtgtctg ctggaagcca   2760
   ggctcagcgc tcctgcctgg acgcatcccg gctatgcagt cccagtccag ggcagcaagg   2820
   caggccccgt ctgcctcttc acccggaggc ctctgcccgc cccactcatg ctcagggaga   2880
   gggtcttctg gctttttccc caggctctgg gcaggcacag gctaggtgcc cctaacccag   2940
   gccctgcaca caaaggggca ggtgctgggc tcagacctgc caagagccat atccgggagg   3000
   accctgcccc tgacctaagc ccaccccaaa ggccaaactc tccactccct cagctcggac   3060
   accttctctc ctcccagatt ccagtaactc ccaatcttct ctctgcagag cccaaatctt   3120
   gtgacaaaac tcacacatgc ccaccgtgcc cagcacctga actcctgggg ggaccgtcag   3180
   tcttcctctt ccccccaaaa cccaaggaca ccctcatgat ctcccggacc cctgaggtca   3240
   catgcgtggt ggtggacgtg agccacgaag accctgaggt caagttcaac tggtacgtgg   3300
   acggcgtgga ggtgcataat gccaagacaa agccgcggga ggagcagtac aacagcacgt   3360
   accgtgtggt cagcgtcctc accgtcctgc accaggactg gctgaatggc aaggagtaca   3420
   agtgcaaggt ctccaacaaa gccctcccag cccccatcga gaaaaccatc tccaaagcca   3480
   aagggcagcc ccgagaacca caggtgtaca ccctgccccc atcccgggag gagatgacca 3540
   agaaccaggt cagcctgacc tgcctggtca aaggcttcta tcccagcgac atcgccgtgg 3600
   agtgggagag caatgggcag ccggagaaca actacaagac cacgcctccc gtgctggact 3660
   ccgacggctc cttcttcctc tatagcaagc tcaccgtgga caagagcagg tggcagcagg 3720
   ggaacgtctt ctcatgctcc gtgatgcatg aggctctgca caaccactac acgcagaaga 3780
   gcctctccct gtctccgggt aaatgagttt aacggatctt aattaatccg agctcggtac 3840
   caagcttaag tttaaaccgc tgatcagcct cgactgtgcc ttctagttgc cagccatctg 3900
   ttgtttgccc ctcccccgtg ccttccttga ccctggaagg tgccactccc actgtccttt 3960
   cctaataaaa tgaggaaatt gcatcgcatt gtctgagtag gtgtcattct attctggggg 4020
   gtggggtggg gcaggacagc aagggggagg attgggaaga caatagcagg catgctgggg 4080
   atgcggtggg ctctatggct tctgaggcgg aaagaaccag ctggggctct agggggtatc 4140
   cccacgcgcc ctgtagcggc gcattaagcg cggcgggtgt ggtggttacg cgcagcgtga 4200
   ccgctacact tgccagcgcc tagcgcccgc tcctttcgct ttcttccctt cctttctcgc 4260
   cacgttcgcc ggctttcccc gtcaagctct aaatcggggg ctccctttag ggttccgatt 4320
   tagtgcttta cggcacctcg accccaaaaa acttgattag ggtgatggtt cacgtagtgg 4380
   gccatcgccc tgatagacgg tttttcgccc tttgacgttg gagtccacgt tctttaatag 4440
   tggactcttg ttccaaactg gaacaacact caaccctatc tcggtctatt cttttgattt 4500
   ataagggatt ttgccgattt cggcctattg gttaaaaaat gagctgattt aacaaaaatt 4560
   taacgcgaat taattctgtg gaatgtgtgt cagttagggt gtggaaagtc cccaggctcc 4620
   ccagcaggca gaagtatgca aagcatgcat ctcaattagt cagcaaccag gtgtggaaag 4680
   tccccaggct ccccagcagg cagaagtatg caaagcatgc atctcaatta gtcagcaacc 4740
   atagtcccgc ccctaactcc gcccatcccg cccctaactc cgcccagttc cgcccattct 4800
   ccgccccatg gctgactaat tttttttatt tatgcagagg ccgaggccgc ctctgcctct 4860
   gagctattcc agaagtagtg aggaggcttt tttggaggcc taggcttttg caaaaagctc 4920
   ccgggagctt ggatatccat tttcggatct gatcaagaga caggatgagg atcgtttcgc 4980
   atgattgaac aagatggatt gcacgcaggt tctccggccg cttgggtgga gaggctattc 5040
   ggctatgact gggcacaaca gacaatcggc tgctctgatg ccgccgtgtt ccggctgtca 5100
   gcgcaggggc gcccggttct ttttgtcaag accgacctgt ccggtgccct gaatgaactg 5160
   caggacgagg cagcgcggct atcgtggctg gccacgacgg gcgttccttg cgcagctgtg 5220
   ctcgacgttg tcactgaagc gggaagggac tggctgctat tgggcgaagt gccggggcag 5280
   gatctcctgt catctcacct tgctcctgcc gagaaagtat ccatcatggc tgatgcaatg 5340
   cggcggctgc atacgcttga tccggctacc tgcccattcg accaccaagc gaaacatcgc 5400
   atcgagcgag cacgtactcg gatggaagcc ggtcttgtcg atcaggatga tctggacgaa 5460
   gagcatcagg ggctcgcgcc agccgaactg ttcgccaggc tcaaggcgcg catgcccgac 5520
   ggcgaggatc tcgtcgtgac ccatggcgat gcctgcttgc cgaatatcat ggtggaaaat 5580
   ggccgctttt ctggattcat cgactgtggc cggctgggtg tggcggaccg ctatcaggac 5640
   atagcgttgg ctacccgtga tattgctgaa gagcttggcg gcgaatgggc tgaccgcttc 5700
   ctcgtgcttt acggtatcgc cgctcccgat tcgcagcgca tcgccttcta tcgccttctt 5760
   gacgagttct tctgagcggg actctggggt tcggtgctac gagatttcga ttccaccgcc 5820
   gccttctatg aaaggttggg cttcggaatc gttttccggg acgccggctg gatgatcctc 5880
   cagcgcgggg atctcatgct ggagttcttc gcccacccca acttgtttat tgcagcttat 5940
   aatggttaca aataaagcaa tagcatcaca aatttcacaa ataaagcatt tttttcactg 6000
   cattctagtt gtggtttgtc caaactcatc aatgtatctt atcatgtctg tataccgtcg 6060
   acctctagct agagcttggc gtaatcatgg tcatagctgt ttcctgtgtg aaattgttat 6120
   ccgctcacaa ttccacacaa catacgagcc ggaagcataa agtgtaaagc ctggggtgcc 6180
   taatgagtga gctaactcac attaattgcg ttgcgctcac tgcccgcttt ccagtcggga 6240
   aacctgtcgt gccagaattg catgaagaat ctgcttaggg ttaggcgttt tgcgctgctt 6300
   cgctaggtgg tcaatattgg ccattagcca tattattcat tggttatata gcataaatca 6360
   atattggcta ttggccattg catacgttgt atccatatca taatatgtac atttatattg 6420
   gctcatgtcc aacattaccg ccatgttgac attgattatt gactagttat taatagtaat 6480
   caattacggg gtcattagtt catagcccat atatggagtt ccgcgttaca taacttacgg 6540
   taaatggccc gcctggctga ccgcccaacg acccccgccc attgacgtca ataatgacgt 6600
   atgttcccat agtaacgcca atagggactt tccattgacg tcaatgggtg gagtatttac 6660
   ggtaaactgc ccacttggca gtacatcaag tgtatcatat gccaagtacg ccccctattg 6720
   acgtcaatga cggtaaatgg cccgcctggc attatgccca gtacatgacc ttatgggact 6780
   ttcctacttg gcagtacatc tacgtattag tcatcgctat taccatggtg atgcggtttt 6840
   ggcagtacat caatgggcgt ggatagcggt ttgactcacg gggatttcca agtctccacc 6900
   ccattgacgt caatgggagt ttgttttggc accaaaatca acgggacttt ccaaaatgtc 6960
   gtaacaactc cgccccattg acgcaaatgg gcggtaggcg tgtacggtgg gaggtctata 7020
   taagcagagc tcgtttagtg aaccgtcaga tcgcctggag acgccatcca cgctgttttg 7080
   acctccatag aagacaccgg gaccgatcca gcctccgcgg ccgggaacgg tgcattggaa 7140
   gcttggtacc ggtgaattag gcgcgccgtc gaggttatcg atccgaccga cgcgttcgcg 7200
   agaggccgca attccctagc caccatggca tgccctggct tcctgtgggc acttgtgatc 7260
   tccacctgtc ttgaattctc catggctgac atccagatga cccagtctcc atcctccctg 7320
   tctgcatctg taggagacag agtcaccatc acttgccggg caagtcagag cattagcagc 7380
   tacttaaatt ggtatcagca gaaaccaggg aaagccccta agctcctgat ctatgctgca 7440
   tccagtttgc aaagtggggt cccatcaagg ttcagtggca gtggatctgg gacagatttc 7500
   actctcacca tcagcagtct gcaacctgaa gattttgcaa cttactactg tcaacagagt 7560
   tacagtaccc ctccaacgtt cggccaaggg accaaggtgg agatcaaacg taagtgcact 7620
   ttgcggccgc taggaagaaa ctcaaaacat caagatttta aatacgcttc ttggtctcct 7680
   tgctataatt atctgggata agcatgctgt tttctgtctg tccctaacat gccctgtgat 7740
   tatccgcaaa caacacaccc aagggcagaa ctttgttact taaacaccat cctgtttgct 7800
   tctttcctca ggaactgtgg ctgcaccatc tgtcttcatc ttcccgccat ctgatgagca 7860
   gttgaaatct ggaactgcct ctgttgtgtg cctgctgaat aacttctatc ccagagaggc 7920
   caaagtacag tggaaggtgg ataacgccct ccaatcgggt aactcccagg agagtgtcac 7980
   agagcaggac agcaaggaca gcacctacag cctcagcagc accctgacgc tgagcaaagc 8040
   agactacgag aaacacaaag tctacgcctg cgaagtcacc catcagggcc tgagctcgcc 8100
   cgtcacaaag agcttcaaca ggggagagtg ttaggtttaa cggatccgag ctcggtacca 8160
   agctcaagtt taaaccgctg atcagcctcg actgtgcctt ctagttgcca gccatctgtt 8220
   gtttgcccct cccccgtgcc ttccttgacc ctggaaggtg ccactcccac tgtcctttcc 8280
   taataaaatg aggaaattgc atcgcattgt ctgagtaggt gtcattctat tctggggggt 8340
   ggggtggggc aggacagcaa gggggaggat tgggaagaca atagcaggca tgctggggat 8400
   gcggtgggct ctatggcttc tgaggcggaa agaaccagct gcattaatga atcggccaac 8460
   gcgcggggag aggcggtttg cgtattgggc gctcttccgc ttcctcgctc actgactcgc 8520
   tgcgctcggt cgttcggctg cggcgagcgg tatcagctca ctcaaaggcg gtaatacggt 8580
   tatccacaga atcaggggat aacgcaggaa agaacatgtg agcaaaaggc cagcaaaagg 8640
   ccaggaaccg taaaaaggcc gcgttgctgg cgtttttcca taggctccgc ccccctgacg 8700
   agcatcacaa aaatcgacgc tcaagtcaga ggtggcgaaa cccgacagga ctataaagat 8760
   accaggcgtt tccccctgga agctccctcg tgcgctctcc tgttccgacc ctgccgctta 8820
   ccggatacct gtccgccttt ctcccttcgg gaagcgtggc gctttctcat agctcacgct 8880
   gtaggtatct cagttcggtg taggtcgttc gctccaagct gggctgtgtg cacgaacccc 8940
   ccgttcagcc cgaccgctgc gccttatccg gtaactatcg tcttgagtcc aacccggtaa 9000
   gacacgactt atcgccactg gcagcagcca ctggtaacag gattagcaga gcgaggtatg 9060
   taggcggtgc tacagagttc ttgaagtggt ggcctaacta cggctacact agaagaacag 9120
   tatttggtat ctgcgctctg ctgaagccag ttaccttcgg aaaaagagtt ggtagctctt 9180
   gatccggcaa acaaaccacc gctggtagcg gtggtttttt tgtttgcaag cagcagatta 9240
   cgcgcagaaa aaaaggatct caagaagatc ctttgatctt ttctacgggg tctgacgctc 9300
   agtggaacga aaactcacgt taagggattt tggtcatgag attatcaaaa aggatcttca 9360
   cctagatcct tttaaattaa aaatgaagtt ttaaatcaat ctaaagtata tatgagtaaa 9420
   cttggtctga cagttaccaa tgcttaatca gtgaggcacc tatctcagcg atctgtctat 9480
   ttcgttcatc catagttgcc tgactccccg tcgtgtagat aactacgata cgggagggct 9540
   taccatctgg ccccagtgct gcaatgatac cgcgagaccc acgctcaccg gctccagatt 9600
   tatcagcaat aaaccagcca gccggaaggg ccgagcgcag aagtggtcct gcaactttat 9660
   ccgcctccat ccagtctatt aattgttgcc gggaagctag agtaagtagt tcgccagtta 9720
   atagtttgcg caacgttgtt gccattgcta caggcatcgt ggtgtcacgc tcgtcgtttg 9780
   gtatggcttc attcagctcc ggttcccaac gatcaaggcg agttacatga tcccccatgt 9840
   tgtgcaaaaa agcggttagc tccttcggtc ctccgatcgt tgtcagaagt aagttggccg 9900
   cagtgttatc actcatggtt atggcagcac tgcataattc tcttactgtc atgccatccg 9960
   taagatgctt ttctgtgact ggtgagtact caaccaagtc attctgagaa tagtgtatgc 10020
   ggcgaccgag ttgctcttgc ccggcgtcaa tacgggataa taccgcgcca catagcagaa 10080
   ctttaaaagt gctcatcatt ggaaaacgtt cttcggggcg aaaactctca aggatcttac 10140
   cgctgttgag atccagttcg atgtaaccca ctcgtgcacc caactgatct tcagcatctt 10200
   ttactttcac cagcgtttct gggtgagcaa aaacaggaag gcaaaatgcc gcaaaaaagg 10260
   gaataagggc gacacggaaa tgttgaatac tca 10293
<210> 100
   <211> 8179
   <212> DNA
   <213> Artificial
<220>
   <223> pCAGGS-IgVKl-39 targeting vector
<400> 100
   atccaggcgc ggatcaataa aagatcatta ttttcaatag atctgtgtgt tggttttttg      60
   tgtgccttgg gggaggggga ggccagaatg aggcgcggcc aagggggagg gggaggccag      120
   aatgaccttg ggggaggggg aggccagaat gaccttgggg gagggggagg ccagaatgag     180
   gcgcggatcc ggagaagttc ctattccgaa gttcctattc ttcaaatagt ataggaactt     240
   cgctcgaggg atcggccatt gaacaagatg gattgcacgc aggttctccg gccgcttggg     300
   tggagaggct attcggctat gactgggcac aacagacaat cggctgctct gatgccgccg     360
   tgttccggct gtcagcgcag gggcgcccgg ttctttttgt caagaccgac ctgtccggtg     420
   ccctgaatga actgcaggac gaggcagcgc ggctatcgtg gctggccacg acgggcgttc 480
   cttgcgcagc tgtgctcgac gttgtcactg aagcgggaag ggactggctg ctattgggcg 540
   aagtgccggg gcaggatctc ctgtcatctc accttgctcc tgccgagaaa gtatccatca 600
   tggctgatgc aatgcggcgg ctgcatacgc ttgatccggc tacctgccca ttcgaccacc 660
   aagcgaaaca tcgcatcgag cgagcacgta ctcggatgga agccggtctt gtcgatcagg 720
   atgatctgga cgaagagcat caggggctcg cgccagccga actgttcgcc aggctcaagg 780
   cgcgcatgcc cgacggcgag gatctcgtcg tgacccatgg cgatgcctgc ttgccgaata 840
   tcatggtgga aaatggccgc ttttctggat tcatcgactg tggccggctg ggtgtggcgg 900
   accgctatca ggacatagcg ttggctaccc gtgatattgc tgaagagctt ggcggcgaat 960
   gggctgaccg cttcctcgtg ctttacggta tcgccgctcc cgattcgcag cgcatcgcct 1020
   tctatcgcct tcttgacgag ttcttctgag gggatcgatc cgctgtaagt ctgcagaaat 1080
   tgatgatcta ttaaacaata aagatgtcca ctaaaatgga agtttttcct gtcatacttt 1140
   gttaagaagg gtgagaacag agtacctaca ttttgaatgg aaggattgga gctacggggg 1200
   tgggggtggg gtgggattag ataaatgcct gctctttact gaaggctctt tactattgct 1260
   ttatgataat gtttcatagt tggatatcat aatttaaaca agcaaaacca aattaagggc 1320
   cagctcattc ctcccactca tgatctatag atctatagat ctctcgtggg atcattgttt 1380
   ttctcttgat tcccactttg tggttctaag tactgtggtt tccaaatgtg tcagtttcat 1440
   agcctgaaga acgagatcag cagcctctgt tccacataca cttcattctc agtattgttt 1500
   tgccaagttc taattccatc agaagctgac tctagatggc gcgtatgcag gttttcgaca 1560
   ttgattattg actagttatt aatagtaatc aattacgggg tcattagttc atagcccata 1620
   tatggagttc cgcgttacat aacttacggt aaatggcccg cctggctgac cgcccaacga 1680
   cccccgccca ttgacgtcaa taatgacgta tgttcccata gtaacgccaa tagggacttt 1740
   ccattgacgt caatgggtgg agtatttacg gtaaactgcc cacttggcag tacatcaagt 1800
   gtatcatatg ccaagtacgc cccctattga cgtcaatgac ggtaaatggc ccgcctggca 1860
   ttatgcccag tacatgacct tatgggactt tcctacttgg cagtacatct acgtattagt 1920
   catcgctatt accatggtcg aggtgagccc cacgttctgc ttcactctcc ccatctcccc 1980
   cccctcccca cccccaattt tgtatttatt tattttttaa ttattttgtg cagcgatggg 2040
   ggcggggggg gggggggcgc gcgccaggcg gggcggggcg gggcgagggg cggggcgggg 2100
   cgaggcggag aggtgcggcg gcagccaatc agagcggcgc gctccgaaag tttcctttta 2160
   tggcgaggcg gcggcggcgg cggccctata aaaagcgaag cgcgcggcgg gcgggagtcg 2220
   ctgcgttgcc ttcgccccgt gccccgctcc gcgccgcctc gcgccgcccg ccccggctct 2280
   gactgaccgc gttactccca caggtgagcg ggcgggacgg cccttctcct ccgggctgta 2340
   attagcgctt ggtttaatga cggctcgttt cttttctgtg gctgcgtgaa agccttaaag   2400
   ggctccggga gggccctttg tgcggggggg agcggctcgg ggggtgcgtg cgtgtgtgtg   2460
   tgcgtgggga gcgccgcgtg cggcccgcgc tgcccggcgg ctgtgagcgc tgcgggcgcg   2520
   gcgcggggct ttgtgcgctc cgcgtgtgcg cgaggggagc gcggccgggg gcggtgcccc   2580
   gcggtgcggg ggggctgcga ggggaacaaa ggctgcgtgc ggggtgtgtg cgtggggggg   2640
   tgagcagggg gtgtgggcgc ggcggtcggg ctgtaacccc cccctgcacc cccctccccg   2700
   agttgctgag cacggcccgg cttcgggtgc ggggctccgt gcggggcgtg gcgcggggct   2760
   cgccgtgccg ggcggggggt ggcggcaggt gggggtgccg ggcggggcgg ggccgcctcg   2820
   ggccggggag ggctcggggg aggggcgcgg cggccccgga gcgccggcgg ctgtcgaggc   2880
   gcggcgagcc gcagccattg ccttttatgg taatcgtgcg agagggcgca gggacttcct   2940
   ttgtcccaaa tctgtgcgga gccgaaatct gggaggcgcc gccgcacccc ctctagcggg   3000
   cgcggggcga agcggtgcgg cgccggcagg aaggaaatgg gcggggaggg ccttcgtgcg   3060
   tcgccgcgcc gccgtcccct tctccctctc cagcctcggg gctgtccgcg gggggacggc   3120
   tgccttcggg ggggacgggg cagggcgggg ttcggcttct ggcgtgtgac cggcggctct   3180
   agaagcgttg gggtgagtac tccctctcaa aagcgggcat gacttctgcg ctaagattgt   3240
   cagtttccaa aaacgaggag gatttgatat tcacctggcc cgcggtgatg cctttgaggg   3300
   tggccgcgtc catctggtca gaaaagacaa tctttttgtt gtcaagcttg aggtgtggca   3360
   ggcttgagat ctggccatac acttgagtga cattgacatc cactttgcct ttctctccac   3420
   aggtgtccac tcccagggcg gcctccggag cgatcgccga tccgcctagg caattgttta   3480
   aatcggccgg ccataacttc gtataatgta tgctatacga agttatggat cctcacagta   3540
   ggtggcatcg ttcctttctg actgcccgcc ccccgcatgc cgtcccgcga tattgagctc   3600
   cgaacctctc gccctgccgc cgccggtgct ccgtcgccgc cgcgccgcca tggaatcgaa   3660
   gccaccatgg atcttaccgg aaaactcgac gcaagaaaaa tcagagagat cctcataaag   3720
   gtcaagaagg gcggaaagat cgccgtgtaa ttctagaccg gttcgagatc caggcgcgga   3780
   tcaataaaag atcattattt tcaatagatc tgtgtgttgg ttttttgtgt gccttggggg   3840
   agggggaggc cagaatgagg cgcggccaag gggggagggg aggccagaat gaccttgggg   3900
   gagggggagg ccagaatgac cttgggggag ggggaggcca gaatgaggcg cgccctccgt   3960
   cgacctataa cttcgtataa tgtatgctat acgaagttat ggcggccgcc accatggaca   4020
   tgagagtgcc cgcccagctc ctggggctcc tgctactctg gctccgaggt aaggatggag   4080
   aacactagga atttactcag ccagtgtgct cagtactgac tggaacttca gggaagttct   4140
   ctgataacat gattaatagt aagaatattt gtttttatgt ttccaatctc aggtgccaga   4200
   tgtgacatcc agatgaccca gagccccagc agcctgagcg ccagcgtggg cgacagagtg 4260
   accatcacct gcagagccag ccagagcatc agcagctacc tgaactggta tcagcagaag 4320
   cccggcaagg cccccaagct gctgatctac gccgccagct ccctgcagag cggcgtgccc 4380
   agcagattca gcggcagcgg ctccggcacc gacttcaccc tgaccatcag cagcctgcag 4440
   cccgaggact tcgccaccta ctactgccag cagagctaca gcaccccccc caccttcggc 4500
   cagggcacca aggtggagat caagagagcc gacgccgctc ccaccgtgtc catcttcccc 4560
   cccagcatgg aacagctgac ctctggcgga gccaccgtgg tctgcttcgt gaacaacttc 4620
   taccccagag acatcagcgt gaagtggaag atcgacggca gcgagcagag ggacggcgtg 4680
   ctggacagcg tgaccgacca ggacagcaag gactccacct acagcatgag cagcaccctg 4740
   agcctgacca aggtggagta cgagaggcac aacctgtaca cctgcgaggt ggtgcacaag 4800
   accagctcca gccccgtggt caagtccttc aaccggaacg agtgttgagc tagcttaaga 4860
   tttaaatagg ccggccgcgt cgacctcgag atccaggcgc ggatcaataa aagatcatta    4920
   ttttcaatag atctgtgtgt tggttttttg tgtgccttgg gggaggggga ggccagaatg    4980
   aggcgcggcc aagggggagg gggaggccag aatgaccttg ggggaggggg aggccagaat 5040
   gaccttgggg gagggggagg ccagaatgag gcgcgccccc gggtaccgag ctcgaattag 5100
   tggatcctca cagtaggtgg catcgttcct ttctgactgc ccgccccccg catgccgtcc 5160
   cgcgatattg agctccgaac ctctcgccct gccgccgccg gtgctccgtc gccgccgcgc 5220
   cgccatggaa tcgcgccggt aaccgaagtt cctatacttt ctagagaata ggaacttcgg 5280
   aataggaact tcaagccggt acccagcttt tgttcccttt agtgagggtt aatttcgagc 5340
   ttggcgtaat catggtcata gctgtttcct gtgtgaaatt gttatccgct cacaattcca 5400
   cacaacatac gagccgggag cataaagtgt aaagcctggg gtgcctaatg agtgagctaa 5460
   ctcacattaa ttgcgttgcg ctcactgccc gctttccagt cgggaaacct gtcgtgccag 5520
   ctgcattaat gaatcggcca acgcgcgggg agaggcggtt tgcgtattgg gcgctcttcc 5580
   gcttcctcgc tcactgactc gctgcgctcg gtcgttcggc tgcggcgagc ggtatcagct 5640
   cactcaaagg cggtaatacg gttatccaca gaatcagggg ataacgcagg aaagaacatg 5700
   tgagcaaaag gccagcaaaa ggccaggaac cgtaaaaagg ccgcgttgct ggcgtttttc 5760
   cataggctcc gcccccctga cgagcatcac aaaaatcgac gctcaagtca gaggtggcga 5820
   aacccgacag gactataaag ataccaggcg tttccccctg gaagctccct cgtgcgctct 5880
   cctgttccga ccctgccgct taccggatac ctgtccgcct ttctcccttc gggaagcgtg 5940
   gcgctttctc atagctcacg ctgtaggtat ctcagttcgg tgtaggtcgt tcgctccaag 6000
   ctgggctgtg tgcacgaacc ccccgttcag cccgaccgct gcgccttatc cggtaactat 6060
   cgtcttgagt ccaacccggt aagacacgac ttatcgccac tggcagcagc cactggtaac 6120
   aggattagca gagcgaggta tgtaggcggt gctacagagt tcttgaagtg gtggcctaac 6180
   tacggctaca ctagaaggac agtatttggt atctgcgctc tgctgaagcc agttaccttc 6240
   ggaaaaagag ttggtagctc ttgatccggc aaacaaacca ccgctggtag cggtggtttt 6300
   tttgtttgca agcagcagat tacgcgcaga aaaaaaggat ctcaagaaga tcctttgatc 6360
   ttttctacgg ggtctgacgc tcagtggaac gaaaactcac gttaagggat tttggtcatg 6420
   agattatcaa aaaggatctt cacctagatc cttttaaatt aaaaatgaag ttttaaatca 6480
   atctaaagta tatatgagta aacttggtct gacagttacc aatgcttaat cagtgaggca 6540
   cctatctcag cgatctgtct atttcgttca tccatagttg cctgactccc cgtcgtgtag 6600
   ataactacga tacgggaggg cttaccatct ggccccagtg ctgcaatgat accgcgagac 6660
   ccacgctcac cggctccaga tttatcagca ataaaccagc cagccggaag ggccgagcgc 6720
   agaagtggtc ctgcaacttt atccgcctcc atccagtcta ttaattgttg ccgggaagct 6780
   agagtaagta gttcgccagt taatagtttg cgcaacgttg ttgccattgc tacaggcatc 6840
   gtggtgtcac gctcgtcgtt tggtatggct tcattcagct ccggttccca acgatcaagg 6900
   cgagttacat gatcccccat gttgtgcaaa aaagcggtta gctccttcgg tcctccgatc 6960
   gttgtcagaa gtaagttggc cgcagtgtta tcactcatgg ttatggcagc actgcataat 7020
   tctcttactg tcatgccatc cgtaagatgc ttttctgtga ctggtgagta ctcaaccaag 7080
   tcattctgag aatagtgtat gcggcgaccg agttgctctt gcccggcgtc aatacgggat 7140
   aataccgcgc cacatagcag aactttaaaa gtgctcatca ttggaaaacg ttcttcgggg 7200
   cgaaaactct caaggatctt accgctgttg agatccagtt cgatgtaacc cactcgtgca 7260
   cccaactgat cttcagcatc ttttactttc accagcgttt ctgggtgagc aaaaacagga 7320
   aggcaaaatg ccgcaaaaaa gggaataagg gcgacacgga aatgttgaat actcatactc 7380
   ttcctttttc aatattattg aagcatttat cagggttatt gtctcatgag cggatacata 7440
   tttgaatgta tttagaaaaa taaacaaata ggggttccgc gcacatttcc ccgaaaagtg 7500
   ccacctaaat tgtaagcgtt aatattttgt taaaattcgc gttaaatttt tgttaaatca 7560
   gctcattttt taaccaatag gccgaaatcg gcaaaatccc ttataaatca aaagaataga 7620
   ccgagatagg gttgagtgtt gttccagttt ggaacaagag tccactatta aagaacgtgg 7680
   actccaacgt caaagggcga aaaaccgtct atcagggcga tggcccacta cgtgaaccat 7740
   caccctaatc aagttttttg gggtcgaggt gccgtaaagc actaaatcgg aaccctaaag 7800
   ggagcccccg atttagagct tgacggggaa agccggcgaa cgtggcgaga aaggaaggga 7860
   agaaagcgaa aggagcgggc gctagggcgc tggcaagtgt agcggtcacg ctgcgcgtaa 7920
   ccaccacacc cgccgcgctt aatgcgccgc tacagggcgc gtcccattcg ccattcaggc 7980
   tgcgcaactg ttgggaaggg cgatcggtgc gggcctcttc gctattacgc cagctggcga   8040
   aagggggatg tgctgcaagg cgattaagtt gggtaacgcc agggttttcc cagtcacgac   8100
   gttgtaaaac gacggccagt gagcgcgcgt aatacgactc actatagggc gaattggggg   8160
   taactaagta aggatcgag                                                8179
<210> 101
   <211> 8188
   <212> DNA
   <213> pCAGGS-IgVL2-14 targeting vector
<400> 101 2
74